(19) Europäisches Patentamt
European Patent Office
Office européen des brevets

(11) Veröffentlichungsnummer: **0 532 456 B1**

# (12) EUROPÄISCHE PATENTSCHRIFT

(45) Veröffentlichungstag der Patentschrift: **29.03.95**

(51) Int. Cl.$^{6}$: **C07D 211/26**, A61K 31/445, C07D 401/06

(21) Anmeldenummer: **92810594.9**

(22) Anmeldetag: **04.08.92**

## (54) 1-Acylpiperidinverbindungen und ihre Verwendung als Substanz P Artagonisten.

(30) Priorität: **12.08.91 CH 2374/91**

(43) Veröffentlichungstag der Anmeldung:
**17.03.93 Patentblatt 93/11**

(45) Bekanntmachung des Hinweises auf die Patenterteilung:
**29.03.95 Patentblatt 95/13**

(84) Benannte Vertragsstaaten:
**AT BE CH DE DK ES FR GB GR IE IT LI LU NL PT SE**

(56) Entgegenhaltungen:
**EP-A- 0 428 434**

(73) Patentinhaber: **CIBA-GEIGY AG**
**Klybeckstrasse 141**
**CH-4002 Basel (CH)**

(72) Erfinder: **Schilling, Walter, Dr.**
**Im Muspenacker**
**CH-4204 Himmelried (CH)**
Erfinder: **Ofner, Silvio, Dr.**
**Hauptstrasse 1**
**CH-4142 Münchenstein (CH)**
Erfinder: **Veenstra, Siem J., Dr.**
**Laufenstrasse 29**
**CH-4053 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft neue 1-Acylpiperidinverbindungen der Formel I

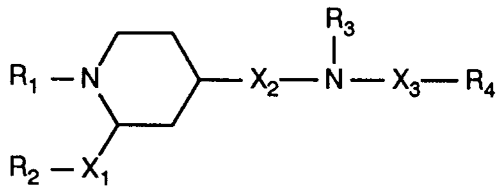

(I),

worin $R_1$ unsubstituiertes oder im Phenylteil durch Niederalkyl, Niederalkoxy, Diniederalkylamino, Halogen und/oder Trifluormethyl substituiertes Phenyl- oder Diphenyl-$C_1$-$C_4$-alkyl, wie Benzyl, 2,4-Dichlorbenzyl, 3,5-Ditrifluormethylbenzyl, 2-Phenylethyl oder 2,2-Diphenylethyl, unsubstituiertes oder im Phenyl durch Halogen und/oder Triazolyl substituiertes Phenoxy-$C_1$-$C_4$-alkyl, Pyridyl- oder Chinolinyl-$C_1$-$C_4$-alkyl, wie 4-Chinolinylmethyl, unsubstituiertes oder durch Niederalkyl, Niederalkoxy, Diniederalkylamino, Halogen und/oder Trifluormethyl substituiertes Benzoyl, wie Benzoyl, 3-Niederalkyl-, 3-Niederalkoxy-, 3-Halogen-, 3-Dimethylamino-, 3,5-Diniederalkyl-, 3,5-Diniederalkoxy-, 3,5-Dihalogen- oder 3,5-Ditrifluormethylbenzoyl, oder in zweiter Linie unsubstituiertes oder durch Niederalkyl, Niederalkoxy, Diniederalkylamino, Halogen und/oder Trifluormethyl substituiertes Naphthoyl, wie 1- oder 2-Naphthoyl, unsubstituiertes oder durch Niederalkyl, Niederalkoxy, Halogen und/oder Trifluormethyl substituiertes Pyridylcarbonyl oder Chinolinylcarbonyl unsubstituiertes oder durch Niederalkyl, Niederalkoxy, Diniederalkylamino, Halogen und/oder Trifluormethyl substituiertes 5-bis 7-gliedriges Cycloalkylcarbonyl, wie Cyclohexylcarbonyl, 3-Methyl-, 3-Methoxy-, 3-Chlor-, 3-Dimethylamino-, 3,5-Dimethyl-, 3,5-Dimethoxy-, 3,5-Dichlor- oder 3,5-Ditrifluormethylcyclohexylcarbonyl, unsubstituiertes oder im Phenyl durch Niederalkyl, Niederalkoxy, Diniederalkylamino, Halogen und/oder Trifluormethyl substituiertes Phenyl- oder Diphenyl-$C_1$-$C_4$-alkanoyl, wie 2,2-Diphenylacetyl oder 2,3-Diphenylpropionyl, unsubstituiertes oder im Phenylteil gegebenenfalls durch Niederalkyl, Niederalkoxy, Diniederalkylamino, Halogen und/oder Trifluormethyl substituiertes N-Phenylcarbamoyl oder eine Gruppe der Formel Ia

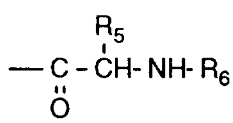

(Ia)

darstellt, in der $R_5$ Wasserstoff, unsubstituiertes oder durch Hydroxy, Mercapto, Amino, gegebenenfalls durch Hydroxy substituiertes Phenyl, Carboxy, Carbamoyl oder Ureido substituiertes $C_1$-$C_4$-Alkyl und $R_6$ $C_2$-$C_7$-Alkanoyl bedeutet, $R_2$ 5- bis 7-gliedriges Cycloalkyl oder einen unsubstituierten oder durch $C_1$-$C_4$-Alkyl, $C_1$-$C_4$-Alkoxy, Halogen und/oder Trifluormethyl substituierten Phenyl-, Naphthyl- oder Pyridylrest darstellt, $R_3$ für Wasserstoff, $C_1$-$C_7$-Alkyl, Carbamoyl, $C_2$-$C_7$-Alkanoyl, Carboxy-$C_1$-$C_4$-alkanoyl oder Carboxy-$C_2$-$C_4$-alkenoyl steht, $R_4$ unsubstituiertes oder durch $C_1$-$C_4$-Alkyl, $C_1$-$C_4$-Alkoxy, Halogen und/oder Trifluormethyl substituiertes Phenyl oder Naphthyl oder unsubstituiertes Pyridyl, Benzofuranyl, Indolyl, 2,3-Dihydroindolyl, Benzimidazolyl, Chinolyl oder 1,2,3,4-Tetrahydrochinolinyl bedeutet, $X_1$ Methylen, Hydroxymethylen, $C_1$-$C_4$-Alkoxymethylen, Carbonyl oder Di-$C_1$-$C_4$-alkoxymethylen oder eine direkte Bindung darstellt, $X_2$ für $C_1$-$C_7$-Alkylen, Carbonyl oder eine direkte Bindung steht und $X_3$ Carbonyl, $C_1$-$C_4$-Alkylen, Carboxy-$C_1$-$C_4$-alkylen, $C_1$-$C_4$-Alkoxycarbonyl-$C_1$-$C_4$-alkylen, Carbamoyl-$C_1$-$C_4$-alkylen oder Hydroxymethyl-$C_1$-$C_4$-alkylen darstellt, und ihre Salze, Verfahren zur Herstellung der erfindungsgemässen Verbindungen, diese enthaltende pharmazeutische Präparate und ihre Verwendung als Arzneimittelwirkstoffe.

Die genannten Aryl-, Aroyl-, Aralkanoyl-, Heteroaryl- und Heteroaroylreste können unsubstituiert oder mono-, di- oder trisubstituiert, insbesondere mono- oder disubstituiert, sein. Aryl-, Aralkyl-, Aryloxyalkyl-, Cycloalkylcarbonyl- und Aroylreste sind vorzugsweise in der angebenen Weise mono- oder disubstituiert, wie 3-mono- oder 3,5-disubstituiert; Heteroaryl-, Heteroaralkyl-, Heteroaralkanoyl- und Heteroaroylreste sind vorzugsweise unsubstituiert.

Cycloalkyl ist beispielsweise 5- bis 7-gliedriges Cycloalkyl, wie insbesondere Cyclohexyl oder in zweiter Linie Cyclopentyl oder Cycloheptyl.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise $C_1$-$C_7$-Alkyl, vorzugsweise $C_1$-$C_4$-Alkyl, wie insbesondere Methyl oder in zweiter Linie Ethyl, Propyl, Isopropyl oder Butyl, kann aber auch Isobutyl, Sekundärbutyl, Tertiärbutyl oder eine $C_5$-$C_7$-Alkyl-, wie Pentyl-, Hexyl- oder Heptylgruppe sein.

Niederalkylen ist beispielsweise $C_1$-$C_7$-Alkylen, vorzugsweise $C_1$-$C_4$-Alkylen, wie Methylen, Ethylen, 1,3-Propylen, 1,4-Butylen oder 1,5-Pentylen.

Gegebenenfalls im Phenyl substituiertes Phenyl- oder Diphenylniederalkyl ist beispielsweise entsprechendes Phenyl- oder Diphenyl-$C_1$-$C_4$-alkyl, wie Benzyl, 2,4-Dichlorbenzyl, 3,5-Ditrifluormethylbenzyl, 2-Phenylethyl oder 2,2-Diphenylethyl.

Im Phenylteil gegebenenfalls substituiertes Phenyl- oder Diphenylniederalkanoyl ist beispielsweise entsprechendes Phenyl- oder Diphenyl-$C_1$-$C_4$-alkanoyl, wie 2,2-Diphenylacetyl oder 2,3-Diphenylpropionyl.

Gegebenenfalls im Phenyl substituiertes Phenoxyniederalkyl ist beispielsweise durch Halogen und/oder Triazolyl substituiertes Phenoxy-$C_1$-$C_4$-alkyl, wie 2-[2-(1H-1,2,4-triazol-1-yl)-4-chlor-phenoxy]ethyl.

Als Heteroarylrest 6- gliedriges monocyclisches oder aus einem 6-gliedrigen und einem 5- oder 6-gliedrigen Ring aufgebautes bicyclisches Azaheteroaryl aufweisendes Heteroarylniederalkyl. ist beispielsweise Pyridyl- oder Chinolinyl-$C_1$-$C_4$-alkyl, wie 4-Chinolinylmethyl.

Niederalkoxy ist beispielsweise $C_1$-$C_7$-Alkoxy, vorzugsweise $C_1$-$C_4$-Alkoxy, wie Methoxy, Ethoxy, Propyloxy, Isopropyloxy oder Butyloxy , kann aber auch Isobutyloxy, Sekundärbutyloxy, Tertiärbutyloxy oder eine Pentyloxy-, Hexyloxy- oder Heptyloxygruppe sein.

Halogen ist beispielsweise Halogen der Atomnummer bis und mit 35, wie Chlor oder Fluor, ferner Brom.

Niederalkoxycarbonyl ist beispielsweise $C_1$-$C_7$-Alkoxycarbonyl, vorzugsweise $C_1$-$C_4$-Alkoxycarbonyl, wie Methoxycarbonyl, Ethoxycarbonyl, Propyloxycarbonyl, Isopropyloxycarbonyl oder Butyloxycarbonyl , kann aber auch Isobutyloxycarbonyl, Sekundärbutyloxycarbonyl, Tertiärbutyloxycarbonyl oder eine Pentyloxycarbonyl-, Hexyloxycarbonyl- oder Heptyloxycarbonylgruppe sein.

N-Niederalkylcarbamoyl ist beispielsweise N-$C_1$-$C_7$-Alkylcarbamoyl, vorzugsweise N-$C_1$-$C_4$-Alkylcarbamoyl, wie Methylcarbamoyl, Ethylcarbamoyl, Propylcarbamoyl, Isopropylcarbamoyl oder Butylcarbamoyl , kann aber auch Isobutylcarbamoyl, Sekundärbutylcarbamoyl, Tertiärbutylcarbamoyl oder eine Pentylcarbamoyl-, Hexylcarbamoyl- oder Heptylcarbamoylgruppe sein.

N,N-Diniederalkylcarbamoyl ist beispielsweise N,N-Di-$C_1$-$C_7$-alkylcarbamoyl, vorzugsweise N,N-Di-$C_1$-$C_4$-alkylcarbamoyl, wie N,N-Dimethylcarbamoyl, N,N-Diethylcarbamoyl, N-Ethyl-N-methyl-carbamoyl, N,N-Dipropylcarbamoyl, N-Methyl-N-propyl-carbamoyl, N-Isopropyl-N-methyl-carbamoyl oder N-Butyl-N-methyl-carbamoyl, kann aber auch N-Isobutyl-N-methyl-carbamoyl, N-Methyl-N-sekundärbutyl-carbamoyl, N-Methyl-N-tertiärbutyl-carbamoyl oder eine N-Methyl-N-pentyl-carbamoyl-, N-Hexyl-N-methyl-carbamoyl- oder N-Heptyl-N-methyl-carbamoylgruppe sein.

In höherer als der $\alpha$-Stellung und in niedrigerer als der $\omega$-Stellung durch Hydroxy substituiertes Niederalkylen ist beispielsweise 1,3-(2-Hydroxy)propylen, 1,4-(2-Hydroxy)butylen, 1,4-(3-Hydroxy)butylen, 1,5-(2-Hydroxy)pentylen, 1,5-(3-Hydroxy)pentylen oder 1,5-(4-Hydroxy)pentylen.

Durch Carboxy substituiertes Niederalkylen ist beispielsweise Carboxymethylen, 1- oder 2-Carboxyethylen, 1,3-(2-Carboxy)propylen, 1,4-(2-Carboxy)butylen, 1,4-(3-Carboxy)butylen, 1,5-(2-Carboxy)pentylen, 1,5-(3-Carboxy)pentylen oder 1,5-(4-Carboxy)pentylen.

Durch Niederalkoxycarbonyl substituiertes Niederalkylen ist beispielsweise Niederalkoxycarbonylmethylen, 1- oder 2-Niederalkoxycarbonylethylen, 1,3-(2-Niederalkoxycarbonyl)propylen, 1,4-(2-Niederalkoxycarbonyl)butylen, 1,4-(3-Niederalkoxycarbonyl)butylen, 1,5-(2-Niederalkoxycarbonyl)pentylen, 1,5-(3-Niederalkoxycarbonyl)pentylen oder 1,5-(4-Niederalkoxycarbonyl)pentylen, wobei Niederalkoxycarbonyl jeweils beispielsweise $C_1$-$C_4$-Alkoxycarbonyl, wie Methoxycarbonyl, Ethoxycarbonyl, Propoxycarbonyl oder Butoxycarbonyl, bedeutet.

Durch Carbamoyl, N-mono- oder N,N-Diniederalkylcarbamoyl substituiertes Niederalkylen ist insbesondere durch Carbamoyl substituiert und bedeutet beispielsweise Carbamoylmethylen, 1-oder 2-Carbamoylethylen, 1,3-(2-Carbamoyl)propylen, 1,4-(2-Carbamoyl)butylen, 1,4-(3-Carbamoyl)butylen, 1,5-(2-Carbamoyl)pentylen, 1,5-(3-Carbamoyl)pentylen oder 1,5-(4-Carbamoyl)pentylen.

Durch Hydroxymethyl substituiertes Niederalkylen ist beispielsweise 2-Hydoxyethyliden, 2,3-(1-Hydroxy)propylen, 1,3-(2-Hydroxymethyl)propylen, 2,4-(1-Hydroxy)butylen, 1,4-(2-Hydroxymethyl)butylen, 1,4-(3-Hydroxymethyl)butylen, 1,5-(2-Hydroxymethyl)pentylen, 1,5-(3-Hydroxymethyl)pentylen oder 1,5-(4-Hydroxymethyl)pentylen.

Niederalkoxymethylen ist beispielsweise $C_1$-$C_4$-Alkoxymethylen, wie Methoxymethylen, Ethoxymethylen, Propyloxymethylen oder Butyloxymethylen.

Diniederalkoxymethylen ist beispielsweise Di-$C_1$-$C_4$-alkoxymethylen, wie Dimethoxymethylen, Diethoxymethylen, Dipropyloxymethylen oder Dibutyloxymethylen.

Niederalkylendioxymethylen ist beispielsweise 5- bis 8-gliedriges, insbesondere 5- oder 6-gliedriges 1,3-Dioxacycloalk-2-yl, wie 1,3-Dioxacyclobut-2-yl, 1,3-Dioxacyclopent-2-yl (1,3-Dioxolan-2-yl), 1,3-Dioxacyclohex-2-yl (1,3-Dioxan-2-yl) oder 1,3-Dioxacyclohept-2-yl.

Die Verbindungen der Formel I weisen basischen oder, sofern $R_3$ und/oder $X_3$ durch Carboxy substituiert ist, amphoteren Charakter und können dementsprechend Säureadditionssalze und gegebenenfalls innere Salze bilden.

Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die erfindungsgemäss bereitgestellten Verbindungen besitzen wertvolle pharmakologische Eigenschaften. Insbesondere zeigen sie eine ausgeprägte antagonistische Wirkung gegen Substanz P und weisen das für Substanz-P-Antagonisten typische Eigenschaftsspektrum auf. So wird durch die Verbindungen der Formel I und ihre pharmazeutisch verwendbaren Salze *in vitro* die Bindung von $^3$H-Substanz-P an der Rinder-Retina im Radiorezeptor-Assay nach H. Bittiger, Ciba Foundation Symposium 91, 196-199 (1982) in Konzentrationen ab etwa 10 µMol/L vollständig gehemmt. *In vivo* hemmen sie ab einer Dosis von etwa 0.01 mg/kg i.v. die durch Substanz-P bewirkte Gefässerweiterung am Meerschweinchenohr, gemessen in Anlehnung an die Versuchsanordnung von Andrews und Helme, Regul. Pept. 25, 267-275 (1989) sowie ab einer Dosis von etwa 1.0 mg/kg i.v. in Anlehnung an die Versuchsanordnung von Lundberg et al, Proc. Nat. Acad. Sci. (USA) 80, 1120-1124 vagal induzierte Bronchospasmen des Meerschweinchens, woraus sich ihre Eignung für die Behandlung von Asthma ergibt. Ihre Verwendbarkeit für die Behandlung von Erkrankungen des zentralen Nervensystems ergibt sich beispielsweise aus ihrer Hemmwirkung auf durch *icv*-applizierten Substanz-P-methylester induzierte Verhaltensänderung der Rennmaus (gerbil) nach A. Vassout et al., Meeting on Substance P, Worcester, Mass (1990) mit einer $ED_{50}$ ab etwa 10 mg/kg s.c., ab etwa 30 mg/kg i.p. und ab etwa 100 mg/kg p.o.

Substanz P ist ein natürlich vorkommendes Undekapeptid der Tachykininfamilie. Es wird in Säugetierorganismus erzeugt und wirkt pharmakologisch als Neuropeptid. Substanz P spielt eine wesentliche Rolle bei verschiedenen Erkrankungen, beispielsweise bei Schmerzzuständen, bei Migräne und bei einigen Störungen des Zentralnervensystems, wie bei Angstzuständen, Schizophrenie und Depressionen sowie bei bestimmten motorischen Störungen, wie bei morbus Parkinson, aber auch bei entzündlichen Erkrankungen, wie bei rheumatoider Arthritis, Iritis und Konjunktivitis, bei Erkrankungen der Atmungsorgane, wie bei Asthma und chronischer Bronchitis, bei Erkrankungen des Gastrointestinalsystems, wie bei ulcerativer Colitis und morbus Crohn, und bei Hypertension.

Es hat deshalb nicht an Versuchen gefehlt, Substanz-P-Antagonisten zu entwickeln. Bei einer Reihe der bislang bekannten Substanz-P-Antagonisten handelt es sich jedoch um peptidische Verbindungen, die metabolisch zu labil sind, um als Arzneimittelwirkstoffe eingesetzt werden zu können.

Die erfindungsgemäss bereitgestellten Substanz-P-Antagonisten der Formel I und ihre pharmazeutisch verwendbaren Salze sind demgegenüber metabolisch stabil und eignen sich dementsprechend vorzüglich zur therapeutischen Behandlung der genannten Erkrankungen.

Die Erfindung betrifft in erster Linie Verbindungen der Formel I, worin $R_1$ unsubstituiertes oder durch $C_1$-$C_4$-Alkyl, wie Methyl, $C_1$-$C_4$-Alkoxy, wie Methoxy, Halogen und/oder Trifluormethyl substituiertes Benzoyl, Naphthoyl oder Phenyl-$C_1$-$C_4$-alkanoyl, unsubstituiertes Pyridylcarbonyl oder Chinolinylcarbonyl oder eine Gruppe der Formel Ia

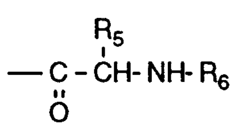

(Ia)

darstellt, in der $R_5$ Wasserstoff, unsubstituiertes oder durch Hydroxy, Mercapto, Amino, gegebenenfalls durch Hydroxy substituiertes Phenyl, Carboxy, Carbamoyl oder Ureido substituiertes $C_1$-$C_4$-Alkyl, beispiels-

weise Methyl, Isopropyl, Isobutyl, Sekundärbutyl, Hydroxymethyl, Mercaptomethyl, 2-Methylmercaptoethyl, 3-Ureidopropyl, 4-Aminobutyl, Carboxymethyl, Carbamoylmethyl, 2-Carboxyethyl, 2-Carbamoylethyl, Benzyl oder 4-Hydroxybenzyl, und $R_6$ $C_2$-$C_7$-Alkanoyl, wie Acetyl, Propionyl, Butyryl oder Pivaloyl, bedeutet, $R_2$ 5- bis 7-gliedriges Cycloalkyl, insbesondere Cyclohexyl oder in zweiter Linie Cyclopentyl oder Cycloheptyl, oder einen unsubstituierten oder durch $C_1$-$C_4$-Alkyl, wie Methyl, $C_1$-$C_4$-Alkoxy, wie Methoxy, Halogen und/oder Trifluormethyl substituierten Phenyl-, Naphthyl- oder Pyridylrest darstellt, $R_3$ für Wasserstoff, $C_1$-$C_7$-Alkyl, wie Methyl, Ethyl, Propyl, Isopropyl oder Butyl, Isobutyl, Sekundärbutyl oder Tertiärbutyl, Carbamoyl, $C_2$-$C_7$-Alkanoyl, wie Acetyl, Propionyl, Butyryl oder Pivaloyl, Carboxy-$C_1$-$C_4$-alkanoyl, wie Succinoyl, Glutaroyl oder Adipoyl, oder Carboxy-$C_3$-$C_5$-alkenoyl, wie Maleyl, Fumaroyl oder Tartroyl, steht, $R_4$ unsubstituiertes oder durch $C_1$-$C_4$-Alkyl, wie Methyl, $C_1$-$C_4$-Alkoxy, wie Methoxy, Halogen und/oder Trifluormethyl substituiertes Phenyl oder Naphthyl oder unsubstituiertes Pyridyl, Benzofuranyl, Indolyl, Benzimidazolyl oder Chinolyl bedeutet, $X_1$ Methylen, Hydroxymethylen, $C_1$-$C_4$-Alkoxymethylen, wie Methoxymethylen, Ethoxymethylen, Propyloxymethylen oder Butyloxymethylen, Carbonyl, Di-$C_1$-$C_4$-alkoxymethylen, wie Dimethoxymethylen, Diethoxymethylen, Dipropyloxymethylen oder Dibutyloxymethylen, oder eine direkte Bindung darstellt, $X_2$ für $C_1$-$C_7$-Alkylen, wie Methylen oder in zweiter Linie Ethylen oder 1,3-Propylen, Carbonyl oder eine direkte Bindung steht und $X_3$ Carbonyl, $C_1$-$C_4$-Alkylen, wie Methylen, Ethylen oder 1,3-Propylen, Carboxy-$C_1$-$C_4$-alkylen, wie 1,3-(2-Carboxy)propylen, 1,4-(2-Carboxy)butylen, 1,4-(3-Carboxy)butylen, $C_1$-$C_4$-Alkoxycarbonyl-$C_1$-$C_4$-alkylen, wie 1,3-(2-$C_1$-$C_4$-Alkoxycarbonyl)propylen, 1,4-(2-$C_1$-$C_4$-Alkoxycarbonyl)butylen, 1,4-(3-$C_1$-$C_4$-Alkoxycarbonyl)butylen, 1,5-(2-$C_1$-$C_4$-Alkoxycarbonyl)pentylen, 1,5-(3-$C_1$-$C_4$-Alkoxycarbonyl)pentylen oder 1,5-(4-$C_1$-$C_4$-Alkoxycarbonyl)pentylen, wobei $C_1$-$C_4$-Alkoxycarbonyl jeweils beispielsweise Methoxycarbonyl, Ethoxycarbonyl, Propoxycarbonyl oder Butoxycarbonyl, bedeutet, Carbamoyl-$C_1$-$C_4$-alkylen, wie 1,3-(2-Carbamoyl)propylen, 1,4-(2-Carbamoyl)butylen, 1,4-(3-Carbamoyl)-butylen, 1,5-(2-Carbamoyl)pentylen, 1,5-(3-Carbamoyl)pentylen oder 1,5-(4-Carbamoyl)pentylen, oder Hydroxymethyl-$C_1$-$C_4$-alkylen, wie 1,3-(2-Hydroxymethyl)propylen, 1,4-(2-Hydroxymethyl)butylen, 1,4-(3-Hydroxymethyl)butylen, 1,5-(2-Hydroxymethyl)pentylen, 1,5-(3-Hydroxymethyl)pentylen oder 1,5-(4-Hydroxymethyl)pentylen, darstellt, und ihre Salze.

Die Erfindung betrifft vorzugsweise Verbindungen der Formel I, worin $R_1$ unsubstituiertes oder durch $C_1$-$C_4$-Alkyl, wie Methyl, $C_1$-$C_4$-Alkoxy, wie Methoxy, Halogen der Atomnummer bis und mit 35, wie Chlor, und/oder Trifluormethyl mono- oder disubstituiertes Benzoyl oder unsubstituiertes Naphthoyl oder Phenyl-$C_1$-$C_4$-alkanoyl bedeutet, $R_2$ unsubstituiertes oder durch $C_1$-$C_4$-Alkyl, wie Methyl, $C_1$-$C_4$-Alkoxy, wie Methoxy, Halogen der Atomnummer bis und mit 35, wie Chlor, und/oder Trifluormethyl mono- oder disubstituiertes Phenyl oder unsubstituiertes Pyridyl darstellt, $R_3$ für Wasserstoff, $C_1$-$C_4$-Alkyl, wie Methyl, Ethyl, Propyl oder Isopropyl, Carbamoyl oder $C_2$-$C_7$-Alkanoyl, wie Acetyl, Propionyl, Butyryl oder Pivaloyl, steht, $R_4$ unsubstituiertes oder durch $C_1$-$C_4$-Alkyl, wie Methyl, $C_1$-$C_4$-Alkoxy, wie Methoxy, Halogen der Atomnummer bis und mit 35, wie Chlor, und/oder Trifluormethyl mono- oder disubstituiertes Phenyl oder unsubstituiertes Naphthyl, Pyridyl, Benzofuranyl, Indolyl, Benzimidazolyl oder Chinolyl bedeutet, $X_1$ Methylen, Hydroxymethylen, Carbonyl oder eine direkte Bindung darstellt, $X_2$ für eine direkte Bindung steht und $X_3$ $C_1$-$C_4$-Alkylen, wie Methylen oder in zweiter Linie Ethylen oder 1,3-Propylen, darstellt, und ihre Salze.

Die Erfindung betrifft in allererster Linie Verbindungen der Formel I, worin $R_1$ unsubstituiertes oder durch $C_1$-$C_4$-Alkyl, wie Methyl, $C_1$-$C_4$-Alkoxy, wie Methoxy, Halogen der Atomnummer bis und mit 35, wie Chlor, und/oder Trifluormethyl mono- oder disubstituiertes Benzoyl oder unsubstituiertes Naphthoyl bedeutet, $R_2$ unsubstituiertes oder durch Halogen der Atomnummer bis und mit 35, wie Chlor, und/oder Trifluormethyl mono- oder disubstituiertes Phenyl darstellt, $R_3$ für Wasserstoff steht, $R_4$ unsubstituiertes Chinolyl bedeutet, $X_1$ Methylen darstellt, $X_2$ für eine direkte Bindung steht und $X_3$ $C_1$-$C_4$-Alkylen, wie Methylen oder in zweiter Linie Ethylen oder 1,3-Propylen, darstellt, und ihre Salze.

Die Erfindung betrifft namentlich die in den Beispielen genannten Verbindungen der Formel I und ihre Salze.

Die Erfindung betrifft ferner ein auf an sich bekannten Methoden beruhendes Verfahren zur Herstellung der erfindungsgemässen Verbindungen. Dieses ist dadurch gekennzeichnet, dass man

a) in eine Verbindung der Formel II

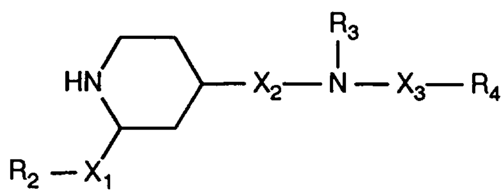

(II),

worin $R_2$, $R_3$, $R_4$, $X_1$, $X_2$ und $X_3$ die angegebenen Bedeutungen haben, den Rest $R_1$ einführt oder
b) Verbindungen der Formeln III und IV

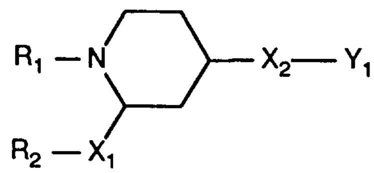

(III)

und

$Y_2$ - $X_3$ - $R_4$ (IV),

worin $Y_1$ eine Gruppe der Formel -N($R_3$)-H und $Y_2$ Hydroxy, reaktionsfähiges verestertes Hydroxy oder, sofern $X_3$ für Carbonyl steht, verethertes Hydroxy darstellt oder $Y_1$ Hydroxy, reaktionsfähig verestertes Hydroxy oder, sofern $X_2$ Carbonyl bedeutet, verethertes Hydroxy und $Y_2$ eine Gruppe der Formel -N($R_3$)-H darstellt, wobei $R_2$, $R_3$, $R_4$, $X_1$, $X_2$ und $X_3$ die angegebenen Bedeutungen haben, oder deren Salze miteinander kondensiert oder
c) zur Herstellung von Verbindungen der Formel I, worin einer der Reste $X_2$ und $X_3$ Alkylen und der andere Alkylen, Carbonyl oder im Falle von $X_2$ eine direkte Bindung bzw. im Falle von $X_3$ einen gegebenenfalls durch Hydroxymethyl oder gegebenenfalls verestertes oder amidiertes Carboxy substituiertes Alkylenrest darstellt, in einer Verbindung der Formel V

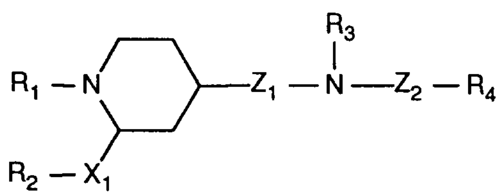

(V),

worin $Z_1$ einen in $\alpha$-Stellung zur Gruppe -N($R_3$)- durch Oxo oder Hydroxy substituierten Alkylenrest und $Z_2$ Alkylen, Carbonyl oder einen gegebenenfalls durch Hydroxymethyl oder gegebenenfalls verestertes oder amidiertes Carboxy substituiertes Alkylenrest bedeutet oder $Z_1$ Alkylen, Carbonyl oder eine direkte Bindung und $Z_2$ in $\alpha$-Stellung zur Gruppe -N($R_3$)- durch Oxo oder Hydroxy substituierten Alkylenrest darstellt und $R_2$, $R_3$, $R_4$, $X_1$, $X_2$ und $X_3$ die angegebenen Bedeutungen haben, oder in einem Salz davon die Oxo- bzw. Hydroxygruppe in $\alpha$-Stellung zur Gruppe -N($R_3$)- reduktiv durch Wasserstoff ersetzt bzw. in einer Verbindung der Formel VI

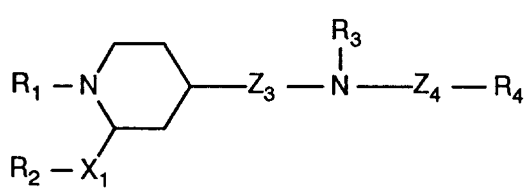

(VI),

worin $Z_3$ einen Rest der Formel -C($R_a$) = C($R_b$)- und $Z_4$ Alkylen, Carbonyl oder einen gegebenenfalls durch Hydroxymethyl oder gegebenenfalls verestertes oder amidiertes Carboxy substituiertes Alkylenrest bedeutet oder $Z_3$ Alkylen, Carbonyl oder eine direkte Bindung und $Z_4$ einen Rest der Formel -C($R_a$) = C-($R_b$)- bedeutet, wobei $R_a$ und $R_b$ jeweils Wasserstoff oder Niederalkyl bedeuten, den Rest der Formel -C-($R_a$) = C($R_b$)- durch Reduktion der Doppelbindung zu dem entsprechenden Rest -CH($R_a$)-CH($R_b$)- reduziert oder
d) zur Herstellung von Verbindungen der Formel I, worin $X_1$ eine Carbonyl- oder Hydroxymethylengruppe bedeutet, Verbindungen der Formeln VII und VIII

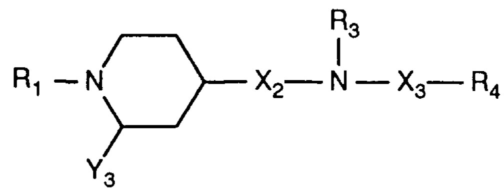 (VII)

und

$Y_4$ - $R_2$ (VIII),

worin einer der Reste $Y_3$ und $Y_4$ Formyl oder eine gegebenenfalls anhydridisierte oder veresterte Carboxygruppe und der andere einen metallischen Rest darstellt und $R_2$, $R_3$, $R_4$, $X_2$ und $X_3$ die angegebenen Bedeutungen haben, miteinander kondensiert oder

e) zur Herstellung von Verbindungen der Formel I, worin $R_3$ für Wasserstoff steht, aus einer Verbindung der Formel IX

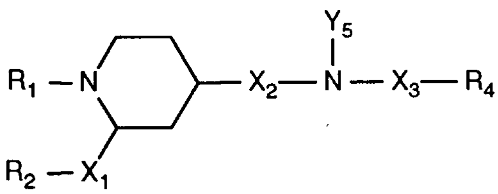 (IX),

worin $Y_5$ eine Aminoschutzgruppe bedeutet und $R_2$, $R_4$, $X_1$, $X_2$ und $X_3$ die angegebenen Bedeutungen haben, oder aus einem Salz davon die Gruppe $Y_5$ abspaltet oder

f) zur Herstellung von Verbindungen der Formel I, worin $X_3$ Alkylen bedeutet, Verbindungen der Formeln X und XI

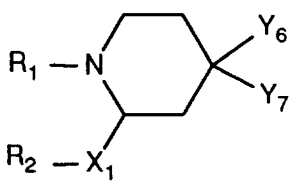 (X) und 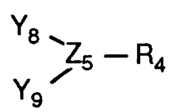 (XI) ,

worin $Y_6$ eine Gruppe der Formel -N($R_3$)-H, $Y_7$ Wasserstoff, $Y_8$ und $Y_9$ gemeinsam Oxo und $Z_5$ einen $X_3$ entsprechenden Alkanylylidenrest bedeuten oder $Y_6$ und $Y_7$ gemeinsam Oxo, $Y_8$ eine Gruppe der Formel -N($R_3$)-H, $Y_9$ Wasserstoff und $Z_5$ einen Rest $X_3$ darstellt,unter reduzierenden Bedingungen miteinander kondensiert und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Die Durchführung der verfahrensgemässen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw.

Verdünnungsmittel, und Reaktions-, wie Temperatur- und Druckbedingungen, sowie gegebenenfalls Schutzgase verwendet.

Die Einführung des Restes $R_1$ gemäss der Verfahrensvariante a) erfolgt in üblicher Weise, beispielsweise durch Umsetzung mit einem den Rest $R_1$ einführenden Mittel, wie einem N-Acylierungsmittel der Formel $R_1$-$Y_a$ (IIa1), worin $R_1$ einen gegebenenfalls substituierten Aroyl-, Heteroaroyl-, Cycloalkylcarbonyl-, Aralkanoyl-, Heteroarylalkanoyl- oder Arylcarbamoylrest oder den Acylrest einer gegebenenfalls N-alkanoylierten $\alpha$-Aminosäure und $Y_a$ gegebenenfalls verethertes Hydroxy, wie Hydroxy, Niederalkoxy oder gegebenenfalls substituiertes Phenoxy, oder reaktionsfähiges verestertes Hydroxy, wie Halogen, insbesondere Chlor, oder

einen Rest der Formel -O-$R_1$, bedeutet, bzw. mit einem Aralkylierungs-, Aryloxyalkylierungs- oder Heteroarylalkylierungsmittel der Formel $R_1$-$Y_b$ (IIa2), worin $R_1$ einen gegebenenfalls substituierten Aralkyl-, Aryloxyalkyl-, Heteroaralkylrest und $Y_b$ reaktionsfähiges verestertes Hydroxy, wie Halogen, z.B. Chlor, Brom oder Jod, oder eine Sulfonyloxygruppe, wie eine Alkan- oder gegebenenfalls substituierte Benzolsulfonyloxygruppe, z.B. Methan-, Ethan-, Benzol-, p-Toluol- oder p-Brombemzolsulfonyloxy, bedeutet, oder durch Umsetzung unter reduzierenden Bedingungen mit einer Verbindung der Formel $R_1 = O$ (IIa3), worin $R_1$ einen gegebenenfalls substituierten Aralkyl-, Aryloxyalkyl-, Heteroaralkylrest bedeutet.

Erforderlichenfalls arbeitet man unter thermischer Zersetzung intermediär gebildeter Ammoniumsalze oder in Gegenwart eines Kondensationsmittels, wie eines wasserbindenden Mittels, oder basischen Kondensationsmittels, und in Gegenwart eines Lösungs- oder Verdünnungsmittels. So wird die Umsetzung mit Säuren der Formel IIa1 (Y= COOH) vorzugsweise in Gegenwart eines wasserbindenden Mittels, wie von N,N-Dicyclohexylcarbodiimid, oder unter thermischer Zersetzung des primär gebildeten Ammoniumsalzes vorgenommen, während die Umsetzung mit Säureanhydriden der Formel IIa1 (Y= Halogen oder -O-(C=O)-$R_1$) und mit Verbindungen der Formel IIa2 vorzugsweise in Gegenwart eines basischen Kondensationsmittels, wie eines Alkalimetallhydroxides oder -carbonates, oder eines tertiären oder sterisch gehinderten sekundären organischen Amins, wie eines Triniederalkylamins, z.B. von Triethylamin oder Diisopropylamin, oder einer aromatischen Stickstoffbase, z.B. von Pyridin, durchgeführt wird.

Bei der Umsetzung mit Verbindungen der Formel IIa3 arbeitet man beispielsweise in Gegenwart von Wasserstoff und eines Hydrierungskatalysators, wie eines Platin- oder Palladiumkatalysators oder von Raney-Nickel, bzw. in Gegenwart eines Dileichtmetallhydrides, wie Natriumborhydrid oder Natriumcyanoborhydrid, vorzugsweise in einem unter den Reaktionsbedingungen inerten Lösungsmittels, wie eines Niederalkanols, wie Methanol oder Ethanol, oder eines Diniederalkyl- oder Niederalkylenethers, wie Diethylether, Dioxan oder Tetrahydrofuran.

Die Ausgangsstoffe der Formel II können in üblicher Weise hergestellt werden, beispielsweise indem man Verbindungen der Formeln

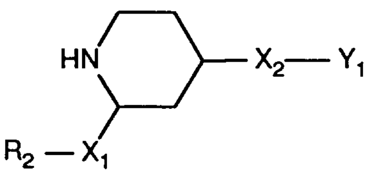
(IIb)

und

$Y_2$-$X_3$-$R_4$ (IV),

miteinander umsetzt, beispielsweise wie nachstehend unter der Verfahrensvariante b) beschrieben.

In Ausgangsstoffen der Formel III oder IV gemäss der Verfahrensvariante b) bedeutet reaktionsfähiges verestertes Hydroxy beispielsweise ein Halogen-, wie Chlor-, Brom- oder Jodatom, oder, sofern $X_3$ von Carbonyl verschieden ist, eine Sulfonyloxygruppe, z.B. Methansulfonyloxy oder p-Toluolsulfonyloxy, oder sofern $X_3$ Carbonyl bedeutet, eine Gruppe der Formel -O-(C=O)-$R_4$. Verethertes Hydroxy bedeutet beispielsweise Niederalkoxy, wie Methoxy oder Ethoxy, oder gegebenenfalls substituiertes Phenyloxy.

Die Umsetzung von Verbindungen der Formeln III und IV erfolgt in üblicher Weise, beispielsweise unter thermischer Zersetzung intermediär gebildeter Ammoniumsalze oder in Gegenwart eines Kondensationsmittels, wie eines wasserbindenden Mittels, oder basischen Kondensationsmittels, und in Gegenwart eines Lösungs- oder Verdünnungsmittels. So wird die Umsetzung mit Säuren der Formel IV bzw. III ($Y_2$ bzw. $Y_1$ = OH) vorzugsweise in Gegenwart eines wasserbindenden Mittels, wie von N,N-Dicyclohexylcarbodiimid, oder unter thermischer Zersetzung des primär gebildeten Ammoniumsalzes vorgenommen, während die Umsetzung mit reaktionsfähigen Estern der Formel IV bzw. III ($Y_2$ bzw. $Y_1$ = reaktionsfähiges verestertes Hydroxy) bzw. mit Säureanhydriden der Formel IV bzw. III ($Y_2$ bzw. $Y_1$ = anhydridisiertes Hydroxy) vorzugsweise in Gegenwart eines basischen Kondensationsmittels, wie eines Alkalimetallhydroxides oder -carbonates, oder eines tertiären oder sterisch gehinderten sekundären organischen Amins, wie eines Triniederalkylamins, z.B. von Triethylamin oder Diisopropylamin, oder einer aromatischen Stickstoffbase, z.B. von Pyridin, durchgeführt wird.

Die Ausgangsstoffe der Formel III können in üblicher Weise hergestellt werden, beispielsweise indem man in eine Verbindung der Formel IIIa

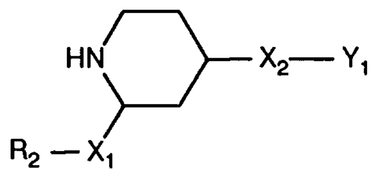

(IIIa)

den Rest $R_1$ einführt, beispielsweise wie vorstehend unter der Verfahrensvariante a) beschrieben.

Der reduktive Ersatz der Oxo- bzw. Hydroxygruppe in $\alpha$-Stellung zur Gruppe $-N(R_3)$-durch Wasserstoff bzw. die Reduktion der Doppelbindung in dem Rest der Formel $-C(R_a) = C(R_b)$- gemäss der Verfahrensvariante c) erfolgt beispielsweise durch katalytische Hydrierung, d.h. Behandlung mit Wasserstoff in Gegenwart eines Hydrierungskatalysators, wie eines Metalles oder einer Metallverbindung eines Metalles der Gruppe VIIIb des Periodischen Systems, wie von Platin, Platinoxid, Palladium/Kohle oder von Raney-Nickel, oder durch Umsetzung mit einem Dileichtmetallhydrid, wie einem Alkalimetalborhydrid, z.B. mit Natriumcyanoborhydrid, oder durch Behandlung mit Ameisensäure.

Ausgangsstoffe der Formel V bzw. VI können beispielsweise durch Kondensation von Verbindungen der Formeln Va und Vb

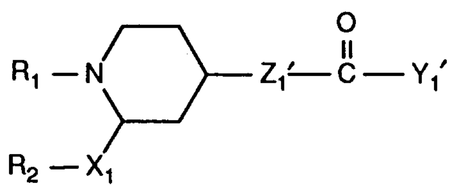

(Va)

und

$H-N(R_3)-Z_2-R_4$ (Vb),

worin $Z_1'$ eine direkte Bindung oder einen um ein C-Atom verkürzten Rest $X_2$ und $Y_1'$ Wasserstoff, Niederalkyl oder freies, verethertes oder reaktionsfähiges verestertes Hydroxy bedeutet und $Z_2$ die angegebene Bedeutung hat, oder von Verbindungen der Formel VIa und VIb

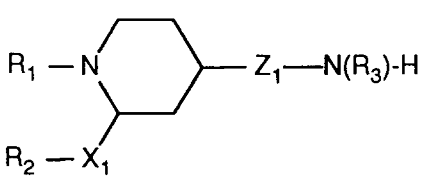

(VIa) und

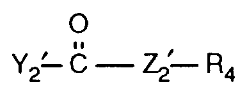

(VIb),

worin $Z_1$ die angegebene Bedeutung hat, $Z_2'$ eine direkte Bindung oder einen um ein C-Atom verkürzten Rest $X_3$ und $Y_2'$ Wasserstoff, Niederalkyl oder freies, verethertes oder reaktionsfähiges verestertes Hydroxy bedeutet, erhalten werden. Ist in Verbindungen der Formel Va $Y_1'$ bzw. in Verbindungen der Formel VIb $Y_2'$ Wasserstoff oder Niederalkyl, werden dabei unter milden Reaktionsbedingungen, insbesondere im basischen oder neutralen Milieu, die entsprechenden Verbindungen der Formel V und unter drastischen Reaktionsbedingungen, insbesondere im sauren Milieu, die entsprechenden Verbindungen der Formel VI gebildet. Im letztgenannten Fall werden dabei intermediär die entsprechenden Verbindungen der Formel V gebildet, aus denen dann durch Wasserabspaltung die entsprechenden Verbindungen der Formel VI gebildet werden. Ausgangsstoffe der Formeln V und VI können auch nebeneinander entstehen. In einer bevorzugten Ausführungsform der Erfindung werden die Zwischenprodukte der Formel V bzw. VI in situ gebildet und ohne Isolierung zu den entsprechenden Verbindungen der Formel I reduziert, indem man die Kondensation von Ausgangsstoffen der Formeln Va und Vb bzw. VIa und VIb in Gegenwart eines der genannten Reduktionsmittel vornimmt.

In Ausgangsstoffen der Formel VII bzw. VIII für die Verfahrensvariante d) bedeutet gegebenenfalls anhydridisiertes oder verestertes Carboxy $Y_3$ bzw. $Y_4$ beispielsweise Halogencarbonyl oder in Falle von $Y_4$ eine Gruppe der Formel $R_2-C(=O)-O-$ und ein metallischer Rest $Y_3$ bzw. $Y_4$ beispielsweise ein Alkalimetallatom oder eine Gruppe der Formel $-M^{II}/2$ oder $M^{II}$-Hal, worin $M^{II}$ ein Metallatom der Gruppe IIb des Periodischen Systems der Elemente, wie Mg oder Zn, bedeutet.

Die Umsetzung von Verbindungen der Formeln VII und VIII erfolgt in üblicher Weise, beispielsweise in einem etherartigen Lösungsmittel, wie einem aliphatischen oder cycloaliphatischen Ether, z.B. in Diethylether, Methoxybutan, Dibutylether, Tetrahydrofuran oder Dioxan.

Ausgangsstoffe der Formel VII, worin $Y_3$ Formyl oder gegebenenfalls anhydridisiertes oder verestertes Carboxy bedeutet, werden beispielsweise hergestellt, indem man Verbindungen der Formeln VIIa und VIIIa

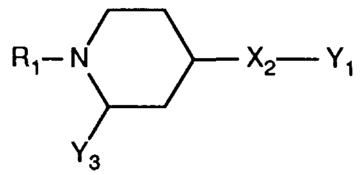
(VIIa)

und

$Y_2$-$X_3$-$R_2$ (VIIIa),

worin $Y_1$ eine Gruppe der Formel -N($R_3$)-H und $Y_2$ Hydroxy, reaktionsfähiges verestertes Hydroxy oder, sofern $X_3$ für Carbonyl steht, veräthertes oder anhydridisiertes Hydroxy darstellt oder $Y_1$ Hydroxy, reaktionsfähig verestertes Hydroxy oder, sofern $X_2$ Carbonyl bedeutet, veräthertes oder anhydridisiertes Hydroxy und $Y_2$ eine Gruppe der Formel -N($R_3$)-H darstellt, wobei $R_2$, $R_3$, $R_4$, $X_1$, $X_2$ und $X_3$ die angegebenen Bedeutungen haben, oder deren Salze miteinander kondensiert, beispielsweise wie unter der Verfahrensvariante b) angegeben.

Ausgangsstoffe der Formeln VII bzw. VIII, worin $Y_3$ bzw. $Y_4$ einen metallischen Rest bedeutet, werden vorzugsweise in situ hergestellt, indem man eine Verbindung der Formel VIIb

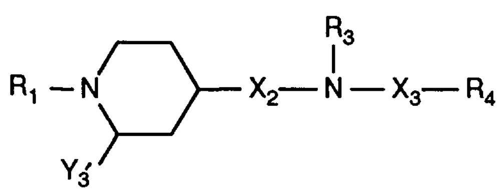
(VIIb),

worin $Y'_3$ ein Halogenatom, insbesondere Chlor, Brom oder Jod, bedeutet, mit einem Metall der Formel $M^{II}$ umsetzt bzw. ausgehend von Verbindungen der Formel $Y'_4$-$R_2$ (VIIIb), worin $Y'_4$ Wasserstoff oder ein Halogenatom, insbesondere Chlor, Brom oder Jod, bedeutet, eine Verbindung der Formel VIIIb, worin $Y'_4$ Wasserstoff ist mit einer metallorganischen Verbindung, beispielsweise einem Metallderivat eines aliphatischen Kohlenwasserstoffes, z.B. mit Butyllithium, bzw. eine Verbindung der Formel VIIIb, worin $Y'_4$ ein Halogenatom bedeutet, mit einem Metall der Formel $M^{II}$ umsetzt.

In Ausgangsstoffen der Formel IX gemäss der Verfahrensvariante e) ist die Aminoschutzgruppe $Y_5$ beispielsweise eine gegebenenfalls halogenierte Niederalkanoylgruppe, wie Trifluoracetyl, oder eine von einem Halbester der Kohlensäure abgeleitete Acylgruppe, wie eine Niederalkoxycarbonyl- oder $\alpha$-Phenylniederalkoxycarbonylgruppe, z.B. Tertiärbutyloxycarbonyl oder Benzyloxycarbonyl, oder eine Silylgruppe, wie Triniederalkylsilyl, z.B. Trimethylsilyl. Die Abspaltung der Aminoschutzgruppe erfolgt in üblicher Weise, beispielsweise durch Säurebehandlung, oder ausgehend von Verbindungen IX, worin $Y_5$ halogeniertes Niederalkanoyl, wie Trifluoracetyl, ist, durch reduktive Abspaltung, beispielsweise durch Behandeln mit einem Dileichtmetallhydrid, wie Natriumborhydrid, vorzugsweise in einem Niederalkanol, wie Methanol.

Die Ausgangsstoffe der Formel IX können beispielsweise in Analogie zur Verfahrensvariante a) hergestellt werden, wobei man von entsprechenden Verbindungen der Formel IXa

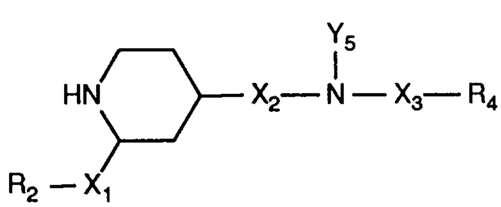
(IXa)

ausgeht.

Die Umsetzung von Verbindungen der Formeln X und XI gemäss der Verfahrensvariante f) erfolgt beispielsweise durch Wasserabspaltung, beispielsweise durch azeotrope Destillation, insbesondere mit Toluol, und anschliessende Reduktion mit Boran oder einem Dileichtmetallhydrid, wie einem Alkalimetalborhydrid, z.B. mit Natriumboranat oder Natriumcyanoborhydrid.

Ausgangsstoffe der Formel X, worin $R_1$ eine der unter Formel I genannten Acylreste, $X_1$ Hydroxymethylen darstellt und $Y_6$ und $Y_7$ gemeinsam für Oxo stehen, und ihre pharmazeutisch verwendbaren Salze weisen die gleichen pharmakologischen Eigenschaften und vergleichbare Wirkungsstärke auf wie die Endstoffe der Formel I.

Die Erfindung betrifft dementsprechend auch 1-Acylpiperidone der Formel X

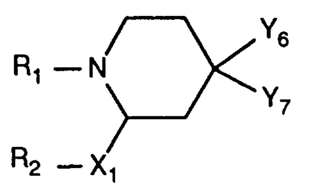

(X),

worin $R_1$ einen gegebenenfalls substituierten Aroyl-, Heteroaroyl-, Cycloalkylcarbonyl-, Aralkanoyl-, Heteroarylalkanoyl-, Aralkoxycarbonyl- oder Arylcarbamoylrest oder den Acylrest einer gegebenenfalls durch Niederalkanoyl oder Carbamoylniederalkanoyl N-substituierten $\alpha$-Aminosäure bedeutet, $R_2$ Cycloalkyl oder einen gegebenenfalls substituierten Aryl- oder Heteroarylrest darstellt, $X_1$ Hydroxymethylen darstellt und $Y_6$ und $Y_7$ gemeinsam für Oxo stehen, und ihre Salze, Verfahren zur Herstellung der erfindungsgemässen Verbindungen der Formel X, diese enthaltende pharmazeutische Präparate und ihre Verwendung als Arzneimittelwirkstoffe.

Dabei hat die Variable $R_2$ vorzugsweise die für die Verbindungen der Formel I, vorzugsweise die vorstehend für besonders bevorzugte Verbindungen der Formel I, angegebene Bedeutung.

Die Erfindung betrifft in allererster Linie diejenigen Verbindungen der Formel X, worin $R_1$ unsubstituiertes oder durch $C_1$-$C_4$-Alkyl, wie Methyl, $C_1$-$C_4$-Alkoxy, wie Methoxy, Halogen der Atomnummer bis und mit 35, wie Chlor, und/oder Trifluormethyl mono- oder disubstituiertes Benzoyl oder unsubstituiertes Naphthoyl bedeutet, $R_2$ unsubstituiertes oder durch Halogen der Atomnummer bis und mit 35, wie Chlor, und/oder Trifluormethyl mono- oder disubstituiertes Phenyl darstellt, $X_1$ Hydroxymethylen darstellt und $Y_6$ und $Y_7$ gemeinsam für Oxo stehen, und ihre Salze.

Die Erfindung betrifft namentlich die in den Beispielen genannten Verbindungen der Formel X und ihre Salze.

Die erfindungsgemässen Verbindungen der Formel X, worin $R_1$ und $R_2$ die angegebenen Bedeutungen haben, $X_1$ Hydroxymethylen darstellt und $Y_6$ und $Y_7$ gemeinsam für Oxo stehen, und ihre Salze werden nach an sich bekannten Methoden hergestellt. Das erfindungsgemässe Verfahren zu ihrer Herstellung ist dadurch gekennzeichnet, dass man eine Verbindung der Formel Xa

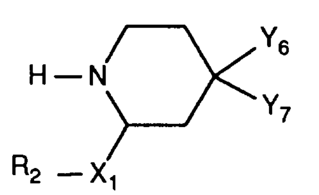

(Xa),

mit einem den Rest $R_1$ einführendem Mittel kondensiert und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Als den Rest $R_1$ einführende Mittel kommen beispielsweise Verbindungen der Formel $R_1$-$Y_{10}$ (Xb) in Betracht, worin $Y_{10}$ reaktionsfähiges verestertes Hydroxy, wie Halogen oder eine Sulfonyloxygruppe, wie Benzol-, p-Toluol- oder Methansulfonyloxy, oder sofern $R_1$ Aroyl-, Heteroaroyl-, Cycloalkylcarbonyl-, Aralkanoyl-, Heteroarylalkanoyl-, Aralkoxycarbonyl- oder Arylcarbamoylrest oder den Acylrest einer gegebenenfalls durch Niederalkanoyl oder Carbamoylniederalkanoyl N-substituierten $\alpha$-Aminosäure bedeutet, verether-

tes Hydroxy, wie Niederalkoxy oder gegebenenfalls, beispielsweise durch Halogen und/oder Nitro, substituiertes Phenoxy, darstellt.

Die Umsetzung erfolgt in üblicher Weise, beispielsweise in Gegenwart eines basischen Kondensationsmittels, wie eines Alkalimetallhydrogencarbonates, z.B. von Natriumhydrogencarbonat, vorzugsweise in einem wasserhaltigen Zweiphasensystem, z.B. in Methylenchlorid/Wasser.

Verbindungen der Formel Xa können ihrerseits hergestellt werden durch Umsetzung eines N-geschützten Piperidin-4-onketals, z.B. von 1-(Tertiärbutyloxycarbonyl)piperindin-4-on-ethylenketal, mit einem Aldehyd der Formel $R_2$-CH = O (Xc), beispielsweise in Gegenwart einer Metall-, wie Alkalimetallkohlenwasserstoffverbindung, vorzugsweise einer Niederalkyllithiumverbindung, z.B. von Sekundärbutyllithium, insbesondere in einem etherartigen Lösungsmittel, wie in Diethylether, bei -30° bis -80° C, z.B. bei -60° bis -75° C.

Verbindungen der Formel X, worin $Y_6$ eine Gruppe der Formel -N($R_3$)-H und $Y_7$ Wasserstoff bedeutet, werden beispielsweise hergestellt, indem man eine Verbindung der Formel $R_2$-$X_1$-Y (Xd), worin Y reaktionsfähiges verestertes Hydroxy, wie Halogen, Niederalkansulfonyloxy oder gegebenenfalls substituiertes Benzolsulfonyloxy bedeutet, in Gegenwart eines Alkalimetallniederalkanolates, wie Natriummethanolat, in einem Niederalkanol, wie Methanol, oder in Gegenwart von Natriumamid in Toluol mit einem But-2-en-1,1-dicarbonsäureniederalkylester der Formel $CH_2$ = CH-$CH_2$-CH(COOR)$_2$ (Xe; R = Niederalkyl) kondensiert, das Reaktionsprodukt der Formel Xf

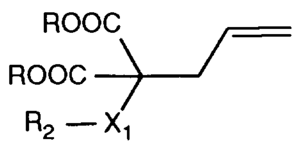

(Xf),

durch Behandlung mit einem Alkalimetallhydroxid, z.B. mit Kaliumhydroxid in wässrigem Methanol, verseift und decarboxyliert, die erhaltene Säure der Formel Xg

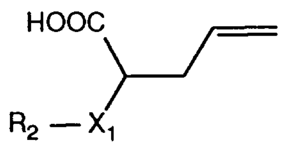

(Xg),

z.B. durch Behandeln mit einem Halogenierungsmittel, wie Oxalylchlorid oder Thionylchlorid, und nachfolgende Umsetzung mit Ammoniak amidiert und anschliessend zum entsprechenden Amin der Formel Xh

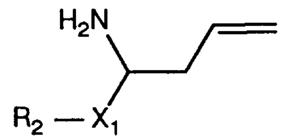

(Xh)

abbaut, dieses nach Schutz der Aminogruppe, beispielsweise durch Acylierung, mit einem Niederalkoxymethylhalid der Formel RO-$CH_2$-Hal (Xi; R = Niederalkyl; Hal = Halogen), z.B. mit Chlordimethylether in Gegenwart von Natriumhydroxid in Dichlormethan/Wasser, kondensiert, das Reaktionsprodukt der Formel Xi

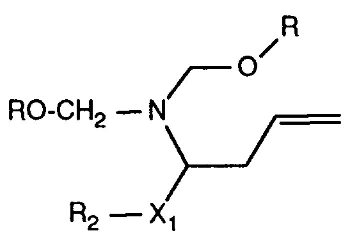

(Xi)

durch Säurebehandlung, beispielsweise Behandlung mit einer Lewissäure, wie Zinntetrachlorid, Eisen-IIIchlorid, Titantetrachlorid, oder Protonensäure, wie Schwefelsäure, Chlorsulfonsäure, p-Toluolsulfonsäure oder Trifluormethylessigsäure oder -methansulfonsäure, in Acetonitril und erforderlichenfalls Acetanhydrid und einem weiteren Lösungsmittel, wie Dichlormethan, Benzol oder Toluol, zur entsprechenden N-geschützten Verbindung der Formel Xj

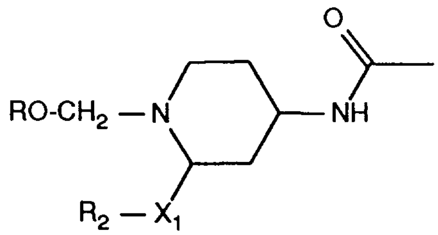

(Xj)

cyclokondensiert, die Aminoschutzgruppe abspaltet, gewünschtenfalls ein erhaltenes Racemat in die Enantiomeren auftrennt, in üblicher Weise die Gruppe $R_1$, beispeilsweise wie vorstehend für die Herstellung von Verbindungen der Formel X, worin $X_1$ Hydroxymethylen und $Y_6$ gemeinsam mit $Y_7$ Oxo darstellt, beschrieben, einführt und die Aminoschutzgruppe durch Säurebehandlung, beispielsweise mit 6N-Salzsäure, abspaltet.

Verfahrensgemäss erhältliche Verbindungen können in üblicher Weise in andere Verbindungen der Formel I überführt werden.

So kann man Verbindungen der Formel I, worin $X_1$ Carbonyl bedeutet, in üblicher Weise zu den entsprechenden Verbindungen der Formel I reduzieren, worin $X_1$ für Hydroxymethylen steht, beispielsweise wie unter der Verfahrensvariante c) bzw. für die Herstellung von Zwischenprodukten der Formeln V und VI beschrieben. In analoger Weise können auch erhaltene Verbindungen der Formel I, worin $X_1$ Hydroxymethylen oder $X_2$ und/oder $X_3$ Carbonyl bedeutet, zu den entsprechenden Verbindungen der Formel I reduziert werden, worin $X_1$, $X_2$ und/oder $X_3$ Methylen darstellt.

In erhaltenen Verbindungen der Formel I, worin $X_1$ ketalisiertes Carbonyl bedeutet, kann die Carbonylgruppe in üblicher Weise, beispielsweise durch Säurebehandlung, freigesetzt werden. Umgekehrt kann Carbonyl $X_1$ durch Umsetzung mit einem entsprechenden Alkohol, wie ein Niederalkanol oder einem Niederalkandiol, ketalisiert werden.

Ferner kann man in erhaltenen Verbindungen der Formel I, worin $R_3$ Wasserstoff bedeutet, einen von Wasserstoff verschiedenen Rest $R_3$ einführen, Alkyl beispielsweise durch übliche Alkylierung, Carbamoyl beispielsweise durch Kondensation mit Isocyansäure oder einem Carbamoylhalogenid und gegebenenfalls wie angegeben substituiertes Alkanoyl bzw. Alkenoyl durch übliche Acylierung. Umgekehrt kann man in erhaltenen Verbindungen der Formel I, worin $R_3$ Alkyl, insbesondere Methyl, ist, die Alkylgruppe durch Behandlung mit einem Halogenameisensäureester, wie -methylester, abspalten.

Weiterhin kann man in erhaltenen Verbindungen der Formel I verestertes oder amidiertes Carboxy als Substituent von Alkanoyl bzw. Alkenoyl $R_3$ oder von Alkylen $X_3$ zu Carboxy hydrolysieren bzw. umgekehrt freies Carboxy verestern bzw. amidieren.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate

aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgen Beispiele dienen zur Illustration der Erfindung; Temperaturen sind in Celsiusgraden, Drucke in mbar angegeben.

Beispiel 1: (2R,4S) und (2R,4R)-2-Benzyl-1-(3,5-dimethylbenzoyl)-N-(2-phenethyl)-4-piperidinamin-hydrochlorid

Zu einem Gemisch von 3.65 g (11.4 mMol) (2R,4RS)-2-Benzyl-1-(3,5-dimethylbenzoyl)-4-piperidinamin in 30 ml Methanol, 935 mg (11.4 mMol) Natriumacetat, 0.65 ml (11.4 mMol) Essigsäure und 1.44 g (12 mMol) Phenylacetaldehyd werden unter Stickstoff bei 0° während 10 Minuten 1.26 g (17.1 mMol)

Natriumcyanoborhydrid (85%) portionsweise zugegeben. Danach rührt man das Reaktionsgemisch 3 Stunden bei Raumtemperatur, gibt nochmals 0.376 g (2.4 mMol) Phenylacetaldehyd zu und rührt 16 Stunden bei 4° aus. Man entfernt das Methanol am Rotationsverdampfer und verteilt das rötliche Reaktionsgemisch zwischen Ether und 1n-Natriumbicarbonatlösung. Die organischen Phasen werden mit Sole gewaschen, über Magnesiumsulfat getrocknet und zur Trockne eingedampft. Man erhält ein Gemisch der Hydrochloride der Titelverbindungen der Formeln

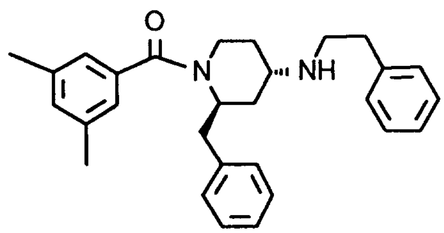

Diastereomer A

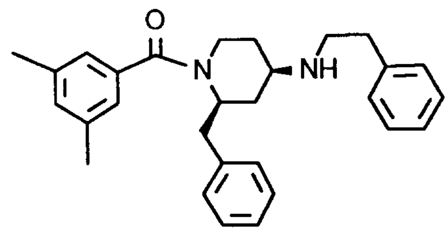

Diastereomer B

als gelbes Öl. Dieses wird mit dem Elutionsmittelgemisch Methylenchlorid/Methanol/conc. Ammoniak (97.5:2.25:0.25) zur Trennung der Diastereomeren an Kieselgel chromatographiert, wobei man die Diastereomeren als freie Basen rein erhält.
DC: Methylenchlorid/Methanol (98:2)
Diastereomer A (2R,4R): $R_f$ = 0.16, Smp. 248-249°C, $[\alpha]_D$ = -56.9° (c = 0.946,Methanol), MS: $M^+$ = 426 (freie Base).
Diastereomer B (2R,4S): $R_f$ = 0.06, Smp. 270°C (Zersetzung), $[\alpha]_D$ = +30.6° (c = 0.759, Methanol), MS: $M^+$ = 426 (freie Base).

Die Ausgangsverbindungen dazu werden wie folgt hergestellt:

a) (R)-3-Benzylamino-4-phenylbuttersäureethylester

Eine Lösung von 42.2 g (0.203 Mol) (R)-3-Amino-4-phenylbuttersäureethylester erhältlich durch Veresterung der bekannten (R)-3-Amino-4-phenylbuttersäure mit Ethanol, 11.6 ml (0.203 Mol) Eisessig, 33.3 g (0.406 Mol) Natriumacetat und 20.9 ml (0.207 Mol) Benzaldehyd in 400 ml Methanol werden bei -5 bis 5° portionenweise mit total 19.1 g Natriumcyanoborhydrid (0.304 Mol) versetzt. Nach beendeter Zugabe lässt man noch 1 Stunde bei Raumtemperatur ausreagieren. Die gelbe Suspension engt man am Rotationsverdampfer fast vollständig ein und verteilt den breiigen Rückstand zwischen Essigester und Wasser, welches man mit Ammoniaklösung auf pH ca. 8 stellt. Die organischen Phasen wäscht man mit Wasser und Sole neutral, trocknet sie über Magnesiumsulfat und engt zur Trockne ein wobei man ein gelbes Öl erhält. Dieses wird an Kieselgel mit Methylenchlorid/Methanol 99:1 chromatographiert, wobei man die Titelverbindung der Formel als schwach gelbes Öl erhält. Durch Zugabe von Oxalsäure zu einer etherischen Lösung der Titelverbindung erhält man das Oxalat.
Smp. 142-143°.
DC: Methylenchlorid/Methanol (95:5): $R_f$ = 0.63
MS: $M^+$- 91 = 206 (60%)
$[\alpha]_D$ = + 3° (c = 1, Ethanol) freie Base
$[\alpha]_D$ = - 0.8° (c = 1, $CHCl_3$) Oxalat

| | | | | |
|---|---|---|---|---|
| $C_{21}H_{25}NO_6$ (Oxalat): | C ber.<br>H ber.<br>N ber. | 65.11%,<br>6.51%,<br>3.62% | gef.<br>gef.<br>gef. | 65.12%<br>6.46%<br>3.77% |

b) (R)-N-Benzyl-N-[(1-ethoxycarbonylmethyl-2-phenyl)ethyl]carbamoyl-essigsäuremethylester

Zu einer im Eiswasserbad gekühlten Lösung von 115.8 g (0.389 Mol) (R)-3-Benzylamino-4-phenylbuttersäure-ethylester, 56.8 ml (0.408 Mol) Triethylamin und 366 mg Dimethylaminopyridin in 630 ml Toluol tropft man während 2 1/2 Stunden eine Lösung von 43.8 ml (0.408 Mol) Malonsäuremonomethylesterchlorid

in 480 ml Toluol, sodass die Temperatur innerhalb 0-5° bleibt. Die Suspension lässt man während 2 Stunden ausreagieren und giesst sie danach auf 500 ml Eiswasser. Die organische Phase wird abgetrennt, nacheinander mit 0.1N Salzsäurelösung, 1N Natriumbicarbonatlösung und Eiswasser gewaschen, danach über Natriumsulfat getrocknet und zur Trockne eingeengt. Das anfallende gelbe Öl chromatographiert man an Kieselgel mit Essigester/Hexan (1:2), wobei man die Titelverbindung erhält.
DC: Essigester/Hexan (1:2), $R_f$ = 0.25
MS: $M^+$ = 397 (3%)
$[\alpha]_D$ = + 19.5 ° (c = 1.3, $CHCl_3$)

c) (6R)-1,6-Dibenzyl-2,4-dioxo-3-piperidincarbonsäuremethylester

Zu einer Lösung von 53.9g (0.135 Mol) (R)-N-Benzyl-N-[(1-ethoxycarbonylmethyl-2-phenyl)ethyl]-carbamoyl-essigsäuremethylester in 520 ml tert.-Butanol gibt man bei Raumtemperatur 15.2g (0.135 Mol) Kaliumtertiärbutanolat und lässt während 1 Stunde ausreagieren. Die hellgelbe Suspension versetzt man bei Raumtemperatur mit 1 Äquivalent (8.1 g) Eisessig und engt auf ca. 100 ml Totalvolumen ein. Das Konzentrat verdünnt man mit 300 ml Wasser und extrahiert 3 mal mit je 300 ml Essigester. Die organischen Phasen wäscht man hiernach mit Wasser und Sole, trocknet die vereinigten organischen Phasen über Natriumsulfat und engt zur Trockne ein, wobei man die Titelverbindung als klares, gelbes Öl erhält, welches ohne weitere Reinigung weiterverwendet wird; DC. Essigester/Methanol (1:1); $R_f$ = 0.3.

d) (6R)-1,6-Dibenzyl-2,4-piperidindion

Eine Lösung von 106.1g (0.301 Mol) (6R)-1,6-Dibenzyl-2,4-dioxo-3-piperidin-carbonsäuremethylester in 298 ml Toluol und 445 ml 10 %-iger (Vol/Vol) Essigsäure wird während 2 1/2 Stunden auf 80° erwärmt. Das Reaktionsgemisch kühlt man auf Raumtemperatur, neutralisiert durch Zugabe von 48 g festem Natriumcarbonat unter Eiswasserkühlung, trennt die Phasen und extrahiert die wässrige Phase nochmals mit 300 ml Essigester. Die vereinigten organischen Phasen wäscht man mit Wasser und mit gesättigter Kochsalzlösung, trocknet sie über Natriumsulfat und engt zur Trockne ein. Das anfallende Öl kristallisiert man aus Ether, wobei man die Titelverbindung erhält.
Smp. 97-97.5°
DC: Essigester/Hexan (2:1), $R_f$ = 0.31
$[\alpha]_D$: + 166.9 ° (c=1, $CHCl_3$)
MS: $M^+$ = 293 (2.4%)

e) (2R,4RS)-1,2-Dibenzyl-4-piperidinamin

(Variante e1)

e1a) 6R)-1,6-Dibenzyl-4-(methoxyimino)-2-piperidon

Eine Lösung von 10 g (0.034 Mol) (6R)-1,6-Dibenzyl-2,4-piperidindion in 68 ml Pyridin versetzt man mit 3.09 g (0.037 Mol) Methoxyamin-hydrochlorid und erhitzt während 1h auf 85°. Die klare gelbe Lösung giesst man auf eiskalte 1N-Salzsäurelösung (pH ca. 3) und extrahiert mit Toluol. Die organischen Extrakte wäscht man mit 1N-Salzsäurelösung, dann mit 1N-Sodalösung und mit Sole. Danach trocknet man über Natriumsulfat und engt zur Trockne ein, wobei man die Titelverbindung in Form wachsartiger Kristalle erhält.
Smp. 63-77°
DC: Essigester/Hexan (1:1), $R_f$ = 0.52
MS: $M^+$: 322 (1.4%)
Auf Grund des $^1H$-NMR ($CDCl_3$) liegt ein *syn/anti*-Gemisch im Verhältnis von ca. 7:3 vor, Methoxysignale des Oximethers bei 3.92 bzw. 3.88 ppm.

e1b) (2R,4RS)-1,2-Dibenzyl-4-piperidinamin

In einer Destillationsapparatur mit aufgesetzter Vigreuxkolonne und mit 40° warmem Wasser durchströmtem Kühler erhitzt man eine Lösung von 9.19 g (0.0285 Mol) (6R)-1,6-Dibenzyl-4-(methoxyimino)-2-piperidon in 90 ml Tetrahydrofuran unter Argon zum Rückfluss. Zu dieser Lösung tropft man innert 20 Minuten 6.1 ml (0.0643 Mol) Boran/Dimethylsulfidkomplex gefolgt von einer zweiten Zugabe von 9 ml

(0.0949 Mol) Boran/Dimethylsulfidkomplex innert 4 Stunden. Während der Zugabe des Boran/Dimethylsulfidkomplexes entweicht das frei werdende Dimethylsulfid durch die Destillationsapparatur.

Nach beendeter Zugabe kühlt man das Reaktionsgemisch im Eiswasserbad auf 0-4° und hydrolysiert überschüssiges Boran durch langsame Zugabe von total 20 ml Methanol. Nach Abklingen der heftigen, exothermen Hydrolyse entfernt man die Lösungsmittel am Wasserstrahlvacuum direkt aus der Apparatur. Danach kocht man den Rückstand während 2 Stunden nach Zugabe von 90 ml 5N-Salzsäurelösung. Die auf Raumtemperatur gekühlte Lösung verdünnt man mit 200 ml Wasser, extrahiert mit Ether zur Entfernung von Säure- und Neutralteil, kühlt danach die Wasserphase im Eiswasserbad, stellt sie mit 5N-Natronlauge auf pH ca. 9 und extrahiert den Baseteil mit Ether/Tetrahydrofuran (2:1). Die organischen Extrakte trocknet man über Magnesiumsulfat und engt zur Trockne ein, wobei man die Rohbase in Form eines gelblichen Öles erhält. Diese wird direkt in die nächste Stufe eingesetzt; DC: Methylenchlorid/Methanol/conc. Ammoniak (90:10:0.4), $R_f = 0.3$.

Durch Lösen in methanolischer Salzsäurelösung und Zugabe von Ether fällt man amorphes Dihydrochlorid der Titelverbindung; Smp. 150-182°.

Variante e2)

e2a) (6R)-1,6-Dibenzyl-2,4-piperidindion-4-ethylenketal

Eine Lösung von 30 g (0.102Mol) (6R)-1,6-Dibenzyl-2,4-piperidindion, 50 ml Ethylenglykol und 1.8 g p-Toluolsulfonsäure-monohydrat in 800 ml Toluol erhitzt man während 3h am Wasserabscheider. Die auf Raumtemperatur gekühlte Lösung wäscht man mit 100 ml 1N-Natriumbicarbonatlösung und Sole, trocknet die organische Phase über Magesiumsulfat und engt zur Trockne ein, wobei man das rohe Ketal als Öl erhält. Dieses chromatographiert man an Kieselgel mit Essigester und kristallisiert das aus der Chromatographie erhaltene Öl aus Ether, wobei man die Titelverbindung in Form weisser Kristalle erhält.
Smp. 91-93°
DC: Essigester/Hexan (3:1), $R_f$ = 0.53
MS: $M^+$: 337

e2b) (2R)-1,2-Dibenzyl-4-piperidon-ethylenketal

Eine Lösung von 10.2 g (0.0302Mol) (6R)-1,6-Dibenzyl-2,4-piperidindion-4-ethylenketal in 100 ml Tetrahydrofuran versetzt man unter Argon innert 10 Minuten mit 7.6 ml (0.0756 Mol) Boran/Dimethylsulfidkomplex und erhitzt für 1h zum Rückfluss. Danach gibt man zur auf Raumtemperatur gekühlten Lösung 40 ml 2N-Natronlauge und erhitzt erneut während 2h zum Rückfluss, entfernt anschliessend das Tetrahydrofuran am Rotationsverdampfer und extrahiert das Reaktionsgemisch mit Ether. Die organischen Extrakte wäscht man mit Natriumbicarbonat, trocknet sie über Magnesiumsulfat und engt zur Trockne ein, wobei man die Titelverbindung erhält; DC: Essigester/Hexan (2:1), $R_f$ = 0.81; MS: $M^+$: 323.

e2c) (2R)-1,2-Dibenzyl-4-piperidon

Eine Lösung von 85.7 g (0.261 Mol) (2R)-1,2-Dibenzyl-4-piperidon-ethylenketal in 170 ml Dioxan und 1000 ml 2.25 M Salzsäurelösung werden während 29 Stunden auf 70° erwärmt. Danach entfernt man das Dioxan im Vacuum, stellt die Wasserphase mit 30 %-iger Natronlauge unter Eiswasserkühlung auf pH ca. 8 und extrahiert mit Ether. Die Etherextrakte wäscht man mit 1N-Natriumbicarbonatlösung, trocknet sie über Natriumsulfat und engt zur Trockne ein, wobei man die Titelverbindung als rötliches Öl erhält, das wegen seiner Instabilität ohne weitere Reinigung weiterverarbeitet wird; DC: Essigester/Hexan (1:1); $R_f = 0.71$; FD-MS: $M^+$: 279

e2d) (2R)-1,2-Dibenzyl-4-(methoxyimino)-piperidin

3 g (0.01071 Mol) (2R)-1,2-Dibenzyl-4-piperidon, 4.4 g (0.0537 Mol) Natriumacetat und 942 mg (0.0113 Mol) Methoxyamin-hydrochlorid löst man in 30 ml Ethanol und erhitzt die 30 Minuten auf 60°. Danach entfernt man das Ethanol im Vacuum und verteilt den Rückstand zwischen Wasser und Essigester, trocknet die organischen Extrakte über Natriumsulfat und engt zur Trockne ein, wobei man das Rohprodukt erhält. Dieses chromatographiert man an Kieselgel mit Essigester/Hexan (3:1), wobei man die Titelverbindung in Form eines Öles erhält.
DC: Essigester/Hexan (1:1), $Rf_1$ = 0.84, $Rf_2$ = 0.76 (*syn–/anti*-Oximether)

MS: $M^+$: 308 (1%), $M^+$-91: 217 (90%).
$^1$H-NMR-Spektrum ($CD_3OD$), $\delta$(ppm) = 3.85 (s, =N-$OCH_3$), 3.825 (s): ca. 1:1

e2e) (2R,4RS)-1,2-Dibenzyl-4-piperidinamin

In eine Lösung von 5.43 g (17.6mMol)(2R)-1,2-Dibenzyl-4(methoxyimino)-piperidin in 60 ml Tetrahydrofuran kondensiert man bei -78° 180 ml über Kaliumhydroxid getrocknetes Ammoniakgas. Zu dieser Lösung gibt man bei -70° 3.7g (69 mMol) Ammoniumchlorid und portionenweise 1.6 g (70.4 mMol) Natrium-Metall. Nach einer Stunde gibt man zur entstandenen Suspension nochmals 3.7 g Ammoniumchlorid und 0.6 g Natrium-Metall und rührt 2 Stunden bei -70° nach. Danach entfernt man das Kühlbad und lässt das Ammoniakgas verdampfen.
Den Rückstand verteilt man zwischen 1N-Natronlauge und Ether, trennt die organische Phase ab, extrahiert die wässrige Phase nach, wäscht die organischen Phasen mit Sole, trocknet die vereinigten organischen Phasen über Natriumsulfat und engt zur Trockne ein, wobei man die Titelverbindung in Form eines gelben Öles erhält; DC: Methylenchlorid/Methanol/conc. Ammoniak (90:9:1), $R_f$ = 0.33); MS: $M^+$ = 280

f) (2R,4RS)-N-(1,2-Dibenzyl-4-piperidyl)-trifluoracetamid-trifluoracetat

Eine Lösung von 6.88 g (24.5 mMol) (2R,4RS)-1,2-Dibenzyl-4-piperidinamin in 20 ml Methylenchlorid wird im Eiswasserbad mit 5.1 ml (36.8 mMol) Trifiuoressigsäure-anhydrid versetzt und danach bei Raumtemperatur während einer Stunde ausgerührt. Das Reaktionsgemisch wird zur Trockne eingeengt, wobei man die Titelverbindung als schwach gelben Schaum erhält; DC: Methylenchlorid/Methanol/conc. Ammoniak (190:9:1), $R_f$ = 0.41 (cis) bzw. 0.57 (trans) Diastereomeres; MS: $M^+$-91 (Benzyl) = 285 (14%).

g) (2R,4RS)-N-(2-Benzyl-4-piperidyl)-trifluoracetamid-trifluoracetat

Zu einer Lösung von 19.3 g (39.4mMol) (2R,4RS)-N-(1,2-Dibenzyl-4piperidyl)-trifluoracetamid-trifluoracetat in 160 ml Dioxan gibt man unter Stickstoff 3.0 g 10% Palladiumkatalysator auf Kohle und hydriert bei Raumtemperatur unter Normaldruck. Das Reaktionsgemisch wird über Celite® vom Katalysator befreit und den Rückstand wäscht man mit Dioxan nach. Das Filtrat wird zur Trockne eingeengt und am Hochvacuum getrocknet, wobei man die Titelverbindung erhält, die ohne weitere Reinigung weiterverwendet wird; DC: Methylenchlorid/Methanol/conc. Ammoniak (90:9:1) $R_f$ = 0.24 bzw. 0.3 (zwei schwach getrennte Diastereomere)

h) (2R,4RS)-N-[2-Benzyl-1-(3,5-dimethylbenzoyl)-4-piperidyl]-trifluoracetamid

Zu einem mit Eiswasser gekühlten Gemisch von 1.39 g (3.44 mMol) (2R,4RS)-N-(2-Benzyl-4-piperidyl)-trifluoracetamid-trifluoracetat und 10ml Toluol/Wasser (1:1) gibt man unter Rühren 710 mg festes Natriumbicarbonat und 712 mg 3,5-Dimethylbenzoylchlorid. Danach lässt man das Reaktionsgemisch auf Raumtemperatur erwärmen, rührt 2 Stunden nach und verteilt zwischen Toluol und 1n-Natriumbicarbonatlösung. Die organischen Phasen werden mit Wasser gewaschen, über Magnesiumsulfat getrocknet und zur Trockne eingeengt. Das farblose Öl wird mit Essigester/Hexan 1:2 an Kieselgel chromatographiert, wobei man die Titelverbindung erhält. Diese wird ohne weitere Reinigung weiterverwendet.
DC: Methylenchlorid/Methanol/conc. Ammoniak (190:9:1) $R_f$ = 0.5, (keine Trennung der beiden Diastereomere unter diesen Bedingungen
MS: $M^+$ = 418 (3%), $M^+$-91 (43%)

i) (2R,4RS)-2-Benzyl-1-(3,5-dimethylbenzoyl)-4-piperidinamin

Eine Lösung von 4.73 g (10.4 mMol) (2R,4RS)-N-[2-Benzyl-1-(3,5-dimethylbenzoyl)-4-piperidyl]-trifluoracetamid in 50 ml Tetrahydrofuran/Methanol 1:1 wird bei Raumtemperatur unter Stickstoff mit 4.1 ml 5n-Natriumhydroxidlösung versetzt und 3 Stunden zum Rückfluss erhitzt. Nach beendeter Reaktion kühlt man das Reaktionsgemisch in Eiswasser, stellt mit 1N-Salzsäurelösung auf pH ca. 1 und entfernt die organischen Lösungsmittel am Rotationsverdampfer. Die verbleibende saure Wasserphase extrahiert man zuerst mit Ether zur Entfernung von Säure- und Neutralteil, stellt sie danach unter Eiswasserkühlung durch Zugabe von 10n Natriumhydroxidlösung auf pH ca. 10 und extrahiert mit Ether. Die organischen Phasen wäscht man mit Sole, trocknet sie über Natriumsulfat und dampft zur Trockne ein, wobei man die freie Base als bräunliches Öl erhält, das ohne weitere Reinigung weiterverwendet wird; DC: Methylenchlorid/Methanol/conc. Ammoniak (90:9:1) $R_f$ = 0.29; MS: $M^+$ = 322 (0.03%), $M^+$-91 = 231 (62%).

Analog dazu erhält man ausgehend von L-Phenylalaninol gemäss der vorstehenden Reaktionssequenz (2S,4RS)-2-Benzyl-1-(3,5-dimethylbenzoyl)-4-piperidinamin.

Beispiel 2: (2R*,4S*)-2-Benzyl-1-(2-naphthoyl)-N-(4-chinolylmethyl)-4-piperidinamin

Zur Lösung von 106 mg (0.182 mMol) (2R*,4S*)-2-Benzyl-1-(2-naphthoyl)-N-(4-chinolylmethyl)-N-trifluoracetyl-4-piperidinamin in 1.5 ml Methanol werden bei 0° während 20 Minuten 28 mg (0.73 mMol) Natriumborhydrid in drei Portionen gegeben und das Gemisch anschliessend 3 Stunden bei 0° gerührt. Danach versetzt man das Reaktionsgemisch mit 0.06 ml (0.81 mMol) Aceton und rührt 10 Minuten aus. Man entfernt das Methanol am Rotationsverdampfer und verteilt den festen weissen Rückstand zwischen Ethylacetat und Wasser. Die organischen Phasen werden mit Sole gewaschen, über Magnesiumsulfat getrocknet und zur Trockne eingedampft. Der erhaltene weisse Schaum wird mit Methylenchlorid/Methanol/conc. Ammoniak (1500:50:1) an Kieselgel chromatographiert. Es wird die Titelverbindung der Formel

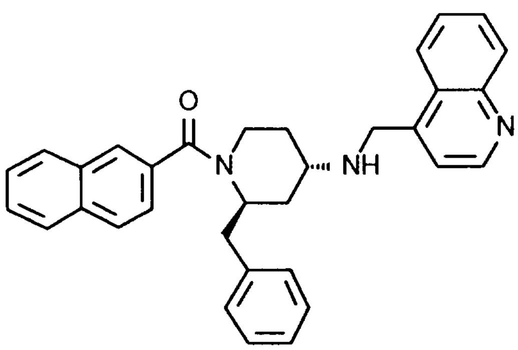

als weisser Schaum erhalten. DC: Methylenchlorid/Methanol/conc. Ammoniak (700:50:1) $R_f$=0.34, FD-MS: $M^+$ = 485.

Die Ausgangsverbindung dazu wird wie folgt hergestellt:

a) 2-Benzyl-N-benzyloxycarbonyl-2,3-dihydro-4-(1H)-pyridon

Zur Lösung von 104 g (0.95 Mol) 4-Methoxypyridin in 1 l wasserfreiem Tetrahydrofuran wird bei -70° unter Argon 165 ml (1.16 Mol) Chlorameisensäurebenzylester innert 20 Minuten zugetropft. Danach verdünnt man die dicke beige Suspension mit 200 ml wasserfreiem Tetrahydrofuran. Nun lässt man das Grignardreagens, hergestellt in 160 ml wasserfreiem Ether aus 35.5 g (1.46 Mol) Magnesiumspänen und 460 ml (1.46 Mol) einer 3 molaren Lösung von Benzylchlorid in wasserfreiem Ether, während 75 Minuten zum Reaktionsgemisch zutropfen und behält die Temperatur bei -70°. Nach weiteren 10 Minuten lässt man auf Raumtemperatur erwärmen. Man verdünnt mit 500 ml Ether, tropft 900 ml 4 N Salzsäure dazu und trennt die Phasen. Die organischen Phasen werden mit Wasser und Sole gewaschen, über Magnesiumsulfat getrocknet und zur Trockne eingedampft. Der Rückstand wird mit Hexar/Ethylacetat (3:1) an Kieselgel chromatographiert. Man erhält die Titelverbindung als farbloses zähflüssiges Öl. DC: Hexan/Ethylacetat (1:3) $R_f$=0.7, IR: 1725, 1665, 1602 $cm^{-1}$.

b) (2R*,4R*)-2-Benzyl-4-hydroxy-piperidin

Man hydriert 150 g (0.467 Mol) 2-Benzyl-N-benzyloxycarbonyl-2,3-dihydro-4-(1H)-pyridon in 1.5 l Methanol mit 7.5 g Pd/C (10 %-ig) als Katalysator, fügt danach 50 g Raney-Nickel und weitere 200 ml Methanol dazu und lässt aushydrieren. Nach Filtration wird am Rotationsverdampfer eingeengt und das bräunliche Öl mit Methylenchlorid/Methanol/conc. Ammoniak (60:10:1) an Kieselgel chromatographiert.

Man erhält die Titelverbindung als halbkristalline Masse, welche ohne zusätzliche Reinigung weiter verwendet wird. Die Kristallisation einer Probe aus Ether/Hexan ergab weisse Kristalle vom Smp. 111-112°. DC: Methylenchlorid/Methanol/conc. Ammoniak (40:10:1) $R_f$=0.55, FD-MS: $M^+$ = 191.

c) (2R*,4R*)-2-Benzyl-1-*t*-butyloxycarbonyl-4-hydroxy-piperidin

Man lässt die Lösung von 28 g (146 mMol) (2R*,4R*)-2-Benzyl-4-hydroxy-piperidin und 35.1 g (161 mMol) Di-*t*ert.-butyl-dicarbonat in 500 ml Chloroform bei 50° während 20 Stunden rühren. Danach wird am Rotationsverdampfer eingeengt und das gelbe Öl an Kieselgel mit Methylenchlorid/Methanol/conc. Ammoniak (2000:50:1) chromatographiert. Die Titelverbindung wird als gelbes Öl erhalten. DC: Methylenchlorid/Methanol/conc. Ammoniak (2000:50:1) $R_f = 0.43$, FD-MS: $M^+ = 291$.

d) (2R*,4R*)-2-Benzyl-1-t-Butyloxycarbonyl-4-methansulfonyloxy-piperidin

Zur Lösung von 62.4 g (214 mMol) (2R*,4R*)-2-Benzyl-1-*t*-butyloxycarbonyl-4-hydroxypiperidin in 75 ml Pyridin tropft man unter Eiskühlung 33.3 ml (428 mMol) Methansulfonsäurechlorid. Nach 30 Minuten bei 0° lässt man die Suspension 3 Stunden bei Raumtemperatur ausrühren. Nach Einengen am Rotationsverdampfer wird das Reaktionsgemisch in Ethylacetat aufgenommen, mit Wasser und Sole gewaschen, über Magnesiumsulfat getrocknet und am Rotationsverdampfer eingedampft. Aus Ether kristallisiert die Titelverbindung in weissen Kristallen; Smp. 110 - 115°; DC: Toluol/Ethylacetat (4:1) $R_f = 0.42$, FD-MS: $M^+ = 369$.

e) (2R*,4S*)-2-Benzyl-1-t-butyloxycarbonyl-piperidin-4-azid

Man lässt das Gemisch aus 98.9 g (267 mMol) (2R*,4R*)-2-Benzyl-1-t-Butyloxycarbonyl-4-methansulfonyloxy-piperidin, 14.4 g (294 mMol) Lithiumazid und 500 ml N,N-Dimethylformamid 3 Stunden bei 80° unter Argon rühren. Das Reaktionsgemisch wird mit Ethylacetat verdünnt und mit Wasser und Sole gewaschen, über Magnesiumsulfat getrocknet und zur Trockne eingedampft. Das bräunliche Öl wird mit Toluol/Ethylacetat (9:1) als Elutionsmittel an Kieselgel chromatographiert. Man erhält die Titelverbindung im Gemisch mit 2-Benzyl-N-*t*-butyloxycarbonyl-1,2,5,6-tetrahydropyridin (Gewichtsverhältnis = 4,2:1 gemäss $^1$H-NMR), welches nicht weiter aufgetrennt wird. DC: Toluol/Ethylacetat (9:1) $R_f = 0.59$, FD-MS: $M^+ = 316$, IR: 2100, 1685 $cm^{-1}$.

f) (2R*,4S*)-2-Benzyl-1-*t*-butyloxycarbonyl-4-piperidinamin

Das Gemisch aus 4.16 g (13.1 mMol) (2R*,4S*)-2-Benzyl-1-*t*-butyloxycarbonyl-4-piperidinazid und 0.99 g (3.62 mMol) 2-Benzyl-N-*t*-butyloxycarbonyl-1,2,5,6-tetrahydropyridin (berechnet aufgrund des $^1$H-NMR-Spektrums) wird in 100 ml Methanol mit Wasserstoff und 1 g 10 %-iger Pd/C hydriert Nach beendeter Wasserstoffaufnahme wird abfiltriert und am Rotationsverdampfer eingeengt. Das braune Öl wird mit Methylenchlorid/Methanol/conc.Ammoniak (350:50:1) an Kieselgel chromatographiert. Man erhalt die Titelverbindung als gelbes Öl. DC: Methylenchlorid/Methanol/conc. Ammoniak (350:50:1) $R_f = 0.4$, FD-MS: $M^+ = 290$.

g) (2R*,4S*)-2-Benzyl-1-*t*-butyloxvcarbonyl-N-(4-chinolylmethyl)-4-piperidinamin

Das Gemisch von 5 g (17.2 mMol) (2R*,4S*)-2-Benzyl-1-*t*-butyloxycarbonyl-4-piperidinamin und 2.7 g (17.2 mMol) Chinolin-4-carboxaldehyd wird in 50 ml Toluol bei Raumtemperatur gerührt und nach 2 Stunden mit 2.8 g (23.3 mMol) wasserfreiem Magnesiumsulfat versetzt. Nach weiteren 16 Stunden wird filtriert und das Filtrat eingeengt. Das braune Öl löst man in 50 ml Methanol und versetzt mit 0.69 g (18.3 mMol) Natriumborhydrid in 4 Portionen. Nach 3 Stunden Rühren bei Raumtemperatur wird das Reaktionsgemisch eingeengt, in Ethylacetat aufgenommen und mit Wasser und Sole gewaschen. Die organischen Phasen werden über Magnesiumsulfat getrocknet und zur Trockne eingedampft. Das braune Öl wird mit Methylenchlorid/Methanol/conc. Ammoniak (850:50:1) an Kieselgel chromatographiert. Man erhält die Titelverbindung als gelbes Öl. DC: Methylenchlorid/Methanol/conc. Ammoniak (700:50:1) $R_f = 0.38$, FD-MS: $M^+ = 431$.

h) (2R*,4S*)-2-Benzyl-1-*t*-butyloxycarbonyl-N-(4-chinolylmethyl)-N-trifluoracetyl-4-piperidinamin

Die Lösung von 6.2 g (14.4 mMol) (2R*,4S*)-2-Benzyl-1-*t*-butyloxycarbonyl-N-(4-chinolylmethyl)-4-piperidinamin und 2.6 ml (18.7 mMol) Triethylamin in 60 ml Methylenchlorid wird bei 0° unter Argon mit 2.2 ml (15.8 mMol) Trifluoracetanhydrid versetzt und das Reaktionsgemisch 3 Stunden bei 0° gerührt. Man verdünnt mit Methylenchlorid und wäscht mit Wasser. Die organischen Phasen werden über Magnesiumsulfat getrocknet und zur Trockne eingedampft. Man erhält die Titelverbindung als DC-reines Produkt als

gelbes Öl. DC:
Methylenchlorid/Methanol/conc. Ammoniak (700:50:1) $R_f = 0.62$, DCI-MS: $(M+H)^+ = 528$.

i) (2R*,4S*)-2-Benzyl-N-(4-chinolylmethyl)-N-trifluoracetyl-4-piperidinamin

Unter Eiskühlung wird zu 7.73 g (14.7 mMol) (2R*,4S*)-2-Benzyl-1-*t*-butyloxycarbonyl-N-(4-chinolylmethyl)-N-trifluoracetyl-4-piperidinamin 250 ml 6 N Chlorwasserstoff in Dioxan während 3 Minuten getropft und das Gemisch anschliessend 1 Stunde bei Raumtemperatur gerührt. Das Reaktionsgemisch wird am Rotationsverdampfer eingeengt, mit 1 N Natriumhydrogencarbonatlösung basisch gestellt und mit Methylenchlorid extrahiert. Die organischen Phasen werden über Magnesiumsulfat getrocknet und zur Trockne eingedampft. Das bräunliche Öl (7.14 g) wird mit Methylenchlorid/Methanol/conc. Ammoniak (700:50:1) an Kieselgel chromatographiert. Man erhält die Titelverbindung als gelbes Öl. DC: Methylenchlorid/Methanol/conc.-Ammoniak (700:50:1) $R_f = 0.42$, DCI-MS: $(M+H)^+ = 428$, IR: 1690 $cm^{-1}$.

j) (2R*,4S*)-2-Benzyl-1-(2-naphthoyl)-N-(4-chinolylmethyl)-N-trifluoracetyl-4-piperidinamin

Zur Lösung von 97 mg (0.56 mMol) 2-Naphthoesäure in 1 ml Toluol wird während 10 Minuten unter Argon bei 50° die Lösung von 58 µl (0.795 mMol) Thionylchlorid in 0.2 ml Toluol zugetropft und das Reaktionsgemisch 2 Stunden bei 80° gerührt. Danach wird am Rotationsverdampfer eingeengt, zweimal mit je 1 ml Toluol versetzt und wieder eingeengt. Man löst das braune Öl in 1 ml Methylenchlorid, gibt es unter Argon bei 0° zu einer Lösung von 200 mg (0.468 mMol) (2R*,4S*)-2-Benzyl-N-(4-chinolylmethyl)-N-trifluoracetyl-4-piperidinamin und rührt 2 Stunden bei 0°. Anschliessend wird das Reaktionsgemisch mit Wasser versetzt und mit Methylenchlorid extrahiert. Die organischen Phasen werden mit Sole gewaschen, über Magnesiumsulfat getrocknet und zur Trockne eingedampft. Das gelbe Öl wird mit Methylenchlorid/Methanol/conc. Ammoniak (1000:50:1) an Kieselgel chromatographiert. Man erhält die Titelverbindung als gelbes Öl. DC: Methylenchlorid/Methanol/conc. Ammoniak (2000:50:1) $R_f = 0.36$, FD-MS: $M^+ = 581$.

Beispiel 3: (2R*,4S*)-2-Benzyl-1-(3-trifluormethylbenzoyl)-N-(4-chinolylmethyl)-4-piperidinamin

Analog Beispiel 2 werden 0.184g (0.307 mMol) (2R*,4S*)-2-Benzyl-1-(3-trifluormethylbenzoyl)-N-(4-chinolylmethyl)-N-trifluoracetyl-4-piperidinamin mit 0.046 g (1.23 mMol) Natriumborhydrid umgesetzt. Man erhält die Titelverbindung der Formel

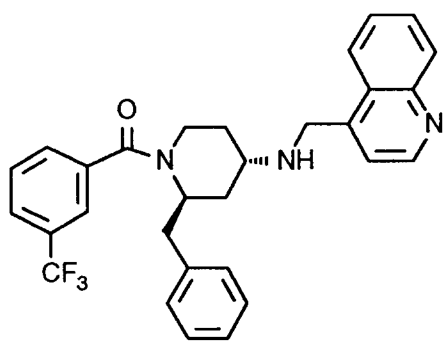

als gelbes Öl. DC: Methylenchlorid/Methanol/conc. Ammoniak (700:50:1) $R_f = 0.28$, FD-MS: $M^+ = 503$.
Die Ausgangsverbindung dazu wird wie folgt hergestellt:

(2R*,4S*)-2-Benzyl-1-(3-trifluormethylbenzoyl)-N-(4-chinolylmethyl)-N-trifluoracetyl-4-piperidinamin

Analog Beispiel 2j werden 106 mg (0.56 mMol) 3-Trifluormethylbenzoesäure zuerst mit 58 µl (0.795 mMol) Thionylchlorid und anschliessend mit 200 mg (0.468 mMol) (2R*,4S*)-2-Benzyl-N-(4-chinolylmethyl)-N-trifluoracetyl-4-piperidinamin zum Produkt umgesetzt. DC: Methylenchlorid/Methanol/conc. Ammoniak (700:50:1) $R_f = 0.56$, FD-MS: $M^+ = 599$.

Beispiel 4: (2R*,4S*)-2-Benzyl-1-(3,5-bis-(trifluormethyl)-benzoyl)-N-(4-chinolylmethyl)-4-piperidinamin

Analog Beispiel 2 werden 0.271 g (0.406 mMol) (2R*,4S*)-2-Benzyl-1-(3,5-bis-(trifluormethyl)-benzoyl)-N-(4-chinolylmethyl)-N-trifluoracetyl-4-piperidinamin mit 0.061 g (1.23 mMol) Natriumborhydrid umgesetzt. Man erhält die Titelverbindung

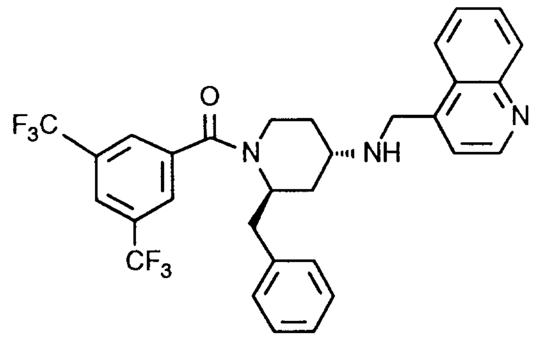

als weissen Schaum. DC: Methylenchlorid/Methanol/conc. Ammoniak (1000:50:1) $R_f = 0.21$, FD-MS: $M^+ = 571$.

Die Ausgangsverbindung dazu wird wie folgt hergestellt:

(2R*,4S*)-2-Benzyl-1-(3,5-bis-(trifluormethyl)-benzoyl)-N-(4-chinolylmethyl)-N-trifluoracetyl-4-piperidinamin

Zur Lösung von 200 mg (467 mMol) (2R*,4S*)-2-Benzyl-N-(4-chinolylmethyl)-N-trifluoracetyl-4-piperidinamin und 113 mg (561 mMol) 3,5-Bis-(trifluormethyl)-benzoesäure in 3 ml Methylenchlorid gibt man 143 mg (561 mMol) Bis-(2-oxo-3-oxazolidinyl)-phosphinsäurechlorid und 144 µl (1.03 mMol) Triethylamin und lässt das Reaktionsgemisch 16 Stunden bei Raumtemperatur rühren. Danach wird mit Methylenchlorid verdünnt, und die organische Phase je einmal mit 10 %-iger Citronensäure, 1N-Natriumhydrogencarbonatlösung und mit Sole gewaschen, über Magnesiumsulfat getrocknet und zur Trockne eingedampft. Der gelbliche Schaum wird mit Methylenchlorid/Methanol/conc. Ammoniak (1000:50:1) an Kieselgel chromatographiert. Man erhält die Titelverbindung als gelbes Öl. DC: Methylenchlorid/Methanol/conc. Ammoniak (1000:50:1) $R_f = 0.34$, FD-MS: $M^+ = 667$.

Beispiel 5: (2R*,4S*)-2-Benzyl-1-(3,5-dimethoxybenzoyl)-N-(4-chinolylmethyl)-4-piperidinamin

Analog Beispiel 2 werden 250 mg (0.423 mMol) (2R*,4S*)-2-Benzyl-1-(3,5-dimethoxybenzoyl)-N-(4-chinolylmethyl)-N-trifluoracetyl-4-piperidinamin mit 64 mg (1.69 mMol) Natriumborhydrid umgesetzt. Man erhält die Titelverbindung

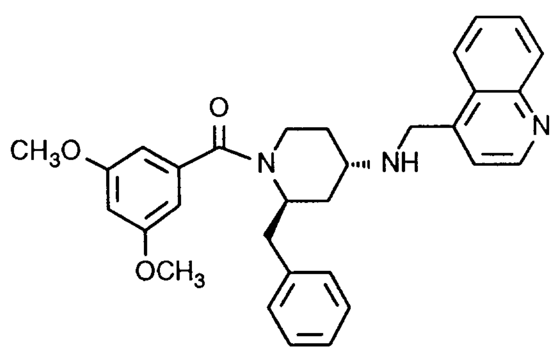

als weissen Schaum. DC: Methylenchlorid/Methanol/conc. Ammoniak (1000:50:1) $R_f = 0.23$, DCI-MS: $(M+H)^+ = 496$.

Die Ausgangsverbindung dazu wird wie folgt hergestellt:

(2R*,4S*)-2-Benzyl-1-(3,5-dimethoxybenzoyl)-N-(4-chinolylmethyl)-N-trifluoracetyl-4-piperidinamin

Analog Beispiel 4 werden 200 mg (0.467 mMol) (2R*,4S*)-2-Benzyl-N-(4-chinolylmethyl)-N-trifluoracetyl-4-piperidinamin mit 102 mg (0.561 mMol) 3,5-Dimethoxybenzoesäure, 143 mg (0.561 mMol) Bis-(2-oxo-3-oxazolidinyl)-phosphinsäurechlorid und 144 µl (1.03 mMol) Triethylamin umgesetzt. Man erhält die Titelverbindung als weissen Schaum. DC: Methylenchlorid/Methanol/conc. Ammoniak (1000:50:1) $R_f = 0.31$, FD-MS: $M^+ = 591$.

Beispiel 6: (2R*,4S*)-2-Benzyl-1-(1-naphthoyl)-N-(4-chinolylmethyl)-4-piperidinamin

Analog Beispiel 2 werden 200 mg (0.344 mMol) (2R*,4S*)-2-Benzyl-1-(1-naphthoyl)-N-(4-chinolylmethyl)-N-trifluoracetyl-4-piperidinamin mit 52 mg (1.37 mMol) Natriumborhydrid umgesetzt. Man erhält die Titelverbindung

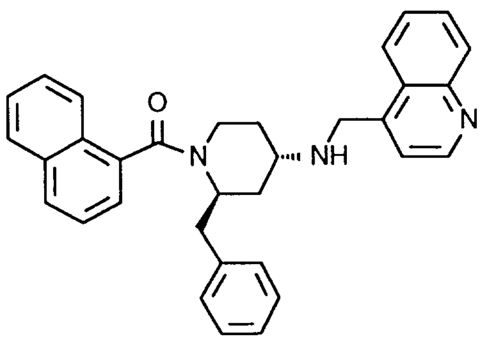

als weissen Schaum. DC: Methylenchlorid/Methanol/conc. Ammoniak (1000:50:1) $R_f = 0.35$, FD-MS: $M^+ = 485$.

Die Ausgangsverbindung dazu wird wie folgt hergestellt:

(2R*,4S*)-2-Benzyl-1-(1-naphthoyl)-N-(4-chinolylmethyl)-N-trifluoracetyl-4-piperidinamin

Zur Lösung von 96 mg (0.561 mMol) 1-Naphthoesäure in 2 ml trockenem Methylenchlorid gibt man bei 0° 92 µl (0.655 mMol) 1-Chlor-N,N,2-trimethyl-1-propen-1-amin und rührt das Gemisch 1 Stunde bei 0° und eine Stunde bei Raumtemperatur. Zu dieser gelben Lösung wird anschliessend die Lösung von 200 mg (0.468 mMol) (2R*,4S*)-2-Benzyl-N-(4-chinolylmethyl)-N-trifluoracetyl-4-piperidinamin und 130 µl (0.936 mMol) Triethylamin in 3 ml Methylenchlorid innert 10 Minuten bei Raumtemperatur getropft. Nach 3 Stunden Rühren bei Raumtemperatur wird mit Wasser versetzt, die organische Phase abgetrennt und noch zweimal mit Wasser gewaschen. Die organischen Phasen werden über Magnesiumsulfat getrocknet und zur Trockne eingedampft. Das gelbe Öl wird mit Methylenchlorid/Methanol/conc. Ammoniak (800:50:1) an Kieselgel chromatographiert. Man erhält die Titelverbindung als gelbes Öl. DC: Methylenchlorid/Methanol/conc. Ammoniak (700:50:1) $R_f = 0.49$, FD-MS: $M^+ = 581$.

Beispiel 7: (2R*,4S*)-2-Benzyl-1-(3,5-dichlorbenzoyl)-N-(4-chinolylmethyl)-4-piperidinamin

Analog Beispiel 2 werden 1.21 g (2.01 mMol) (2R*,4S*)-2-Benzyl-1-(3,5-dichlorbenzoyl)-N-(4-chinolylmethyl)-N-trifluoracetyl-4-piperidinamin mit 0.305 mg (8.06 mMol) Natriumborhydrid umgesetzt. Man erhält die Titelverbindung

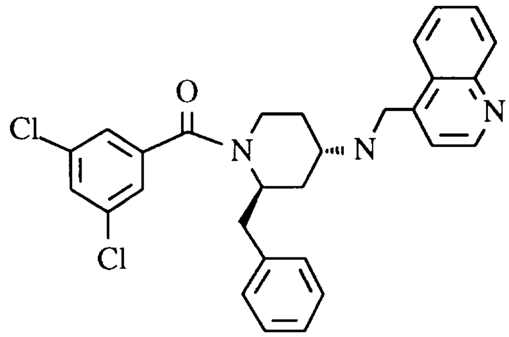

als weissen Schaum; DC: Methylenchlorid/Methanol/conc. Ammoniak (700:50:1) $R_f = 0.37$; FD-MS: $M^+ = 503$.

Die Ausgangsverbindung dazu wird wie folgt hergestellt:

(2R*,4S*)-2-Benzyl-1-(3,5-dichlorbenzoyl)-N-(4-chinolylmethyl)-N-trifluoracetyl-4-piperidinamin

Analog Beispiel 2j werden 1.11 g (5.85 mMol) 3,5-Dichlorbenzoesäure zuerst mit 0.63 ml (8.77 mMol) Thionylchlorid und anschliessend mit 1 g (2.34 mMol) (2R*,4S*)-2-Benzyl-N-(4-chinolylmethyl)-N-trifluoracetyl-4-piperidinamin zum Produkt umgesetzt. DC: Methylenchlorid/Methanol/conc. Ammoniak (700:50:1) $R_f = 0.65$, FD-MS: $M^+ = 599$.

Beispiel 8: (2R*,4S*)-2-Benzyl-1-(2-chinolinylcarbonyl)-N-(4-chinolylmethyl)-4-piperidinamin

Analog Beispiel 2 werden 155 mg (0.266 mMol) (2R*,4S*)-2-Benzyl-1-(2-chinolinylcarbonyl)-N-(4-chinolylmethyl)-N-trifluoracetyl-4-piperidinamin mit 40 mg (1.06 mMol) Natriumborhydrid umgesetzt. Man erhält die Titelverbindung

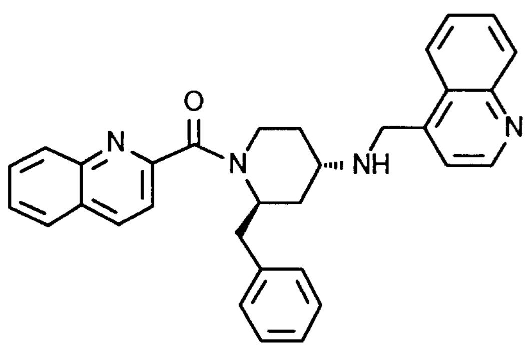

als weissen Schaum. DC: Methylenchlorid/Methanol/conc. Ammoniak (700:50:1) $R_f = 0.42$, FD-MS: $M^+ = 486$.

Die Ausgangsverbindung dazu wird wie folgt hergestellt:

(2R*,4S*)-2-Benzyl-1-(2-chinolinylcarbonyl)-N-(4-chinolylmethyl)-N-trifluoracetyl-4-piperidinamin

Analog Beispiel 2j werden 97 mg (0.56 mMol) Chinolin-2-carbonsäure zuerst mit 58 µl (0.795 mMol) Thionylchlorid und anschliessend mit 200 mg (0.468 mMol) (2R*,4S*)-2-Benzyl-N-(4-chinolylmethyl)-N-trifluoracetyl-4-piperidinamin zum Produkt umgesetzt. DC: Methylenchlorid/Methanol/conc. Ammoniak (700:50:1) $R_f = 0.45$, FD-MS: $M^+ = 582$.

Beispiel 9: (2R*,4S*)-2-Benzyl-1-(4-chlor-phenylacetyl)-N-(4-chinolylmethyl)-4-piperidinamin

Analog Beispiel 2 werden 256 mg (0.441 mMol) (2R*,4S*)-2-Benzyl-1-(4-chlor-phenylacetyl)-N-(4-chinolylmethyl)-N-trifluoracetyl-4-piperidinamin mit 66 mg (1.76 mMol) Natriumborhydrid umgesetzt. Man erhält die Titelverbindung

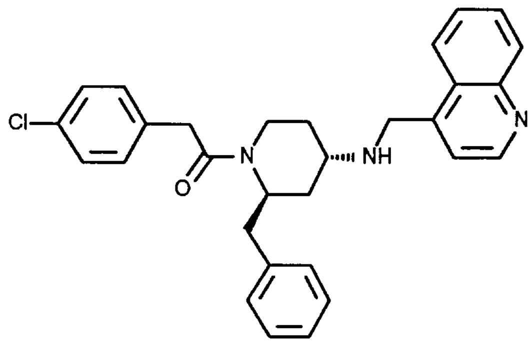

als weissen Schaum. DC: Methylenchlorid/Methanol/conc. Ammoniak (700:50:1) $R_f = 0.48$, FD-MS: $M^+ = 484$.

Die Ausgangsverbindung dazu wird wie folgt hergestellt:

(2R*,4S*)-2-Benzyl-1-(4-chlor-phenylacetyl)-N-(4-chinolylmethyl)-N-trifluoracetyl-4-piperidinamin

Analog Beispiel 2j werden 96 mg (0.56 mMol) 4-Chlor-phenylessigsäure zuerst mit 58 µl (0.795 mMol) Thionylchlorid und anschliessend mit 200 mg (0.468 mMol) (2R*,4S*)-2-Benzyl-N-(4-chinolylmethyl)-N-trifluoracetyl-4-piperidinamin zum Produkt umgesetzt. DC: Methylenchlorid/Methanol/conc. Ammoniak (700:50:1) $R_f = 0.39$, FD-MS: $M^+ = 580$.

Beispiel 10: (2R*,4S*)-2-Benzyl-1-(benzyloxycarbonyl)-N-(4-chinolylmethyl)-4-piperidinamin

Analog Beispiel 2 werden 80 mg (0.142 mMol) (2R*,4S*)-2-Benzyl-1-(benzyloxycarbonyl)-N-(4-chinolylmethyl)-N-trifluoracetyl-4-piperidinamin mit 22 mg (0.57 mMol) Natriumborhydrid umgesetzt. Man erhält die Titelverbindung

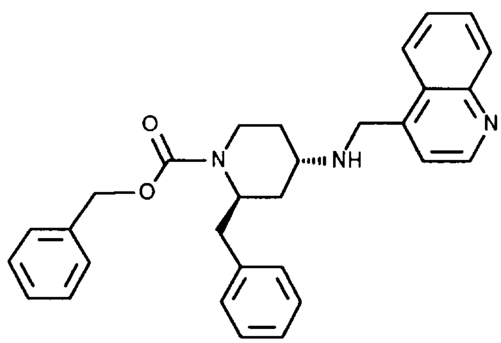

als farbloses Öl. DC: Methylenchlorid/Methanol/conc. Ammoniak (700:50:1) $R_f = 0.43$, FD-MS: $M^+ = 465$.

Die Ausgangsverbindung dazu wird wie folgt hergestellt:

(2R*,4S*)-2-Benzyl-1-(benzyloxycarbonyl)-N-(4-chinolylmethyl)-N-trifluoracetyl-4-piperidinamin

Zur Lösung von 200 mg (0.468 mMol) (2R*,4S*)-2-Benzyl-N-(4-chinolylmethyl)-N-trifluoracetyl-4-piperidinamin in 4 ml Methylenchlorid gibt man bei 0°67 µl (0.468 mMol) Chlorameisensäurebenzylester und 72 µl (0.515 mMol) Triethylamin und lässt das Gemisch bei dieser Temperatur 16 Stunden rühren. Danach werden nochmals 34 µl (0.234 mMol) Chlorameisensäurebenzylester und 36 µl (0.257 mMol) Triethylamin zugefügt und 3 Stunden bei Raumtemperatur gerührt. Danach wird mit Methylenchlorid verdünnt und die organische Phase mit Sole gewaschen, über Magnesiumsulfat getrocknet und zur Trockne eingedampft. Das Öl wird mit Methylenchlorid/Methanol/conc. Ammoniak (1000:50:1) an Kieselgel chromatographiert. Man erhält die Titelverbindung als Öl. DC: Methylenchlorid/Methanol/conc. Ammoniak (700:50:1) $R_f = 0.61$, FD-MS: $M^+ = 561$.

Beispiel 11: (2R*,4S*)-2-Benzyl-1-(2-phenylethyl)-N-(4-chinolylmethyl)-4-piperidinamin

Analog Beispiel 2 werden 215 mg (0.494 mMol) (2R*,4S*)-2-Benzyl-1-(2-phenylethyl)-N-(4-chinolylmethyl)-N-trifluoracetyl-4-piperidinamin mit 77 mg (1.97 mMol) Natriumborhydrid umgesetzt. Man erhält die Titelverbindung

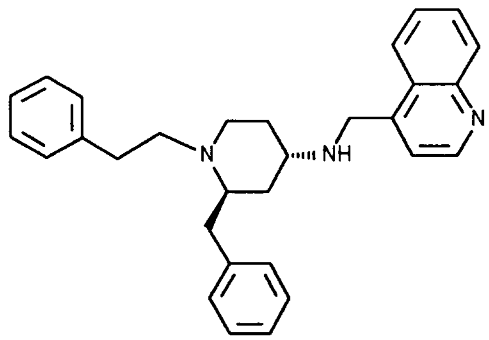

als Öl. DC: Methylenchlorid/Methanol/conc. Ammoniak (700:50:1) $R_f = 0.34$, FD-MS: $M^+ = 435$.

Die Ausgangsverbindung dazu wird wie folgt hergestellt:

(2R*,4S*)-2-Benzyl-1-(2-phenylethyl)-N-(4-chinolylmethyl)-N-trifluoracetyl-4-piperidinamin

209 µl (0.936 mMol) Phenylacetaldehyd tropft man innert 10 Minuten bei Raumtemperatur zu einer Lösung von 100 mg (0.233 mMol) (2R*,4S*)-2-Benzyl-N-(4-chinolylmethyl)-N-trifluoracetyl-4-piperidinamin, 58 mg (0.702 mMol) Natriumacetat, 44 mg (0.702 mMol) Natriumcyanoborhydrid und 67 µl (1.17 mMol) Essigsäure in 2 ml Ethanol. Man lässt das Reaktionsgemisch 16 Stunden bei Raumtemperatur rühren. Nach Eindampfen am Rotationsverdampfer wird der Rückstand in Ethylacetat aufgenommen, die organische Phase mit Wasser und mit Sole gewaschen, über Magnesiumsulfat getrocknet und zur Trockne eingedampft. Das gelbe Öl wird mit Methylenchlorid/Methanol/conc. Ammoniak (2000:50:1) an Kieselgel chromatographiert. Man erhält die Titelverbindung als Öl. DC: Methylenchlorid/Methanol/conc. Ammoniak (2000:50:1) $R_f = 0.33$, FD-MS: $M^+ = 531$.

Beispiel 12: (2R*,4S*)-2-Benzyl-1-(2-naphthylacetyl)-N-(4-chinolylmethyl)-4-piperidinamin

Analog Beispiel 2 werden 160 mg (0.269 mMol) (2R*,4S*)-2-Benzyl-1-(2-naphthylacetyl)-N-(4-chinolylmethyl)-N-trifluoracetyl-4-piperidinamin mit 42 mg (1.13 mMol) Natriumborhydrid umgesetzt. Man erhält die Titelverbindung

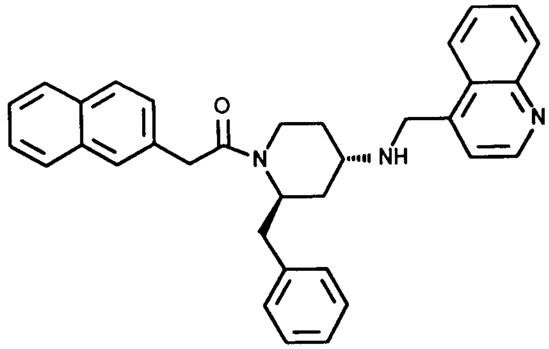

als farbloses Öl. DC: Methylenchlorid/Methanol/conc. Ammoniak (700:50:1) $R_f = 0.27$, FD-MS: $M^+ = 499$.

Die Ausgangsverbindung dazu wird wie folgt hergestellt:

(2R*,4S*)-2-Benzyl-1-(2-naphthylacetyl)-N-(4-chinolylmethyl)-N-trifluoracetyl-4-piperidinamin

Analog Beispiel 6 werden 105 mg (0.561 mMol) 2-Naphthylessigsäure zuerst mit 92 µl (0.655 mMol) 1-Chlor-N,N,2-trimethyl-1-propen-1-amin und anschliessend mit 200 mg (0.468 mMol) (2R*,4S*)-2-Benzyl-N-(4-chinolylmethyl)-N-trifluoracetyl-4-piperidinamin und 130 µl (0.936 mMol) Triethylamin zum Produkt umgesetzt. DC: Methylenchlorid/Methanol/conc. Ammoniak (700:50:1) $R_f = 0.56$, FD-MS: $M^+ = 595$.

Beispiel 13: (2R*,4S*)-2-Benzyl-1-(4-chinolylmethyl)-N-(4-chinolylmethyl)-4-piperidinamin

Analog Beispiel 2 werden 128 mg (0.225 mMol) (2R*,4S*)-2-Benzyl-1-(4-chinolylmethyl)-N-(4-chinolylmethyl)-N-trifluoracetyl-4-piperidinamin mit 34 mg (0.872 mMol) Natriumborhydrid umgesetzt. Man erhält die Titelverbindung

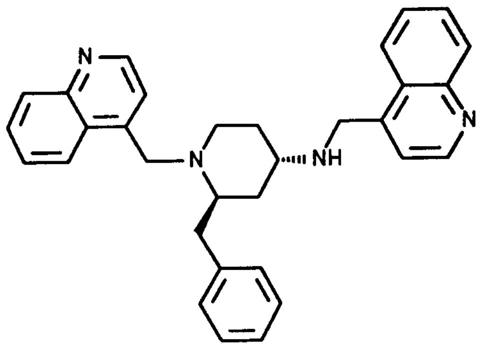

als gelbes Öl. DC: Methylenchlorid/Methanol/conc. Ammoniak (700:50:1) $R_f = 0.45$, FD-MS: $M^+ = 490$.

Die Ausgangsverbindung dazu wird wie folgt hergestellt:

(2R*,4S*)-2-Benzyl-1-(4-chinolylmethyl)-N-(4-chinolylmethyl)-N-trifluoracetyl-4-piperidinamin

Analog Beispiel 11 werden 200 mg (0.468 mMol) (2R*,4S*)-2-Benzyl-N-(4-chinolylmethyl)-N-trifluoracetyl-4-piperidinamin mit 88 mg (1.4 mMol) Natriumcyanoborhydrid, 115 mg (1.4 mMol) Natriumacetat, 134 µl (2.34 mMol) Essigsäure und 294 mg (1.87 mMol) Chinolin-4-carboxaldehyd zum Produkt umgesetzt. DC: Methylenchlorid/Methanol/conc. Ammoniak (700:50:1) $R_f = 0.33$, FD-MS: $M^+ = 568$.

Beispiel 14: (2R*,4S*)-2-Benzyl-1-(2,4-dichlorbenzyl)-N-(4-chinolylmethyl)-4-piperidinamin

Analog Beispiel 2 werden 128 mg (0.218 mMol) (2R*,4S*)-2-Benzyl-1-(2,4-dichlorbenzyl)-N-(4-chinolylmethyl)-N-trifluoracetyl-4-piperidinamin mit 34 mg (0.920 mMol) Natriumborhydrid umgesetzt. Man erhält die Titelverbindung

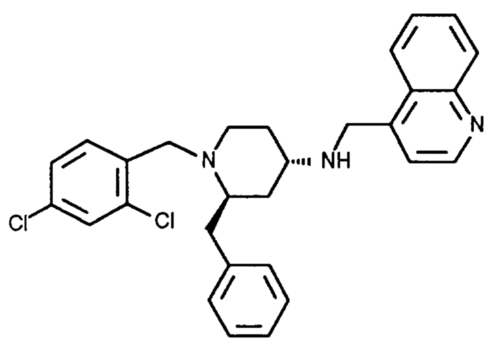

als gelbes Öl. DC: Methylenchlorid/Methanol/conc. Ammoniak (700:50:1) $R_f = 0.25$, FD-MS: $M^+ = 472$.

Die Ausgangsverbindung dazu wird wie folgt hergestellt:

a) (2R*,4S*)-2-Benzyl-1-(4-chinolylmethyl)-N-(4-chinolylmethyl)-N-trifluoracetyl-4-piperidinamin

Analog Beispiel 11 werden 200 mg (0.468 mMol) (2R*,4S*)-2-Benzyl-N-(4-chinolylmethyl)-N-trifluoracetyl-4-piperidinamin mit 88 mg (1.4 mMol) Natriumcyanoborhydrid, 115 mg (1.4 mMol) Natriumacetat, 134 µl (2.34 mMol) Essigsäure und 294 mg (1.87 mMol) Chinolin-4-carboxaldehyd zum Produkt umgesetzt. DC: Methylenchlorid/Methanol/conc. Ammoniak (700:50:1) $R_f = 0.33$, FD-MS: $M^+ = 568$.

Beispiel 15: (2R*,4S*)-2-Benzyl-1-(2,2-diphenylethyl)-N-(4-chinolylmethyl)-4-piperidinamin

Analog Beispiel 2 werden 170 mg (0.280 mMol) (2R*,4S*)-2-Benzyl-1-(2,2-diphenylethyl)-N-(4-chinolylmethyl)-N-trifluoracetyl-4-piperidinamin mit 42 mg (1.12 mMol) Natriumborhydrid umgesetzt. Man erhält die Titelverbindung

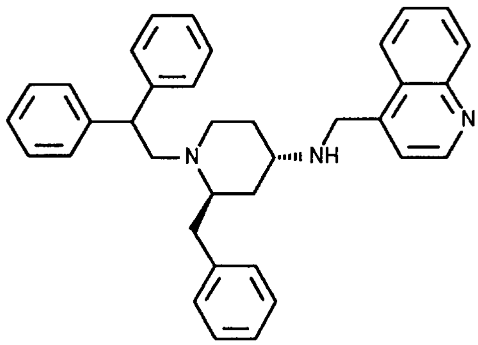

als weissen Schaum. DC: Methylenchlorid/Methanol/conc. Ammoniak (1000:50:1) $R_f = 0.28$, FD-MS: $M^+ = 511$.

Die Ausgangsverbindung dazu wird wie folgt hergestellt:

(2R*,4S*)-2-Benzyl-1-(2,2-diphenylethyl)-N-(4-chinolylmethyl)-N-trifluoracetyl-4-piperidinamin

Analog Beispiel 11 werden 200 mg (0.468 mMol) (2R*,4S*)-2-Benzyl-N-(4-chinolylmethyl)-N-trifluoracetyl-4-piperidinamin mit 88 mg (1.4 mMol) Natriumcyanoborhydrid, 115 mg (1.4 mMol) Natriumacetat, 134 µl (2.34 mMol) Essigsäure und 335 µl (1.87 mMol) Diphenylacetaldehyd zum Produkt umgesetzt. DC: Methylenchlorid/Methanol/conc. Ammoniak (2000:50:1) $R_f = 0.50$, FD-MS: $M^+ = 607$.

Beispiel 16: (2R*,4S*)-2-Benzyl-1-(phenylcarbamoyl)-N-(4-chinolylmethyl)-4-piperidinamin

Analog Beispiel 2 werden 210 mg (0.384 mMol) (2R*,4S*)-2-Benzyl-1-(phenylcarbamoyl)-N-(4-chinolylmethyl)-N-trifluoracetyl-4-piperidinamin mit 58 mg (1.54 mMol) Natriumborhydrid umgesetzt. Man erhält die Titelverbindung

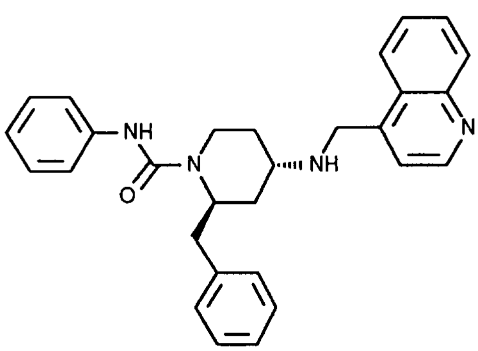

als weissen Schaum. DC: Methylenchlorid/Methanol/conc. Ammoniak (1000:50:1) $R_f = 0.33$, FD-MS: $M^+ = 450$.

Die Ausgangsverbindung dazu wird wie folgt hergestellt:

(2R*,4S*)-2-Benzyl-1-(phenylcarbamoyl)-N-(4-chinolylmethyl)-N-trifluoracetyl-4-piperidinamin

Zur Lösung von 72 mg (0.608 mMol) Phenylisocyanat in 5 ml Toluol gibt man die Lösung von 200 mg (0.468 mMol) (2R*,4S*)-2-Benzyl-N-(4-chinolylmethyl)-N-trifluoracetyl-4-piperidinamin und rührt das Reaktionsgemisch 2 Stunden bei 100°. Die weisse Suspension wird abgekühlt und filtriert. Man erhält die Titelverbindung als weisse Kristalle vom Schmelzpunkt 245° (Zersetzung). DC: Methylenchlorid/Methanol/conc. Ammoniak (700:50:1) $R_f = 0.76$, FD-MS: $M^+ = 546$.

Beispiel 17: (2R*,4S*)-2-Benzyl-1-(diphenylacetyl)-N-(4-chinolylmethyl)-4-piperidinamin

Analog Beispiel 2 werden 235 mg (0.378 mMol) (2R*,4S*)-2-Benzyl-1-(diphenylacetyl)-N-(4-chinolylmethyl)-N-trifluoracetyl-4-piperidinamin mit 58 mg (1.51 mMol) Natriumborhydrid umgesetzt. Man erhält die Titelverbindung

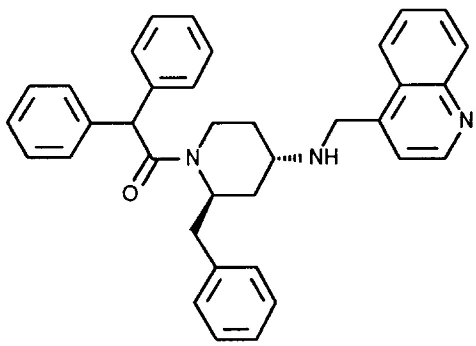

als weissen Schaum. DC: Methylenchlorid/Methanol/conc. Ammoniak (700:50:1) $R_f = 0.49$, FD-MS: $M^+ = 525$.

Die Ausgangsverbindung dazu wird wie folgt hergestellt:

(2R*,4S*)-2-Benzyl-1-(diphenylacetyl)-N-(4-chinolylmethyl)-N-trifluoracetyl-4-piperidinamin

Analog Beispiel 2j werden 248 mg (1.17 mMol) Diphenylessigsäure zuerst mit 128 µl (1.76 mMol) Thionylchlorid und anschliessend mit 200 mg (0.468 mMol) (2R*,4S*)-2-Benzyl-N-(4-chinolylmethyl)-N-trifluoracetyl-4-piperidinamin zum Produkt umgesetzt. DC: Methylenchlorid/Methanol/conc. Ammoniak (700:50:1) $R_f = 0.45$, FD-MS: $M^+ = 621$.

Beispiel 18: (2R*,4S*)-2-Benzyl-1-(2-pyridylacetyl)-N-(4-chinolylmethyl)-4-piperidinamin

Analog Beispiel 2 werden 180 mg (0.329 mMol) (2R*,4S*)-2-Benzyl-1-(2-pyridylacetyl)-N-(4-chinolylmethyl)-N-trifluoracetyl-4-piperidinamin mit 50 mg (1.32 mMol) Natriumborhydrid umgesetzt. Man erhält die Titelverbindung

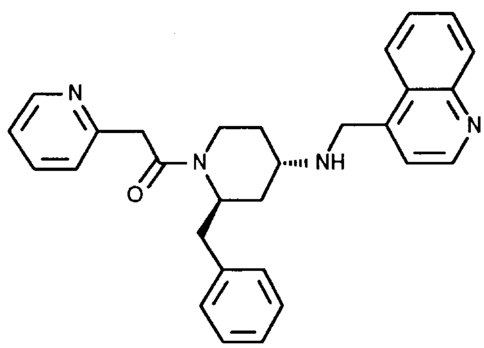

als weissen Schaum. DC: Methylenchlorid/Methanol/conc. Ammoniak (700:50:1) $R_f = 0.28$, FD-MS: $M^+ = 450$.

Die Ausgangsverbindung dazu wird wie folgt hergestellt:

(2R*,4S*)-2-Benzyl-1-(2-pyridylacetyl)-N-(4-chinolylmethyl)-N-trifluoracetyl-4-piperidinamin

Analog Beispiel 4 werden 200 mg (0.467 mMol) (2R*,4S*)-2-Benzyl-N-(4-chinolylmethyl)-N-trifluoracetyl-4-piperidinamin mit 98 mg (0.561 mMol) 2-Pyridylessigsäure-hydrochlorid, 143 mg (0.562 mMol) Bis-(2-oxo-3-oxazolidinyl)-phosphinsäurechlorid und 209 µl (1.50 mMol) Triethylamin umgesetzt. Man erhält die Titelverbindung als weissen Schaum. DC: Methylenchlorid/Methanol/conc. Ammoniak (700:50:1) $R_f = 0.60$, FD-MS: $M^+ = 546$.

Beispiel 19: (2R*,4S*)-2-Benzyl-1-(4-pyridylacetyl)-N-(4-chinolylmethyl)-4-piperidinamin

Analog Beispiel 2 werden 200 mg (0.366 mMol) (2R*,4S*)-2-Benzyl-1-(4-pyridylacetyl)-N-(4-chinolylmethyl)-N-trifluoracetyl-4-piperidinamin mit 55 mg (1.46 mMol) Natriumborhydrid umgesetzt. Man erhält die Titelverbindung

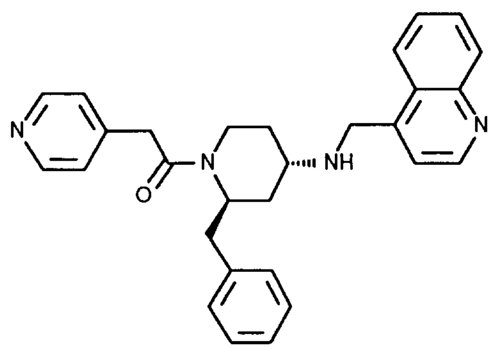

als weissen Schaum. DC: Methylenchlorid/Methanol/conc. Ammoniak (700:50:1) $R_f = 0.31$, FD-MS: $M^+ = 450$.

Die Ausgangsverbindung dazu wird wie folgt hergestellt:

(2R*,4S*)-2-Benzyl-1-(4-pyridylacetyl)-N-(4-chinolylmethyl)-N-trifluoracetyl-4-piperidinamin

Analog Beispiel 4 werden 200 mg (0.468 mMol) (2R*,4S*)-2-Benzyl-N-(4-chinolylmethyl)-N-trifluoracetyl-4-piperidinamin mit 98 mg (0.561 mMol) 4-Pyridylessigsäure-hydrochlorid, 143 mg (0.562 mMol) Bis-(2-oxo-3-oxazolidinyl)-phosphinsäurechlorid und 209 µl (1.50 mMol) Triethylamin umgesetzt. Man erhält die Titelverbindung als weissen Schaum. DC: Methylenchlorid/Methanol/conc. Ammoniak (700:50:1) $R_f = 0.56$, FD-MS: $M^+ = 546$.

Beispiel 20: (2R*,4S*)-2-Benzyl-1-(2,3-diphenylpropionyl)-N-(4-chinolylmethyl)-4-piperidinamin

Analog Beispiel 2 werden 340 mg (0.535 mMol) (2R*,4S*)-2-Benzyl-1-(2,3-diphenylpropionyl)-N-(4-chinolylmethyl)-N-trifluoracetyl-4-piperidinamin mit 81 mg (2.14 mMol) Natriumborhydrid umgesetzt. Man erhält die Titelverbindung

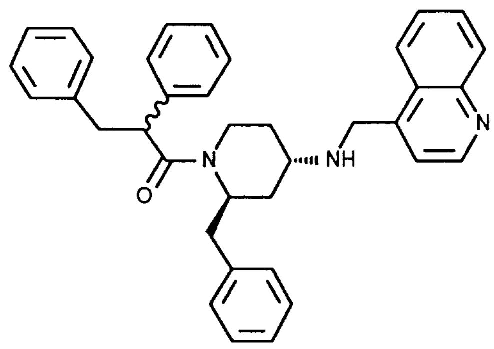

als Diastereomerengemisch in Form von weissem Schaum. DC: Methylenchlorid/Methanol/conc. Ammoniak (1000:50:1) $R_f = 0.37$, FD-MS: $M^+ = 539$.

Die Ausgangsverbindung dazu wird wie folgt hergestellt:

(2R*,4S*)-2-Benzyl-1-(2,3-diphenylpropionyl)-N-(4-chinolylmethyl)-N-trifluoracetyl-4-piperidinamin

Analog Beispiel 4 werden 300 mg (0.702 mMol) (2R*,4S*)-2-Benzyl-N-(4-chinolylmethyl)-N-trifluoracetyl-4-piperidinamin mit 190 mg (0.842 mMol) (R,S)-2,3-Diphenylpropionsäure, 214 mg (0.842 mMol) Bis-(2-oxo-3-oxazolidinyl)-phosphinsäurechlorid und 216 µl (1.54 mMol) Triethylamin umgesetzt. Man erhält die Titelverbindung als weissen Schaum. DC: Methylenchlorid/Methanol/conc. Ammoniak (700:50:1) $R_f = 0.74$, FD-MS: $M^+ = 635$.

Beispiel 21: (2R*,4S*)-2-Benzyl-1-((3S)-(2,3,4,9-tetrahydro-1H-pyrido[3,4-b]indol-3-yl)-carbonyl)-N-(4-chinolylmethyl)-4-piperidinamin

Analog Beispiel 2 werden 197 mg (0.315 mMol) des Diastereomerengemisches (2R*,4S*)-2-Benzyl-1-(-(3S)-(2,3,4,9-tetrahydro-1H-pyrido[3,4-b]indol-3-yl)-carbonyl)-N-(4-chinolylmethyl)-N-trifluoracetyl-4-piperidinamin mit 48 mg (1.26 mMol) Natriumborhydrid umgesetzt. Man erhält die Titelverbindung

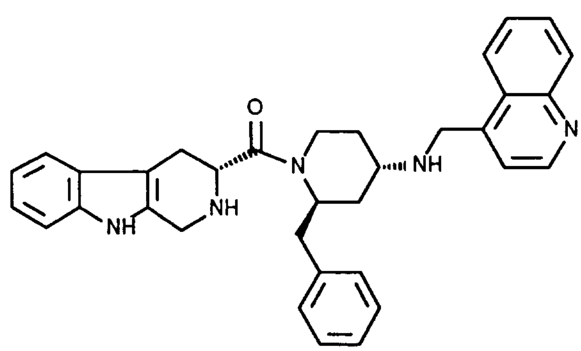

als Diastereomerengemisch in Form von weissem Schaum. DC: Methylenchlorid/Methanol/conc. Ammoniak (350:50:1) $R_f = 0.50$, FD-MS: $M^+ = 529$.

Die Ausgangsverbindung dazu wird wie folgt hergestellt:

(2R*,4S*)-2-Benzyl-1-((3S)-(2,3,4,9-tetrahydro-1H-pyrido[3,4-b]indol-3-yl)-carbonyl)N-(4-chinolylmethyl)-N-trifluoracetyl-4-piperidinamin

Zur Lösung von 338 mg (0.399 mMol) (2R*,4S*)-2-Benzyl-1-((3S)-(2-(9-fluorenylmethyloxycarbonyl)-2,3,4,9-tetrahydro-1H-pyrido[3,4-b]indol-3-yl)-carbonyl)-N-(4-chinolylmethyl)-N-trifluoracetyl-4-piperidinamin in 3 ml N,N-Dimethylformamid gibt man 1.97 ml (19.9 mMol) Piperidin und rührt das Gemisch 2 Stunden bei Raumtemperatur. Dann wird am Rotationsverdampfer eingeengt und der Rückstand zur Trennung der Diastereomeren mit Methylenchlorid/Methanol/conc. Ammoniak (2000:50:1) an Kieselgel chromatographiert.

DC: Methylenchlorid/Methanol/conc. Ammoniak (700:50:1)
Diastereomer A : $R_f = 0.21$, FD-MS: $M^+ = 625$,
Diastereomer B : $R_f = 0.13$, FD-MS: $M^+ = 625$.

Beispiel 22: (2R*,4S*)-2-Benzyl-1-(3-methoxybenzoyl)-N-(4-chinolylmethyl)-4-piperidinamin

Analog Beispiel **2** werden 230 mg (0.409 mMol) (2R*,4S*)-2-Benzyl-1-(3-methoxybenzoyl)-N-(4-chinolylmethyl)-N-trifluoracetyl-4-piperidinamin mit 81 mg (2.14 mMol) Natriumborhydrid umgesetzt. Man erhält die Titelverbindung der Formel

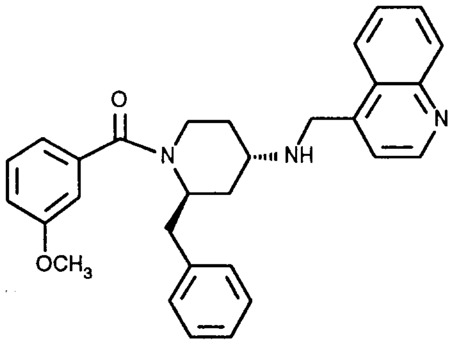

als weissen Schaum. DC: Methylenchlorid/Methanol/conc. Ammoniak (1000:50:1) $R_f = 0.26$, FD-MS: $M^+ = 465$.

Die Ausgangsverbindung dazu wird wie folgt hergestellt:

(2R*,4S*)-2-Benzyl-1-(3-methoxybenzoyl)-N-(4-chinolylmethyl)-N-trifluoracetyl-4-piperidinamin

Analog Beispiel 4 werden 200 mg (0.468 mMol) (2R*,4S*)-2-Benzyl-N-(4-chinolylmethyl)-N-trifluoracetyl-4-piperidinamin mit 85 mg (0.561 mMol) 3-Methoxybenzoesäure, 143 mg (0.561 mMol) Bis-(2-oxo-3-oxazolidinyl)-phosphinsäurechlorid und 144 µl (1.03 mMol) Triethylamin umgesetzt. Man erhält die Titelverbindung als weissen Schaum. DC: Methylenchlorid/Methanol/conc. Ammoniak (1000:50:1) $R_f = 0.45$, FD-MS: $M^+ = 561$.

Beispiel 23: (2R*,4S*)-2-Benzyl-1-(3-N,N-dimethylaminobenzoyl)-N-(4-chinolylmethyl)-4-piperidinamin

Analog Beispiel 2 werden 225 mg (0.391 mMol) (2R*,4S*)-2-Benzyl-1-(3-N,N-dimethylaminobenzoyl)-N-(4-chinolylmethyl)-N-trifluoracetyl-4-piperidinamin mit 59 mg (1.56 mMol) Natriumborhydrid umgesetzt. Man erhält die Titelverbindung

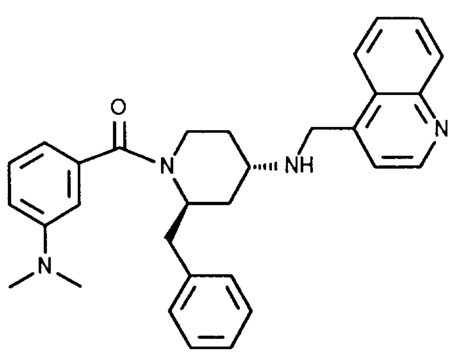

als weissen Schaum. DC: Methylenchlorid/Methanol/conc. Ammoniak (700:50:1) $R_f = 0.36$, FD-MS: $M^+ = 478$.

Die Ausgangsverbindung dazu wird wie folgt hergestellt:

(2R*,4S*)-2-Benzyl-1-(3-N,N-dimethylaminobenzoyl)N-(4-chinolylmethyl)-N-trifluoracetyl-4-piperidinamin

Analog Beispiel 4 werden 200 mg (0.468 mMol) (2R*,4S*)-2-Benzyl-N-(4-chinolylmethyl)-N-trifluoracetyl-4-piperidinamin mit 93 mg (0.561 mMol) 3-N,N-Dimethylaminobenzoesäure, 143 mg (0.561 mMol) Bis-(2-oxo-3-oxazolidinyl)-phosphinsäurechlorid und 144 µl (1.03 mMol) Triethylamin umgesetzt. Man erhält die Titelverbindung als gelbes Öl. DC: Methylenchlorid/Methanol/conc. Ammoniak (1000:50:1) $R_f = 0.65$, FD-MS: $M^+ = 574$.

Beispiel 24: (2R*,4S*)-2-Benzyl-1-(cis,cis-3,5-dimethylcyclohexylcarbonyl)-N-(4-chinolylmethyl)-4-piperidinamin

Analog Beispiel 2 werden 195 mg (0.345 mMol) (2R*,4S*)-2-Benzyl-1-(cis,cis-3,5-dimethylcyclohexylcarbonyl)-N-(4-chinolylmethyl)-N-trifluoracetyl-4-piperidinamin mit 52 mg (1.38 mMol) Natriumborhydrid umgesetzt. Man erhält die Titelverbindung

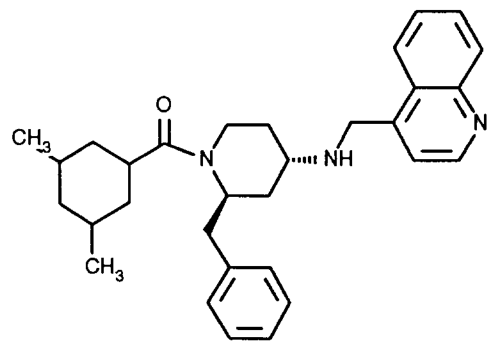

als weissen Schaum. DC: Methylenchlorid/Methanol/conc. Ammoniak (1000:50:1) $R_f = 0.24$, FD-MS: $M^+ = 469$.

Die Ausgangsverbindung dazu wird wie folgt hergestellt:

(2R*,4S*)-2-Benzyl-1-(cis,cis-3,5-dimethylcyclohexylcarbonyl)N-(4-chinolylmethyl)-N-trifluoracetyl-4-piperidinamin

Analog Beispiel 4 werden 200 mg (0.468 mMol) (2R*,4S*)-2-Benzyl-N-(4-chinolylmethyl)-N-trifluoracetyl-4-piperidinamin mit 87 mg (0.561 mMol) cis,cis-3,5-Dimethylcyclohexylcarbonsäure (hergestellt nach der Methode von H. van Bekkum et. al., Koninkl. Ned. Akad. Wetenschap, Proc. Ser. B. 64, 161 (1961), 143 mg (0.561 mMol) Bis-(2-oxo-3-oxazolidinyl)-phosphinsäurechlorid und 144 µl (1.03 mMol) Triethylamin umgesetzt. Man erhält die Titelverbindung als gelbes Öl. DC: Methylenchlorid/Methanol/conc. Ammoniak (1000:50:1) $R_f = 0.32$, FD-MS: $M^+ = 565$.

Beispiel 25: (2R*,4S*)-2-Benzyl-1-(3,5-bis-(trifluorrnethvl)-benzyl)-N-(4-chinolylmethyl)-4-piperidinamin

Analog Beispiel 2 werden 280 mg (28 mMol) (2R*,4S*)-2-Benzyl-1-(3,5-bis-(trifluormethyl)-benzyl)-N-(4-chinolylmethyl)-N-trifluoracetyl-4-piperidinamin mit 65 mg (1.71 mMol) Natriumborhydrid umgesetzt. Man erhält die Titelverbindung

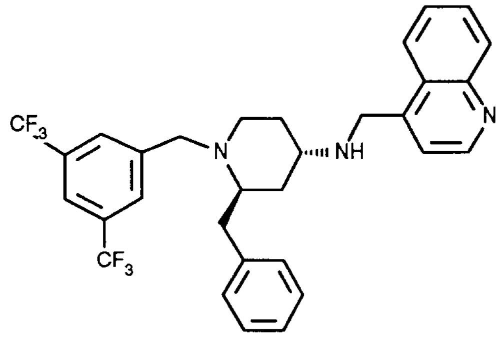

als weissen Schaum. DC: Methylenchlorid/Methanol/conc. Ammoniak (1000:50:1) $R_f = 0.35$, FD-MS: $M^+ = 557$.

Die Ausgangsverbindung dazu wird wie folgt hergestellt:

(2R*,4S*)-2-Benzyl-1-(3,5-bis-(trifluormethyl)-benzyl)-N-(4-chinolylmethyl)-N-trifluoracetyl4-piperidinamin

Man rührt das Gemisch aus 129 µl (0.702 mMol) 3,5-Bis-(trifluorrnethyl)-benzylbromid, 194 mg (1.40 mMol) Kaliumcarbonat und 300 mg (0.702 mMol) (2R*,4S*)-2-Benzyl-N-(4-chinolylmethyl)-N-trifluoracetyl-4-piperidinamin in 3 ml N,N-Dimethylformamid während 17 Stunden bei 60° . Darauf wird die Suspension abfiltriert, mit Aceton gut nachgewaschen und das Filtrat am Rotationsverdampfer eingeengt. Den Rückstand nimmt man in Methylenchlorid auf und wäscht nacheinander mit 10% wässriger Zitronensäure, 1 N Natriumhydrogencarbonatlösung, Wasser und Sole und trocknet über Magnesiumsulfat. Das gelbe Öl wird mit Methylenchlorid/Methanol/conc. Ammoniak (3000:50:1) an Kieselgel chromatographiert. Man erhält die Titelverbindung als gelbes Öl. DC: Methylenchlorid/Methanol/conc. Ammoniak (3000:50:1) $R_f = 0.36$, FD-MS: $M^+ = 653$.

Beispiel 26: (2S*,4R*)-2-Benzyl-1-[2-(5-chlor-1H-1,2,4-triazol-1-yl)phenoxyethyl]-N-(4-chinolylmethyl)-4-piperidinamin

Analog Beispiel 2 werden 100 mg (0.54 mMol) (2R*,4S*)-2-Benzyl-[2-(5-chlor-1H-1,2,4-triazol-1-yl)-phenoxyethyl]-N-(4-chinolylmethyl)-N-trifluoracetyl-4-piperidinamin mit 23 mg (0.62 mMol) Natriumborhydrid umgesetzt. Man erhält die Titelverbindung

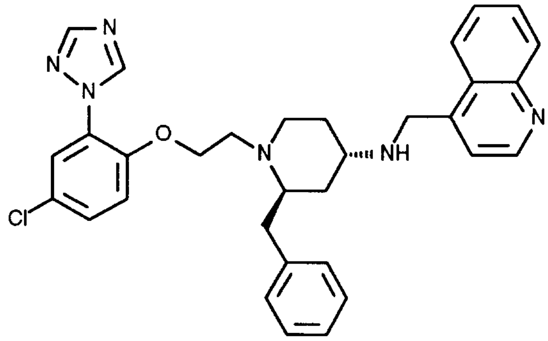

als weissen Schaum. DC: Methylenchlorid/Methanol/conc. Ammoniak (1000:50:1) $R_f = 0.23$, FD-MS: $M^+ = 553$.

Die Ausgangsverbindung dazu wird wie folgt hergestellt:

(2R*,4S*)-2-Benzyl-1-[2-(5-chlor-1H-1,2,4-triazol-1-yl)phenoxyethyl]-N-(4-chinolylmethyl)-N-trifluoracetyl-4-piperidinamin

Analog Beispiel 24 werden 200 mg (0.468 mMol) (2R*,4S*)-2-Benzyl-N-(4-chinolylmethyl)-N-trifluoracetyl-4-piperidinamin mit 141 mg (0.468 mMol) 2-(5-chlor-(1H-1,2,4-triazol-1-yl)phenoxy)ethylbromid und 129 mg (0.936 mMol) Kaliumcarbonat umgesetzt. Man erhält die Titelverbindung als weissen Schaum. DC:

Methylenchlorid/Methanol/conc. Ammoniak (1000:50:1) $R_f = 0.23$, FD-MS: $M^+ = 649$.

Beispiel 27: Diastereomer A von (2R*,4S*)-2-Benzyl-1-((S)-phenylalanyl)-N-(4-chinolylmethyl)-4-piperidinamin

Analog Beispiel 2 werden 112 mg (0.195 mMol) des Diastereomeren A von (2R*,4S*)-2-Benzyl-1-((S)-phenylalanyl)-N-(4-chinolylmethyl)-N-trifluoracetyl-4-piperidinamin mit 30 mg (0.801 mMol) Natriumborhydrid umgesetzt. Man erhält die Titelverbindung

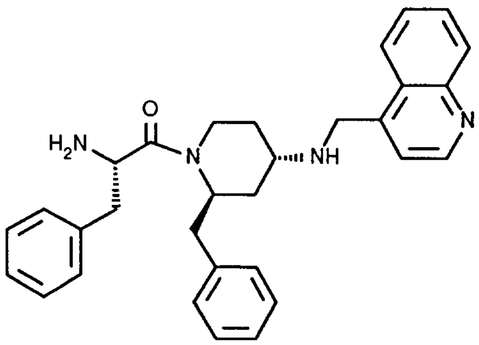

als weissen Schaum. DC: Methylenchlorid/Methanol/conc. Ammoniak (700:50:1) $R_f = 0.21$, FD-MS: $M^+ = 478$.

Die Ausgangsverbindungen dazu werden wie folgt hergestellt:

a) (2R*,4S*)-2-Benzyl-1-((S)-N-tert.-butyloxycarbonyl-phenylalanyl)-N-(4-chinolylmethyl)-N-trifluoracetyl-4-piperidinamin

Analog Beispiel 4 werden 300 mg (0.702 mMol) (2R*,4S*)-2-Benzyl-N-(4-chinolylmethyl)-N-trifluoracetyl-4-piperidinamin mit 223 mg (0.842 mMol) (S)-N-tert.-Butyloxycarbonyl-phenylalanin, 214 mg (0.842 mMol) Bis-(2-oxo-3-oxazolidinyl)-phosphinsäurechlorid und 215 µl (1.54 mMol) Triethylamin umgesetzt. Man erhält die Titelverbindung als Diastereomerengemisch in Form eines gelben Öls. DC: Methylenchlorid/Methanol/conc. Ammoniak (2000:50:1) $R_f = 0.37$, FD-MS: $M^+ = 674$.

b) Diastereomere von (2R*,4S*)-2-Benzyl-1-((S)-phenylalanyl)-N-(4-chinolylmethyl)-N-trifluoracetyl-4-piperidinamin

Zu 920 mg (1.36 mMol) (2R*,4S*)-2-Benzyl-1-((S)-N-tert.-butyloxycarbonyl-phenylalanyl)-N-(4-chinolylmethyl)-N-trifluoracetyl-4-piperidinamin gibt man 3.1 ml (4.09 mMol) Trifluoressigsäure und rührt das Reaktionsgemisch 2 Stunden bei Raumtemperatur. Dann wird am Rotationsverdampfer eingeengt, der Rückstand in Wasser aufgenommen, bei 0 °C mit 1 N Natriumhydrogencarbonatlösung basisch gestellt und mit Methylenchlorid extrahiert. Die organischen Phasen werden mit Sole gewaschen, über Magnesiumsulfat getrocknet und zur Trockne eingedampft. Der Rückstand wird zur Trennung der Diastereomeren mit Methylenchlorid/Methanol/conc. Ammoniak (2500:50:1) an Kieselgel chromatographiert. DC: Methylenchlorid/Methanol/conc. Ammoniak (1000:50:1)
Diastereomer A: $R_f = 0.24$, FD-MS: $M^+ = 574$,
Diastereomer B: $R_f = 0.22$, FD-MS: $M^+ = 574$.
Die Mischfraktionen von Diastereomer A und B wurden nicht weiter aufgetrennt.

Beispiel 28: Diastereomer B von (2R*,4S*)-2-Benzyl-1-((S)-phenylalanyl)-N-(4-chinolylmethyl)-4-piperidinamin

Analog Beispiel 2 werden 115 mg (0.200 mMol) des Diastereomeren B von (2R*,4S*)-2-Benzyl-1-((S)-phenylalanyl)-N-(4-chinolylmethyl)-N-trifluoracetyl-4-piperidinamin mit 30 mg (0.801 mMol) Natriumborhydrid umgesetzt. Man erhält die Titelverbindung

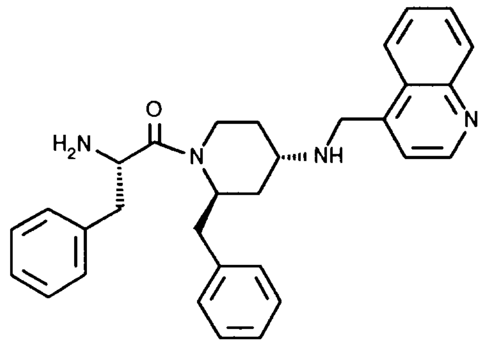

als weissen Schaum. DC: Methylenchlorid/Methanol/conc. Ammoniak (700:50:1) $R_f = 0.20$, FD-MS: $M^+ =$ 478.

Zur Ausgangsverbindung dazu siehe Beispiel 27a.

Beispiel 29: Diastereomer A von (2R*,4S*)-2-Benzyl-1-((R)-phenylalanyl)-N-(4-chinolylmethyl)-4-piperidinamin

Analog Beispiel 2 werden 174 mg (0.303 mMol) des Diastereomeren A von (2R*,4S*)-2-Benzyl-1-((R)-phenylalanyl)-N-(4-chinolylmethyl)-N-trifluoracetyl-4-piperidinamin mit 46 mg (1.211 mMol) Natriumborhydrid umgesetzt. Man erhält die Titelverbindung

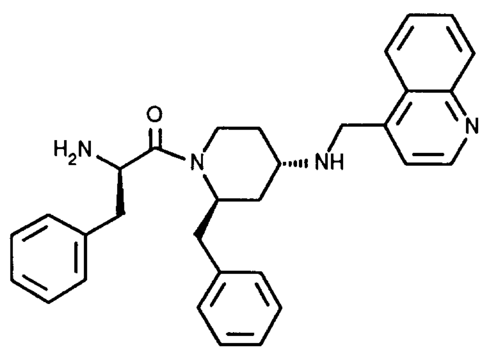

als weissen Schaum. DC: Methylenchlorid/Methanol/conc. Ammoniak (700:50:1) $R_f = 0.28$, FD-MS: $M^+ =$ 478.

Die Ausgangsverbindungen dazu werden wie folgt hergestellt:

Diastereomere von (2R*,4S*)-2-Benzyl-1-((R)-phenylalanyl)-N-(4-chinolylmethyl)-N-trifluoracetyl-4-piperidinamin

Analog Beispiel 27b werden 1.10 g (1.63 mMol) (2R*,4S*)-2-Benzyl-1-((R)-N-tert.-butyloxycarbonyl-phenylalanyl)-N-(4-chinolylmethyl)-N-trifluoracetyl-4-piperidinamin mit 3.8 ml (48.8 mMol) Trifluoressigsäure behandelt. Man erhält die Diastereomeren der Titelverbindung. DC: Methylenchlorid/Methanol/conc. Ammoniak (1000:50:1)
Diastereomer A: $R_f = 0.52$, FD-MS: $M^+ =$ 574,
Diastereomer B: $R_f = 0.50$, FD-MS: $M^+ =$ 574.
Die Mischfraktionen von Diastereomer A und B wurden nicht weiter aufgetrennt.

Beispiel 30: Diastereomer B von (2R*,4S*)-2-Benzyl-1-((R)-phenylalanyl)-N-(4-chinolylmethyl)-4-piperidinamin

Analog Beispiel 2 werden 92 mg (0.160 mMol) des Diastereomeren B von (2R*,4S*)-2-Benzyl-1-((R)-phenylalanyl)-N-(4-chinolylmethyl)-N-trifluoracetyl-4-piperidinamin mit 25 mg (0.640 mMol) Natriumborhydrid umgesetzt. Man erhält die Titelverbindung

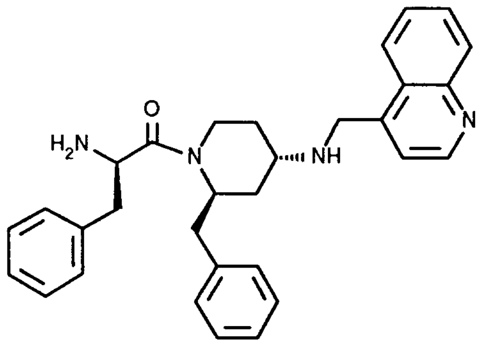

als weissen Schaum. DC: Methylenchlorid/Methanol/conc. Ammoniak (350:50:1) $R_f = 0.49$, FD-MS: $M^+ =$ 478.

Zur Ausgangsverbindung dazu siehe Beispiel 27a.

Beispiel 31: (2R*,4S*)-2-Benzyl-1-((S)-N-acetyl-phenylalanyl)-N-(4-chinolylmethyl)-4-piperidinamin

Analog Beispiel 2 werden 160 mg (0.259 mMol) des Diastereomerengemisches von (2R*,4S*)-2-Benzyl-1-((S)-N-acetyl-phenylalanyl)-N-(4-chinolylmethyl)-N-trifluoracetyl-4-piperidinamin mit 39 mg (1.04 mMol) Natriumborhydrid umgesetzt. Man erhält die Titelverbindung

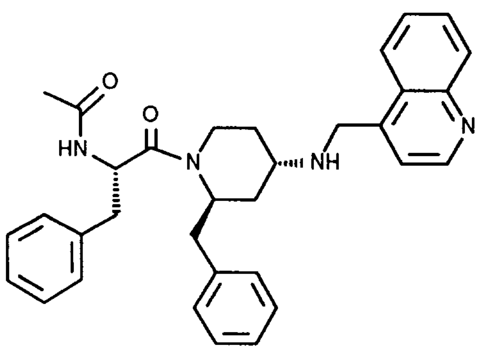

als Diastereomerengemisch als weissen Schaum. DC: Methylenchlorid/Methanol/conc. Ammoniak (1000:50:1) $R_f = 0.22$, FD-MS: $M^+ =$ 520.

Die Ausgangsverbindung dazu wird wie folgt hergestellt:

(2R*,4S*)-2-Benzyl-1-((S)-N-acetyl-phenylalanyl)-N-(4-chinolylmethyl)-N-trifluoracetyl-4-piperidinamin

Zur Lösung von 180 mg (0.313 mMol) des Diastereomerengemisches von (2R*,4S*)-2-Benzyl-1-((S)-phenylalanyl)-N-(4-chinolylmethyl)-N-trifluoracetyl-4-piperidinamin in 2 ml Pyridin werden bei 0° 35 µl (0.376 mMol) Essigsäureanhydrid zugefügt und das Gemisch 2.5 Stunden bei 0° gerührt. Nach Einengen am Rotationsverdampfer wird der ölige Rückstand in Methylenchlorid aufgenommen, mit 5% wässriger Zitronensäure und mit 1 N Natriumhydrogencarbonatlösung gewaschen. Die organischen Phasen werden über Magnesiumsulfat getrocknet und zur Trockne eingedampft. Man erhält die Titelverbindung als weissen Schaum. DC: Methylenchlorid/Methanol/conc. Ammoniak (1000:50:1) $R_f = 0.39$, FD-MS: $M^+ =$ 616.

Beispiel 32: (2R*,4S*)-2-Benzyl-1-((R)-N-acetyl-phenylalanyl)-N-(4-chinolylmethyl)-4-piperidinamin

Analog Beispiel 2 werden 185 mg (0.411 mMol) des Diastereomerengemisches von (2R*,4S*)-2-Benzyl-1-((R)-N-acetyl-phenylalanyl)-N-(4-chinolylmethyl)-N-trifluoracetyl-4-piperidinamin mit 62 mg (1.64 mMol) Natriumborhydrid umgesetzt. Man erhält die Titelverbindung

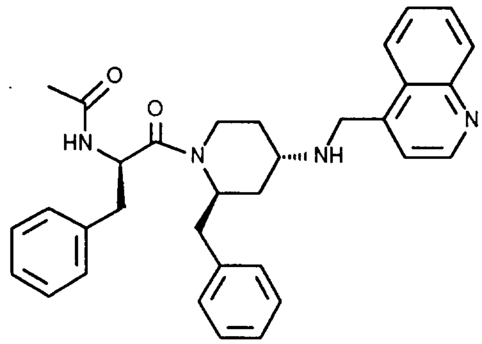

als Diastereomerengemisch (weisser Schaum). DC: Methylenchlorid/Methanol/conc. Ammoniak (700:50:1) $R_f = 0.42$, FD-MS: $M^+ = 520$.

Die Ausgangsverbindung dazu wird wie folgt hergestellt:

(2R*,4S*)-2-Benzyl-1-((R)-N-acetyl-phenylalanyl)N-(4-chinolylmethyl)-N-trifluoracetyl-4-piperidinamin

Analog Beispiel 31 werden 200 mg (0.348 mMol) des Diastereomerengemisches von (2R*,4S*)-2-Benzyl-1-((R)-phenylalanyl)-N-(4-chinolylmethyl)-N-trifluoracetyl-4-piperidinamin mit 39 µl (0.417 mMol) Essigsäureanhydrid umgesetzt. Man erhält die Titelverbindung als Diastereomerengemisch (weisser Schaum). DC: Methylenchlorid/Methanol/conc. Ammoniak (1000:50:1) $R_f = 0.28$, FD-MS: $M^+ = 616$.

Beispiel 33: (2R*,4S*)-2-Benzyl-1-((S)-N-(4-carboxamido-butyroyl)-phenylalanyl)-N-(4-chinolylmethyl)-4-piperidinamin

Analog Beispiel 2 werden 152 mg (0.221 mMol) des Diastereomerengemisches von (2R*,4S*)-2-Benzyl-1-((S)-N-(4-carboxamido-butyroyl)-phenylalanyl)-N-(4-chinolylmethyl)-N-trifluoracetyl-4-piperidinamin mit 34 mg (0.88 mMol) Natriumborhydrid umgesetzt. Man erhält die Titelverbindung

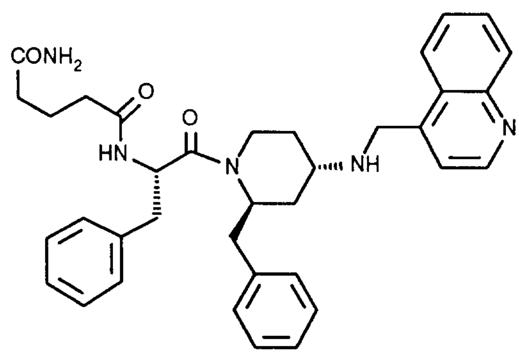

als Diastereomerengemisch als weissen Schaum. DC: Methylenchlorid/Methanol/conc. Ammoniak (350:50:1) $R_f = 0.50$, FD-MS: $M^+ = 591$.

Die Ausgangsverbindung dazu wird wie folgt hergestellt:

(2R*,4S*)-2-Benzyl-1-((S)-N-(4-carboxamido-butyroyl)-phenylalanyl)-N-(4-chinolylmethyl)-N-trifluoracetyl-4-piperidinamin

Zur Lösung von 200 mg (0.348 mMol) des Diastereomerengemisches von (2R*,4S*)-2-Benzyl-1-((S)-phenylalanyl)-N-(4-chinolylmethyl)-N-trifluoracetyl-4-piperidinamin in 2 ml Methylenchlorid werden bei 0° 179 µl (1.04 mMol) Diisopropylethylamin und 108 mg (0.348 mMol) Glutarsäure-mono-2,4,5-trichlorphenylester-amid zugefügt und die weisse Suspension 2 Stunden bei 0° und 16 Stunden bei Raumtemperatur gerührt. Nach Einengen der nun farblosen Lösung am Rotationsverdampfer wird der ölige Rückstand in Methylenchlorid aufgenommen, mit 5% wässriger Zitronensäure und mit 1 N Natriumhydrogencarbonatlösung gewaschen. Die organischen Phasen werden über Magnesiumsulfat getrocknet und zur Trockne eingedampft. Man erhält die Titelverbindung als weissen Schaum. DC: Methylenchlorid/Methanol/conc.

Ammoniak (1000:50:1) $R_f = 0.13$, FD-MS: $M^+ = 687$.

Beispiel 34: (2R*,4S*)-2-Benzyl-1-((R)-N-(4-carboxamido-butyroyl)-phenylalanyl)-N-(4-chinolylmethyl)-4-piperidinamin

Analog Beispiel 2 werden 210 mg (0.305 mMol) des Diastereomerengemisches von (2R*,4S*)-2-Benzyl-1-((R)-N-(4-carboxamido-butyroyl)-phenylalanyl)-N-(4-chinolylmethyl)-N-trifluoracetyl-4-piperidinamin mit 46 mg (1.22 mMol) Natriumborhydrid umgesetzt. Man erhält die Titelverbindung

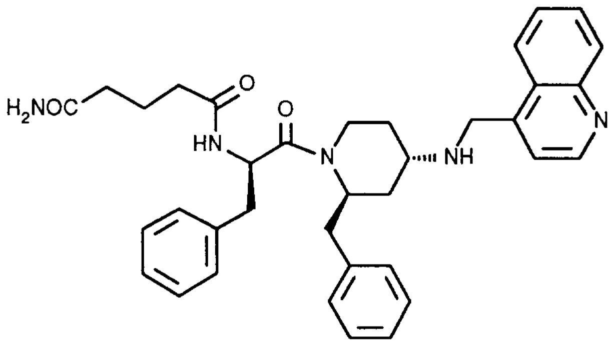

als Diastereomerengemisch als weissen Schaum. DC: Methylenchlorid/Methanol/conc. Ammoniak (350:50:1) $R_f = 0.56$, FD-MS: $M^+ = 591$.

Die Ausgangsverbindung dazu wird wie folgt hergestellt:

(2R*,4S*)-2-Benzyl-1-[(R)-N-(4-carboxamido-butyroyl)-phenylalanyl]-N-(4-chinolylmethyl)-N-trifluoracetyl-4-piperidinamin

Analog Beispiel 33 werden 222 mg (0.386 mMol) des Diastereomerengemisches von (2R*,4S*)-2-Benzyl-1-[(R)-phenylalanyl)-N-(4-chinolylmethyl)-N-trifluoracetyl-4-piperidinamin mit 198 µl (1.16 mMol) Diisopropylethylamin und 120 mg (0.386 mMol) Glutarsäure-mono-2,4,5-trichlorphenylester-amid umgesetzt. Man erhält die Titelverbindung als Diastereomerengemisch als weissen Schaum. DC: Methylenchlorid/Methanol/conc. Ammoniak (700:50:1) $R_f = 0.38$, FD-MS: $M^+ = 687$.

Beispiel 35: (2R*,4S*)-2-Benzyl-1-benzoyl-N-(4-chinolylmethyl)-4-piperidinamin

Analog Beispiel 2 werden 234 mg (0.440 mMol) (2R*,4S*)-2-Benzyl-1-benzoyl-N-(4-chinolylmethyl)-N-trifluoracetyl-4-piperidinamin mit 67 mg (1.76 mMol) Natriumborhydrid umgesetzt. Man erhält die Titelverbindung

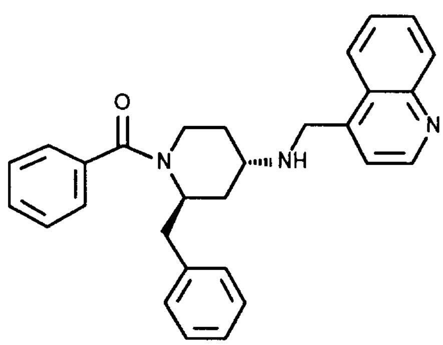

als weissen Schaum. DC: Methylenchlorid/Methanol/conc. Ammoniak (700:50:1) $R_f = 0.33$, FD-MS: $M^+ = 435$.

Die Ausgangsverbindung dazu wird wie folgt hergestellt:

(2R*,4S*)-2-Benzyl-1-benzoyl-N-(4-chinolylmethyl)-N-trifluoracetyl-4-piperidinamin

Zur Lösung von 200 mg (0.468 mMol) (2R*,4S*)-2-Benzyl-N-(4-chinolylmethyl)-N-trifluoracetyl-4-piperidinamin in 4 ml Methylenchlorid gibt man bei 0° 72 µl (0.515 mMol) Triethylamin und 54 µl (0.468 mMol) Benzoylchlorid. Man rührt das Reaktionsgemisch 3 Stunden bei 0°, versetzt mit Wasser und extrahiert mit Methylenchlorid. Die organischen Phasen werden über Magnesiumsulfat getrocknet und zur Trockne eingedampft. Das gelbe Öl wird mit Methylenchlorid/Methanol/conc. Ammoniak (1000:50:1) an Kieselgel chromatographiert. Man erhält die Titelverbindung als weissen Schaum. DC: Methylenchlorid/Methanol/conc. Ammoniak (700:50:1) $R_f = 0.72$, FD-MS: $M^+ = 531$.

Beispiel 36: (2R*,4S*)-2-Benzyl-1-(3-chlorbenzoyl)-N-(4-chinolylmethyl)-4-piperidinamin

Analog Beispiel 2 werden 254 mg (0.449 mMol) (2R*,4S*)-2-Benzyl-1-(3-chlorbenzoyl)-N-(4-chinolylmethyl)-N-trifluoracetyl-4-piperidinamin mit 68 mg (1.80 mMol) Natriumborhydrid umgesetzt. Man erhält die Titelverbindung

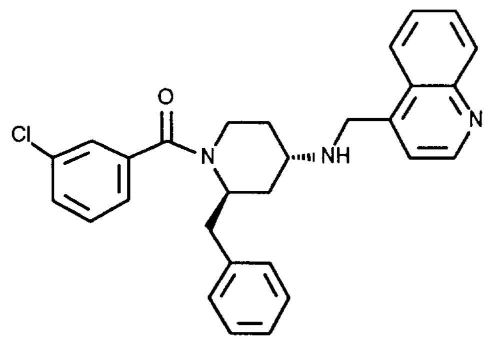

als weissen Schaum. DC: Methylenchlorid/Methanol/conc. Ammoniak (700:50:1) $R_f = 0.29$, FD-MS: $M^+ = 470$.

Die Ausgangsverbindung dazu wird wie folgt hergestellt:

(2R*,4S*)-2-Benzyl-1-(4-chlorbenzoyl)-N-(4-chinolylmethyl)-N-trifluoracetyl-4-piperidinamin

Analog Beispiel 35 werden 200 mg (0.468 mMol) (2R*,4S*)-2-Benzyl-N-(4-chinolylmethyl)-N-trifluoracetyl-4-piperidinamin mit 72 µl (0.515 mMol) Triethylamin und 60 µl (0.468 mMol) 3-Chlorbenzoylchlorid zum Produkt umgesetzt. DC: Methylenchlorid/Methanol/conc. Ammoniak (700:50:1) $R_f = 0.71$, FD-MS: $M^+ = 566$.

Beispiel 37: (2R*,4S*)-2-Benzyl-1-(3,5-dichlorbenzoyl)-N-(4-chinolylmethyl)-N-(3-carboxamido-propionyl)-4-piperidinamin

Man gibt 92 mg (0.99 mMol) 1-Hydroxybenzotriazol und 138 mg (0.899 mMol) N,N'-Dicyclohexylcarbodiimid zu einer Lösung von 181 mg (0.299 mMol) (2R*,4S*)-2-Benzyl-1-(3,5-dichlorbenzoyl)-N-(4-chinolylmethyl)-N-(3-carboxy-propionyl)-4-piperidinamin in 2 ml Tetrahydrofuran. Nach einer halben Stunde Rühren bei Raumtemperatur fügt man 2.1 ml (15 mMol) einer 7 M Lösung von Ammoniak in Ethanol dazu und rührt 36 Stunden bei Raumtemperatur. Man engt am Rotationsverdampfer ein, schlemmt den Rückstand in Methylenchlorid/Ether (1:1) auf und filtriert die weisse Suspension ab. Das Filtrat wird am Rotationsverdampfer eingeengt und das gelbe Öl mit Methylenchlorid/Methanol/conc. Ammoniak (1000:50:1) an Kieselgel chromatographiert. Man erhält die Titelverbindung

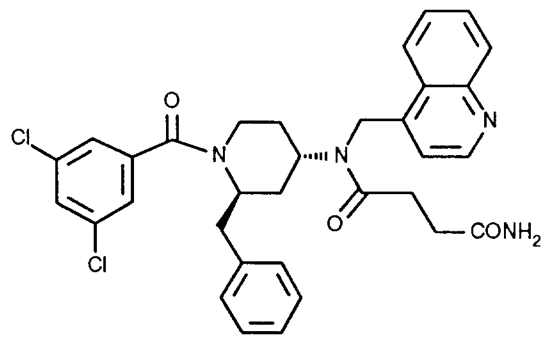

als weisse feste Substanz. DC: Methylenchlorid/Methanol/ conc. Ammoniak (700:50:1) $R_f = 0.23$, FD-MS: $M^+ =$ 602, 604.

Als Nebenprodukt wird aus der Chromatographie (2R*,4S*)-2-Benzyl-1-(3,5-dichlorbenzoyl)-N-(4-chinolylmethyl)-N-(3-N-cyclohexyl-carbamido-propionyl)-4-piperidinamin erhalten. DC: Methylenchlorid/Methanol/conc. Ammoniak (700:50:1) $R_f = 0.40$, FD-MS: $M^+ =$ 684, 686.

Beispiel 38: (2R,4S)-2-Benzyl-1-(3,5-bis-(trifluormethyl)-benzoyl)-N-(4-chinolylmethyl)-4-piperidinamin

Analog Beispiel 2g werden 3.35 g (7.78 mMol) (2R,4S)-2-Benzyl-1-(3,5-bis-(trifluormethyl)-benzoyl)-4-piperidinamin mit 1.34 g (8.56 mMol) Chinolin-4-carboxaldehyd und 1.3 g Magnesiumsulfat in 30 ml Toluol umgesetzt und anschliessend mit 324 mg (8.56 mMol) Natriumborhydrid in 25 ml Methanol reduziert. Man erhält die Titelverbindung

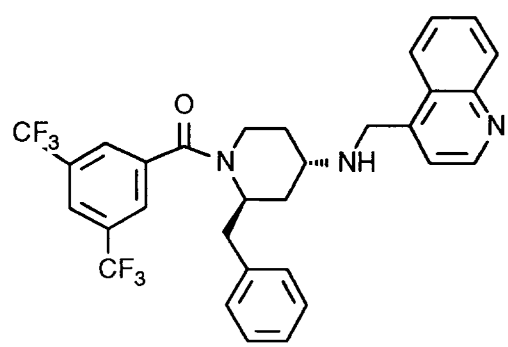

(3.5 g, 79%) als weissen Schaum. DC: Methylenchlorid/Methanol/conc. Ammoniak (1000:50:1) $R_f = 0.21$, FD-MS: $M^+ = 571$, $[\alpha]_D = +0.7$ (c = 1, MeOH), IR: 1635 $cm^{-1}$.

Die Ausgangsverbindung dazu wird wie folgt hergestellt:

a) (2R,4R)-2-Benzyl-1-*t*-butyloxycarbonyl-4-hydroxy-piperidin

Zur Lösung von 26.9 g (92.3 mMol) (2R*,4R*)-2-Benzyl-1-*t*-butyloxycarbonyl-4-hydroxy-piperidin in 200 ml Pyridin werden bei 0 °C 24 g (111 mMol) (-)-Camphansäurechlorid geben und das heterogen werdende Gemisch 2 Stunden bei 0 °C und anschliessend 16 Stunden bei RT gerührt. Nach Einengen am Rotationsverdampfer wird das Reaktionsgemisch in Methylenchlorid aufgenommen, zweimal mit 10% Zitronensäure, einmal mit Wasser und einmal mit Sole gewaschen, über Magnesiumsulfat getrocknet und am Rotationsverdampfer eingedampft.

Man erhält 47.8 g des diastereomeren Gemisches der Camphansäureester als oranges Öl. Diastereomer A: $R_f = 0.52$; Diastereomer B: $R_f = 0.47$.

Dieses wird mit Toluol/Ethylacetat (9:1) an Kieselgel chromatographiert und die diastereomeren Ester aus Hexan kristallisiert.

Man erhält das Diastereomer A in weissen Kristallen (14.2 g, 33%); Smp.: 114 - 115 °C und das Diastereomer B in weissen Kristallen (15.3 g, 35%); Smp.: 138 - 139 °C.

Die Lösung des Diastereomeren B in 300 ml Methanol wird mit 130 ml 0.5N Natronlauge versetzt und das Reaktionsgemisch während 18 Stunden bei RT gerührt. Nach Einengen am Rotationsverdampfer wird

das Reaktionsgemisch in Methylenchlorid aufgenommen, mit Wasser und Sole gewaschen, über Magnesiumsulfat getrocknet und am Rotationsverdampfer eingedampft. Man erhält die Titelverbindung als gelbes Öl (9.3 g, 98%). DC: Toluol/Ethylacetat (7:3) $R_f = 0.34$, FD-MS: $M^+$ = 291, $[\alpha]_D$ = +32° (c = 1, Methanol).

b) (2R,4R)-2-Benzyl-1-*t*-butyloxycarbonyl-4-(O-methylsulfonyl)-hydroxypiperidin

Analog Beispiel 2d werden 9.3 g (32 mMol) (2R,4R)-2-Benzyl-1-*t*-butyloxycarbonyl-4-hydroxy-piperidin mit 5 ml (63.8 mMol) Methansulfonsäurechlorid in 10 ml Pyridin umgesetzt. Man erhält die Titelverbindung (11 g, 93%) als farblose Nadeln. Smp.: 137 °C, DC: Toluol/Ethylacetat (4:1) $R_f = 0.42$, $[\alpha]_D$ = +21° (c = 1, MeOH).

c) (2R,4S)-2-Benzyl-1-*t*-butyloxycarbonyl-4-piperidinazid

Analog Beispiel 2e werden 10.9 g (29.6 mMol) (2R,4R)-2-Benzyl-1-*t*-butyloxycarbonyl-4-(O-methylsultonyl)-hydroxypiperidin mit 1.6 g (32.6 mMol) Lithiumazid in 60 ml N,N-Dimethylformamid umgesetzt.

Man erhält die Titelverbindung im Gemisch mit 2-Benzyl-N-*t*-butyloxycarbonyl-1,2,5,6-tetrahydropyridin (9.2 g, Gewichtsverhältnis laut NMR: 4.7:1), welches nicht weiter aufgetrennt wird. DC: Toluol/Ethylacetat (9:1) $R_f = 0.59$.

d) (2R,4S)-2-Benzyl-4-piperidinazid

Das Gemisch aus Beispiel 38c (berechneter Gehalt an (2R,4S)-2-Benzyl-1-*t*-butyloxycarbonyl-4-piperidinazid: 7.58 g (80%)) wird mit 36 ml Trifluoressigsäure versetzt und 90 Minuten bei RT gerührt. Danach wird am Rotationsverdampfer eingeengt, der Rückstand in Methylenchlorid aufgenommen und mit 2N Natronlauge gewaschen. Die organischen Phasen werden über Magnesiumsulfat getrocknet und zur Trockene eingedampft. Der Rückstand wird mit Methylenchlorid/Methanol/conc. Ammoniak (1000:50:1) an Kieselgel chromatographiert. Die Titelverbindung (4.7 g, 92%) wird als gelbes Öl erhalten. DC: Methylenchlorid/Methanol/conc. Ammoniak (350:50:1) $R_f = 0.63$, IR: 2100 $cm^{-1}$, $[\alpha]_D$ = -28.8° (c = 1, Methanol).

e) (2R,4S)-2-Benzyl-1-(3,5-bis-(trifluormethyl)-benzoyl)-4-piperidinazid

Analog Beispiel 4a werden 2.2 g (10.2 mMol) (2R,4S)-2-Benzyl-4-piperidinazid mit 2.5 g (12.2 mMol) 3,5-Bis-(trifluormethyl)-benzoesäure, 3.1 g (12.2 mMol) Bis-(2-oxo-3-oxazolidinyl)-phosphinsäurechlorid und 3.1 ml (22.4 mMol) Triethylamin. umgesetzt. Man erhält die Titelverbindung (4.16 g, 90%) als gelbes Öl. DC: Toluol/Ethylacetat (9:1) $R_f = 0.45$, FD-MS: $M^+$ = 456, $[\alpha]_D$ = + 5.1° (c = 1, Methanol).

f) (2R,4S)-2-Benzyl-1-(3,5-bis-(trifluormethyl)-benzoyl)-4-piperidinamin

Analog Beispiel 2f werden 4.1 g (9.0 mMol) (2R,4S)-2-Benzyl-1-(3,5-bis-(trifluormethyl)-benzoyl)-4-piperidinazid mit 10% Pd/C hydriert. Man erhält die Titelverbindung (3.38 g, 87%) als Öl. DC: Methylenchlorid/Methanol/conc. Ammoniak (350:50:1) $R_f = 0.47$, FD-MS: $M^+$ = 430, $[\alpha]_D$ = - 3.0° (c = 1, Methanol).

Beispiel 39: (2R,4S)-2-Benzyl-1-(3,5-dichlorbenzoyl)-N-(4-chinolylmethyl)-4-piperidinamin

Analog Beispiel 2g werden 1.95 g (5.37 mMol) (2R,4S)-2-Benzyl-1-(3,5-dichlorbenzoyl)-4-piperidinamin mit 0.93 g (5.90 mMol) Chinolin-4-carboxaldehyd und 0.9 g Magnesiumsulfat in 18 ml Toluol umgesetzt und anschliessend mit 223 mg (5.90 mMol) Natriumborhydrid in 18 ml Methanol reduziert. Man erhält die Titelverbindung

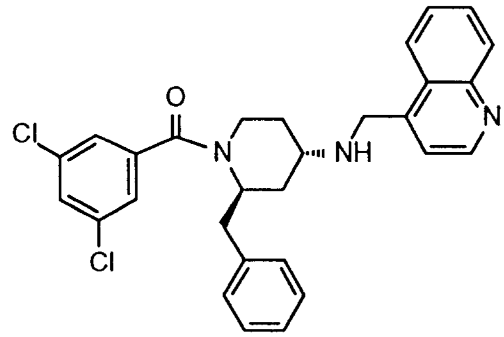

(2.2 g, 82%) als weissen Schaum. DC: Methylenchlorid/Methanol/conc. Ammoniak (700:50:1) $R_f = 0.35$, FD-MS: $M^+ =$ 503, 505, $[\alpha]_D =$ - 19.3 (c = 1, MeOH), IR: 1635, 1595, 1565 $cm^{-1}$.

Die Ausgangsverbindung dazu wird wie folgt hergestellt:

a) (2R,4S)-2-Benzyl-1-(3,5-dichlorbenzoyl)-4-piperidinazid

Zur Lösung von 2.4 g (11.1 mMol) (2R,4S)-2-Benzyl-4-piperidinazid und 2.2 ml (15.5 mMol) Triethylamin in 35 ml Methylenchlorid tropft man bei 0 °C die Lösung von 2.8 g (13.3 mMol) 3,5-Dichlorbenzoylchlorid. Nach 18 Stunden Rühren bei 0 °C wird am Rotationsverdampfer eingeengt, und das gelbe Öl zwischen Methylenchlorid und Wasser verteilt. Die organischen Phasen werden mit Sole gewaschen, über Magnesiumsulfat getrocknet und zur Trockene eingedampft. Das erhaltene Öl wird mit Toluol/Ethylacetat (9:1) an Kieselgel chromatographiert. Es wird die Titelverbindung (4.04 g, 94 %) als halbkristalline Masse erhalten. DC: Toluol/Ethylacetat (9:1) $R_f = 0.51$; FD-MS: $M^+ = 388$, 390; $[\alpha]_D =$ + 33.4 ° (c = 1, MeOH).

b) (2R,4S)-2-Benzyl-1-(3,5-dichlorbenzoyl)-4-piperidinamin

Analog Beispiel 2f werden 4.02 g (10.3 mMol) (2R,4S)-2-Benzyl-1-(3,5-dichlorbenzoyl)-4-piperidinazid mit 10% Pd/C hydriert. Man erhält die Titelverbindung (1.97 g, 52%) als Öl. DC: Methylenchlorid/Methanol/conc. Ammoniak (350:50:1) $R_f = 0.40$, FD-MS: $M^+ =$ 362, 364; IR: 3660, 3360, 1630 $cm^{-1}$; $[\alpha]_D =$ + 22.7 ° (c = 1, Methanol).

Beispiel 40: (2R*,4S*)-2-Benzyl-1-(2,4-dichlorbenzoyl)-N-(4-chinolylmethyl)-4-piperidinamin

Die Lösung von 195 mg (0.325 mMol) (2R*,4S*)-2-Benzyl-1-(2,4-dichlorbenzoyl)-N-(4-chinolylmethyl)-N-trifluoracetyl-4-piperidinamin und 26 mg (0.649 mMol) Natriumhydroxid in 2 ml Methanol und 2 ml Tetrahydrofuran lässt man 18 Stunden bei 0 °C rühren. Danach wird das Reaktionsgemisch eingeengt, in Methylenchlorid aufgenommen und mit Wasser und Sole gewaschen. Die organischen Phasen werden über Magnesiumsulfat getrocknet und zur Trockene eingedampft. Das gelbe Öl wird mit Methylenchlorid/Methanol/conc. Ammoniak (800:50:1) an Kieselgel chromatographiert. Man erhält die Titelverbindung

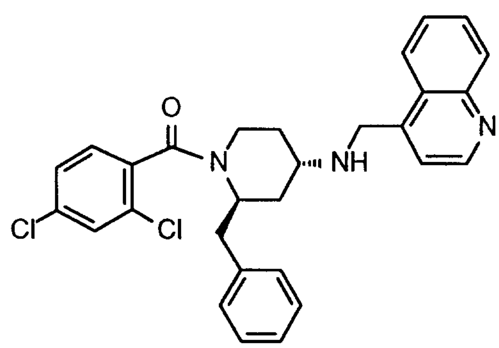

(157 mg, 96%) als weissen Schaum. DC: Methylenchlorid/Methanol/conc. Ammoniak (700:50:1) $R_f = 0.45$, FD-MS: $M^+ =$ 503,505.

Die Ausgangsverbindung dazu wird wie folgt hergestellt:

(2R*,4S*)-2-Benzyl-1-(2,4-dichlorbenzoyl)-N-(4-chinolylmethyl)-N-trifluoracetyl-4-piperidinamin

Analog Beispiel 35a werden 200 mg (0.468 mMol) (2R*,4S*)-2-Benzyl-N-(4-chinolylmethyl)-N-trifluoracetyl-4-piperidinamin mit 91 µl (0.655 mMol) Triethylamin und 78 µl (0.561 mMol) 2,4-Dichlorbenzoylchlorid zur Titelverbindung umgesetzt (240 mg, 86%). DC: Methylenchlorid/Methanol/conc. Ammoniak (700:50:1) $R_f = 0.57$, FD-MS: $M^+ =$ 599, 601.

Beispiel 41: (2R*,4S*)-2-Benzyl-1-(phenylacetyl)-N-(4-chinolylmethyl)-4-piperidinamin

Analog Beispiel 40 werden 192 mg (0.352 mMol) (2R*,4S*)-2-Benzyl-1-(2-phenylacetyl)-N-(4-chinolylmethyl)-N-trifluoracetyl-4-piperidinamin mit 141 µl (0.704 mMol) 5N Natronlauge in 1 ml Tetrahydrofuran und 1 ml Methanol umgesetzt. Man erhält die Titelverbindung

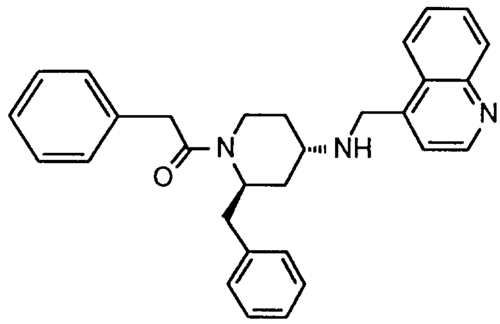

(73 mg, 46%) als weissen Schaum. DC: Methylenchlorid/Methanol/conc. Ammoniak (700:50:1) $R_f = 0.43$, FD-MS: $M^+ =$ 449.

Die Ausgangsverbindung dazu wird wie folgt hergestellt:

(2R*,4S*)-2-Benzyl-1-(phenylacetyl)-N-(4-chinolylmethyl)-N-trifluoracetyl-4-piperidinamin

Analog Beispiel 35a werden 200 mg (0.468 mMol) (2R*,4S*)-2-Benzyl-N-(4-chinolylmethyl)-N-trifluoracetyl-4-piperidinamin mit 72 µl (0.515 mMol) Triethylamin und 62 µl (0.468 mMol) Phenylacetylchlorid zur Titelverbindung umgesetzt (208 mg, 81%). DC: Methylenchlorid/Methanol/conc. Ammoniak (700:50:1) $R_f = 0.54$, FD-MS: $M^+ =$ 545.

Beispiel 42: (2R*,4S*)-2-Benzyl-1-(2,6-dichlorbenzoyl)-N-(4-chinolylmethyl)-4-piperidinamin

Analog Beispiel 40 werden 138 mg (0.230 mMol) (2R*,4S*)-2-Benzyl-1-(2,6-dichlorbenzoyl)-N-(4-chinolylmethyl)-N-trifluoracetyl-4-piperidinamin mit 18.4 mg (0.460 mMol) Natriumhydroxid in 1.5 ml Tetrahydrofuran und 1.5 ml Methanol umgesetzt. Man erhalt die Titelverbindung

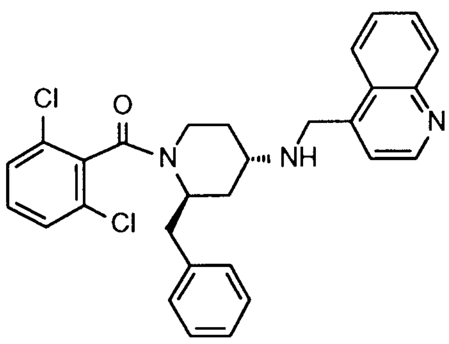

(56 mg, 48%) als weissen Schaum. DC: Methylenchlorid/Methanol/conc. Ammoniak (700:50:1) $R_f = 0.50$, FD-MS: $M^+ =$ 503, 505.

Die Ausgangsverbindung dazu wird wie folgt hergestellt:

(2R*,4S*)-2-Benzyl-1-(2,6-dichlorbenzoyl)-N-(4-chinolylmethyl)-N-trifluoracetyl-4-piperidinamin

Analog Beispiel 35a werden 200 mg (0.468 mMol) (2R*,4S*)-2-Benzyl-N-(4-chinolylmethyl)-N-trifluoracetyl-4-piperidinamin mit 91 µl (0.655 mMol) Triethylamin und 80 µl (0.561 mMol) 2,6-Dichlorbenzoylchlorid zur Titelverbindung umgesetzt (158 mg, 56%). DC: Methylenchlorid/Methanol/conc. Ammoniak (700:50:1) $R_f = 0.62$, FD-MS: $M^+$ = 599, 601.

Beispiel 43: (2R*,4S*)-2-Benzyl-1-(3,5-dibrombenzoyl)-N-(4-chinolylmethyl)-4-piperidinamin.

Analog Beispiel 2 werden 0.166g (0.241 mMol) (2R*,4S*)-2-Benzyl-1-(3,5-dibrombenzoyl)-N-(4-chinolylmethyl)-N-trifluoracetyl-4-piperidinamin mit 0.037 g (0.96 mMol) Natriumborhydrid umgesetzt. Man erhalt die Titelverbindung

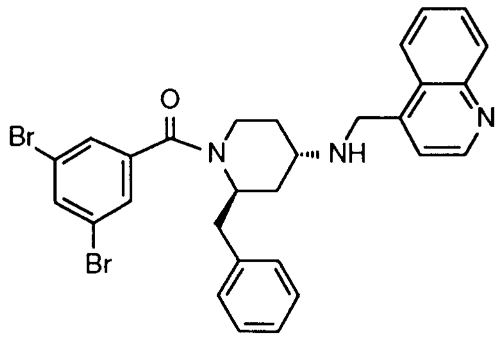

(0.094 g, 66%) als weissen Schaum. DC: Methylenchlorid/Methanol/conc. Ammoniak (700:50:1) $R_f = 0.23$, FD-MS: $M^+$ = 591, 593, 595
Die Ausgangsverbindung dazu wird wie folgt hergestellt:

(2R*,4S*)-2-Benzyl-1-(3,5-dibrombenzoyl)-N-(4-chinolylmethyl)-N-trifluoracetyl-4-piperidinamin

Analog Beispiel 2a werden 197 mg (0.70 mMol) 3,5-Dibrombenzoesäure (hergestellt nach: J. Organometallic Chem. 215, 281 (1981)) zuerst mit 2 ml (27 mMol) Thionylchlorid und anschliessend mit 200 mg (0.468 mMol) (2R*,4S*)-2-Benzyl-N-(4-chinolylmethyl)-N-trifluoracetyl-4-piperidinamin und 130 µl (0.936 mMol) Triethylamin zur Titelverbindung umgesetzt (168 mg, 52%). DC: Methylenchlorid/Methanol/conc. Ammoniak (700:50:1) $R_f = 0.60$, FD-MS: $M^+$ = 687, 689, 691.

Beispiel 44: (2R*,4S*)-2-Benzyl-1-(9-fluorenoyl)-N-(4-chinolylmethyl)-4-piperidinamin.

Analog Beispiel 2 werden 0.238 g (0.384 mMol) (2R*,4S*)-2-Benzyl-1-(9-fluorenoyl)-N-(4-chinolylmethyl)-N-trifluoracetyl-4-piperidinamin mit 0.066 g (1.54 mMol) Natriumborhydrid umgesetzt. Man erhalt die Titelverbindung

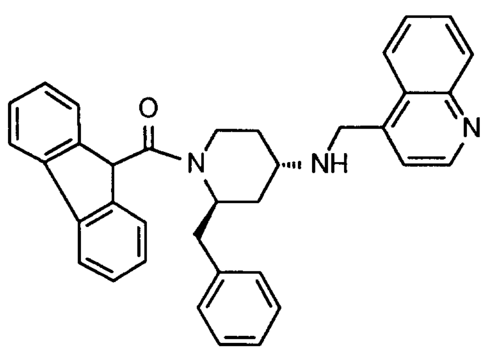

(0.155 g, 79%) als weissen Schaum. DC: Methylenchlorid/Methanol/conc. Ammoniak (700:50:1) $R_f = 0.36$, FD-MS: $M^+$ = 523.

Die Ausgangsverbindung dazu wird wie folgt hergestellt:

(2R*,4S*)-2-Benzyl-1-(9-fluorenoyl)-N-(4-chinolylmethyl)-N-trifluoracetyl-4-piperidinamin

Analog Beispiel 4a werden 200 mg (0.467 mMol) (2R*,4S*)-2-Benzyl-N-(4-chinolylmethyl)-N-trifluoracetyl-4-piperidinamin mit 78 mg (0.562 mMol) 9-Fluorencarbonsäure, 143 mg (0.561 mMol) Bis-(2-oxo-3-oxazolidinyl)-phosphinsäurechlorid und 144 µl (1.03 mMol) Triethylamin umgesetzt. Man erhalt die Titelverbindung (241 mg, 82%) als Öl. DC: Methylenchlorid/Methanol/conc. Ammoniak (1000:50:1) $R_f = 0.58$, FD-MS: $M^+ = 619$.

Beispiel 45: (2R*,4S*)-2-Benzyl-1-(3-toluoyl)-N-(4-chinolylmethyl)-4-piperidinamin.

Analog Beispiel 2 werden 0.251 g (0.460 mMol) (2R*,4S*)-2-Benzyl-1-(3-toluoyl)-N-(4-chinolylmethyl)-N-trifluoracetyl-4-piperidinamin mit 0.070 g (1.84 mMol) Natriumborhydrid umgesetzt. Man erhalt die Titelverbindung

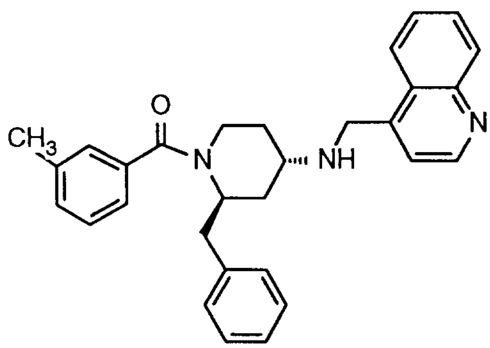

(0.172 g, 83%) als weissen Schaum. DC: Methylenchlorid/Methanol/conc. Ammoniak (700:50:1) $R_f = 0.27$, FD-MS: $M^+ = 449$.

Die Ausgangsverbindung dazu wird wie folgt hergestellt:

(2R*,4S*)-2-Benzyl-1-(3-toluoyl)-N-(4-chinolylmethyl)-N-trifluoracetyl-4-piperidinamin

Analog Beispiel 2a werden 96 mg (0.70 mMol) m-Toluylsäure zuerst mit 2 ml (27 mMol) Thionylchlorid und anschliessend mit 200 mg (0.468 mMol) (2R*,4S*)-2-Benzyl-N-(4-chinolylmethyl)-N-trifluoracetyl-4-piperidinamin und 118 µl (0.842 mMol) Triethylamin zur Titelverbindung umgesetzt (251 mg, 98%). DC: Methylenchlorid/Methanol/conc. Ammoniak (700:50:1) $R_f = 0.51$, FD-MS: $M^+ = 545$.

Beispiel 46: (2R*,4S*)-2-Benzyl-1-(3-brombenzoyl)-N-(4-chinolylmethyl)-4-piperidinamin.

Analog Beispiel 2 werden 0.271 g (0.444 mMol) (2R*,4S*)-2-Benzyl-1-(3-brombenzoyl)-N-(4-chinolylmethyl)-N-trifluoracetyl-4-piperidinamin mit 0.067 g (1.78 mMol) Natriumborhydrid umgesetzt. Man erhält die Titelverbindung

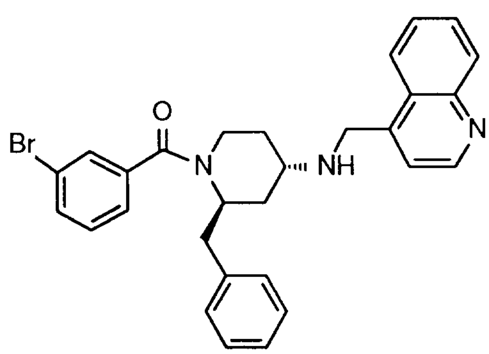

(0.212 g, 93%) als Öl. DC: Methylenchlorid/Methanol/conc. Ammoniak (700:50:1) $R_f = 0.29$, FD-MS: $M^+ =$ 513, 515.

Die Ausgangsverbindung dazu wird wie folgt hergestellt:

(2R*,4S*)-2-Benzyl-1-(3-brombenzoyl)-N-(4-chinolylmethyl)-N-trifluoracetyl-4-piperidinamin

Analog Beispiel 2a werden 141 mg (0.70 mMol) m-Brombenzoesäure zuerst mit 2 ml (27 mMol) Thionylchlorid und anschliessend mit 200 mg (0.468 mMol) (2R*,4S*)-2-Benzyl-N-(4-chinolylmethyl)-N-trifluoracetyl-4-piperidinamin und 118 µl (0.842 mMol) Triethylamin zur Titelverbindung umgesetzt (271 mg, 95%). DC: Methylenchlorid/Methanol/conc. Ammoniak (700:50:1) $R_f = 0.71$, FD-MS: $M^+ =$ 609, 611.

Beispiel 47: (2R*,4S*)-2-Benzyl-1-(3,5-dihydroxybenzoyl)-N-(4-chinolylmethyl)-4-piperidinamin.

Analog Beispiel 2 werden 0.097 g (0.172 mMol) (2R*,4S*)-2-Benzyl-1-(3,5-dihydroxybenzoyl)-N-(4-chinolylmethyl)-N-trifluoracetyl-4-piperidinamin mit 0.026 g (0.688 mMol) Natriumborhydrid umgesetzt. Man erhält die Titelverbindung

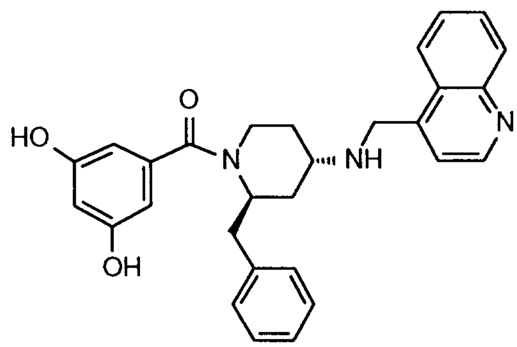

(0.023 g, 29%) als weissen Schaum. DC: Methylenchlorid/Methanol/conc. Ammoniak (350:50:1) $R_f = 0.57$, FD-MS: $M^+ =$ 467.

Die Ausgangsverbindung dazu wird wie folgt hergestellt:

(2R*,4S*)-2-Benzyl-1-(3,5-dihydroxybenzoyl)-N-(4-chinolylmethyl)-N-trifluoracetyl-4-piperidinamin

Analog Beispiel 4a werden 200 mg (0.467 mMol) (2R*,4S*)-2-Benzyl-N-(4-chinolylmethyl)-N-trifluoracetyl-4-piperidinamin mit 87 mg (0.562 mMol) 3,5-Dihydroxybenzoesäure, 143 mg (0.561 mMol) Bis-(2-oxo-3-oxazolidinyl)-phosphinsäurechlorid und 144 µl (1.03 mMol) Triethylamin umgesetzt. Man erhält die Titelverbindung (99 mg, 38%) als weissen Schaum. DC: Methylenchlorid/Methanol/conc. Ammoniak (700:50:1) $R_f = 0.41$, FD-MS: $M^+ =$ 563.

Beispiel 48: (2R*,4S*)-2-Benzyl-1-(3-cyanbenzoyl)-N-(4-chinolylmethyl)-4-piperidinamin.

Analog Beispiel 2 werden 0.248 g (0.446 mMol) (2R*,4S*)-2-Benzyl-1-(3-cyanbenzoyl)-N-(4-chinolylmethyl)-N-trifluoracetyl-4-piperidinamin mit 0.068 g (1.78 mMol) Natriumborhydrid umgesetzt. Man erhält die Titelverbindung

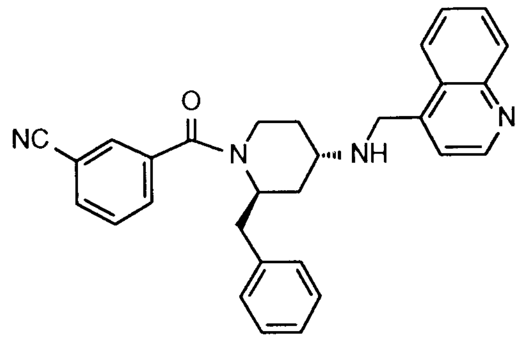

(0.157 g, 62%) als weissen Schaum. DC: Methylenchlorid/Methanol/conc. Ammoniak (700:50:1) $R_f = 0.51$, FD-MS: $M^+ = 460$.

Die Ausgangsverbindung dazu wird wie folgt hergestellt:

(2R*,4S*)-2-Benzyl-1-(3-cyanbenzoyl)-N-(4-chinolylmethyl)-N-trifluoracetyl-4-piperidinamin

Analog Beispiel 4a werden 200 mg (0.467 mMol) (2R*,4S*)-2-Benzyl-N-(4-chinolylmethyl)-N-trifluoracetyl-4-piperidinamin mit 69 mg (0.514 mMol) 3-Cyanbenzoesäure, 143 mg (0.561 mMol) Bis-(2-oxo-3-oxazolidinyl)-phosphinsäurechlorid und 144 µl (1.03 mMol) Triethylamin umgesetzt. Man erhält die Titelverbindung (250 mg, 96%) als weissen Schaum. DC: Methylenchlorid/Methanol/conc. Ammoniak (700:50:1) $R_f = 0.53$, FD-MS: $M^+ = 556$.

Beispiel 49: (2R*,4S*)-2-Benzyl-1-(2-chlorbenzoyl)-N-(4-chinolylmethyl)-4-piperidinamin.

Analog Beispiel 2 werden 0.165 g (0.291 mMol) (2R*,4S*)-2-Benzyl-1-(2-chlorbenzoyl)-N-(4-chinolylmethyl)-N-trifluoracetyl-4-piperidinamin mit 0.044 g (1.16 mMol) Natriumborhydrid umgesetzt. Man erhält die Titelverbindung

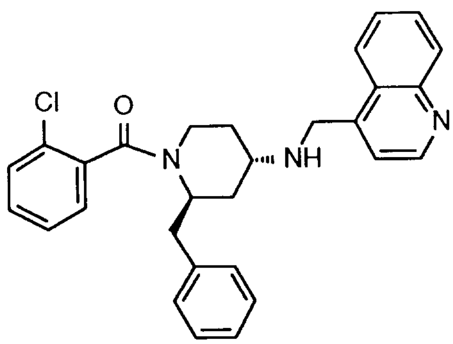

(0.109 g, 80%) als weissen Schaum. DC: Methylenchlorid/Methanol/conc. Ammoniak (1000:50:1) $R_f = 0.33$, MS: $M^+ = 469, 471$; IR: 3680, 1640, 1605, 1580 $cm^{-1}$.

Die Ausgangsverbindung dazu wird wie folgt hergestellt:

(2R*,4S*)-2-Benzyl-1-(2-chlorbenzoyl)-N-(4-chinolylmethyl)-N-trifluoracetyl-4-piperidinamin

Analog Beispiel 4a werden 200 mg (0.467 mMol) (2R*,4S*)-2-Benzyl-N-(4-chinolylmethyl)-N-trifluoracetyl-4-piperidinamin mit 88 mg (0.560 mMol) 2-Chlorbenzoesäure, 143 mg (0.561 mMol) Bis-(2-oxo-3-oxazolidinyl)-phosphinsäurechlorid und 144 µl (1.03 mMol) Triethylamin umgesetzt. Man erhält die Titelverbindung (179 mg, 68%) als weissen Schaum. DC: Methylenchlorid/Methanol/conc. Ammoniak (2000:50:1) $R_f = 0.42$, FD-MS: $M^+ = 565, 567$.

Beispiel 50: (2R*,4S*)-2-Benzyl-1-(4-chlorbenzoyl)-N-(4-chinolylmethyl)-4-piperidinamin.

Analog Beispiel 2 werden 0.202 g (0.291 mMol) (2R*,4S*)-2-Benzyl-1-(4-chlorbenzoyl)-N-(4-chinolylmethyl)-N-trifluoracetyl-4-piperidinamin mit 0.054 g (1.43 mMol) Natriumborhydrid umgesetzt. Man erhält die Titelverbindung

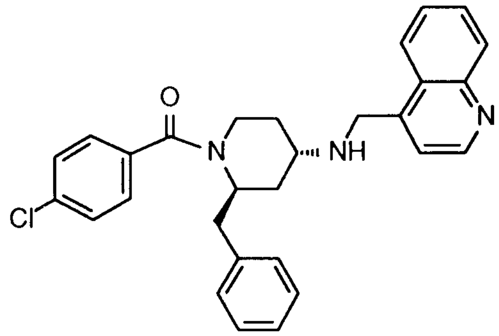

(0.136 g, 81%) als weissen Schaum. DC: Methylenchlorid/Methanol/conc. Ammoniak (1000:50:1) $R_f = 0.17$, FD-MS: $M^+ =$ 469, 471; IR: 3675, 1625, 1595, 1570 $cm^{-1}$.

Die Ausgangsverbindung dazu wird wie folgt hergestellt:

(2R*,4S*)-2-Benzyl-1-(4-chlorbenzoyl)-N-(4-chinolylmethyl)-N-trifluoracetyl-4-piperidinamin

Analog Beispiel 4a werden 200 mg (0.467 mMol) (2R*,4S*)-2-Benzyl-N-(4-chinolylmethyl)-N-trifluoracetyl-4-piperidinamin mit 88 mg (0.560 mMol) 4-Chlorbenzoesäure, 143 mg (0.561 mMol) Bis-(2-oxo-3-oxazolidinyl)-phosphinsäurechlorid und 144 µl (1.03 mMol) Triethylamin umgesetzt. Man erhalt die Titelverbindung (210 mg, 80%) als weissen Schaum. DC: Methylenchlorid/Methanol/conc. Ammoniak (1000:50:1) $R_f = 0.44$, FD-MS: $M^+ =$ 565, 567.

Beispiel 51: (2R*,4S*)-2-Benzyl-1-(9-fluorenyl)-N-(4-chinolylmethyl)-4-piperidinamin

Analog Beispiel 40 werden 130 mg (0.219 mMol) (2R*,4S*)-2-Benzyl-1-(9-fluorenyl)-N-(4-chinolylmethyl)-N-trifluoracetyl-4-piperidinamin mit 500 µl (0.500 mMol) 1N Natronlauge in 1 ml Tetrahydrofuran und 1 ml Methanol umgesetzt Man erhält die Titelverbindung

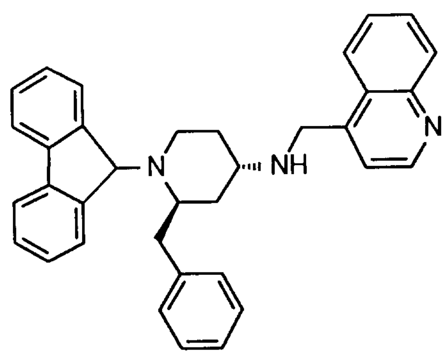

(49 mg, 45%) als weissen Schaum. DC: Toluol/Ethylacetat (1:1) $R_f = 0.26$; FD-MS: $M^+ = 495$.

Die Ausgangsverbindung dazu wird wie folgt hergestellt:

(2R*,4S*)-2-Benzyl-1-(9-fluorenyl)-N-(4-chinolylmethyl)-N-trifluoracetyl-4-piperidinamin

Analog Beispiel 25a werden 200 mg (0.467 mMol) (2R*,4S*)-2-Benzyl-N-(4-chinolylmethyl)-N-trifluoracetyl-4-piperidinamin mit 138 mg (0.561 mMol) 9-Bromfluoren und 155 mg (1.12 mMol) Kaliumcarbonat in 2.5 ml Aceton umgesetzt. Man erhalt die Titelverbindung (131 mg, 47%) als Öl. DC: Toluol/Ethylacetat (1:1) $R_f = 0.43$; FD-MS: $M^+ = 591$.

Beispiel 52: (2R*,4S*)-2-Benzyl-1-(3,5-dichlorbenzoyl)-N-(4-chinolylmethyl)-N-methyl 4-piperidinamin

Zur Lösung von 150 mg (0.297 mMol) (2S*,4R*)-2-Benzyl-1-(3,5-dichlorbenzoyl)-N-(4-chinolylmethyl)-4-piperidinamin in 2 ml 1,2-Dimethoxyäthan werden bei 0 °C 13.4 mg (0.446 mMol) einer 80%igen Suspension von Natriumhydrid in Mineralöl (mit Hexan aufgeschlemmt und abdekantiert) in einer Portion zugegeben. Nach 10 Minuten bei 0 °C lässt man 30 Minuten bei RT rühren, kühlt wieder auf 0 °C ab und gibt 22 µl (0.357 mMol) Methyliodid dazu. Nun wird 96 Stunden bei RT gerührt. Man zieht das Lösungsmittel am Rotationsverdampfer ab und chromatographiert den Rückstand mit Toluol/Ethylacetat (1:1) an Kieselgel. Man erhält die Titelverbindung

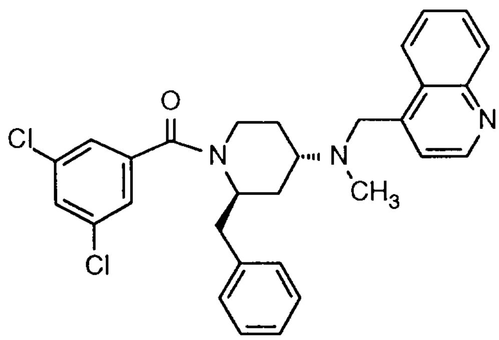

(20 mg, 13%) als weissen Schaum. DC: Toluol/Ethylacetat (1:1) $R_f = 0.45$; FD-MS: $M^+ = 517, 519$.

Beispiel 53: (2R*,4S*)-2-Benzyl-1-(3,5-dichlorbenzoyl)-N-(4-chinolylmethyl)-N-cyclohexylcarbamoyl-4-piperidinamin

Analog Beispiel 16a werden 200 mg (0.396 mMol) (2S*,4R*)-2-Benzyl-1-(3,5-dichlorbenzoyl)-N-(4-chinolylmethyl)-4-piperidinamin mit 66 µl (0.515 mMol) Cyclohexylisocyanat umgesetzt. Man erhält die Titelverbindung

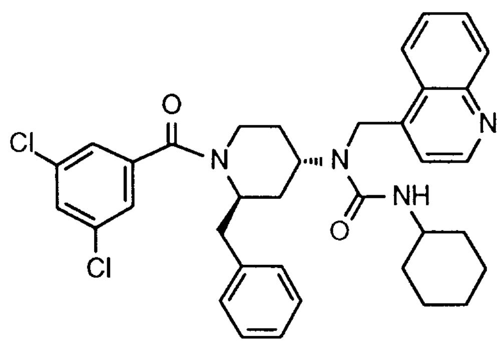

als weisse Kristalle (165 mg, 66%) vom Schmelzpunkt 229 °C (Zersetzung). DC: Methylenchlorid/Methanol/conc. Ammoniak (700:50:1) $R_f = 0.44$, FD-MS: $M^+ = 628, 630$.

Beispiel 54: (2R*,4S*)-2-Benzyl-1-(3,5-dichlorbenzoyl)-N-(4-chinolylmethyl)-N-phenylcarbamoyl-4-piperidinamin

Analog Beispiel 16a werden 200 mg (0.396 mMol) (2S*,4R*)-2-Benzyl-1-(3,5-dichlorbenzoyl)-N-(4-chinolylmethyl)-4-piperidinamin mit 45 mg (0.377 mMol) Phenylisocyanat umgesetzt. Man erhält die Titelverbindung

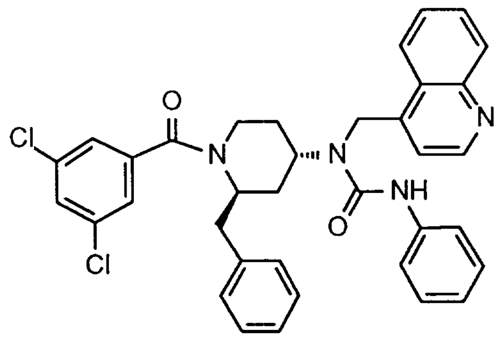

als festen Rückstand (129 mg, 52%). DC: Methylenchlorid/Methanol/conc. Ammoniak (700:50:1) $R_f = 0.42$, FD-MS: $M^+$ = 622, 624.

Beispiel 55: (2R*,4S*)-2-Benzyl-1-(3,5-dichlorbenzoyl)-N-(2-phenylethyl)-4-piperidinamin

Analog Beispiel 2 werden 0.130 g (0.231 mMol) (2R*,4S*)-2-Benzyl-1-(3,5-dichlorbenzoyl)-N-(2-phenylethyl)-N-trifluoracetyl-4-piperidinamin mit 0.035 g (0.923 mMol) Natriumborhydrid umgesetzt. Man erhält die Titelverbindung

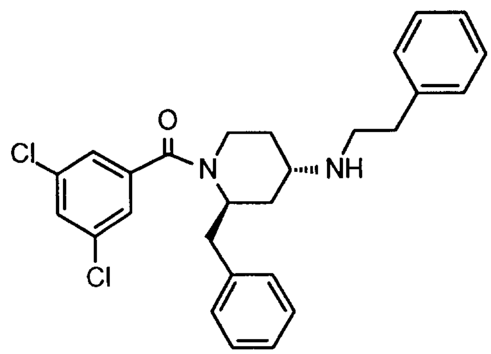

(0.101 g, 94%) als Öl. DC: Methylenchlorid/Methanol/conc. Ammoniak (700:50:1) $R_f = 0.34$, FD-MS: $M^+$ = 466, 468.

Die Ausgangsverbindung dazu wird wie folgt hergestellt:

a) (2R*,4S*)-2-Benzyl-1-*t*-butyloxycarbonyl-N-(2-phenylethyl)-4-piperidinamin

Analog Beispiel 11a werden 1g (3.44 mMol) (2R*,4S*)-2-Benzyl-1-*t*-butyloxycarbonyl-4-piperidinamin mit 1 ml (4.48 mMol) Phenylacetaldehyd, 0.433 g (6.89 mMol) Natriumcyanoborhydrid, 0.791 g (9.64 mMol) Natriumacetat und 434 µl Essigsäure zur Titelverbindung umgesetzt (805 mg, 60%). DC: Methylenchlorid/Methanol/conc. Ammoniak (700:50:1) $R_f = 0.35$, FD-MS: $M^+$ = 394.

b) (2R*,4S*)-2-Benzyl-1-*t*-butyloxycarbonyl-N-(2-phenylethyl)-N-trifluoracetyl-4-piperidinamin

Analog Beispiel 2h werden 618 mg (1.57 mMol) (2R*,4S*)-2-Benzyl-1-*t*-butyloxycarbonyl-N-(4-chinolylmethyl)-4-piperidinamin mit 240 µl (1.72 mMol) Trifluoressigsäureanhydrid und 284 µl (2.04 mMol) Triethylamin umgesetzt. Man erhält die Titelverbindung
als weissen Schaum (572 mg, 75%). DC: Toluol/Ethylacetat (9:1) $R_f = 0.47$, FD-MS: $(M+H)^+$ = 491.

c) (2R*,4S*)-2-Benzyl-N-(2-phenylethyl)-N-trifluoracetyl-4-piperidinamin

Analog Beispiel 38d werden 615 mg (1.25 mMol) (2R*,4S*)-2-Benzyl-1-*t*-butyloxycarbonyl-N-(2-phenylethyl)-N-trifluoracetyl-4-piperidinamin mit 1.9 ml (25 mMol) Trifluoressigsäure umgesetzt. Man erhält die Titelverbindung als Öl (308 mg, 63%). DC: Methylenchlorid/Methanol/conc. Ammoniak (700:50:1) $R_f = 0.63$, FD-MS: $M^+$ = 390.

d) (2R*,4S*)-2-Benzyl-1-(3,5-dichlorbenzoyl)-N-(2-phenylethyl)-N-trifluoracetyl-4-piperidinamin

Analog Beispiel 2a werden 189 mg (0.992 mMol) 3,5-Dichlorbenzoesäure zuerst mit 0.108 ml (1.49 mMol) Thionylchlorid und anschliessend mit 155 mg (0.397 mMol) (2R*,4S*)-2-Benzyl-N-(2-phenylethyl)-N-trifluoracetyl-4-piperidinamin und 166 µl (1.19 mMol) Triethylamin zur Titelverbindung umgesetzt (135 mg, 60%). DC: Methylenchlorid/Methanol/conc. Ammoniak (1000:50:1) $R_f = 0.70$, FD-MS: $M^+$ = 562, 564.

Beispiel 56: (2R*,4S*)-2-Benzyl-1-(3,5-bis-(trifluormethyl)-benzoyl)-N-(2-phenylethyl)-4-piperidinamin

Analog Beispiel 2 werden 0.190 g (0.301 mMol) (2R*,4S*)-2-Benzyl-1-(3,5-bis-(trifluormethyl)-benzoyl)-N-(2-phenylethyl)-N-trifluoracetyl-4-piperidinamin mit 0.046 g (1.21 mMol) Natriumborhydrid umgesetzt. Man erhält die Titelverbindung

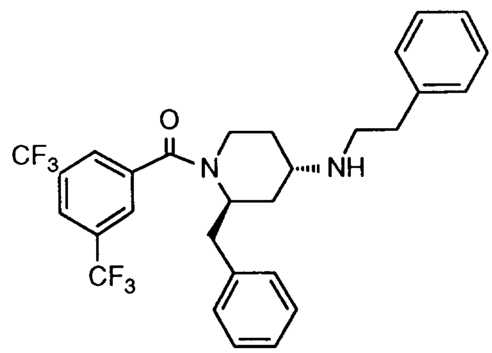

(0.123 g, 76%) als Öl. DC: Methylenchlorid/Methanol/conc. Ammoniak (1000:50:1) $R_f = 0.22$, FD-MS: $M^+$ = 534, IR: 1630 $cm^{-1}$.

Die Ausgangsverbindung dazu wird wie folgt hergestellt:

(2R*,4S*)-2-Benzyl-1-(3,5-bis-(trifluormethyl)-benzoyl)-N-(2-phenylethyl)-N-trifluoracetyl-4-piperidinamin

Analog Beispiel 4a werden 150 mg (0.384 mMol) (2R*,4S*)-2-Benzyl-N-(2-phenylethyl)-N-trifluoracetyl-4-piperidinamin mit 93 mg (0.461 mMol) 3,5-Bis-(trifluormethyl)-benzoesäure, 117 mg (0.461 mMol) Bis-(2-oxo-3-oxazolidinyl)-phosphinsäurechlorid und 118 µl (0.845 mMol) Triethylamin umgesetzt. Man erhält die Titelverbindung (191 mg, 79%) als Öl. DC: Methylenchlorid/Methanol/conc. Ammoniak (2000:50:1) $R_f = 0.79$, FD-MS: $M^+$ = 630.

Beispiel 57: (2R*,4S*)-2-Benzyl-1-(3,5-dichlorbenzoyl)-N-(2-naphthoyl)-4-piperidinamin

Analog Beispiel 2a werden 142 mg (0.825 mMol) 2-Naphthoesäure zuerst mit 2 ml (27 mMol) Thionylchlorid und anschliessend mit 200 mg (0.550 mMol) (2R*,4S*)-2-Benzyl-1-(3,5-dichlorbenzoyl)-4-piperidinamin und 138 µl (0.991 mMol) Triethylamin zur Titelverbindung

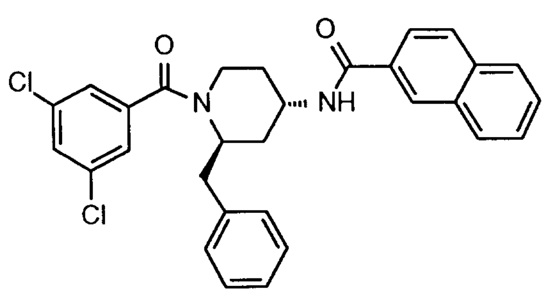

umgesetzt (272 mg, 96%). DC: Methylenchlorid/Methanol/conc. Ammoniak (1000:50:1) $R_f = 0.73$, FD-MS: $(M+H)^+$ = 516, 518, 520, IR: 3420, 1625 $cm^{-1}$.

Die Ausgangsverbindung dazu wird wie folgt hergestellt:

a) (2R*,4S*)-2-Benzyl-4-piperidinazid

Analog Beispiel 38d werden 15g (38.3 mMol) des Gemisches aus Beispiel 2e mit 70 ml Trifluoressigsäure umgesetzt. Man erhält die Titelverbindung (7.15 g, 87%) als Öl. DC: Methylenchlorid/Methanol/conc. Ammoniak (350:50:1) $R_f = 0.57$, FD-MS: $M^+$ = 216.

b) (2R*,4S*)-2-Benzyl-1-(3,5-dichlorbenzoyl)-4-piperidinazid

Analog Beispiel 2a werden 6.62 g (34.7 mMol) 3,5-Dichlorbenzoesäure zuerst mit 3.78 ml (52.0 mMol) Thionylchlorid und anschliessend mit 3.0 g (13.9 mMol) (2R*,4S*)-2-Benzyl-4-piperidinazid und 5.8 ml (41.6 mMol) Triethylamin zur Titelverbindung umgesetzt (5.18 g, 96%). DC: Methylenchlorid/Methanol/conc. Ammoniak (2000:50:1) $R_f = 0.77$, DCI-MS: $(M + H)^+$ = 389, 391.

c) (2R*,4S*)-2-Benzyl-1-(3,5-dichlorbenzoyl)-4-piperidinamin

Analog Beispiel 2f werden 11.0 g (28.3 mMol) (2R*,4S*)-2-Benzyl-1-(3,5-dichlorbenzoyl)-4-piperidinazid mit 10% Pd/C hydriert. Man erhält die Titelverbindung (8.76 g, 85%) als Öl. DC: Methylenchlorid/Methanol/conc. Ammoniak (350:50:1) $R_f = 0.40$, FD-MS: $M^+$ = 362, 364.

Beispiel 58: (2R*,4S*)-2-Benzyl-1-(3,5-dichlorbenzoyl)-N-(3,5-dimethylbenzoyl)-4-piperidinamin

Analog Beispiel 2a werden 124 mg (0.825 mMol) 3,5-Dimethylbenzoesäure zuerst mit 2 ml (27 mMol) Thionylchlorid und anschliessend mit 200 mg (0.550 mMol) (2R*,4S*)-2-Benzyl-1-(3,5-dichlorbenzoyl)-4-piperidinamin und 138 µl (0.991 mMol) Triethylamin zum Produkt

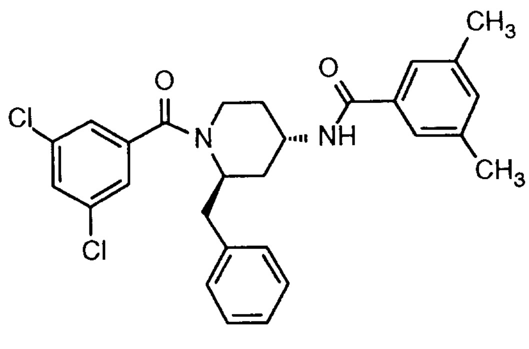

umgesetzt (200 mg, 73%). DC: Methylenchlorid/Methanol/conc. Ammoniak (2000:50:1) $R_f = 0.61$, FD-MS: $(M + H)^+$ = 494, 496; IR: 3420, 1625 $cm^{-1}$

Beispiel 59: (2R*,4S*)-2-Benzyl-1-(3,5-dichlorbenzoyl)-N-(4-chinolylcarbonyl)-4-piperidinamin

Analog Beispiel 4a werden 200 mg (0.550 mMol) (2R*,4S*)-2-Benzyl-1-(3,5-dichlorbenzoyl)-4-piperidinamin mit 105 mg (0.660 mMol) Chinolin-4-carbonsäure, 168 mg (0.661 mMol) Bis-(2-oxo-3-oxazolidinyl)-phosphinsäurechlorid und 169 µl (1.21 mMol) Triethylamin umgesetzt. Man erhält die Titelverbindung

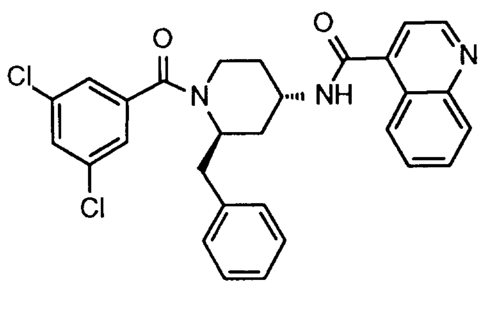

(278 mg, 97%) als Öl. DC: Methylenchlorid/Methanol/conc. Ammoniak (700:50:1) $R_f = 0.45$, FD-MS: $M^+ =$ 517, 519; IR: 3395, 1755, 1620 $cm^{-1}$.

Beispiel 60: (2R*,4S*)-2-Benzyl-1-(3,5-dichlorbenzoyl)-N-(3-indolylcarbonyl)-4-piperidinamin

Analog Beispiel 4a werden 200 mg (0.550 mMol) (2R*,4S*)-2-Benzyl-1-(3,5-dichlorbenzoyl)-4-piperidinamin mit 106 mg (0.661 mMol) Indol-3-carbonsäure, 168 mg (0.661 mMol) Bis-(2-oxo-3-oxazolidinyl)-phosphinsäurechlorid und 169 µl (1.21 mMol) Triethylamin umgesetzt. Man erhält die Titelverbindung

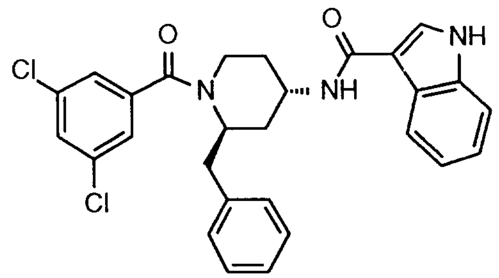

(92 mg, 33%) als Öl. DC: Methylenchlorid/Methanol/conc. Ammoniak (350:50:1) $R_f = 0.61$, FD-MS: $M^+ =$ 505, 507; IR: 3450, 3260, 1770, 1635 $cm^{-1}$.

Beispiel 61: (2R*,4S*)-2-Benzyl-1-(3,5-dichlorbenzoyl)-N-(2-indolylcarbonyl)-4-piperidinamin

Analog Beispiel 4a werden 200 mg (0.550 mMol) (2R*,4S*)-2-Benzyl-1-(3,5-dichlorbenzoyl)-4-piperidinamin mit 106 mg (0.661 mMol) Indol-2-carbonsäure, 168 mg (0.661 mMol) Bis-(2-oxo-3-oxazolidinyl)-phosphinsäurechlorid und 169 µl (1.21 mMol) Triethylamin umgesetzt. Man erhält die Titelverbindung

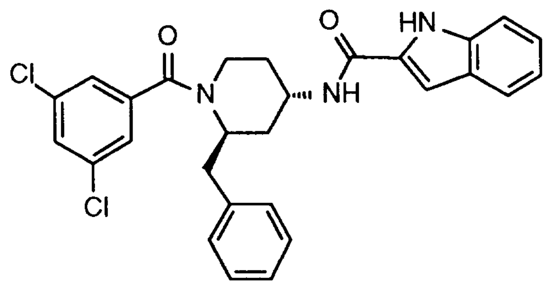

(89 mg, 32%) als weisse Kristalle vom Schmelzpunkt 254 °C. DC: Methylenchlorid/Methanol/conc. Ammoniak (2000:50:1) $R_f = 0.56$, FD-MS: $M^+ =$ 505, 507; IR: 3430, 3290, 1625 $cm^{-1}$.

Beispiel 62: (2R*,4S*)-2-Benzyl-1-(3,5-dichlorbenzoyl)-N-(5-methoxy-2-indolylcarbonyl)-4-piperidinamin

Analog Beispiel 4a werden 200 mg (0.550 mMol) (2R*,4S*)-2-Benzyl-1-(3,5-dichlorbenzoyl)-4-piperidinamin mit 126 mg (0.661 mMol) 5-Methoxy-indol-2-carbonsäure, 168 mg (0.661 mMol) Bis-(2-oxo-3-oxazolidinyl)-phosphinsäurechlorid und 169 µl (1.21 mMol) Triethylamin umgesetzt. Man erhält die Titelverbindung

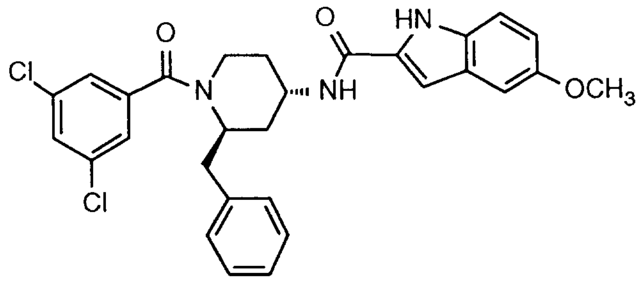

(118 mg, 41%) als weisse Kristalle vom Schmelzpunkt 251 °C. DC: Methylenchlorid/Methanol/conc. Ammoniak (2000:50:1) $R_f = 0.81$, FD-MS: $M^+ =$ 535, 537; IR: 3440, 3280, 1625 $cm^{-1}$.

Beispiel 63: (2R*,4S*)-2-Benzyl-1-(3,5-dichlorbenzoyl)-N-(1-naphthoyl)-4-piperidinamin

Analog Beispiel 39a werden 150 mg (0.431 mMol) (2R*,4S*)-2-Benzyl-1-(3,5-dichlorbenzoyl)-4-piperidinamin mit 75 µl (0.495 mMol) 1-Naphthoylchlorid und 81 µl (0.578 mMol) Triethylamin umgesetzt. Man erhält die Titelverbindung

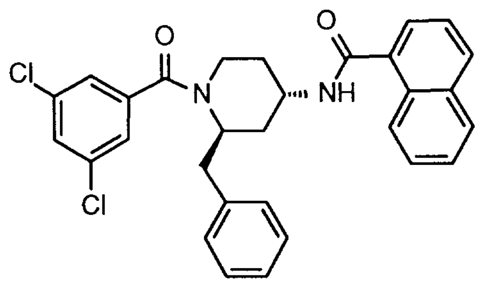

(208 mg, 97%) als Öl. DC: Methylenchlorid/Methanol/conc. Ammoniak (1000:50:1) $R_f = 0.85$, FD-MS: $M^+ =$ 516, 518; IR: 3680, 3400, 1620 $cm^{-1}$.

Beispiel 64: (2R*,4S*)-2-Benzyl-1-(3,5-dichlorbenzoyl)-N-(phenylacetyl)-4-piperidinamin

Analog Beispiel 39a werden 200 mg (0.551 mMol) (2R*,4S*)-2-Benzyl-1-(3,5-dichlorbenzoyl)-4-piperidinamin mit 88 µl (0.661 mMol) Phenylacetylchlorid und 108 µl (0.771 mMol) Triethylamin umgesetzt. Man erhält die Titelverbindung

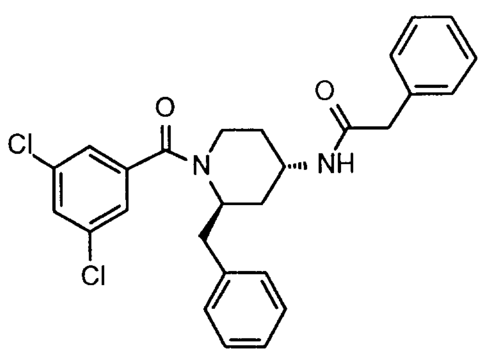

(127 mg, 48%) als weissen Schaum. DC: Methylenchlorid/Methanol/conc. Ammoniak (1000:50:1) $R_f = 0.56$, FD-MS: $M^+ =$ 480, 482; IR: 3660, 3405, 1665, 1630 $cm^{-1}$.

Beispiel 65: (2R*,4S*)-2-Benzyl-1-(3,5-dichlorbenzoyl)-N-(2-methoxybenzyl)-4-piperidinamin

Analog Beispiel 2g werden 200 mg (0.551 mMol) (2R*,4S*)-2-Benzyl-1-(3,5-dichlorbenzoyl)-4-piperidinamin mit 75 mg (0.551 mMol) 2-Methoxybenzaldehyd und 90 mg Magnesiumsulfat in 2 ml Toluol umgesetzt und anschliessend mit 22 mg (0.584 mMol) Natriumborhydrid in 2 ml Methanol reduziert. Man erhält die Titelverbindung

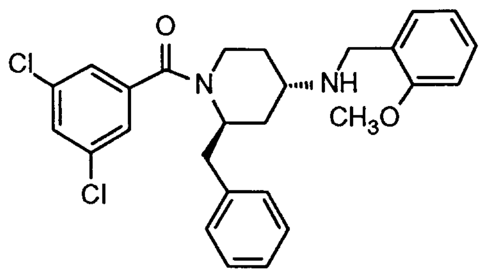

(170 mg, 64%) als weissen Schaum. DC: Methylenchlorid/Methanol/conc. Ammoniak (700:50:1) $R_f = 0.66$, FD-MS: $M^+$ = 482, 482. IR: 1620 $cm^{-1}$.

Beispiel 66: (2R*,4S*)-2-Benzyl-1-(3,5-dichlorbenzoyl)-N-(3-(N-acetyl)-indolylmethyl)-4-piperidinamin

Analog Beispiel 2g werden 200 mg (0.551 mMol) (2R*,4S*)-2-Benzyl-1-(3,5-dichlorbenzoyl)-4-piperidinamin mit 113 mg (0.606 mMol) N-Acetylindol-3-carboxaldehyd und 90 mg Magnesiumsulfat in 2 ml Toluol umgesetzt und anschliessend mit 31 mg (0.826 mMol) Natriumborhydrid in 3 ml Methanol reduziert. Man erhält die Titelverbindung

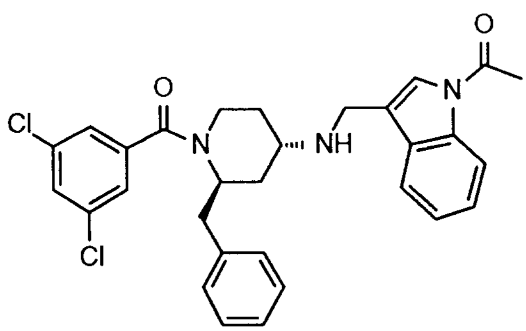

(30 mg, 10%) als Öl. DC: Methylenchlorid/Methanol/conc. Ammoniak (1000:50:1) $R_f = 0.57$, FD-MS: $M^+$ = 533, 535. IR: 1720, 1680, 1635 $cm^{-1}$.

Beispiel 67: (2R*,4S*)-2-Benzyl-1-(3,5-dichlorbenzoyl)-N-(2-benzo[b]furanylmethyl)-4-piperidinamin

Analog Beispiel 2g werden 200 mg (0.551 mMol) (2R*,4S*)-2-Benzyl-1-(3,5-dichlorbenzoyl)-4-piperidinamin mit 97 mg (0.661 mMol) Benzofuran-2-carboxaldehyd und 90 mg Magnesiumsulfat in 2 ml Toluol umgesetzt und anschliessend mit 22 mg (0.584 mMol) Natriumborhydrid in 2 ml Methanol reduziert. Man erhält die Titelverbindung

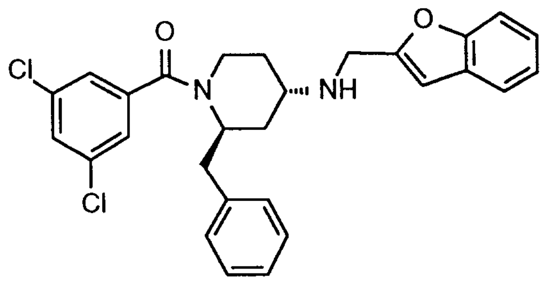

(150 mg, 55%) als Öl. DC: Methylenchlorid/Methanol/conc. Ammoniak (2000:50:1) $R_f = 0.18$, FD-MS: $M^+ =$ 492, 494. IR: 1630 $cm^{-1}$.

Beispiel 68: (2R*,4S*)-2-Benzyl-1-(3,5-dichlorbenzoyl)-N-[(3-methylbenzo[b]thiophen-2-ylmethyl]-4-piperidinamin

Analog Beispiel 2g werden 200 mg (0.551 mMol) (2R*,4S*)-2-Benzyl-1-(3,5-dichlorbenzoyl)-4-piperidinamin mit 116 mg (0.661 mMol) 3-Methylbenzo[b]thiophen-2-carboxaldehyd und 90 mg Magnesiumsulfat in 2 ml Toluol umgesetzt und anschliessend mit 22 mg (0.584 mMol) Natriumborhydrid in 2 ml Methanol reduziert. Man erhält die Titelverbindung

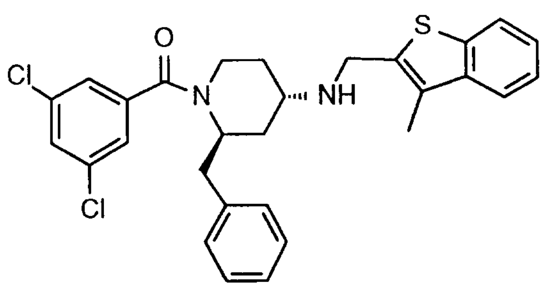

(75 mg, 25%) als weissen Schaum. DC: Methylenchlorid/Methanol/conc. Ammoniak (2000:50:1) $R_f = 0.38$, FD-MS: $M^+ =$ 522, 524. IR: 1630 $cm^{-1}$.

Beispiel 69: (2R*,4S*)-2-Benzyl-1-(3,5-dichlorbenzoyl)-N-(5-methoxyindol-3-ylmethyl)-4-piperidinamin

Analog Beispiel 2g werden 200 mg (0.551 mMol) (2R*,4S*)-2-Benzyl-1-(3,5-dichlorbenzoyl)-4-piperidinamin mit 116 mg (0.661 mMol) 5-Methoxyindol-3-carboxaldehyd und 90 mg Magnesiumsulfat in 2 ml Toluol umgesetzt und anschliessend mit 22 mg (0.584 mMol) Natriumborhydrid in 2 ml Methanol reduziert. Man erhält die Titelverbindung

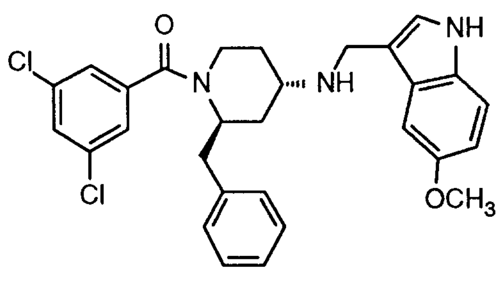

(98 mg, 34%) als weissen Schaum. DC: Methylenchlorid/Methanol/conc. Ammoniak (1000:50:1) $R_f = 0.42$, FD-MS: $M^+ =$ 521, 523. IR: 3460, 1630 $cm^{-1}$.

Beispiel 70: (2R*,4S*)-2-Benzyl-1-(3,5-dichlorbenzoyl)-N-(3-indolylmethyl)-4-piperidinamin

Analog Beispiel 2g werden 200 mg (0.551 mMol) (2R*,4S*)-2-Benzyl-1-(3,5-dichlorbenzoyl)-4-piperidinamin mit 80 mg (0.551 mMol) Indol-3-carboxaldehyd und 90 mg Magnesiumsulfat in 2 ml Toluol umgesetzt und anschliessend mit 22 mg (0.584 mMol) Natriumborhydrid in 2 ml Methanol reduziert. Man erhält die Titelverbindung

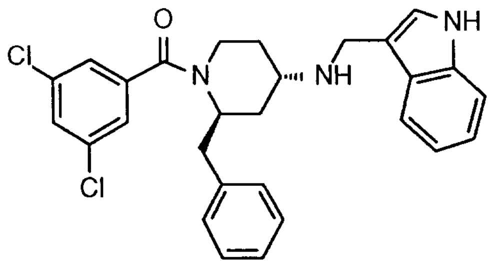

(75 mg, 28%) als weissen Schaum. DC: Methylenchlorid/Methanol/conc. Ammoniak (400:50:1) $R_f = 0.49$, FD-MS: $M^+ =$ 491, 493. IR: 3460, 1630 $cm^{-1}$.

Beispiel 71: (2R*,4S*)-2-Benzyl-1-(3,5-dichlorbenzoyl)-N-phenylcarbamoyl-4-piperidinamin

Analog Beispiel 16a werden 200 mg (0.551 mMol) (2R*,4S*)-2-Benzyl-1-(3,5-dichlorbenzoyl)-4-piperidinamin mit 85 mg (0.716 mMol) Phenylisocyanat umgesetzt. Man erhält die Titelverbindung

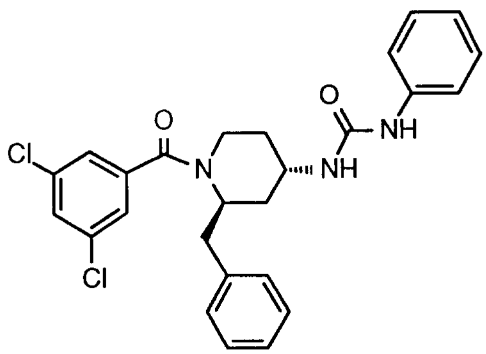

als weissen Schaum (160 mg, 60%). DC: Toluol/Ethylacetat (1:1) $R_f = 0.40$, FD-MS: $M^+ =$ 481, 483; IR: 1600 - 1690 $cm^{-1}$.

Beispiel 72: (2R*,4S*)-2-Benzyl-1-(3,5-dichlorbenzoyl)-N-diphenylmethyl-4-piperidinamin

Die Lösung von 200 mg (0.551 mMol) (2R*,4S*)-2-Benzyl-1-(3,5-dichlorbenzoyl)-4-piperidinamin und 110 mg (0.606 mMol) Benzophenon in 5 ml Toluol wird 18 Stunden am Rückfluss gehalten. Danach engt man am Rotationsverdampfer ein, löst das Reaktionsgemisch in 3 ml Methanol und fügt bei RT 69 mg (1.10 mMol) Natriumcyanoborhydrid dazu. Mit 80 µl Essigsäure wird das Reaktionsgemisch auf pH = 5 gestellt und 68 Stunden bei RT gerührt. Man erhält die Titelverbindung

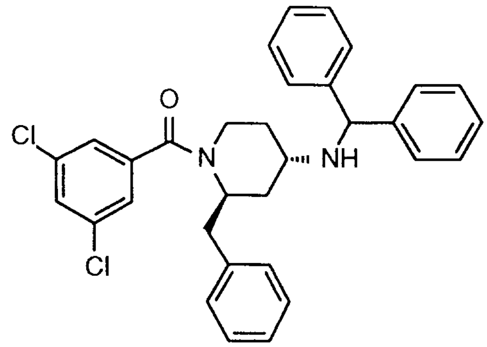

als weissen Schaum (120 mg, 41%). DC: Toluol/Ethylacetat (7:3) $R_f = 0.79$, FD-MS: $M^+ =$ 528, 530; IR: 1630 $cm^{-1}$.

Beispiel 73: (2R*,4S*)-2-Benzyl-1-(3,5-dichlorbenzoyl)-N-(3,4-dihydro-2H-1-benzopyran-2-carbonyl)-4-piperidinamin

Zur Lösung von 108 mg (0.606 mMol) 3,4-Dihydro-2H-1-benzopyran-2-carbonsäure in 3 ml Tetrahydrofuran gibt man bei 0 °C 125 mg (0.606 mMol) N,N'-Dicyclohexylcarbodiimid, rührt das Reaktionsgemisch eine Stunde und fügt 177 µl (1.27 mMol) Triethylamin und 200 mg (0.551 mMol) (2R*,4S*)-2-Benzyl-1-(3,5-dichlorbenzoyl)-4-piperidinamin dazu. Man lässt auf RT erwärmen und rührt 16 Stunden bei dieser Temperatur. Man engt am Rotationsverdampfer ein, schlemmt den Rückstand in Methylenchlorid/Ether (1:1) auf und filtriert die weisse Suspension ab. Nach Einengen am Rotationsverdampfer wird das Reaktionsgemisch in Methylenchlorid aufgenommen, zweimal mit 10% Zitronensäure, einmal mit Wasser, einmal mit 2N Natronlauge und einmal mit Sole gewaschen, über Magnesiumsulfat getrocknet und am Rotationsverdampfer eingedampft. Das gelbe Öl wird mit Toluol/Ethylacetat (7:3) an Kieselgel chromatographiert. Man erhält das Diastereomerengemisch der Titelverbindung

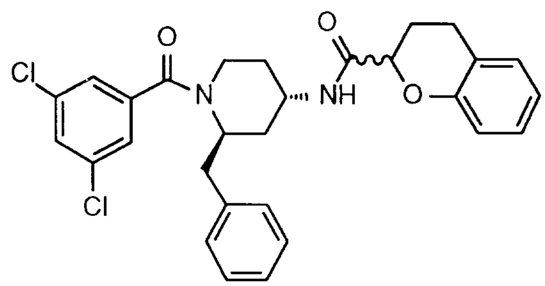

als weissen Schaum (69 mg, 24%). DC: Toluol/Ethylacetat (1:1) $R_f = 0.56$, FD-MS: $M^+ =$ 522, 524; IR: 3410, 1675, 1630 $cm^{-1}$.

Beispiel 74: (2R*,4S*)-2-Benzyl-1-(4-methoxybenzoyl)-N-(4-chinolylmethyl)-4-piperidinamin

Analog Beispiel 2 werden 0.175 g (0.321 mMol) (2R*,4S*)-2-Benzyl-1-(4-methoxybenzoyl)-N-(4-chinolylmethyl)-N-trifluoracetyl-4-piperidinamin mit 0.047 g (1.25 mMol) Natriumborhydrid umgesetzt. Man erhält die Titelverbindung

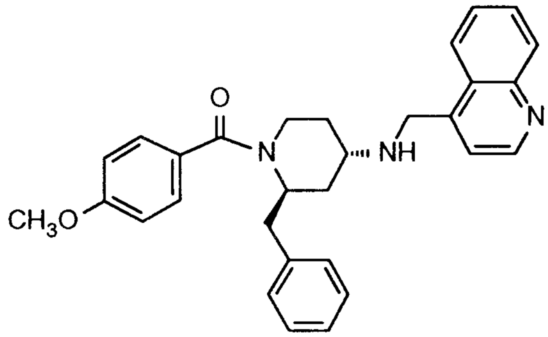

(0.100 g, 69%) als weissen Schaum. DC: Methylenchlorid/Methanol/conc. Ammoniak (1000:50:1) $R_f = 0.21$, FD-MS: $M^+ = 465$; IR: 1620 $cm^{-1}$.

Die Ausgangsverbindung dazu wird wie folgt hergestellt:

(2R*,4S*)-2-Benzyl-1-(4-methoxybenzoyl)-N-(4-chinolylmethyl)-N-trifluoracetyl-4-piperidinamin

Analog Beispiel 4a werden 200 mg (0.467 mMol) (2R*,4S*)-2-Benzyl-N-(4-chinolylmethyl)-N-trifluoracetyl-1-piperidinamin mit 85 mg (0.562 mMol) p-Anissäure, 143 mg (0.561 mMol) Bis-(2-oxo-3-oxazolidinyl)-phosphinsäurechlorid und 91 µl (0.655 mMol) Triethylamin umgesetzt. Man erhält die Titelverbindung (170 mg. 65%) als weissen Schaum. DC: Methylenchlorid/Methanol/conc. Ammoniak (1000:50:1) $R_f = 0.37$, FD-MS: $M^+ = 561$.

Beispiel 75:

In analoger Weise wie in den Beispielen 1 bis 74 beschrieben kann man ferner herstellen:
(2R*,4S*)-2-Benzyl-1-(3,5-dimethylbenzoyl)-N-benzyl-N-carbamoyl-4-piperidinamin;
(2R,4S)-2-Benzyl-1-(3,5-dimethylbenzoyl)-N-(3-phenylpropyl)4-piperidinamin;
(2S,4S)-2-Benzyl-1-(3,5-dimethylbenzoyl)-N-(3-phenylpropyl)-4-piperidinamin;
(2R,4S)-2-Benzyl-1-(3,5-dimethylbenzoyl)-N-(2-methoxybenzyl)-4-piperidinamin;
(2S,4S)-2-Benzyl-1-(3,5-dimethylbenzoyl)-N-(2-methoxybenzyl)-4-piperidinamin;
(2R,4S)-2-Benzyl-1-(3,5-dimethylbenzoyl)-N-[2-(2-methoxyphenyl)ethyl]-4-piperidinamin;
(2S,4S)-2-Benzyl-1-(3,5-dimethylbenzoyl)-N-[2-(2-methoxyphenyl)ethyl]-4-piperidinamin;
(2R,4S)-2-Benzyl-1-(3,5-dimethylbenzoyl)-N-[3-(2-methoxyphenyl)propyl]-4-piperidinamin;
(2S,4S)-2-Benzyl-1-(3,5-dimethylbenzoyl)-N-[3-(2-methoxyphenyl)propyl]-4-piperidinamin;
(2R,4S)-2-Benzyl-1-(3,5-dimethylbenzoyl)-N-(2-trifluormethylbenzyl)-4-piperidinamin;
(2S,4S)-2-Benzyl-1-(3,5-dimethylbenzoyl)-N-(2-trifluormethylbenzyl)-4-piperidinamin;
(2R,4S)-2-Benzyl-1-(3,5-dimethylbenzoyl)-N-[2-(2-trifluormethylphenyl)ethyl]-4-piperidinamin;
(2S,4S)-2-Benzyl-1-(3,5-dimethylbenzoyl)-N-[2-(2-trifluormethylphenyl)ethyl]-4-piperidinamin;
(2R,4S)-2-Benzyl-1-(3,5-dimethylbenzoyl)-N-[3-(2-trifluormethylphenyl)propyl]-4-piperidinamin;
(2S,4S)-2-Benzyl-1-(3,5-dimethylbenzoyl)-N-[3-(2-trifluormethylphenyl)propyl]-4-piperidinamin;
(2R,4S)-2-Benzyl-1-(3,5-dimethylbenzoyl)-N-(3-trifluormethylbenzyl)-4-piperidinamin;
(2S,4S)-2-Benzyl-1-(3,5-dimethylbenzoyl)-N-(3-trifluormethylbenzyl)-4-piperidinamin;
(2R,4S)-2-Benzyl-1-(3,5-dimethylbenzoyl)-N-[2-(3-trifluormethylphenyl)ethyl]-4-piperidinamin;
(2S,4S)-2-Benzyl-1-(3,5-dimethylbenzoyl)-N-[2-(3-trifluormethylphenyl)ethyl]-4-piperidinamin;
(2R,4S)-2-Benzyl-1-(3,5-dimethylbenzoyl)-N-[3-(3-trifluormethylphenyl)propyl]-4-piperidinamin;
(2S,4S)-2-Benzyl-1-(3,5-dimethylbenzoyl)-N-[3-(3-trifluormethylphenyl)propyl]-4-piperidinamin;
(2R,4S)-2-Benzyl-1-(3,5-dimethylbenzoyl)-N-(4-trifluormethylbenzyl)-4-piperidinamin;
(2S,4S)-2-Benzyl-1-(3,5-dimethylbenzoyl)-N-(4-trifluormethylbenzyl)4-piperidinamin;
(2R,4S)-2-Benzyl-1-(3,5-dimethylbenzoyl)-N-[2-(4-trifluormethylphenyl)ethyl]-4-piperidinamin;
(2S,4S)-2-Benzyl-1-(3,5-dimethylbenzoyl)-N-[2-(4-trifluormethylphenyl)ethyl]-4-piperidinamin;
(2R,4S)-2-Benzyl-1-(3,5-dimethylbenzoyl)-N-[3-(4-trifluormethylphenyl)propyl]-4-piperidinamin;
(2S,4S)-2-Benzyl-1-(3,5-dimethylbenzoyl)-N-[3-(4-trifluormethylphenyl)propyl]-4-piperidinamin;
(2R,4S)-2-Benzyl-1-(3,5-dimethylbenzoyl)-N-(2,3-dimethoxybenzyl)-4-piperidinamin;
(2S,4S)-2-Benzyl-1-(3,5-dimethylbenzoyl)-N-(2,3-dimethoxybenzyl)-4-piperidinamin;
(2R,4S)-2-Benzyl-1-(3,5-dimethylbenzoyl)-N-[2-(2,3-dimethoxyphenyl)ethyl]-4-piperidinamin;

(2S,4S)-2-Benzyl-1-(3,5-dimethylbenzoyl)-N-[2-(2,3-dimethoxyphenyl)ethyl]-4-piperidinamin;
(2R,4S)-2-Benzyl-1-(3,5-dimethylbenzoyl)-N-[3-(2,3-dimethoxyphenyl)propyl]-4-piperidinamin;
(2S,4S)-2-Benzyl-1-(3,5-dimethylbenzoyl)-N-[3-(2,3-dimethoxyphenyl)propyl]-4-piperidinamin;
(2R,4S)-2-Benzyl-1-(3,5-dimethylbenzoyl)-N-(2,4-dimethoxybenzyl)-4-piperidinamin;
(2S,4S)-2-Benzyl-1-(3,5-dimethylbenzoyl)-N-(2,4-dimethoxybenzyl)-4-piperidinamin;
(2R,4S)-2-Benzyl-1-(3,5-dimethylbenzoyl)-N-[2-(2,4-dimethoxyphenyl)ethyl]-4-piperidinamin;
(2S,4S)-2-Benzyl-1-(3,5-dimethylbenzoyl)-N-[2-(2,4-dimethoxyphenyl)ethyl]-4-piperidinamin;
(2R,4S)-2-Benzyl-1-(3,5-dimethylbenzoyl)-N-[3-(2,4-dimethoxyphenyl)propyl]-4-piperidinamin;
(2S,4S)-2-Benzyl-1-(3,5-dimethylbenzoyl)-N-[3-(2,4-dimethoxyphenyl)propyl]-4-piperidinamin;
(2R,4S)-2-Benzyl-1-(3,5-dimethylbenzoyl)-N-(2,5-dimethoxybenzyl)-4-piperidinamin;
(2S,4S)-2-Benzyl-1-(3,5-dimethylbenzoyl)-N-(2,5-dimethoxybenzyl)-4-piperidinamin;
(2R,4S)-2-Benzyl-1-(3,5-dimethylbenzoyl)-N-[2-(2,5-dimethoxyphenyl)ethyl]-4-piperidinamin;
(2S,4S)-2-Benzyl-1-(3,5-dimethylbenzoyl)-N-[2-(2,5-dimethoxyphenyl)ethyl]-4-piperidinamin;
(2R,4S)-2-Benzyl-1-(3,5-dimethylbenzoyl)-N-[3-(2,5-dimethoxyphenyl)propyl]-4-piperidinamin;
(2S,4S)-2-Benzyl-1-(3,5-dimethylbenzoyl)-N-[3-(2,5-dimethoxyphenyl)propyl]4-piperidinamin;
(2R,4S)-2-Benzyl-1-(3,5-dimethylbenzoyl)-N-(2,6-dimethoxybenzyl)-4-piperidinamin;
(2S,4S)-2-Benzyl-1-(3,5-dimethylbenzoyl)-N-(2,6-dimethoxybenzyl)-4-piperidinamin;
(2R,4S)-2-Benzyl-1-(3,5-dimethylbenzoyl)-N-[2-(2,6-dimethoxyphenyl)ethyl]-4-piperidinamin;
(2S,4S)-2-Benzyl-1-(3,5-dimethylbenzoyl)-N-[2-(2,6-dimethoxyphenyl)ethyl]-4-piperidinamin;
(2R,4S)-2-Benzyl-1-(3,5-dimethylbenzoyl)-N-[3-(2,6-dimethoxyphenyl)propyl]-4-piperidinamin;
(2S,4S)-2-Benzyl-1-(3,5-dimethylbenzoyl)-N-[3-(2,6-dimethoxyphenyl)propyl]-4-piperidinamin;
(2R,4S)-2-Benzyl-1-(3,5-dimethylbenzoyl)-N-(2,3-methylendioxybenzyl)-4-piperidinamin;
(2S,4S)-2-Benzyl-1-(3,5-dimethylbenzoyl)-N-(2,3-methylendioxybenzyl)-4-piperidinamin;
(2R,4S)-2-Benzyl-1-(3,5-dimethylbenzoyl)-N-[2-(2,3-methylendioxyphenyl)ethyl]-4-piperidinamin;
(2S,4S)-2-Benzyl-1-(3,5-dimethylbenzoyl)-N-[2-(2,3-methylendioxyphenyl)ethyl]-4-piperidinamin;
(2R,4S)-2-Benzyl-1-(3,5-dimethylbenzoyl)-N-[3-(2,3-methylendioxyphenyl)propyl]-4-piperidinamin;
(2S,4S)-2-Benzyl-1-(3,5-dimethylbenzoyl)-N-[3-(2,3-methylendioxyphenyl)propyl]-4-piperidinamin;
(2R,4S)-2-Benzyl-1-(3,5-dimethylbenzoyl)-N-(2,4-methylendioxybenzyl)-4-piperidinamin;
(2S,4S)-2-Benzyl-1-(3,5-dimethylbenzoyl)-N-(2,4-methylendioxybenzyl)-4-piperidinamin;
(2R,4S)-2-Benzyl-1-(3,5-dimethylbenzoyl)-N-[2-(2,4-methylendioxyphenyl)ethyl]-4-piperidinamin;
(2S,4S)-2-Benzyl-1-(3,5-dimethylbenzoyl)-N-[2-(2,4-methylendioxyphenyl)ethyl]-4-piperidinamin;
(2R,4S)-2-Benzyl-1-(3,5-dimethylbenzoyl)-N-[3-(2,4-methylendioxyphenyl)propyl]-4-piperidinamin;
(2S,4S)-2-Benzyl-1-(3,5-dimethylbenzoyl)-N-[3-(2,4-methylendioxyphenyl)propyl]-4-piperidinamin;
(2R,4S)-2-Benzyl-1-(3,5-dimethylbenzoyl)-N-(indol-2-ylmethyl)-4-piperidinamin;
(2S,4S)-2-Benzyl-1-(3,5-dimethylbenzoyl)-N-(indol-2-ylmethyl)-4-piperidinamin;
(2R,4S)-2-Benzyl-1-(3,5-dimethylbenzoyl)-N-(indol-3-ylmethyl)-4-piperidinamin;
(2S,4S)-2-Benzyl-1-(3,5-dimethylbenzoyl)-N-(indol-3-ylmethyl)-4-piperidinamin;
(2S,4S)-2-Benzyl-1-(3,5-dimethylbenzoyl)-N-(chinolin-2-ylmethyl)-4-piperidinamin;
(2S,4S)-2-Benzyl-1-(3,5-dimethylbenzoyl)-N-(chinolin-3-ylmethyl)-4-piperidinamin;
(2R,4S)-2-Benzyl-1-(3,5-dimethylbenzoyl)-N-(2-chlorbenzyl)-4-piperidinamin;
(2S,4S)-2-Benzyl-1-(3,5-dimethylbenzoyl)-N-(2-chlorbenzyl)-4-piperidinamin;
(2R,4S)-2-Benzyl-1-(3,5-dimethylbenzoyl)-N-[2-(2-chlorphenyl)ethyl]-4-piperidinamin;
(2S,4S)-2-Benzyl-1-(3,5-dimethylbenzoyl)-N-[2-(2-chlorphenyl)ethyl]-4-piperidinamin;
(2R,4S)-2-Benzyl-1-(3,5-dimethylbenzoyl)-N-[3-(2-chlorphenyl)propyl]-4-piperidinamin;
(2S,4S)-2-Benzyl-1-(3,5-dimethylbenzoyl)-N-[3-(2-chlorphenyl)propyl]-4-piperidinamin;
(2R,4S)-2-Benzyl-1-(3,5-dimethylbenzoyl)-N-(3-chlorbenzyl)-4-piperidinamin;
(2S,4S)-2-Benzyl-1-(3,5-dimethylbenzoyl)-N-(3-chlorbenzyl)-4-piperidinamin;
(2R,4S)-2-Benzyl-1-(3,5-dimethylbenzoyl)-N-[2-(3-chlorphenyl)ethyl]-4-piperidinamin;
(2S,4S)-2-Benzyl-1-(3,5-dimethylbenzoyl)-N-[2-(3-chlorphenyl)ethyl]-4-piperidinamin;
(2R,4S)-2-Benzyl-1-(3,5-dimethylbenzoyl)-N-[3-(3-chlorphenyl)propyl]-4-piperidinamin;
(2S,4S)-2-Benzyl-1-(3,5-dimethylbenzoyl)-N-[3-(3-chlorphenyl)propyl]-4-piperidinamin;
(2R,4S)-2-Benzyl-1-(3,5-dimethylbenzoyl)-N-(4-chlorbenzyl)-4-piperidinamin;
(2S,4S)-2-Benzyl-1-(3,5-dimethylbenzoyl)-N-(4-chlorbenzyl)-4-piperidinamin;
(2R,4S)-2-Benzyl-1-(3,5-dimethylbenzoyl)-N-[2-(4-chlorphenyl)ethyl]-4-piperidinamin;
(2S,4S)-2-Benzyl-1-(3,5-dimethylbenzoyl)-N-[2-(4-chlorphenyl)ethyl]-4-piperidinamin;
(2R,4S)-2-Benzyl-1-(3,5-dimethylbenzoyl)-N-[3-(4-chlorphenyl)propyl]-4-piperidinamin;
(2S,4S)-2-Benzyl-1-(3,5-dimethylbenzoyl)-N-[3-(4-chlorphenyl)propyl]-4-piperidinamin;
(2R,4S)-2-Benzyl-1-(3,5-dimethylbenzoyl)-N-(4-methoxynaphth-1-ylmethyl)-4-piperidinamin;

(2S,4S)-2-Benzyl-1-(3,5-dimethylbenzoyl)-N-(4-methoxynaphth-1-ylmethyl)-4-piperidinamin;
(2R,4S)-2-Benzyl-1-(3,5-dimethylbenzoyl)-N-(3,4-ethylendioxybenzyl)-4-piperidinamin;
(2S,4S)-2-Benzyl-1-(3,5-dimethylbenzoyl)-N-(3,4-ethylendioxybenzyl)-4-piperidinamin;
(2R,4S)-2-Benzyl-1-(3,5-dimethylbenzoyl)-N-(chinolin-2-ylmethyl)-4-piperidinamin;
(2R,4S)-2-Benzyl-1-(3,5-dimethylbenzoyl)-N-(chinolin-3-ylmethyl)-4-piperidinamin;
(2R,4S)-2-Benzyl-1-(3,5-ditrifluormethylbenzoyl)-N-(indol-2-ylmethyl)-4-piperidinamin;
(2S,4S)-2-Benzyl-1-(3,5-ditrifluormethylbenzoyl)-N-(indol-2-ylmethyl)-4-piperidinamin;
(2R,4S)-2-Benzyl-1-(3,5-ditrifluormethylbenzoyl)-N-(indol-3-ylmethyl)-4-piperidinamin;
(2S,4S)-2-Benzyl-1-(3,5-ditrifluormethylbenzoyl)-N-(indol-3-ylmethyl)-4-piperidinamin;
(2R,4S)-2-Benzyl-1-(3,5-ditrifluormethylbenzoyl)-N-(chinolin-2-ylmethyl)-4-piperidinamin;
(2S,4S)-2-Benzyl-1-(3,5-ditrifluormethylbenzoyl)-N-(chinolin-2-ylmethyl)-4-piperidinamin;
(2R,4S)-2-Benzyl-1-(3,5-ditrifluormethylbenzoyl)-N-(chinolin-3-ylmethyl)-4-piperidinamin;
(2S,4S)-2-Benzyl-1-(3,5-ditrifluormethylbenzoyl)-N-(chinolin-3-ylmethyl)-4-piperidinamin;
(2R,4S)-2-Benzyl-1-(3,5-ditrifluormethylbenzoyl)-N-(2-chlorbenzyl)-4-piperidinamin;
(2S,4S)-2-Benzyl-1-(3,5-ditrifluormethylbenzoyl)-N-(2-chlorbenzyl)-4-piperidinamin;
(2R,4S)-2-Benzyl-1-(3,5-ditrifluormethylbenzoyl)-N-[2-(2-chlorphenyl)ethyl]-4-piperidinamin;
(2S,4S)-2-Benzyl-1-(3,5-ditrifluormethylbenzoyl)-N-[2-(2-chlorphenyl)ethyl]-4-piperidinamin;
(2R,4S)-2-Benzyl-1-(3,5-ditrifluormethylbenzoyl)-N-[3-(2-chlorphenyl)propyl]-4-piperidinamin;
(2S,4S)-2-Benzyl-1-(3,5-ditrifluormethylbenzoyl)-N-[3-(2-chlorphenyl)propyl]-4-piperidinamin;
(2R,4S)-2-Benzyl-1-(3,5-ditrifluormethylbenzoyl)-N-(3-chlorbenzyl)-4-piperidinamin;
(2S,4S)-2-Benzyl-1-(3,5-ditrifluormethylbenzoyl)-N-(3-chlorbenzyl)-4-piperidinamin;
(2R,4S)-2-Benzyl-1-(3,5-ditrifluormethylbenzoyl)-N-[2-(3-chlorphenyl)ethyl]-4-piperidinamin;
(2S,4S)-2-Benzyl-1-(3,5-ditrifluormethylbenzoyl)-N-[2-(3-chlorphenyl)ethyl]-4-piperidinamin;
(2R,4S)-2-Benzyl-1-(3,5-ditrifluormethylbenzoyl)-N-[3-(3-chlorphenyl)propyl]-4-piperidinamin;
(2S,4S)-2-Benzyl-1-(3,5-ditrifluormethylbenzoyl)-N-[3-(3-chlorphenyl)propyl]-4-piperidinamin;
(2R,4S)-2-Benzyl-1-(3,5-ditrifluormethylbenzoyl)-N-(4-chlorbenzyl)-4-piperidinamin;
(2S,4S)-2-Benzyl-1-(3,5-ditrifluormethylbenzoyl)-N-(4-chlorbenzyl)-4-piperidinamin;
(2R,4S)-2-Benzyl-1-(3,5-ditrifluormethylbenzoyl)-N-[2-(4-chlorphenyl)ethyl]-4-piperidinamin;
(2S,4S)-2-Benzyl-1-(3,5-ditrifluormethylbenzoyl)-N-[2-(4-chlorphenyl)ethyl]-4-piperidinamin;
(2R,4S)-2-Benzyl-1-(3,5-ditrifluormethylbenzoyl)-N-[3-(4-chlorphenyl)propyl]-4-piperidinamin;
(2S,4S)-2-Benzyl-1-(3,5-ditrifluormethylbenzoyl)-N-[3-(4-chlorphenyl)propyl]-4-piperidinamin;
(2R,4S)-2-Benzyl-1-(3,5-ditrifluormethylbenzoyl)-N-(4-methoxynaphth-1-ylmethyl)-4-piperidinamin;
(2S,4S)-2-Benzyl-1-(3,5-ditrifluormethylbenzoyl)-N-(4-methoxynaphth-1-ylmethyl)-4-piperidinamin;
(2R,4S)-2-Benzyl-1-(3,5-ditrifluormethylbenzoyl)-N-(3,4-ethylendioxybenzyl)-4-piperidinamin;
(2S,4S)-2-Benzyl-1-(3,5-ditrifluormethylbenzoyl)-N-(3,4-ethylendioxybenzyl)-4-piperidinamin;
(2R,4S)-2-Benzyl-1-(3,5-dichlorbenzoyl)-N-(indol-2-ylmethyl)-4-piperidinamin;
(2S,4S)-2-Benzyl-1-(3,5-dichlorbenzoyl)-N-(indol-2-ylmethyl)-4-piperidinamin;
(2R,4S)-2-Benzyl-1-(3,5-dichlorbenzoyl)-N-(indol-3-ylmethyl)-4-piperidinamin;
(2S,4S)-2-Benzyl-1-(3,5-dichlorbenzoyl)-N-(indol-3-ylmethyl)-4-piperidinamin;
(2R,4S)-2-Benzyl-1-(3,5-dichlorbenzoyl)-N-(chinolin-2-ylmethyl)-4-piperidinamin;
(2S,4S)-2-Benzyl-1-(3,5-dichlorbenzoyl)-N-(chinolin-2-ylmethyl)-4-piperidinamin;
(2R,4S)-2-Benzyl-1-(3,5-dichlorbenzoyl)-N-(chinolin-3-ylmethyl)-4-piperidinamin;
(2S,4S)-2-Benzyl-1-(3,5-dichlorbenzoyl)-N-(chinolin-3-ylmethyl)-4-piperidinamin;
(2R,4S)-2-Benzyl-1-(3,5-dichlorbenzoyl)-N-(2-chlorbenzyl)-4-piperidinamin;
(2S,4S)-2-Benzyl-1-(3,5-dichlorbenzoyl)-N-(2-chlorbenzyl)-4-piperidinamin;
(2R,4S)-2-Benzyl-1-(3,5-dichlorbenzoyl)-N-[2-(2-chlorphenyl)ethyl]-4-piperidinamin;
(2S,4S)-2-Benzyl-1-(3,5-dichlorbenzoyl)-N-[2-(2-chlorphenyl)ethyl]-4-piperidinamin;
(2R,4S)-2-Benzyl-1-(3,5-dichlorbenzoyl)-N-[3-(2-chlorphenyl)propyl]-4-piperidinamin;
(2S,4S)-2-Benzyl-1-(3,5-dichlorbenzoyl)-N-[3-(2-chlorphenyl)propyl]-4-piperidinamin;
(2R,4S)-2-Benzyl-1-(3,5-dichlorbenzoyl)-N-(3-chlorbenzyl)-4-piperidinamin;
(2S,4S)-2-Benzyl-1-(3,5-dichlorbenzoyl)-N-(3-chlorbenzyl)-4-piperidinamin;
(2R,4S)-2-Benzyl-1-(3,5-dichlorbenzoyl)-N-[2-(3-chlorphenyl)ethyl]-4-piperidinamin;
(2S,4S)-2-Benzyl-1-(3,5-dichlorbenzoyl)-N-[2-(3-chlorphenyl)ethyl]-4-piperidinamin;
(2R,4S)-2-Benzyl-1-(3,5-dichlorbenzoyl)-N-[3-(3-chlorphenyl)propyl]-4-piperidinamin;
(2S,4S)-2-Benzyl-1-(3,5-dichlorbenzoyl)-N-[3-(3-chlorphenyl)propyl]-4-piperidinamin;
(2R,4S)-2-Benzyl-1-(3,5-dichlorbenzoyl)-N-(4-chlorbenzyl)-4-piperidinamin;
(2S,4S)-2-Benzyl-1-(3,5-dichlorbenzoyl)-N-(4-chlorbenzyl)-4-piperidinamin;
(2R,4S)-2-Benzyl-1-(3,5-dichlorbenzoyl)-N-[2-(4-chlorphenyl)ethyl]-4-piperidinamin;

(2S,4S)-2-Benzyl-1-(3,5-dichlorbenzoyl)-N-[2-(4-chlorphenyl)ethyl]-4-piperidinamin;
(2R,4S)-2-Benzyl-1-(3,5-dichlorbenzoyl)-N-[3-(4-chlorphenyl)propyl]-4-piperidinamin;
(2S,4S)-2-Benzyl-1-(3,5-dichlorbenzoyl)-N-[3-(4-chlorphenyl)propyl]-4-piperidinamin;
(2R,4S)-2-Benzyl-1-(3,5-dichlorbenzoyl)-N-(4-methoxynaphth-1-ylmethyl)-4-piperidinamin;
(2S,4S)-2-Benzyl-1-(3,5-dichlorbenzoyl)-N-(4-methoxynaphth-1-ylmethyl)-4-piperidinamin;
(2R,4S)-2-Benzyl-1-(3,5-dichlorbenzoyl)-N-(3,4-ethylendioxybenzyl)-4-piperidinamin;
(2S,4S)-2-Benzyl-1-(3,5-dichlorbenzoyl)-N-(3,4-ethylendioxybenzyl)-4-piperidinamin;
(2R,4S)-2-Benzyl-1-(3,5-dimethoxybenzoyl)-N-(indol-2-ylmethyl)-4-piperidinamin;
(2S,4S)-2-Benzyl-1-(3,5-dimethoxybenzoyl)-N-(indol-2-ylmethyl)-4-piperidinamin;
(2R,4S)-2-Benzyl-1-(3,5-dimethoxybenzoyl)-N-(indol-3-ylmethyl)-4-piperidinamin;
(2S,4S)-2-Benzyl-1-(3,5-dimethoxybenzoyl)-N-(indol-3-ylmethyl)-4-piperidinamin;
(2R,4S)-2-Benzyl-1-(3,5-dimethoxybenzoyl)-N-(chinolin-2-ylmethyl)-4-piperidinamin;
(2S,4S)-2-Benzyl-1-(3,5-dimethoxybenzoyl)-N-(chinolin-2-ylmethyl)-4-piperidinamin;
(2R,4S)-2-Benzyl-1-(3,5-dimethoxybenzoyl)-N-(chinolin-3-ylmethyl)-4-piperidinamin;
(2S,4S)-2-Benzyl-1-(3,5-dimethoxybenzoyl)-N-(chinolin-3-ylmethyl)-4-piperidinamin;
(2R,4S)-2-Benzyl-1-(3,5-dimethoxybenzoyl)-N-(2-chlorbenzyl)-4-piperidinamin;
(2S,4S)-2-Benzyl-1-(3,5-dimethoxybenzoyl)-N-(2-chlorbenzyl)-4-piperidinamin;
(2R,4S)-2-Benzyl-1-(3,5-dimethoxybenzoyl)-N-[2-(2-chlorphenyl)ethyl]-4-piperidinamin;
(2S,4S)-2-Benzyl-1-(3,5-dimethoxybenzoyl)-N-[2-(2-chlorphenyl)ethyl]-4-piperidinamin;
(2R,4S)-2-Benzyl-1-(3,5-dimethoxybenzoyl)-N-[3-(2-chlorphenyl)propyl]-4-piperidinamin;
(2S,4S)-2-Benzyl-1-(3,5-dimethoxybenzoyl)-N-[3-(2-chlorphenyl)propyl]-4-piperidinamin;
(2R,4S)-2-Benzyl-1-(3,5-dimethoxybenzoyl)-N-(3-chlorbenzyl)-4-piperidinamin;
(2S,4S)-2-Benzyl-1-(3,5-dimethoxybenzoyl)-N-(3-chlorbenzyl)-4-piperidinamin;
(2R,4S)-2-Benzyl-1-(3,5-dimethoxybenzoyl)-N-[2-(3-chlorphenyl)ethyl]-4-piperidinamin;
(2S,4S)-2-Benzyl-1-(3,5-dimethoxybenzoyl)-N-[2-(3-chlorphenyl)ethyl]-4-piperidinamin;
(2R,4S)-2-Benzyl-1-(3,5-dimethoxybenzoyl)-N-[3-(3-chlorphenyl)propyl]-4-piperidinamin;
(2S,4S)-2-Benzyl-1-(3,5-dimethoxybenzoyl)-N-[3-(3-chlorphenyl)propyl]-4-piperidinamin;
(2R,4S)-2-Benzyl-1-(3,5-dimethoxybenzoyl)-N-(4-chlorbenzyl)-4-piperidinamin;
(2S,4S)-2-Benzyl-1-(3,5-dimethoxybenzoyl)-N-(4-chlorbenzyl)-4-piperidinamin;
(2R,4S)-2-Benzyl-1-(3,5-dimethoxybenzoyl)-N-[2-(4-chlorphenyl)ethyl]-4-piperidinamin;
(2S,4S)-2-Benzyl-1-(3,5-dimethoxybenzoyl)-N-[2-(4-chlorphenyl)ethyl]-4-piperidinamin;
(2R,4S)-2-Benzyl-1-(3,5-dimethoxybenzoyl)-N-[3-(4-chlorphenyl)propyl]-4-piperidinamin;
(2S,4S)-2-Benzyl-1-(3,5-dimethoxybenzoyl)-N-[3-(4-chlorphenyl)propyl]-4-piperidinamin;
(2R,4S)-2-Benzyl-1-(3,5-dimethoxybenzoyl)-N-(4-methoxynaphth-1-ylmethyl)-4-piperidinamin;
(2S,4S)-2-Benzyl-1-(3,5-dimethoxybenzoyl)-N-(4-methoxynaphth-1-ylmethyl)-4-piperidi namin;
(2R,4S)-2-Benzyl-1-(3,5-dimethoxybenzoyl)-N-(3,4-ethylendioxybenzyl)-4-piperidinamin und
(2S,4S)-2-Benzyl-1-(3,5-dimethoxybenzoyl)-N-(3,4-ethylendioxybenzyl)-4-piperidinamin.

Beispiel 76: (2R,4S)- und (2R,4R)-2-Benzyl-1-(3,5-dimethylbenzoyl)-N-(4-chinolylmethyl)-4-piperidinamin-dihydrochlorid

Analog Beispiel 1 erhält man ausgehend von 1.26 g (3.9 mMol) (2R,4R/S)-2-Benzyl-1-(3,5-dimethylbenzoyl)-4-piperidinamin, erhalten als Gemisch von ca 70% (2R,4R)- und ca 30% (2R,4S)-Diastereomeren durch Reduktion mittels Borandimethylsulfid gemäss Beispiel e1b, und unter Verwendung von insgesamt 2.26 g (14.4 mMol) Chinolin-4-carboxaldehyd ein Gemisch der Hydrochloride der beiden diastereomeren Titelverbindungen.

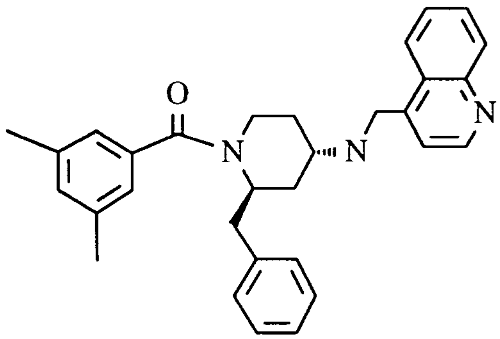

Diasteromer A

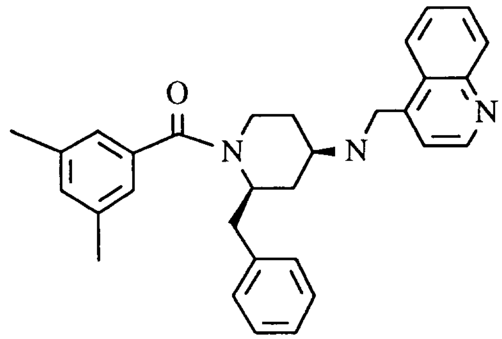

Diastereomer B

DC: Methylenchlorid/Methanol/conc. Ammoniak (90:9:1)
Diastereomer A (2R,4R): $R_f$ = 0.5 , MS: $M^+$ = 463
Diastereomer B (2R,4S): $R_f$ = 0.45, MS: $M^+$ = 463, Smp. 144-145°, $[\alpha]_D$ = +25° (c=0.94 in Ethanol)
Diese werden gelöst in Essigester mit etherischer HCl-Lösung behandelt , wobei man das Dihydrochlorid der Titelverbindung erhält.
DC: Methylenchlorid/Methanol/conc. Ammoniak (90:9:1)
Diastereomer A (2R,4R): $R_f$ = 0.5, Smp. 172-174°, $[\alpha]_D$ = -55.7° (c=1, Ethanol)
Diastereomer B (2R,4S): $R_f$ = 0.45, Smp. 174-176°, $[\alpha]_D$ = +18° (c=1, Ethanol)

Beispiel 77: (2S,4R) und (2S,4S)-2-Benzyl-1-(3,5-dimethylbenzoyl)-N-(2-phenethyl)-4-piperidinamin-hydrochlorid

Analog Beispiel 1 erhält man ausgehend von 1.87 g (5.8 mMol) (2S,4R/S)-2-Benzyl-1-(3,5-dimethylbenzoyl)-4-piperidinamin, erhalten als Gemisch von ca 70% (2S,4S)- und ca 30% (2S,4R)-Diastereomeren durch Reduktion mittels Borandimethylsulfid gemäss Beispiel e1b, und unter Verwendung von Phenylacetaldehyd die Hydrochloride der beiden diastereomeren Titelverbindungen.
DC: Methylenchlorid/Methanol (98:2)
Diastereomer A (2S,4S): $R_f$= 0.16, Smp. 250-251°C, $[\alpha]_D$ = +56.2° (c= 0.980,Methanol), MS: $M^+$ = 426 (freie Base).
Diastereomer B (2S,4R): $R_f$= 0.06, Smp. 250°C (Zersetzung), $[\alpha]_D$ = -29.7° (c= 0.768, Methanol), MS: $M^+$ = 426 (freie Base).

Beispiel 78: (2R,4S)-2-Benzyl-1-(3,5-dimethylbenzoyl)-N-(3-chinolylmethyl)-4-piperidinamin

Analog Beispiel 1 erhält man ausgehend von 511 mg (1.59 mMol) (2R,4RS)-2-Benzyl-1-(3,5-dimethylbenzoyl)-4-piperidinamin unter Verwendung von Chinolin-3-carbaldehyd die Titelverbindung durch Kristallisation aus Hexan/Ethylacetat (1:1) in Form farbloser Kristalle.
DC: Methylenchlorid/Methanol/konz. Ammoniak (90:9:1)
$R_f$= 0.5, Smp. 91-93°C, MS: $M^+$ = 463 (freie Base), $[\alpha]_D$ = +0.7° (c=1.09, Methanol)

Beispiel 79: (2R,4S)-2-Benzyl-1-(3,5-dimethylbenzoyl)-N-(2-chinolylmethyl)-4-piperidinamin-dihydrochlorid

Analog Beispiel 1 erhält man ausgehend von 541 mg (1.68 mMol) (2R,4RS)-2-Benzyl-1-(3,5-dimethylbenzoyl)-4-piperidinamin unter Verwendung von Chinolin-2-carbaldehyd die Titelverbindung.
DC: Methylenchlorid/Methanol/konz. Ammoniak (90:9:1)
$R_f$=0.5, Zersetzungspunkt: ab 110°C, MS: $M^+$ = 463 (freie Base), $[\alpha]_D$ = +4.8° (c=1.105, Methanol)

Beispiel 80: (2R,4S)- und (2R,4R)-2-Benzyl-1-(3,5-dimethylbenzoyl)-N-benzyl-4-piperidinamin-hydrochlorid

Analog Beispiel 1 erhalt man ausgehend von 0.748 g (2.32mMol) (2R,4R/S)-2-Benzyl-1-(3,5-dimethylbenzoyl)-4-piperidinamin, erhalten als Gemisch von ca 70% (2R,4R)- und ca 30% (2R,4S)-Diastereomeren durch Reduktion mittels Borandimethylsulfid gemäss Beispiel e1b, und unter Verwendung von Benzaldehyd

die Hydrochloride der beiden diastereomeren Titelverbindungen.
DC: Methylenchlorid/Methanol/konz. Ammoniak (95:4.5:0.5)
Diastereomer A (2R,4R): $R_f$ = 0.45, Smp. 244-246 °C, $[\alpha]_D$ = -50.4 ° (c = 0.979,Chloroform), MS: $M^+$ = 412 (freie Base).
Diastereomer B (2R,4S): $R_f$ = 0.33, amorph.
$[\alpha]_D$ = +7.9 ° (c = 1.0, Chloroform), MS: $M^+$ = 412 (freie Base).

Beispiel 81: (2S,4R)- und (2S,4S)-2-Benzyl-1-(3,5-dimethylbenzoyl)-N-benzyl-4-piperidinamin-hydrochlorid

Analog Beispiel 1 erhält man ausgehend von 4.5 g (13.95mMol) (2S,4R/S)-2-Benzyl-1-(3,5-dimethylbenzoyl)-4-piperidinamin, erhalten als Gemisch von ca 70% (2S,4S)- und ca 30% (2S,4R)-Diastereomeren durch Reduktion mittels Borandimethylsulfid gemäss Beispiel e1b, und unter Verwendung von Benzaldehyd die Hydrochloride der beiden diastereomeren Titelverbindungen.
DC: Methylenchlorid/Methanol/konz. Ammoniak (95:4.5:0.5)
Diastereomer A (2S,4S): $R_f$ = 0.45, Smp. 246-247 °C, $[\alpha]_D$ = +51.2 ° (c = 0.672,Chloroform), MS: $M^+$ = 412 (freie Base).
Diastereomer B (2S,4R): $R_f$ = 0.33, amorph.
$[\alpha]_D$ = -7.7 ° (c = 0.784, Chloroform), MS: $M^+$ = 412 (freie Base).

Beispiel 82: (2R,4S)- und (2R,4R)-2-Benzyl-1-(3,5-dimethylbenzoyl)-N-(4-pyridylmethyl)-4-piperidinamin-dihydrochlorid

Analog Beispiel 1 erhält man ausgehend von 100mg (0.279 mMol) (2R,4R/S)-2-Benzyl-1-(3,5-dimethylbenzoyl)-4-piperidinamin, erhalten als Gemisch von ca 70% (2R,4R)- und ca 30% (2R,4S)-Diastereomeren durch Reduktion mittels Borandimethylsulfid gemäss Beispiel e1b, und unter Verwendung von Pyridin-4-carbaldehyd die beiden diastereomeren Titelverbindungen.
DC: Methylenchlorid/Methanol/konz. Ammoniak (90:9:1)
Diastereomer A (2R,4R): $R_f$ = 0.68, ab 142 °C Zersetzung,
$[\alpha]_D$ = -57.3 ° (c = 0,508, Ethanol), MS: $M^+$ = 413 (freie Base).
Diastereomer B (2R,4S): $R_f$ = 0.44, ab 145 °C Zersetzung,
$[\alpha]_D$ = +23.0 ° (c = 0.300, Ethanol), MS: $M^+$ = 413 (freie Base).

Beispiel 83: (2R,4S)- und (2R,4R)-2-Benzyl-1-(3,5-dimethylbenzoyl)-N-(3-pyridylmethyl)-4-piperidinamin-dihydrochlorid

Analog Beispiel 1 erhält man ausgehend von 100mg (0.279 mMol) (2R,4R/S)-2-Benzyl-1-(3,5-dimethylbenzoyl)-4-piperidinamin, erhalten als Gemisch von ca 70% (2R,4R)- und ca 30% (2R,4S)-Diastereomeren durch Reduktion mittels Borandimethylsulfid gemäss Beispiel e1b, und unter Verwendung von Pyridin-3-carbaldehyd die beiden diastereomeren Titelverbindungen.
DC: Methylenchlorid/Methanol/konz. Ammoniak (90:9:1)
Diastereomer A (2R,4R): $R_f$ = 0.68, ab 105 °C Zersetzung,
$[\alpha]_D$ = -52.6 ° (c = 1.06, Ethanol), MS: $M^+$ = 413 (freie Base).
Diastereomer B (2R,4S): $R_f$ = 0.44, ab 105 °C Zersetzung,
$[\alpha]_D$ = +22.6 ° (c = 1.03, Ethanol), MS: $M^+$ = 413 (freie Base).

Beispiel 84: (2S,4R) und (2S,4S)-2-Benzyl-1-(3,5-dimethylbenzoyl)-N-(4-chinolylmethyl)-4-piperidinamin

Analog Beispiel 1 erhält man ausgehend von 12.1g (37.5mMol) (2S,4RS)-2-Benzyl-1-(3,5-dimethylbenzoyl)-4-piperidinamin, erhalten als Gemisch von ca 70% (2S,4S)- und ca 30% (2S,4R)-Diastereomeren durch Reduktion mittels Borandimethylsulfid gemäss Beispiel e1b, und unter Verwendung von Chinolin-4-carbaldehyd die beiden diastereomeren Titelverbindungen.
DC: Methylenchlorid/Methanol/konz. Ammoniak (90:9:1)
Diastereomer A (2S,4R): $R_f$ = 0.59 ,Smp.144-145 °C (freie Base) $[\alpha]_D$ = -25.1 ° (c = 1.0, Ethanol), MS: $M^+$ = 463 (freie Base)

Beispiel 85: (2R*,4S*,1'R*)-N-Benzyl-1-(3,5-dimethylbenzoyl)-2-(1'-hydroxy-1'-benzyl)-4-piperidinamin (Diastereomer A) und (2R*,4R*,1'R*)-N-Benzyl-1-(3,5-dimethylbenzoyl)-2-(1'-hydroxy-1'-phenyl-methyl)-4-piperidinamin (Diastereomer B)

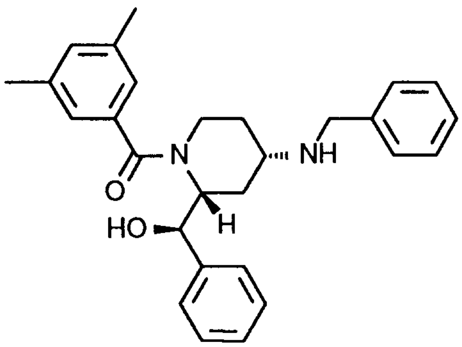

A

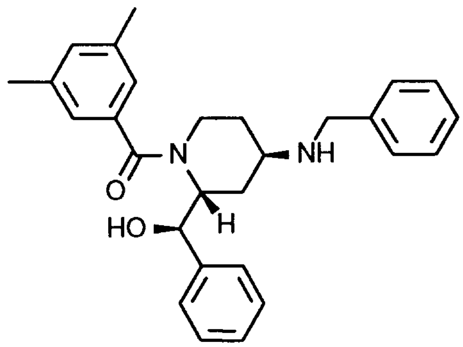

B

Eine Lösung vom 400 mg (1.18 mMol) (2R*,1'R*-1-(3,5-dimethylbenzoyl)-2-(1'-hydroxy-1'-phenyl-methyl)-4-piperidon und 139 mg (1.3 mMol) Benzylamin in Toluol/Hexan wird unter azeotroper Entfernung von Wasser 18 Stunden zum Rückfluss erhitzt. Die Reaktionsmischung wird unter vermindertem Druck eingeengt und das zurückbleibende Öl in Methanol aufgenommen, bei 0 bis 5° mit 111 mg Natriumcyanoborhydrid (85 % in Mineralöl) versetzt und 4 Stunden bei 25° gerührt. Das Lösungsmittel wird unter vermindertem Druck abgezogen und das Rohprodukt in einem Gemisch von Essigsäureethylester und 10 %-iger Natriumcarbonatlösung aufgenommen. Die organische Phase wird abgetrennt, über Natriumsulfat getrocknet und unter vermindertem Druck eingedampft. Chromatographie an Silicagel mit Essigsäureethylester als Laufmittel ergibt die beiden Titelverbindungen; TLC (Essigsäureethylester):
Diastereomer A: $R_f$ = 0.28; Smp. 159-160°
Diastereomer B: $R_f$ = 0.09; Smp. 190-192°.

Das Ausgangsmaterial kann folgendermassen hergestellt werden:

a) 1-tert.-Butoxycarbonyl-4-piperidon-ethylenketal

Zu einer Lösung von 14.3 g 4-Piperidonethylenketal in 100 ml Toluol werden unter Rühren bei 0 bis 5° langsam 26.1 g Ditertiärbutyldicarbonat, gelöst in 20 ml Toluol zugegeben. Man lässt 2 Stunden bei Raumtemperatur rühren und dampft dann unter vermindertem Druck ein und destilliert unter vermindertem Druck. Man erhält 22.2 g der Titelverbindung, Sdp. 83-85° (0.2 Torr). TLC (Essigsäureethylester/Hexan; 1:3): $R_f$ = 0.20.

b) (2R*,1'R*)-1-tert.-Butoxycarbonyl-2-(1'-hydroxy-1'-phenyl-methyl)-4-piperidonethylenketal (Diastereomer A) und (2R*,1'S*)-1-tert.-Butoxycarbonyl-2-(1'-hydroxy-1'-phenyl-methyl)-4-piperidonethylenketal(Diastereomer B)

24.3 g ( 100mMol) 1-tert.-Butoxycarbonyl-4-piperidonethylenketal und 32.8 ml Tetramethylethylendiamin werden in 100 ml Diethylether gelöst, auf -70° gekühlt und langsam mit 87.5 ml (120 mMol) einer Lösung von Sekundärbutyllithium (1.4-molare Lösung in Cyclohexan/Isopentan) versetzt. Man lässt 4 Stunden bei -70° nachrühren, fügt dann in einem Guss 12.72 g (120 mMol) Benzaldehyd hinzu und lässt auf 0° erwärmen. Das Reaktionsgemisch wird mit gesättigter Ammoniumchloridlösung versetzt und mit Essigsäureethylester ausgeschüttelt. Die organische Phase wird abgetrennt, mit gesättigter Kochsalzlösung gewaschen, über Natriumsulfat getrocknet und unter vermindertem Druck eingedampft. Chromatographie an Silicagel mit Essigsäureethylester als Laufmittel ergibt 12.2 g an Diastereomer A und 21.6 g an Diastereomer B der Titelverbindung. TLC (Essigsäureethylester/Hexan; 1:1):
Diastereomer A (2R*,1'R*): $R_f$ = 0.43, Smp. 133-134°
Diastereomer B (2R*,1'S*): $R_f$ = 0.34, Smp. 114-116°

c) 2-(1'-Hydroxy-1'-phenyl-methyl)-4-piperidon

Eine Suspension von 2.6 g (7.44 mMol)(2R*,1'R*)-1-tert.-Butoxycarbonyl-2-(1'-hydroxy-1'-phenyl-methyl)-4-piperidonethylenketal in 30 ml 6N Salzsäure wird 1 Stunde auf 60° erhitzt, dann abgekühlt, mit Natriumcarbonat neutralisiert und mit Essigsäureethylester ausgeschüttelt. Die organische Phase wird über Natriumsulfat getrocknet und unter vermindertem Druck eingedampft. Man erhält die Titelverbindung vom Smp. 124-126°; $R_f$ = 0.26 (Methylenchlorid/Methanol/25 %-ige wässrige Ammoniaklösung, 90:9.5:0.5).

d) (2R*,1'R*)-1-(3,5-Dimethylbenzoyl)-2-(1'-hydroxy-1'-phenyl-methyl)-4-piperidon

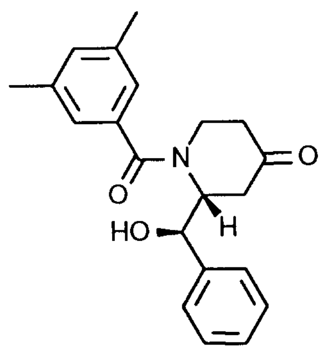

Das wie vorstehend unter c) beschrieben alhltene rohe 2-(1'-Hydroxy-1'-phenyl-methyl)-4-piperidon wird in einem Gemisch von 20 ml Dichlormethan und 20 ml gesättigter Natriumhydrogencarbonat-Lösung aufgenommen, unter Rühren auf 0-5° gekühlt und innerhalb von 1.5 Stunden tropfenweise mit 1.5 g (8.9 mMol) 3,5-Dimethylbenzoylchlorid versetzt. Man lässt 1 Stunde nachrühren, verdünnt mit Essigsäureethylester, wäscht nacheinander mit 1N-HCl und gesättigter Kochsalzlösung, trocknet über Natriumsulfat und dampft unter vermindertem Druck zur Trockne ein. Die Titelverbindung kann durch Chromatographie an Silicagel mit Essigsäureethylester/Hexan (1:1) als Laufmittel gereinigt werden. TLC (Essigsäureethylester/Hexan; 1:1): $R_f$ = 0.19, FD-MS: M+ = 338.

In analoger Weise wie vorstehend unter b), c) und d) beschrieben kann man auch die nachtehenden Verbindungen herstellen:
(2R*,1'R*)-1-(3,5-Bistrifluormethylbenzoyl)-2-{1'-hydroxy-1'-(4-chlorphenyl)methyl}-4-piperidon, TLC (Essigester/Hexan; 1:1): $R_f$ = 0.35; FD-MS: 479,481;
(2R*,1'R*)-1-(3,5-Dimethylbenzoyl)-2-{1'-hydroxy-1'-(3,4-dichlorphenyl)methyl}-4-piperidon, TLC-(Essigester/Hexan;1:1): $R_f$ = 0.16, Smp.: 222-223°;
(2R*,1'S*)-1-(3,5-Bistrifluormethylbenzoyl)-2-{1'-hydroxy-1'-(4-chlorphenyl)methyl}-4-piperidon
(2R*,1'R*)-1-(3,5-Bistrifluormethylbenzoyl)-2-{1'-hydroxy-1'-(3,4-dichlorphenyl)methyl}-4-piperidon
(2R*,1'R*)-1-(3,5-Dimethylbenzoyl)-2-{1'-hydroxy-1'-(4-methoxyphenyl)methyll}-4-piperidon
(2R*,1'S*)-1-(3,5-Dimethylbenzoyl)-2-{1'-hydroxy-1'-(4-methoxyphenyl)methyl}-4-piperidon
(2R*,1'R*)-1-(3,5-Dimethylbenzoyl)-2-{1'-hydroxy-1'-(3-methoxyphenyl)methyl}-4-piperidon
(2R*,1'S*)-1-(3,5-Dimethylbenzoyl)-2-{1'-hydroxy-1'-(3-methoxyphenyl)methyl}-4-piperidon
(2R*,1'R*)-1-(3,5-Dimethylbenzoyl)-2-{1'-hydroxy-1'-(4-trifluormethylphenyl)methyl}-4-piperidon
(2R*,1'S*)-1-(3,5-Dimethylbenzoyl)-2-{1'-hydroxy-1'-(4-trifluormethylphenyl)methyl}-4-piperidon
(2R*,1'R*)-1-(3,5-Dimethylbenzoyl)-2-{1'-hydroxy-1'-(4-chlor-3-trifluormethylphenyl)methyl}-4-piperidon und
(2R*,1'S*)-1-(3,5-Dimethylbenzoyl)-2-{1'-hydroxy-1'-(4-chlor-3-trifluormethylphenyl)-methyl}-4-piperidon.

Beispiel 86: (2R*,4S*,1'R*)-2-(1'-Hydroxy-1'-phenyl-methyl)-1-(3,5-dimethylbenzoyl)-N-(4-chinolylmethyl)-4-piperidinamin (Diastereomer A) und (2R*,4R*,1'R*)-1-(3,5-dimethylbenzoyl)-2-(1'-hydroxy-1'-phenyl-methyl)--N-(4-chinolylmethyl)-4-piperidinamin (Diastereomer B)

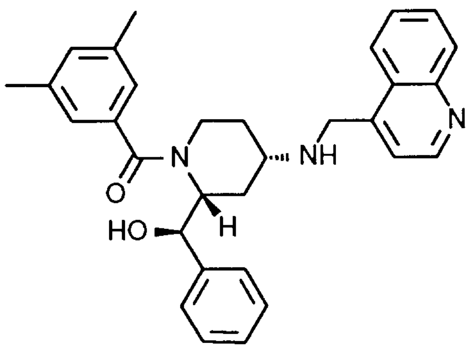

A

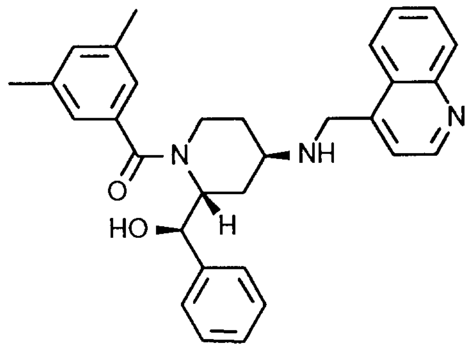

B

Die Titelverbindung kann in analoger Weise wie in Beispiel 85 beschrieben ausgehend von 4-Chinolinmethylamin hergestellt werden. Sie wird durch Säulenchromatographie an Silicagel mit Essigsäureethylester und Essigsäureethylester/Methanol (50:1) als Laufmittel in die Diastereomeren aufgetrennt und gereinigt. TLC (Essigsäureethylester):
Diastereomer A (2R*,4S*,1'R*): $R_f$ = 0.08, FD-MS: M+ = 479
Diastereomer B (2R*,4R*,1'R*): $R_f$ = 0.01, FD-MS: M+ = 479

Beispiel 87: (2R*,4S*,1'S*)-1-(3,5-Dimethylbenzoyl)-2-(1'-hydroxy-1'-phenyl-methyl)-N-(4-chinolylmethyl)-4-piperidinamin (Diastereomer A) und (2R*,4R*,1'S*)-1-(3,5-Dimethyl-benzoyl)-2-(1'-hydroxy-1'-phenyl-methyl)-N-(4-chinolylmethyl)-4-piperidinamin (Diastereomer B)

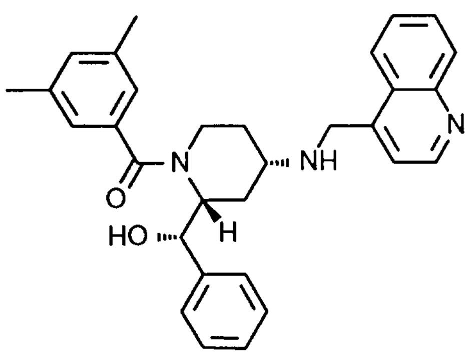

A

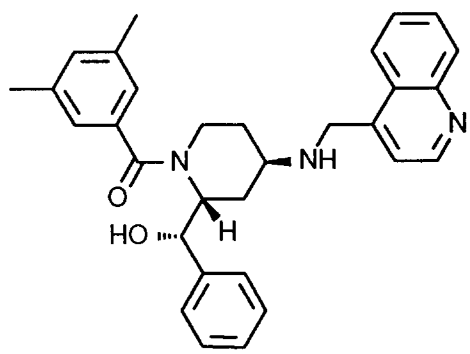

B

Die Titelverbindung kann in analoger Weise wie in Beispiel 85 beschrieben ausgehend von 420 mg (1.24 mMol) (2R*,1'S*)-1-(3,5-Dimethylbenzoyl)-2-(1'-hydroxy-1'-phenyl-methyl)-4-chinolylmethylamin hergestellt werden. Sie wird durch Säulenchromatographie an Silicagel mit Essigsäureethylester und Dichlormethan/Methanol/35%-ige Ammoniaklösung (95:4.5:0.5) als Laufmittel in die Diastereomeren aufgetrennt und gereinigt. TLC (Essigsäureethylester):
Diastereomer A (2R*,4S*,1'S*): $R_f$ = 0.08, FD-MS: M+ = 479
Diastereomer B (2R*,4R*,1'S*): $R_f$ = 0.01, FD-MS: M+ = 479
Das Ausgangsmaterial kann z.B. folgendermassen hergestellt werden:

a) (2R*,1'S*)-1-(3,5-Dimethylbenzoyl)-2-(1'-hydroxy-1'-phenyl-methyl)-4-piperidon

Die Titelverbindung wird in analoger Weise wie in Beispiel 1c beschrieben aus 860 mg ( 2.46 mMol) (2R*,1'S*)-1-tert.-Butoxycarbonyl-2-(1'-hydroxy-1'-phenyl-methyl)-4-piperidonethylenketal (Beispiel 1b) hergestellt und durch Säulenchromatographie an Silicagel mit Essigsäureethylester/Hexan (2:3) als Laufmittel gereinigt werden. TLC (Essigsäureethylester/Hexan; 1:1): $R_f$ = 0.24; FD-MS: M+ = 337.

Beispiel 88: (2R*,4S*,1'R*)-2-{1'-Hydroxy-1'-(4-chlorphenyl)methyl}-1-(3,5-dimethylbenzoyl)-N-(4-chinolylmethyl)-4-piperidinamin (Diastereomer A) und (2R*,4R*,1'R*)-1-(3,5-dimethylbenzoyl)-2-{1'-hydroxy-1'-(4-chlorphenyl)methyl}-N-(4-chinolylmethyl)-4-piperidinamin (Diastereomer B)

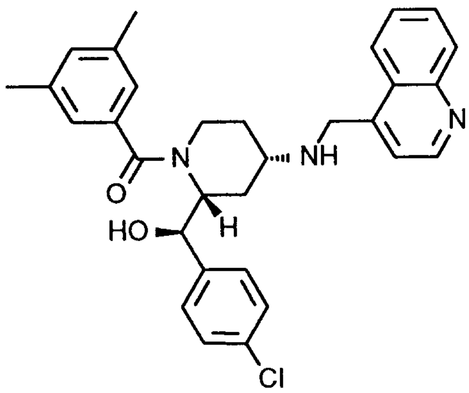

A

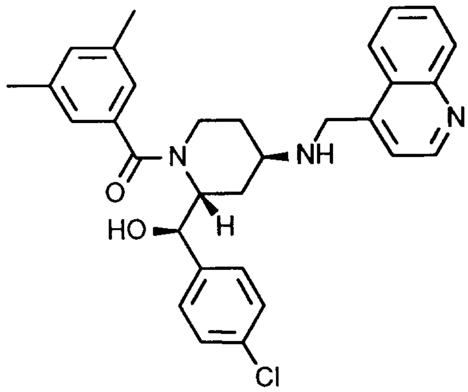

B

Die Titelverbindung kann in analoger Weise wie in Beispiel 1 aus 310 mg (0.833 mMol) (2R*,1'R*)-1-(3,5-Dimethylbenzoyl)-2-{1'-hydroxy-1'-(4-chlorphenyl)methyl}-4-piperidon und 145 mg (0.92 mMol) 4-Chinolylmethylamin hergestellt und durch Säulenchromatographie an Silicagel mit Essigsäureethylester/Isopropanol (95:5 - 90:10) als Laufmittel aufgetrennt und gereinigt werden. TLC (Dichlormethan/Methanol/25 %-ige Ammoniaklösung (90:**9.5**:0.5)
Diastereomer A (2R*,4S*,1'R*): $R_f$ = 0.47, FD-MS: (M+1)+ = 514
Diastereomer B (2R*,4R*,1'R*): $R_f$ = 0.35, FD-MS: M+ = 513
Das Ausgangsmaterial kann z.B. folgendermassen hergestellt werden:

a) (2R*,1'R*)-1-tert.-Butoxycarbonyl-2-{1'-hydroxy-1'-(4-chlorphenyl)methyl}-4-piperidonethylenketal (Diastereomer A) und (2R*,1'S*)-1-tert.-Butoxycarbonyl-2-{1'-hydroxy-1'-(4-chlorphenyl)methyl}-4-piperidonethylenketal (Diastereomer B)

Die Titelverbindung kann in analoger Weise wie in Beispiel 1b beschrieben ausgehend von 4-Chlorbenzaldehyd hergestellt, durch Säulenchromatographie an Silicagel mit Essigsäureethylester/Hexan (1:3) als Laufmittel aufgetrennt und gereinigt werden sowie aus Essigsäureethylester/Hexan kristallisiert werden. TLC (Essigsäureethylester/Hexan; 1:1):
Diastereomer A (2R*,1'R*): $R_f$ = 0.44, Smp. 129-130 °
Diastereomer B (2R*,1'S*): $R_f$ = 0.35, Smp. 160-161 °

b) (2R*,1'R*)-1-(3;5-Dimethylbenzoyl)-2-{1'-hydroxy-1'(4-chlorphenyl)methyl}-4-piperidon

Die Titelverbindung kann in analoger Weise wie in Beispiel 1c beschrieben hergestellt werden und kristallisiert aus Essigsäureethylester. Smp. 222-225 °; TLC (Essigsäureethylester/Hexan; 1.1): $R_f$ = 0.17

Beispiel 89: (2R*,4S*,1'S*)-1-(3,5-Dimethylbenzoyl)-2-{1'-hydroxy-1'-(4-chlorphenyl)-methyl}-N-(4-chinolylmethyl)-4-piperidinamin (Diastereomer A) und (2R*,4R*,1'S*)-1-(3,5-Dimethylbenzoyl)-2-{1'-hydroxy-1'-(4-chlorphenyl)methyl}-N-(4-chinolylmethyl)-4-piperidinamin (Diastereomer B)

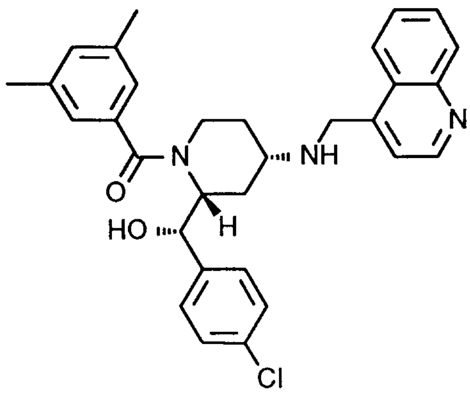

A

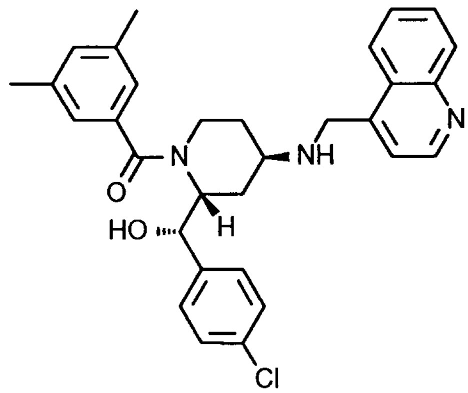

B

Die Titelverbindung kann in analoger Weise wie in Beispiel 1 ausgehend von (2R*,1'S*)-1-(3,5-Dimethylbenzoyl)-2-{1'-hydroxy-1'-(4-chlorphenyl)methyl}-4-piperidon als Ausgangsmaterial hergestellt und durch Säulenchromatographie an Silicagel mit Dichlormethan/Methanol/25 %-ige Ammoniaklösung (95:4.5:0.5) als Laufmittel aufgetrennt und gereinigt werden. TLC (Essigsäure):
Diastereomer A (2R*,4S*,1'S*): $R_f$ = 0.24, FD-MS: M+ = 514
Diastereomer B (2R*,4R*,1'S*): $R_f$ = 0.06, FD-MS: M+ = 514
Das Ausgangsmaterial kann beispielsweise wie folgt hergestellt werden:

a) (2R*,1'S*)1-(3,5-Dimethylbenzoyl)-2-{1'-hydroxy-1'-(4-chlorphenyl)methyl}-4-piperidon

Die Titelverbindung wird in analoger Weise wie in Beispiel 1c beschrieben ausgehend von (2R*,1'S*)-1-tert.-Butoxycarbonyl-2-{1'-hydroxy-1'-(4-chlorphenyl)methyl}-4-piperidonethylenketal (Beispiel 4a, Diastereomer B) hergestellt. Smp. 195-197°; TLC (Essigsäureethylester/Hexan; 1:1): $R_f$ = 0.26

Beispiel 90: (2R*,4S*,1'S*)-1-(3,5-Dimethylbenzoyl)-2-{1'-hydroxy-1'-(3,4-dichlorphenyl)-methyl}-N-(4-chinolylmethyl)-4-piperidinamin (Diastereomer A) und (2R*,4R*,1'S*)-1-(3,5-dimethylbenzoyl)-2-{1'-hydroxy-1'-(3,4-dichlorphenyl)-methyl}-N-(4-chinolylmethyl)-4-piperidinamin (Diastereomer B)

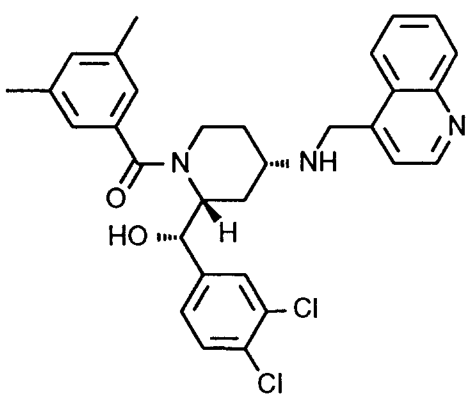

A

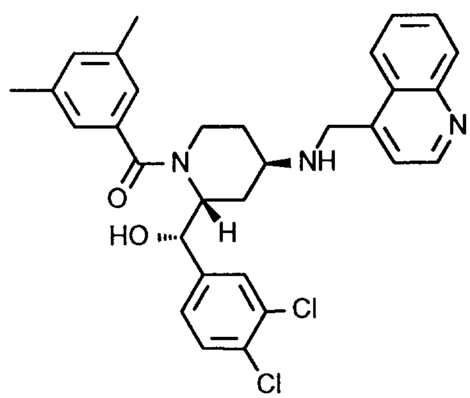

B

Die Titelverbindung wird in analoger Weise wie in Beispiel 1 beschrieben ausgehend von (2R*,1'S*)-1-(3,5-Dimethylbenzoyl)-2-{1'-hydroxy-1'-(3,4-dichlorphenyl)methyl}-4-piperidon hergestellt und durch Säulenchromatographie an Silicagel mit Dichlormethan/Methanol/25 %-ige Ammoniaklösung (93:6.5:0.5) als Laufmittel aufgetrennt. TLC (Dichlormethan/Methanol/25 %-ige Ammoniaklösung; 90:9.5:0.5):
Diastereomer A (2R*,4S*,1'S*): $R_f$ = 0.38; Smp. 138-140°
Diastereomer B (2R*,4R*,1'S*): $R_f$ = 0.22; Smp. 188-190°
Das Ausgangsmaterial kann beispielsweise wie folgt hergestellt werden.

a) (2R*,1'R*)-1-tert.-Butoxycarbonyl-2-{1'-hydroxy-1'-(3,4-dichlorphenyl)methyl}-4-piperidonethylenketal (Diastereomer A) und (2R*,1'S*)-1-tert.-Butoxycarbonyl-2-{1'-hydroxy-1'-(3,4-dichlorphenyl)methyl}-4-piperidonethylenketal (Diastereomer B)

Die Titelverbindung wird in analoger Weise wie in Beispiel 1b beschrieben ausgehend von 3,4-Dichlorbenzaldehyd statt Benzaldehyd erhalten. Die Diastereomeren werden durch Säulenchromatographie an Silicagel mit Essigsäureethylester/Hexan (1:3) als Laufmittel aufgetrennt und aus Essigsäureethylester/Hexan kristallisiert. TLC (Essigsäureethylester/Hexan; 1:1):
Diastereomer A (2R*,1'R*): $R_f$ = 0.57, IR-Spektrum (Methylenchlorid): 3700-3300, 1680 $cm^{-1}$
Diastereomer B (2R*,1'S*): $R_f$ = 0.48, Smp. 160-162°

b) (2R*,1'S*)-1-(3,5-Dimethylbenzoyl)-2-{1'-hydroxy-1'-(3,4-dichlorphenyl)methyl}-4-piperidon

Die Titelverbindung wird in analoger Weise wie in Beispiel 1c aus (2R*,1'S*)-1-tert.-Butoxycarbonyl-2-{1'-hydroxy-1'-(3,4-dichlorphenyl)methyl}-4-piperidonethylenketal hergestellt und aus Essigsäureethylester/Hexan kristallisiert; Smp. 152-152.5°; TLC (Essigsäureethylester/Hexan; 1:1), $R_f$ = 0.24

Beispiel 91: (2R*,4S*)-N-Benzyl-1-(3,5-dimethylbenzoyl)-2-benzoyl-4-piperidinamin

Eine Lösung von 30 mg (2R*,4R*,1'R*)-N-Benzyl-N-trifluoracetyl-1-(3,5-dimethylbenzoyl)-2-benzoyl-4-piperidinamin in 5 ml Methanol und 1 ml 5N Natriumhydroxidlösung wird 10 Minuten auf 60° erhitzt. Man lässt auf Raumtemperatur abkühlen, verdünnt mit 10%-iger wässriger Natriumhydrogencarbonatlösung und schüttelt zweimal mit Dichlormethan aus. Die organischen Phasen werden vereinigt, über Natriumsulfat getrocknet und unter vermindertem Druck zur Trockne eingedampft. Die Titelverbindung der Formel

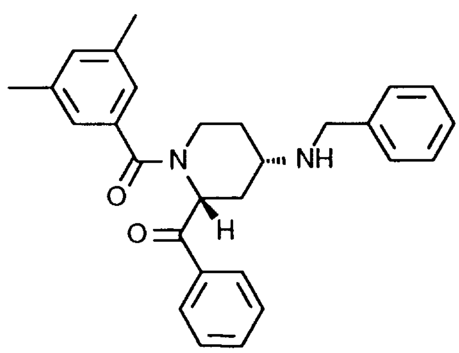

wird durch Säulenchromatographie an Silicagel mit Essigsäureethylester als Laufmittel erhalten. TLC (Essigsäureethylester): $R_f$ = 0.17; IR-Spektrum (Methylenchlorid): 1685, 1625, 1500 $cm^{-1}$.

Das Ausgangsmaterial kann beispielsweise folgendermassen erhalten werden:

a) (2R*,4R*,1'R*)-N-Benzyl-N-trifluoracetyl-1-(3,5-dimethylbenzoyl)-2-benzoyl-4-piperidinamin

Eine Lösung von 80 mg (0.19 mMol) (2R*,4R*,1'R*)-N-Benzyl-1-(3,5-dimethylbenzoyl)-2-(1'-hydroxy-1'-phenyl-methyl)-4-piperidinamin (Beispiel 1, Diastereomer B) in 1 ml Pyridin wird bei 0° mit 0.167 ml Trifluoracetanhydrid versetzt und 2 Stunden bei 0° gerührt. Das Reaktionsgemisch wird mit Diethylether und Wasser verdünnt und in die Phasen getrennt. Die organische Phase wird mit 4N Salzsäure und gesättigter Kochsalzlösung gewaschen, über Natriumsulfat getrocknet und unter vermindertem Druck zur Trockne eingedampft. Das Rohprodukt wird zur Entfernung zweifach trifluoracetylierter Nebenprodukte in 3

ml Ethanol und 0.5 ml Triethylamin auf 55° erhitzt und erneut unter vermindertem Druck eingedampft. Der Rückstand wird in 3 ml Dichlormethan, enthaltend 3Å Molekularsieb aufgenommen und mit 5 mg Tetrapropylammoniumperruthenat und 100 mg Morpholin-N-oxid versetzt. Man lässt 16 Stunden rühren, filtriert, verdünnt mit Dichlormethan, wäscht nacheinander mit Natriumhydrogensulfatlösung, gesättigter Kochsalzlösung und 5%-iger Kupfersulfatlösung, trocknet über Natriumsulfat, dampft unter vermindertem Druck ein und kristallisiert aus Diethylether/Hexan; Smp. 138-139°; TLC (Essigsäureethylester/Hexan; 1:1): $R_f$ = 0.71.

Beispiel 92: (2R*,4S*)-2-(4-Chlorbenzyl)-1-(3,5-dimethylbenzoyl)-N-(4-chinolinylmethyl)-4-piperidinamin

Ein Gemisch von 550 mg (1.54 mMol) (2R*,4S*)-2-(4-Chlorbenzyl)-1-(3,5-dimethylbenzoyl)-4-piperidinamin und 242 mg (1.54 mMol) 4-Chinolinecarboxaldehyd wird in 30 ml Toluol gelöst und unter vermindertem Druck zur Trockne eingedampft. Dies wird noch zweimal wiederholt. Der Rückstand wird in 10 ml Ethanol aufgenommen, mit 70 mg (1.85 mMol) Natriumboranat versetzt und 3 Stunden bei 25° gerührt. Man säuert mit 1N Salzsäure an und lässt 1 Stunde nachrühren. Das Reaktionsgemisch wird in gesättigte wässrige Natriumcarbonatlösung eingegossen und mit Essigsäureethylester extrahiert. Die organische Phase wird mit gesättigter Kochsalzlösung gewaschen, über Natriumsulfat getrocknet und unter vermindertem Druck zur Trockne eingedampft. Das Rohprodukt wird zweimal aus Essigsäureethylester umkristallisiert und ergibt die Titelverbindung der Formel

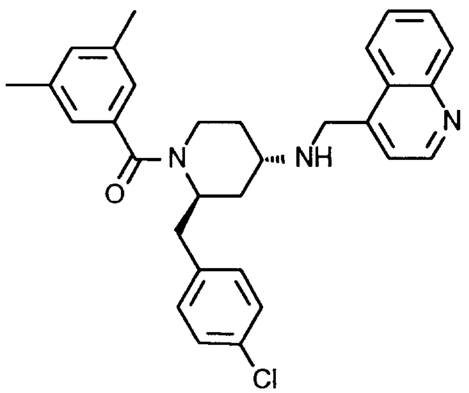

in Form weisser Kristalle; Smp. 148-9°; MS: M+ 497.

Die Ausgangsstoffe können beispielsweise wie folgt hergestellt werden:

a) N-5-(4-Chlorphenyl)pent-1-en-4-yl-3.5-dimethylbenzamid

Zu einer bei 0° gerührten Lösung von 5.0 g (25.6 mMol) N-[1-(4-Chlorphenyl)pent-4-en-2-yl]amin und 5.33 ml (38.4 mMol ) Triethylamin in 100 ml Dichlormethan werden innerhalb von 2 Stunden 4.3 g (25.6 mMol) 3,5-Dimethylbenzoylchlorid zugegeben. Das Reaktionsgemisch wird noch 1 Stunde weitergerührt, mit 1N Salzsäure versetzt und mit Dichlormethan extrahiert. Die organische Phase wird mit gesättigter Kochsalzlösung neutralgewaschen, über Natriumsulfat getrocknet und unter vermindertem Druck eingedampft. Das Rohprodukt wird aus Essigsäureethylester/Hexan kristallisiert und ergibt 7.36 g (88 %) weisse Kristalle; Smp. 116-118°; TLC (Hexan/Essigsäureethylester; 3:1): $R_f$ = 0.37

b) N-{5-(4-Chlorphenyl)pent-1-en-4-yl}-N-ethoxymethyl-3,5-dimethylbenzamid

Zu einer heftig gerührten Lösung von 5.5 g (16.8 mMol) N-{5-(4-Chlorphenyl)pent-1-en-4-yl}-3,5-dimethylbenzamid und 100 mg Benzyltributylammoniumchlorid in 15 ml 50 %-iger wässriger Natriumhydroxidlösung und 15 ml Dichlormethan werden bei 0-5° innerhalb von 2 Stunden 2.36 ml (25.2 mMol) Chlormethylethylether in kleinen Portionen zugegeben. Die organische Phase wird in Dichlormethan und Wasser aufgenommen, die organische Phase abgetrennt, über Natriumsulfat getrocknet und unter vermindertem Druck zur Trockne eingedampft. Der ölige Rückstand wird an Silicagel mit Essigsäureethylester/Hexan (1:4) als Laufmittel chromatographisch gereinigt; TLC (Essigsäureethylester/Hexan; 1:3): $R_f$ = 0.50;

$^1$H-NMR (300 MHz, $CDCl_3$): Gemisch von Rotameren, $\delta$ = 7.31-7.18 (m, 4H), 7.04-6.85 (m, 2.6H), 6.42 (br. s, 0.4H), 5.92-5.60 (m, 1H), 5.20-5.02 (m, 2H), 4.54-4.24 (m, 2H), 3.96-3.67 (m, 1H), 3.25-2.40 (m, 6H), 2.28

(s, ca 5H), 2.24 (s, ca 1H), 1.34-1.21 (m, ca 0.5H), 1.08 (t, J = 7, ca 2.5H).

c) (2R*,4S*)-2-(4-Chlorbenzyl)-1-(3,5-dimethylbenzoyl)-N-acetyl-4-piperidinamin

Zu einer auf -20° gekühlten Lösung von 1.0 g N-{5-(4-Chlorphenyl)pent-1-en-4-yl}-N-ethoxymethyl-3,5-dimethylbenzamid in Acetonitril gibt man nacheinander 0.61 ml Zinntetrachlorid und 0.24 ml Acetanhydrid. Die Reaktionsmischung wird 2 Stunden bei - 20° und 1 Stunde bei 25° nachgerührt, in gesättigte wässrige Natriumhydrogencarbonatlösung gegossen und mit Essigsäureethylester ausgezogen. Die organische Phase wird abgetrennt, über Natriumsulfat getrocknet und unter vermindertem Druck zur Trockne eingedampft. Die rohe Titelverbindung (oranges Öl) wird durch Chromatographie an Silicagel mit Dichlormethan/Methanol/25 %-ige Ammoniaklösung (95:5:0.1) als Laufmittel gereinigt. TLC (Dichlormethan/Methanol/25 %-ige Ammoniaklösung; 90:10:0.1): $R_f$ = 0.45; FD-MS: M+ = 398

d) (2R*,4S*)-2-(4-Chlorbenzyl)-1-(3,5-dimethylbenzoyl)-4-piperidinamin

Eine Suspension von 730 mg (1.83 mMol) (2R*,4S*)-2-(4-Chlorbenzyl)-1-(3,5-dimethylbenzoyl)-N-acetyl-4-piperidinamin in 6N Salzsäure wird 16 Stunden auf 100° erhitzt, wobei der Ausgangsstoff in Lösung geht. Das Reaktionsgemisch wird mit 10 %-iger wässriger Natriumcarbonatlösung basisch gestellt und mit Essigsäureethylester ausgezogen. Die vereinigten organischen Phasen werden über Natriumsulfat getrocknet und unter vermindertem Druck zur Trockne eingedampft. Die rohe Titelverbindung wird an Silicagel mit Dichlormethan/Methanol/25 %-ige Ammoniaklösung (90:10:0.1) als Laufmittel chromatographisch gereinigt und als fast farbloses Harz erhalten. TLC (Dichlormethan/Methanol/25 %-ige Ammoniaklösung; 90:10:0.1): $R_f$ = 0.26; FD-MS: (M+1)+ = 357

Beispiel 93: (2R*,4S*)-2-(3,4-Dichlorbenzyl)-1-(3,5-dimethylbenzoyl)-N-(4-chinolylmethyl)-4-piperidinamin

Die Titelverbindung der Formel

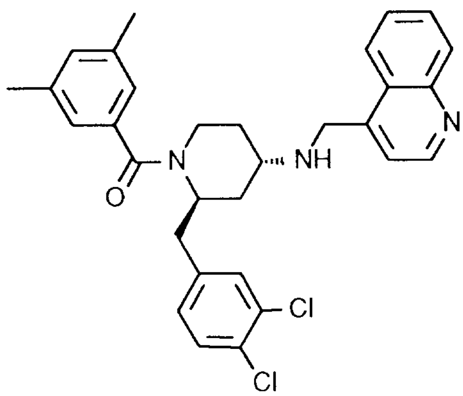

kann in analoger Weise wie in Beispiel 8 ausgehend von (2R*,4S*)-2-(3,4-Dichlorbenzyl)-1-(3,5-dimethylbenzoyl)-4-piperidinamin als Ausgangsstoff hergestellt werden: Smp. 121-124°; TLC (Dichlormethan/Methanol/25 %-ige Ammoniaklösung; 90:9.5:0.5): $R_f$ = 0.30; FD-MS: M+ = 531/533

Der Ausgangsstoff kann wie folgt hergestellt werden:

a) N-{5-(3,4-Dichlorphenyl)pent-1-ene-4-yl}-3,5-dimethylbenzamid

Die Titelverbindung wird in 93 %-iger Ausbeute in analoger Weise wie in Beispiel 8a beschrieben erhalten; Smp. 155-157°; IR-Spektrum (KBr): 3230, 1625, 1595 $cm^{-1}$; TLC (Hexan/Essigsäureethylester; 2:1): $R_f$ = 0.46

b) N-{5-(3,4-Dichlorphenyl)pent-1-ene-4-yl}-N-ethoxymethyl-3,5-dimethylbenzamid

Die Titelverbindung wird in analoger Weise wie in Beispiel 8b beschrieben erhalten; IR-Spektrum (Film): 1640, 1600 $cm^{-1}$; TLC (Hexan/Essigsäureethylester; 2:1): $R_f$ = 0.58

c) (2R*,4S*)-N-Acetyl-2-(3,4-dichlorbenzyl)-1-(3,5-dimethylbenzoyl)-4-piperidinamin

Die Titelverbindung wird in analoger Weise wie in Beispiel 8c beschrieben erhalten; IR-Spektrum (KBr): 3260, 1655, 1605, 1595, 1540 $cm^{-1}$ (KBr); TLC (Dichlormethan/Methanol; 10:1); $R_f$ = 0.32

d) (2R*,4S*)-2-(3,4-Dichlorbenzyl)-1-(3,5-dimethylbenzoyl)-4-piperidinamin

Die Titelverbindung wird in analoger Weise wie in Beispiel 8d beschrieben erhalten; TLC (Dichlormethan/Methanol/25 %-ige Ammoniaklösung; 300:25:3): $R_f$ = 0.46

Beispiel 94: (2R*,4S*)-1-(3,5-Dimethylbenzoyl)-2-phenyl-N-(4-chinolylmethyl)-4-piperidinamin

Eine Suspension von 211 mg (0.60 mMol) (2R*,4S*)-N-Acetyl-1-(3,5-dimethylbenzoyl)-2-phenyl-4-piperidinamin in 8 ml 6N Salzsäure wird unter Inertgas 16 Stunden auf 100° erhitzt, wobei sich der Ausgangsstoff vollständig auflöst. Man lässt auf Raumtemperatur abkühlen, neutralisiert mit Natriumcarbonatlösung und extrahiert mit Essigsäureethylester. Säulenchromatographie an Silicagel mit Dichlormethan/Methanol (95:5 bis 90:10) als Laufmittel ergibt 135 mg Amin, welches ohne weitere Reinigung mit 60 mg (0.38 mMol) Chinolin-4-carboxaldehyd in Toluol unter azeotroper Wasserentfernung weiterumgesetzt wird. Mach Abziehen des Lösungsmittels bleibt ein Öl zurück. Dieses wird in Ethanol aufgenommen und bei 0° mit 14 mg ( 0.38 mMol) Natriumborhydrid versetzt. Nach 1.5 Stunden wird das Reaktionsgemisch mit 1N Salzsäure behandelt, 1 Stunde bei 25° gerührt und schliesslich mit 10 % iger wässriger Natriumcarbonatlösung neutralisiert. Man schüttelt mit Essigsäureethylester aus, trennt die organische Phase ab, trocknet über Natriumsulfat und dampft unter vermindertem Druck zur Trockne ein. Die Titelverbindung der Formel

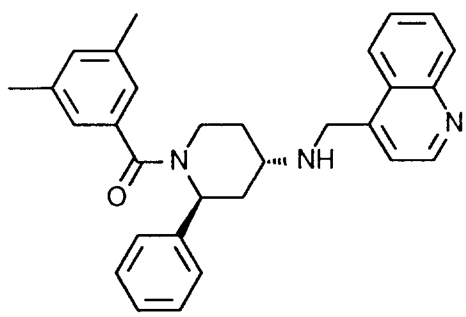

wird durch Säulenchromatographie an Silicagel mit Dichlormethan/Isopropanol (9:1) als Laufmittel isoliert; TLC (Dichlormethan/Isopropanol; 9:1): $R_f$ = 0.51; FD-MS: M+ = 449
Der Ausgangsstoff kann wie folgt hergestellt werden:

a) (2R*,4S*)-N-Acetyl-1-benzyloxycarbonyl-2-phenyl-4-piperidinamin

Zu einer Lösung von 2.03 g (9.90 mMol) N-But-3-en-1-yl-O-benzylcarboxamid und 1.14 g (10.7 mMol) Benzaldehyd in 1 ml (810.6 mMol) Acetanhydrid und 20 ml Acetonitril werden bei -20° 1.41 ml (12.0 mMol) Zinntetrachlorid zugegeben. Man hält 16 Stunden bei - 20 , behandelt dann mit 10 %-iger wässriger Natriumhydrogencarbonatlösung und extrahiert mit Essigsäureethylester. Die organische Phase wird über Natriumsulfat getrocknet und unter vermindertem Druck zur Trockne eingedampft. Die rohe Titelverbindung wird aus Essigester/Hexan kristallisiert; Smp. 139-140°; CI-MS: (M+H)+ = 353, $(M+NH_4)+$ = 370.

b) (2R*,4S*)-N-Acetyl-1-(3,5-dimethylbenzoyl)-2-phenyl-4-piperidinamin

Eine Lösung von 496 mg (1.41 mMol) (2R*,4S*)-N-Acetyl-1-benzyloxycarbonyl-2-phenyl-4-piperidinamin in 30 ml Ethanol und 8 ml 1N Salzsäure wird mit 50 mg 10 % -ige Pd/C-Katalysator versetzt und unter einer Wasserstoffatmosphäre gerührt, bis kein Wasserstoff mehr aufgenommen wird. Der Katalysator wird über Diatomeenerde abfiltriert und das Filtrat unter vermindertem Druck zur Trockne eingedampft. Der Rückstand wird in 5 ml Dichlormethan und 5 ml 10 %-iger wässriger Natriumhydrogencarbonatlösung aufgenommen und unter Rühren bei 0° innerhalb von 1 Stunde langsam mit 285 mg (1.69 mMol) 3,5-Dimethylbenzoylchlorid versetzt. Das Reaktionsgemisch wird mit Dichlormethan extrahiert. Die organische

Phase wird abgetrennt, über Natriumsulfat getrocknet und unter vermindertem Druck zur Trockne eingedampft. Die Titelverbindung kristallisiert aus Essigsäureethylester; Smp. 201-203°; CI-MS: (M+H)+ = 351.

Beispiel 95: (2R*,4S*)-1-(3,5-Dichlorbenzoyl)-2-phenyl-N-(4-chinolinmethyl)-4-piperidinamin

Die Titelverbindung der Formel

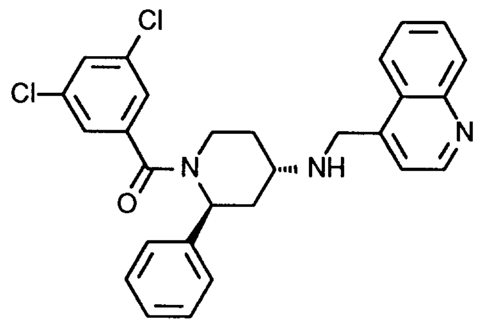

kann in analoger Weise wie in Beispiel 10 beschrieben erhalten werden; TLC (Dichlormethan/Methanol/25 %-ige Ammoniaklösung; 90:9.5:0:5): $R_f$ = 0.46; FD-MS: M+ = 489.

Das Ausgangsmaterial kann wie folgt hergestellt werden:

a) (2R*,4S*)-N-Acetyl-1-(3,5-dichlorbenzoyl)-2-phenyl-4-piperidinamin

Die Titelverbindung wird in analoger Weise wie in Beispiel 10b, ausgehend von 3,5-Dichlorbenzoylchlorid erhalten werden; Smp. 161-163°; TLC (Dichlormethan/Methanol/25 %-ige Ammoniaklösung; 90:9.5:0.5): $R_f$ = 0.55.

Beispiel 96: (2R*,4S*)-1-(1-Naphthoyl)-2-phenyl-N-(4-chinolylmethyl)-4-piperidinamin

Die Titelverbindung der Formel

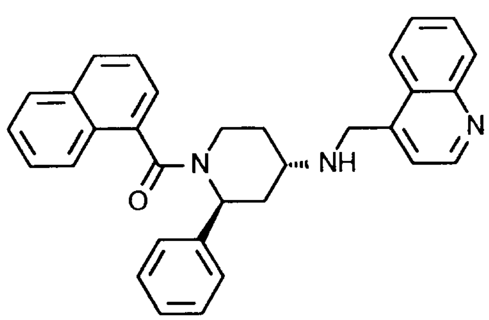

wird in analoger Beispiel wie in Beispiel 11 beschrieben erhalten; TLC (Dichlormethan/Methanol/25 %-ige Ammoniaklösung, 90:9.5:0.5): $R_f$ = 0.41; FD-MS: M+ = 471

Das Ausgangsmaterial wird wie folgt erhalten:

a) (2R*,4S*)-N-Acetyl-1-(1-naphthoyl)-2-phenyl-4-piperidinamin

Die Titelverbindung wird in analoger Weise wie in Beispiel 10b beschrieben erhalten; TLC (Dichlormethan/Methanol/25 %-ige Ammoniaklösung, 90:9.5:0.5): $R_f$ = 0.42.

Beispiel 97: (2R*,4S*)-1-(3,5-Dimethylbenzoyl)-2-(1-naphthyl)-N-(4-chinolylmethyl)-4-piperidinamin

Die Titelverbindung der Formel

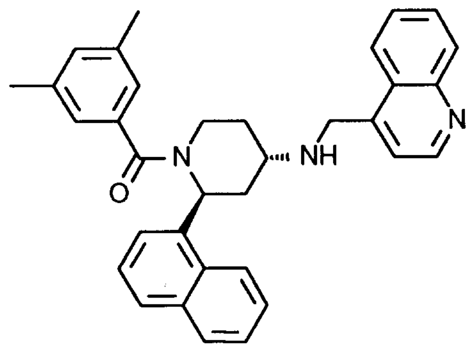

wird in analoger Weise wie in Beispiel 10 beschrieben erhalten; TLC (Dichlormethan/Methanol/25 %-ige Ammoniaklösung, 90:9.5:0.5): $R_f$ = 0.50 FD-MS: M+ = 499
Das Ausgangsmaterial wird wie folgt erhalten:

a) (2R*,4S*)-N-Acetyl-1-benzyloxycarbonyl-2-(1-naphthyl)-4-piperidinamin

Die Titelverbindung wird in analoger Weise wie in Beispiel 10a beschrieben, ausgehend von Naphthalin-1-carboxaldehyd statt Benzaldehyd erhalten; (Dichlormethan/Methanol/25 %-ige Ammoniaklösung, 90:9.5:0.5): $R_f$ = 0.31 FD-MS: M+ = 402.

b) (2R*,4S*)-(N)-Acetyl-1-(3,5-dimethylbenzoyl)-2-(1-naphthyl)-4-piperidinamin

Die Titelverbindung wird in analoger Weise wie in Beispiel 10b beschrieben erhalten; TLC (Dichlormethan/Methanol/25 %-ige Ammoniaklösung, 90:9.5:0.5): $R_f$ = 0.26 FD-MS: M+ = 400.

Beispiel 98:

In analoger Weise wie in den Beispielen 85 bis 97 beschrieben kann man auch die folgenden Verbindungen herstellen:
(2R*,4S*)-1-(3,5-Dimethylbenzoyl)-2-(2-naphthyl)-N-(4-chinolinylmethyl)-4-piperidinamin;
(2R*,4S*)-1-(3,5-Dimethylbenzoyl)-2-(4-methoxyphenylmethyl)-N-(4-chinolinylmethyl)-4-piperidinamin;
(2R*,4S*)-1-(3,5-Dimethylbenzoyl)-2-(3-methoxyphenylmethyl)-N-(4-chinolinylmethyl)-4-piperidinamin;
(2R*,4S*)-1-(3,5-Dimethylbenzoyl)-2-(4-nitrobenzyl)-N-(4-chinolinylmethyl)-4-piperidinamin;
(2R*,4S*)-1-(3,5-Dimethylbenzoyl)-2-(4-trifluormethylphenylmethyl)-N-(4-chinolinylmethyl)-4-piperidinamin;
(2R*,4S*)-1-(3,5-Dimethylbenzoyl)-2-(2,4-dichlorphenylmethyl)-N-(4-chinolinylmethyl)-4-piperidinamin;
(2R*,4S*)-1-(3,5-Dimethylbenzoyl)-2-(2-phenylethyl)-N-(4-chinolinylmethyl)-4-piperidinamin;
(2R*,4S*)-1-(3,5-Dimethylbenzoyl)-2-(2-phenylethenyl)-N-(4-chinolinylmethyl)-4-piperidinamin;
(2R*,4S*)-1-(3,5-Dimethylbenzoyl)-2-benzoyl-N-(4-chinolinylmethyl)-4-piperidinamin;
(2R*,4S*)-1-(3,5-Dimethylbenzoyl)-2-(4-chlorbenzoyl)-N-(4-chinolinylmethyl)-4-piperidinamin;
(2R*,4S*)-1-(3,5-Dimethylbenzoyl)-2-(2-naphthyl)-N-(4-chinolinylmethyl)-4-piperidinamin;
(2R*,4S*)-1-(3,5-Dimethylbenzoyl)-2-(4-methoxybenzyl)-N-(4-chinolinylmethyl)-4-piperidinamin;
(2R*,4S*)-1-(3,5-Dimethylbenzoyl)-2-(3-methoxybenzyl)-(4-chinolinylmethyl)-4-piperidinamin;
(2R*,4S*)-1-(3,5-Dimethylbenzoyl)-2-(4-nitrobenzyl)-(4-chinolinylmethyl)-4-piperidinamin;
(2R*,4S*)-1-(3,5-Dimethylbenzoyl)-2-(4-trifluormethylbenzyl)-(4-chinolinylmethyl)-4-piperidinamin;
(2R*,4S*)-1-(3,5-Dimethylbenzoyl)-2-(2,4-dichlorbenzyl)-(4-chinolinylmethyl)-4-piperidinamin;
(2R*,4S*)-1-(3,5-Dimethylbenzoyl)-2-(2-phenylethyl)-(4-chinolinylmethyl)-4-piperidinamin;
(2R*,4S*)-1-(3,5-Dimethylbenzoyl)-2-(2-phenylethenyl)-N-(4-chinolinylmethyl)-4-piperidinamin;
(2R*,4S*)-1-(3,5-Dimethylbenzoyl)-2-(benzoylmethyl)-N-(4-chinolinylmethyl)-4-piperidinamin;
(2R*,4S*)-1-(3,5-Dimethylbenzoyl)-2-(4-chlorbenzoylmethyl)-N-(4-chinolinylmethyl)-4-piperidinamin.

Beispiel 99:

Tabletten, enthaltend je 50 mg (2R,4S)-2-Benzyl-1-(3,5-dimethylbenzoyl)-N-(2-phenethyl)-4-piperidinaminoder ein Salz, z.B. das Hydrochlorid, davon können wie folgt hergestellt werden:

Zusammensetzung (10000 Tabletten)

| | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 100:

Lacktabletten, enthaltend je 100 mg (2R,4S)-2-Benzyl-1-(3,5-dimethylbenzoyl)-N-(2-phenethyl)-4-piperidinamin oder ein Salz, z.B. das Hydrochlorid, davon können wie folgt hergestellt werden:

Zusammensetzung (für 1000 Lacktabletten)

| | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 101:

Gelatinesteckkapseln, enthaltend 100 mg Wirkstoff, z.B. (2R,4S)-2-Benzyl-1-(3,5-dimethylbenzoyl)-N-(2-phenethyl)-4-piperidinaminoder ein Salz, z.B. das Hydrochlorid, davon, können z.B. folgendermassen hergestellt werden:

Zusammensetzung (für 1000 Kapseln)

| | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3-minütigem weiteren Mischen werden je 390 mg der erhaltenen Formulierung in Gelatinesteckkapseln der Grösse 0 abgefüllt.

Beispiel 102:

In analoger Weise wie in den vorstehenden Beispielen 99 bis 101 beschrieben kann man auch pharmazeutische Präparate, enthaltend eine andere Verbindung der Formel I gemäss einem der vorhergehenden Herstellungsbeispiele herstellen.

**Patentansprüche**
**Patentansprüche für folgende Vertragsstaaten : AT, BE, CH, DE, DK, FR, GB, IE, IT, LI, LU, NL, SE**

**1.** Eine 1-Acylpiperidinverbindung der Formel I

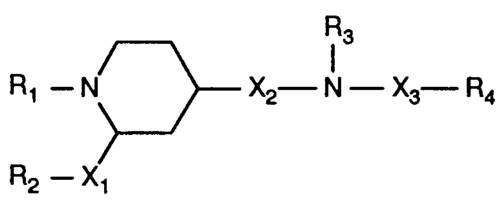

(I),

worin $R_1$ unsubstituiertes oder im Phenylteil durch Niederalkyl, Niederalkoxy, Diniederalkylamino, Halogen und/oder Trifluormethyl substituiertes Phenyl- oder Diphenyl-$C_1$-$C_4$-alkyl, unsubstituiertes oder im Phenyl durch Halogen und/oder Triazolyl substituiertes Phenoxy-$C_1$-$C_4$-alkyl, Pyridyl- oder Chinoliny-$C_1$-$C_4$-alkyl, unsubstituiertes oder durch Niederalkyl, Niederalkoxy, Diniederalkylamino, Halogen und/oder Trifluormethyl substituiertes Benzoyl, unsubstituiertes oder durch Niederalkyl, Niederalkoxy, Diniederalkylamino, Halogen und/oder Trifluormethyl substituiertes Naphthoyl, unsubstituiertes oder durch Niederalkyl, Niederalkoxy, Halogen und/oder Trifluormethyl substituiertes Pyridylcarbonyl oder Chinolinylcarbonyl, unsubstituiertes oder durch Niederalkyl, Niederalkoxy, Diniederalkylamino, Halogen und/oder Trifluormethyl substituiertes 5-bis 7-gliedriges Cycloalkylcarbonyl, unsubstituiertes oder im Phenyl durch Niederalkyl, Niederalkoxy, Diniederalkylamino, Halogen und/oder Trifluormethyl substituiertes Phenyl- oder Diphenyl-$C_1$-$C_4$-alkanoyl, unsubstituiertes oder im Phenylteil gegebenenfalls im Phenyl durch Niederalkyl, Niederalkoxy, Diniederalkylamino, Halogen und/oder Trifluormethyl substituiertes N-Phenylcarbamoyl oder eine Gruppe der Formel Ia

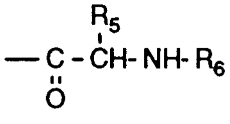

(Ia)

darstellt, in der $R_5$ Wasserstoff, unsubstituiertes oder durch Hydroxy, Mercapto, Amino, gegebenenfalls

durch Hydroxy substituiertes Phenyl, Carboxy, Carbamoyl oder Ureido substituiertes $C_1$-$C_4$-Alkyl und $R_6$ $C_2$-$C_7$-Alkanoyl bedeutet, $R_2$ 5- bis 7-gliedriges Cycloalkyl oder einen unsubstituierten oder durch aromatisch gebundenes $C_1$-$C_4$-Alkyl, $C_1$-$C_4$-Alkoxy, Halogen und/oder Trifluormethyl substituierten Phenyl-, Naphthyl- oder Pyridylrest darstellt, $R_3$ für Wasserstoff, $C_1$-$C_7$-Alkyl, Carbamoyl, $C_2$-$C_7$-Alkanoyl, Carboxy-$C_1$-$C_4$-alkanoyl oder Carboxy-$C_2$-$C_4$-alkenoyl steht, $R_4$ unsubstituiertes oder durch $C_1$-$C_4$-Alkyl, $C_1$-$C_4$-Alkoxy, Halogen und/oder Trifluormethyl substituiertes Phenyl oder Naphthyl oder unsubstituiertes Pyridyl, Benzofuranyl, Indolyl, 2,3-Dihydroindolyl, Benzimidazolyl, Chinolyl oder 1,2,3,4-Tetrahydrochinolinyl bedeutet, $X_1$ Methylen, Hydroxymethylen, $C_1$-$C_4$-Alkoxymethylen, Carbonyl oder Di-$C_1$-$C_4$-alkoxymethylen oder eine direkte Bindung darstellt, $X_2$ für $C_1$-$C_7$-Alkylen, Carbonyl oder eine direkte Bindung steht und $X_3$ Carbonyl, $C_1$-$C_4$-Alkylen, Carboxy-$C_1$-$C_4$-alkylen, $C_1$-$C_4$-Alkoxycarbonyl-$C_1$-$C_4$-alkylen, Carbamoyl-$C_1$-$C_4$-alkylen oder Hydroxymethyl-$C_1$-$C_4$-alkylen darstellt, oder ein Salz davon. Niederalkyl ist $C_1$-$C_7$-Alkyl.

**2.** Eine Verbindung gemäss Anspruch 1 der Formel I, worin $R_1$ unsubstituiertes oder durch $C_1$-$C_4$-Alkyl, $C_1$-$C_4$-Alkoxy, Halogen und/oder Trifluormethyl substituiertes Benzoyl, Naphthoyl oder Phenyl-$C_1$-$C_4$-alkanoyl, unsubstituiertes Pyridylcarbonyl oder Chinolinylcarbonyl oder eine Gruppe der Formel Ia

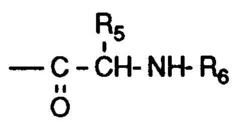

(Ia)

darstellt, in der $R_5$ Wasserstoff, unsubstituiertes oder durch Hydroxy, Mercapto, Amino, gegebenenfalls durch Hydroxy substituiertes Phenyl, Carboxy, Carbamoyl oder Ureido substituiertes $C_1$-$C_4$-Alkyl und $R_6$ $C_2$-$C_7$-Alkanoyl bedeutet, $R_2$ 5- bis 7-gliedriges Cycloalkyl oder einen unsubstituierten oder durch aromatisch gebundenes $C_1$-$C_4$-Alkyl, $C_1$-$C_4$-Alkoxy, Halogen und/oder Trifluormethyl substituierten Phenyl-, Naphthyl- oder Pyridylrest darstellt, $R_3$ für Wasserstoff, $C_1$-$C_7$-Alkyl, Carbamoyl, $C_2$-$C_7$-Alkanoyl, Carboxy-$C_1$-$C_4$-alkanoyl oder Carboxy-$C_3$-$C_5$-alkenoyl steht, $R_4$ unsubstituiertes oder durch $C_1$-$C_4$-Alkyl, $C_1$-$C_4$-Alkoxy, Halogen und/oder Trifluormethyl substituiertes Phenyl oder Naphthyl oder unsubstituiertes Pyridyl, Benzofuranyl, Indolyl, Benzimidazolyl oder Chinolyl bedeutet, $X_1$ Methylen, Hydroxymethylen, $C_1$-$C_4$-Alkoxymethylen, Carbonyl, Di-$C_1$-$C_4$-alkoxymethylen oder eine direkte Bindung darstellt, $X_2$ für $C_1$-$C_7$-Alkylen, Carbonyl oder eine direkte Bindung steht und $X_3$ Carbonyl, $C_1$-$C_4$-Alkylen, Carboxy-$C_1$-$C_4$-alkylen, $C_1$-$C_4$-Alkoxycarbonyl-$C_1$-$C_4$alkylen, Carbamoyl-$C_1$-$C_4$-alkylen, oder Hydroxymethyl-$C_1$-$C_4$-alkylen darstellt, oder ein Salz davon.

**3.** Eine Verbindung gemäss Anspruch 1 der Formel I, worin $R_1$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Benzoyl oder unsubstituiertes Naphthoyl oder Phenyl-$C_1$-$C_4$-alkanoyl bedeutet, $R_2$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Phenyl oder unsubstituiertes Pyridyl darstellt, $R_3$ für Wasserstoff, $C_1$-$C_4$-Alkyl, Carbamoyl oder $C_2$-$C_7$-Alkanoyl, steht, $R_4$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Phenyl oder unsubstituiertes Naphthyl, Pyridyl, Benzofuranyl, Indolyl, Benzimidazolyl oder Chinolyl bedeutet, $X_1$ Methylen, Hydroxymethylen, Carbonyl oder eine direkte Bindung darstellt, $X_2$ für eine direkte Bindung steht und $X_3$ $C_1$-$C_4$-Alkylen, darstellt, oder ein Salz davon.

**4.** Eine Verbindung gemäss Anspruch 1 der Formel I, worin $R_1$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Benzoyl oder unsubstituiertes Naphthoyl bedeutet, $R_2$ unsubstituiertes oder durch Halogen der Atomnummer bis und mit 35 und/oder Trifluormethyl mono- oder disubstituiertes Phenyl darstellt, $R_3$ für Wasserstoff steht, $R_4$ unsubstituiertes Chinolyl bedeutet, $X_1$ Methylen darstellt, $X_2$ für eine direkte Bindung steht und $X_3$ $C_1$-$C_4$-Alkylen darstellt, oder ein Salz davon.

**5.** (2R*,4S*)-2-Benzyl-1-(2-naphthoyl)-N-(4-chinolylmethyl)-4-piperidinamin oder ein Salz davon.

**6.** (2R*,4S*)-2-Benzyl-1-(3-trifluormethylbenzoyl)-N-(4-chinolylmethyl)-4-piperidinamin oder ein Salz davon.

7. (2R*,4S*)-2-Benzyl-1-(3,5-bis-(trifluormethyl)-benzoyl)-N-(4-chinolylmethyl)-4-piperidinamin oder ein Salz davon.

8. (2R*,4S*)-2-Benzyl-1-(3,5-dimethoxybenzoyl)-N-(4-chinolylmethyl)-4-piperidinamin oder ein Salz davon.

9. (2R*,4S*)-2-Benzyl-1-(1-naphthoyl)-N-(4-chinolylmethyl)-4-piperidinamin oder ein Salz davon.

10. (2R*,4S*)-2-Benzyl-1-(3,5-dichlorbenzoyl)-N-(4-chinolylmethyl)-4-piperidinamin oder ein Salz davon.

11. (2R*,4S*)-2-Benzyl-1-(2-chinolinylcarbonyl)-N-(4-chinolylmethyl)-4-piperidinamin oder ein Salz davon.

12. (2R*,4S*)-2-Benzyl-1-(4-chlor-phenylacetyl)-N-(4-chinolylmethyl)-4-piperidinamin oder ein Salz davon.

13. (2R*,4S*)-2-Benzyl-1-(benzyloxycarbonyl)-N-(4-chinolylmethyl)-4-piperidinamin oder ein Salz davon.

14. (2R*,4S*)-2-Benzyl-1-(2-phenylethyl)-N-(4-chinolylmethyl)-4-piperidinamin oder ein Salz davon.

15. (2R*,4S*)-2-Benzyl-1-(2-naphtylacetyl)-N-(4-chinolylmethyl)-4-piperidinamin oder ein Salz davon.

16. (2R*,4S*)-2-Benzyl-1-(4-chinolylmethyl)-N-(4-chinolylmethyl)-4-piperidinamin oder ein Salz davon.

17. (2R*,4S*)-2-Benzyl-1-(2,4-dichlorbenzyl)-N-(4-chinolylmethyl)-4-piperidinamin oder ein Salz davon.

18. (2R*,4S*)-2-Benzyl-1-(2,2-diphenylethyl)-N-(4-chinolylmethyl)-4-piperidinamin oder ein Salz davon.

19. (2R*,4S*)-2-Benzyl-1-(phenylcarbamoyl)-N-(4-chinolylmethyl)-4-piperidinamin oder ein Salz davon.

20. (2R*,4S*)-2-Benzyl-1-(diphenylacetyl)-N-(4-chinolylmethyl)-4-piperidinamin oder ein Salz davon.

21. (2R*,4S*)-2-Benzyl-1-(2-pyridylacetyl)-N-(4-chinolylmethyl)-4-piperidinamin oder ein Salz davon.

22. (2R*,4S*)-2-Benzyl-1-(4-pyridylacetyl)-N-(4-chinolylmethyl)-4-piperidinamin oder ein Salz davon.

23. (2R*,4S*)-2-Benzyl-1-(2,3-diphenylpropionyl)-N-(4-chinolylmethyl)-4-piperidinamin oder ein Salz davon.

24. (2R*,4S*)-2-Benzyl-1-((3S)-(2,3,4,9-tetrahydro-1H-pyrido[3,4-b]indol-3-yl)-carbonyl)-N-(4-chinolylmethyl)-4-piperidinamin oder ein Salz davon.

25. (2R*,4S*)-2-Benzyl-1-(3-methoxybenzoyl)-N-(4-chinolylmethyl)-4-piperidinamin oder ein Salz davon.

26. (2R*,4S*)-2-Benzyl-1-(3-N,N-dimethylaminobenzoyl)-N-(4-chinolylmethyl)-4-piperidinamin oder ein Salz davon.

27. (2R*,4S*)-2-Benzyl-1-(cis,cis-3,5-dimethylcyclohexylcarbonyl)-N-(4-chinolylmethyl)-4-piperidinamin oder ein Salz davon.

28. (2R*,4S*)-2-Benzyl-1-(3,5-bis-(trifluormethyl)-benzyl)-N-(4-chinolylmethyl)-4-piperidinamin oder ein Salz davon.

29. (2S*,4R*)-2-Benzyl-1-[2-(5-chlor-1H-1,2,4-triazol-1-yl)phenoxyethyl]-N-(4-chinolylmethyl)-4-piperidinamin oder ein Salz davon.

30. (2R*,4S*)-2-Benzyl-1-((S)-phenylalanyl)-N-(4-chinolylmethyl)-4-piperidinamin oder ein Salz davon.

31. (2R*,4S*)-2-Benzyl-1-((R)-phenylalanyl)-N-(4-chinolylmethyl)-4-piperidinamin oder ein Salz davon.

32. (2R*,4S*)-2-Benzyl-1-((S)-N-acetyl-phenylalayl)-N-(4-chinolylmethyl)-4-piperidinamin oder ein Salz davon.

**33.** (2R*,4S*)-2-Benzyl-1-((R)-N-acetyl-phenylalanyl)-N-(4-chinolylmethyl)-4-piperidinamin oder ein Salz davon.

**34.** (2R*,4S*)-2-Benzyl-1-((S)-N-(4-carboxamido-butyroyl)-phenylalanyl)-N-(4-chinolylmethyl)-4-piperidinamin oder ein Salz davon.

**35.** (2R*,4S*)-2-Benzyl-1-((R)-N-(4-carboxamido-butyroyl)-phenylalanyl)-N-(4-chinolylmethyl)-4-piperidinamin oder ein Salz davon.

**36.** (2R*,4S*)-2-Benzyl-1-benzoyl-N-(4-chinolylmethyl)-4-piperidinamin oder ein Salz davon.

**37.** (2R*,4S*)-2-Benzyl-1-(3-chlorbenzoyl)-N-(4-chinolylmethyl)-4-piperidinamin oder ein Salz davon.

**38.** (2R*,4S*)-2-Benzyl-1-(3,5-dichlorbenzoyl)-N-(4-chinolylmethyl)-N-(3-carboxamido-propionyl)-4-piperidinamin oder ein Salz davon.

**39.** (2R,4S)- oder (2R,4R)-2-Benzyl-1-(3,5-dimethylbenzoyl)-N-(4-chinolylmethyl)-4-piperidinamin oder ein Salz davon.

**40.** (2S,4R) und (2S,4S)-2-Benzyl-1-(3,5-dimethylbenzoyl)-N-(2-phenethyl)-4-piperidinamin oder ein Salz davon.

**41.** (2R,4S)-2-Benzyl-1-(3,5-dimethylbenzoyl)-N-(3-chinolylmethyl)-4-piperidinamin oder ein Salz davon.

**42.** (2R,4S)-2-Benzyl-1-(3,5-dimethylbenzoyl)-N-(2-chinolylmethyl)-4-piperidinamin oder ein Salz davon.

**43.** (2R,4S)- oder (2R,4R)-2-Benzyl-1-(3,5-dimethylbenzoyl)-N-benzyl-4-piperidinamin oder ein Salz davon.

**44.** (2S,4R)- oder (2S,4S)-2-Benzyl-1-(3,5-dimethylbenzoyl)-N-benzyl-4-piperidinamin oder ein Salz davon.

**45.** (2R,4S)- oder (2R,4R)-2-Benzyl-1-(3,5-dimethylbenzoyl)-N-(4-pyridylmethyl)-4-piperidinamin oder ein Salz davon.

**46.** (2R,4S)- oder (2R,4R)-2-Benzyl-1-(3,5-dimethylbenzoyl)-N-(3-pyridylmethyl)-4-piperidinamin oder ein Salz davon.

**47.** (2S,4R)- und (2S,4S)-2-Benzyl-1-(3,5-dimethylbenzoyl)-N-(4-chinolylmethyl)-4-piperidinamin oder ein Salz davon.

**48.** (2R*,4S*)-2-Benzyl-1-(3,5-dimethylbenzoyl)-N-benzyl-N-carbamoyl-4-piperidinamin oder ein Salz davon.

**49.** (2R,4S)-2-Benzyl-1-(3,5-dimethylbenzoyl)-N-(3-phenylpropyl)-4-piperidinamin;
(2S,4S)-2-Benzyl-1-(3,5-dimethylbenzoyl)-N-(3-phenylpropyl)-4-piperidinamin;
(2R,4S)-2-Benzyl-1-(3,5-dimethylbenzoyl)-N-(2-methoxybenzyl)-4-piperidinamin;
(2S,4S)-2-Benzyl-1-(3,5-dimethylbenzoyl)-N-(2-methoxybenzyl)-4-piperidinamin;
(2R,4S)-2-Benzyl-1-(3,5-dimethylbenzoyl)-N-[2-(2-methoxyphenyl)ethyl]-4-piperidinamin;
(2S,4S)-2-Benzyl-1-(3,5-dimethylbenzoyl)-N-[2-(2-methoxyphenyl)ethyl]-4-piperidinamin;
(2R,4S)-2-Benzyl-1-(3,5-dimethylbenzoyl)-N-[3-(2-methoxyphenyl)propyl]-4-piperidinamin;
(2S,4S)-2-Benzyl-1-(3,5-dimethylbenzoyl)-N-[3-(2-methoxyphenyl)propyl]-4-piperidinamin;
(2R,4S)-2-Benzyl-1-(3,5-dimethylbenzoyl)-N-(2-trifluormethylbenzyl)-4-piperidinamin;
(2S,4S)-2-Benzyl-1-(3,5-dimethylbenzoyl)-N-(2-trifluormethylbenzyl)-4-piperidinamin;
(2R,4S)-2-Benzyl-1-(3,5-dimethylbenzoyl)-N-[2-(2-trifluormethylphenyl)ethyl]-4-piperidinamin;
(2S,4S)-2-Benzyl-1-(3,5-dimethylbenzoyl)-N-[2-(2-trifluormethylphenyl)ethyl]-4-piperidinamin;
(2R,4S)-2-Benzyl-1-(3,5-dimethylbenzoyl)-N-[3-(2-trifluormethylphenyl)propyl]-4-piperidinamin;
(2S,4S)-2-Benzyl-1-(3,5-dimethylbenzoyl)-N-[3-(2-trifluormethylphenyl)propyl]-4-piperidinamin;
(2R,4S)-2-Benzyl-1-(3,5-dimethylbenzoyl)-N-(3-trifluormethylbenzyl)-4-piperidinamin;
(2S,4S)-2-Benzyl-1-(3,5-dimethylbenzoyl)-N-(3-trifluormethylbenzyl)-4-piperidinamin;
(2R,4S)-2-Benzyl-1-(3,5-dimethylbenzoyl)-N-[2-(3-trifluormethylphenyl)ethyl]-4-piperidinamin;

(2S,4S)-2-Benzyl-1-(3,5-dimethylbenzoyl)-N-[2-(3-trifluormethylphenyl)ethyl]-4-piperidinamin;
(2R,4S)-2-Benzyl-1-(3,5-dimethylbenzoyl)-N-[3-(3-trifluormethylphenyl)propyl]-4-piperidinamin;
(2S,4S)-2-Benzyl-1-(3,5-dimethylbenzoyl)-N-[3-(3-trifluormethylphenyl)propyl]-4-piperidinamin;
(2R,4S)-2-Benzyl-1-(3,5-dimethylbenzoyl)-N-(4-trifluormethylbenzyl)-4-piperidinamin;
(2S,4S)-2-Benzyl-1-(3,5-dimethylbenzoyl)-N-(4-trifluormethylbenzyl)-4-piperidinamin;
(2R,4S)-2-Benzyl-1-(3,5-dimethylbenzoyl)-N-[2-(4-trifluormethylphenyl)ethyl]-4-piperidinamin;
(2S,4S)-2-Benzyl-1-(3,5-dimethylbenzoyl)-N-[2-(4-trifluormethylphenyl)ethyl]-4-piperidinamin;
(2R,4S)-2-Benzyl-1-(3,5-dimethylbenzoyl)-N-[3-(4-trifluormethylphenyl)propyl]-4-piperidinamin;
(2S,4S)-2-Benzyl-1-(3,5-dimethylbenzoyl)-N-[3-(4-trifluormethylphenyl)propyl]-4-piperidinamin;
(2R,4S)-2-Benzyl-1-(3,5-dimethylbenzoyl)-N-(2,3-dimethoxybenzyl)-4-piperidinamin;
(2S,4S)-2-Benzyl-1-(3,5-dimethylbenzoyl)-N-(2,3-dimethoxybenzyl)-4-piperidinamin;
(2R,4S)-2-Benzyl-1-(3,5-dimethylbenzoyl)-N-[2-(2,3-dimethoxyphenyl)ethyl]-4-piperidinamin;
(2S,4S)-2-Benzyl-1-(3,5-dimethylbenzoyl)-N-[2-(2,3-dimethoxyphenyl)ethyl]-4-piperidinamin;
(2R,4S)-2-Benzyl-1-(3,5-dimethylbenzoyl)-N-[3-(2,3-dimethoxyphenyl)propyl]-4-piperidinamin;
(2S,4S)-2-Benzyl-1-(3,5-dimethylbenzoyl)-N-[3-(2,3-dimethoxyphenyl)propyl]-4-piperidinamin;
(2R,4S)-2-Benzyl-1-(3,5-dimethylbenzoyl)-N-(2,4-dimethoxybenzyl)-4-piperidinamin;
(2S,4S)-2-Benzyl-1-(3,5-dimethylbenzoyl)-N-(2,4-dimethoxybenzyl)-4-piperidinamin;
(2R,4S)-2-Benzyl-1-(3,5-dimethylbenzoyl)-N-[2-(2,4-dimethoxyphenyl)ethyl]-4-piperidinamin;
(2S,4S)-2-Benzyl-1-(3,5-dimethylbenzoyl)-N-[2-(2,4-dimethoxyphenyl)ethyl]-4-piperidinamin;
(2R,4S)-2-Benzyl-1-(3,5-dimethylbenzoyl)-N-[3-(2,4-dimethoxyphenyl)propyl]-4-piperidinamin;
(2S,4S)-2-Benzyl-1-(3,5-dimethylbenzoyl)-N-[3-(2,4-dimethoxyphenyl)propyl]-4-piperidinamin;
(2R,4S)-2-Benzyl-1-(3,5-dimethylbenzoyl)-N-(2,5-dimethoxybenzyl)-4-piperidinamin;
(2S,4S)-2-Benzyl-1-(3,5-dimethylbenzoyl)-N-(2,5-dimethoxybenzyl)-4-piperidinamin;
(2R,4S)-2-Benzyl-1-(3,5-dimethylbenzoyl)-N-[2-(2,5-dimethoxyphenyl)ethyl]-4-piperidinamin;
(2S,4S)-2-Benzyl-1-(3,5-dimethylbenzoyl)-N-[2-(2,5-dimethoxyphenyl)ethyl]-4-piperidinamin;
(2R,4S)-2-Benzyl-1-(3,5-dimethylbenzoyl)-N-[3-(2,5-dimethoxyphenyl)propyl]-4-piperidinamin;
(2S,4S)-2-Benzyl-1-(3,5-dimethylbenzoyl)-N-[3-(2,5-dimethoxyphenyl)propyl]-4-piperidinamin;
(2R,4S)-2-Benzyl-1-(3,5-dimethylbenzoyl)-N-(2,6-dimethoxybenzyl)-4-piperidinamin;
(2S,4S)-2-Benzyl-1-(3,5-dimethylbenzoyl)-N-(2,6-dimethoxybenzyl)-4-piperidinamin;
(2R,4S)-2-Benzyl-1-(3,5-dimethylbenzoyl)-N-[2-(2,6-dimethoxyphenyl)ethyl]-4-piperidinamin;
(2S,4S)-2-Benzyl-1-(3,5-dimethylbenzoyl)-N-[2-(2,6-dimethoxyphenyl)ethyl]-4-piperidinamin;
(2R,4S)-2-Benzyl-1-(3,5-dimethylbenzoyl)-N-[3-(2,6-dimethoxyphenyl)propyl]-4-piperidinamin;
(2S,4S)-2-Benzyl-1-(3,5-dimethylbenzoyl)-N-[3-(2,6-dimethoxyphenyl)propyl]-4-piperidinamin;
(2R,4S)-2-Benzyl-1-(3,5-dimethylbenzoyl)-N-(2,3-methylendioxybenzyl)-4-piperidinamin;
(2S,4S)-2-Benzyl-1-(3,5-dimethylbenzoyl)-N-(2,3-methylendioxybenzyl)-4-piperidinamin;
(2R,4S)-2-Benzyl-1-(3,5-dimethylbenzoyl)-N-[2-(2,3-methylendioxyphenyl)ethyl]-4-piperidinamin;
(2S,4S)-2-Benzyl-1-(3,5-dimethylbenzoyl)-N-[2-(2,3-methylendioxyphenyl)ethyl]-4-piperidinamin;
(2R,4S)-2-Benzyl-1-(3,5-dimethylbenzoyl)-N-[3-(2,3-methylendioxyphenyl)propyl]-4-piperidinamin;
(2S,4S)-2-Benzyl-1-(3,5-dimethylbenzoyl)-N-[3-(2,3-methylendioxyphenyl)propyl]-4-piperidinamin;
(2R,4S)-2-Benzyl-1-(3,5-dimethylbenzoyl)-N-(2,4-methylendioxybenzyl)-4-piperidinamin;
(2S,4S)-2-Benzyl-1-(3,5-dimethylbenzoyl)-N-(2,4-methylendioxybenzyl)-4-piperidinamin;
(2R,4S)-2-Benzyl-1-(3,5-dimethylbenzoyl)-N-[2-(2,4-methylendioxyphenyl)ethyl]-4-piperidinamin;
(2S,4S)-2-Benzyl-1-(3,5-dimethylbenzoyl)-N-[2-(2,4-methylendioxyphenyl)ethyl]-4-piperidinamin;
(2R,4S)-2-Benzyl-1-(3,5-dimethylbenzoyl)-N-[3-(2,4-methylendioxyphenyl)propyl]-4-piperidinamin;
(2S,4S)-2-Benzyl-1-(3,5-dimethylbenzoyl)-N-[3-(2,4-methylendioxyphenyl)propyl]-4-piperidinamin;
(2R,4S)-2-Benzyl-1-(3,5-dimethylbenzoyl)-N-(indol-2-ylmethyl)-4-piperidinamin;
(2S,4S)-2-Benzyl-1-(3,5-dimethylbenzoyl)-N-(indol-2-ylmethyl)-4-piperidinamin;
(2R,4S)-2-Benzyl-1-(3,5-dimethylbenzoyl)-N-(indol-3-ylmethyl)-4-piperidinamin;
(2S,4S)-2-Benzyl-1-(3,5-dimethylbenzoyl)-N-(indol-3-ylmethyl)-4-piperidinamin;
(2S,4S)-2-Benzyl-1-(3,5-dimethylbenzoyl)-N-(chinolin-2-ylmethyl)-4-piperidinamin;
(2S,4S)-2-Benzyl-1-(3,5-dimethylbenzoyl)-N-(chinolin-3-ylmethyl)-4-piperidinamin;
(2R,4S)-2-Benzyl-1-(3,5-dimethylbenzoyl)-N-(2-chlorbenzyl)-4-piperidinamin;
(2S,4S)-2-Benzyl-1-(3,5-dimethylbenzoyl)-N-(2-chlorbenzyl)-4-piperidinamin;
(2R,4S)-2-Benzyl-1-(3,5-dimethylbenzoyl)-N-[2-(2-chlorphenyl)ethyl]-4-piperidinamin;
(2S,4S)-2-Benzyl-1-(3,5-dimethylbenzoyl)-N-[2-(2-chlorphenyl)ethyl]-4-piperidinamin;
(2R,4S)-2-Benzyl-1-(3,5-dimethylbenzoyl)-N-[3-(2-chlorphenyl)propyl]4-piperidinamin;
(2S,4S)-2-Benzyl-1-(3,5-dimethylbenzoyl)-N-[3-(2-chlorphenyl)propyl]-4-piperidinamin;
(2R,4S)-2-Benzyl-1-(3,5-dimethylbenzoyl)-N-(3-chlorbenzyl)-4-piperidinamin;

(2S,4S)-2-Benzyl-1-(3,5-dimethylbenzoyl)-N-(3-chlorbenzyl)-4-piperidinamin;
(2R,4S)-2-Benzyl-1-(3,5-dimethylbenzoyl)-N-[2-(3-chlorphenyl)ethyl]-4-piperidinamin;
(2S,4S)-2-Benzyl-1-(3,5-dimethylbenzoyl)-N-[2-(3-chlorphenyl)ethyl]-4-piperidinamin;
(2R,4S)-2-Benzyl-1-(3,5-dimethylbenzoyl)-N-[3-(3-chlorphenyl)propyl]-4-piperidinamin;
(2S,4S)-2-Benzyl-1-(3,5-dimethylbenzoyl)-N-[3-(3-chlorphenyl)propyl]-4-piperidinamin;
(2R,4S)-2-Benzyl-1-(3,5-dimethylbenzoyl)-N-(4-chlorbenzyl)-4-piperidinamin;
(2S,4S)-2-Benzyl-1-(3,5-dimethylbenzoyl)-N-(4-chlorbenzyl)-4-piperidinamin;
(2R,4S)-2-Benzyl-1-(3,5-dimethylbenzoyl)-N-[2-(4-chlorphenyl)ethyl]-4-piperidinamin;
(2S,4S)-2-Benzyl-1-(3,5-dimethylbenzoyl)-N-[2-(4-chlorphenyl)ethyl]-4-piperidinamin;
(2R,4S)-2-Benzyl-1-(3,5-dimethylbenzoyl)-N-[3-(4-chlorphenyl)propyl]-4-piperidinamin;
(2S,4S)-2-Benzyl-1-(3,5-dimethylbenzoyl)-N-[3-(4-chlorphenyl)propyl]-4-piperidinamin;
(2R,4S)-2-Benzyl-1-(3,5-dimethylbenzoyl)-N-(4-methoxynaphth-1-ylmethyl)-4-piperidinamin;
(2S,4S)-2-Benzyl-1-(3,5-dimethylbenzoyl)-N-(4-methoxynaphth-1-ylmethyl)-4-piperidinamin;
(2R,4S)-2-Benzyl-1-(3,5-dimethylbenzoyl)-N-(3,4-ethylendioxybenzyl)-4-piperidinamin;
(2S,4S)-2-Benzyl-1-(3,5-dimethylbenzoyl)-N-(3,4-ethylendioxybenzyl)-4-piperidinamin;
(2R,4S)-2-Benzyl-1-(3,5-dimethylbenzoyl)-N-(indol-2-ylmethyl)-4-piperidinamin;
(2S,4S)-2-Benzyl-1-(3,5-dimethylbenzoyl)-N-(indol-2-ylmethyl)-4-piperidinamin;
(2R,4S)-2-Benzyl-1-(3,5-dimethylbenzoyl)-N-(indol-3-ylmethyl)-4-piperidinamin;
(2S,4S)-2-Benzyl-1-(3,5-dimethylbenzoyl)-N-(indol-3-ylmethyl)-4-piperidinamin;
(2R,4S)-2-Benzyl-1-(3,5-dimethylbenzoyl)-N-(chinolin-2-ylmethyl)-4-piperidinamin;
(2S,4S)-2-Benzyl-1-(3,5-dimethylbenzoyl)-N-(chinolin-2-ylmethyl)-4-piperidinamin;
(2R,4S)-2-Benzyl-1-(3,5-dimethylbenzoyl)-N-(chinolin-3-ylmethyl)-4-piperidinamin;
(2S,4S)-2-Benzyl-1-(3,5-dimethylbenzoyl)-N-(chinolin-3-ylmethyl)-4-piperidinamin;
(2R,4S)-2-Benzyl-1-(3,5-dimethylbenzoyl)-N-(2-chlorbenzyl)-4-piperidinamin;
(2S,4S)-2-Benzyl-1-(3,5-dimethylbenzoyl)-N-(2-chlorbenzyl)-4-piperidinamin;
(2R,4S)-2-Benzyl-1-(3,5-dimethylbenzoyl)-N-[2-(2-chlorphenyl)ethyl]-4-piperidinamin;
(2S,4S)-2-Benzyl-1-(3,5-dimethylbenzoyl)-N-[2-(2-chlorphenyl)ethyl]-4-piperidinamin;
(2R,4S)-2-Benzyl-1-(3,5-dimethylbenzoyl)-N-[3-(2-chlorphenyl)propyl]-4-piperidinamin;
(2S,4S)-2-Benzyl-1-(3,5-dimethylbenzoyl)-N-[3-(2-chlorphenyl)propyl]-4-piperidinamin;
(2R,4S)-2-Benzyl-1-(3,5-dimethylbenzoyl)-N-(3-chlorbenzyl)-4-piperidinamin;
(2S,4S)-2-Benzyl-1-(3,5-dimethylbenzoyl)-N-(3-chlorbenzyl)-4-piperidinamin;
(2R,4S)-2-Benzyl-1-(3,5-dimethylbenzoyl)-N-[2-(3-chlorphenyl)ethyl]-4-piperidinamin;
(2S,4S)-2-Benzyl-1-(3,5-dimethylbenzoyl)-N-[2-(3-chlorphenyl)ethyl]-4-piperidinamin;
(2R,4S)-2-Benzyl-1-(3,5-dimethylbenzoyl)-N-[3-(3-chlorphenyl)propyl]-4-piperidinamin;
(2S,4S)-2-Benzyl-1-(3,5-dimethylbenzoyl)-N-[3-(3-chlorphenyl)propyl]-4-piperidinamin;
(2R,4S)-2-Benzyl-1-(3,5-dimethylbenzoyl)-N-(4-chlorbenzyl)-4-piperidinamin;
(2S,4S)-2-Benzyl-1-(3,5-dimethylbenzoyl)-N-(4-chlorbenzyl)-4-piperidinamin;
(2R,4S)-2-Benzyl-1-(3,5-dimethylbenzoyl)-N-[2-(4-chlorphenyl)ethyl]-4-piperidinamin;
(2S,4S)-2-Benzyl-1-(3,5-dimethylbenzoyl)-N-[2-(4-chlorphenyl)ethyl]-4-piperidinamin;
(2R,4S)-2-Benzyl-1-(3,5-dimethylbenzoyl)-N-[3-(4-chlorphenyl)propyl]-4-piperidinamin;
(2S,4S)-2-Benzyl-1-(3,5-dimethylbenzoyl)-N-[3-(4-chlorphenyl)propyl]-4-piperidinamin;
(2R,4S)-2-Benzyl-1-(3,5-dimethylbenzoyl)-N-(4-methoxynaphth-1-ylmethyl)-4-piperidinamin;
(2S,4S)-2-Benzyl-1-(3,5-dimethylbenzoyl)-N-(4-methoxynaphth-1-ylmethyl)-4-piperidinamin;
(2R,4S)-2-Benzyl-1-(3,5-dimethylbenzoyl)-N-(3,4-ethylendioxybenzyl)-4-piperidinamin;
(2S,4S)-2-Benzyl-1-(3,5-dimethylbenzoyl)-N-(3,4-ethylendioxybenzyl)-4-piperidinamin;
(2R,4S)-2-Benzyl-1-(3,5-ditrifluormethylbenzoyl)-N-(chinolin-2-ylmethyl)-4-piperidinamin;
(2S,4S)-2-Benzyl-1-(3,5-ditrifluormethylbenzoyl)-N-(chinolin-2-ylmethyl)-4-piperidinamin;
(2R,4S)-2-Benzyl-1-(3,5-ditrifluormethylbenzoyl)-N-(chinolin-3-ylmethyl)-4-piperidinamin;
(2S,4S)-2-Benzyl-1-(3,5-ditrifluormethylbenzoyl)-N-(chinolin-3-ylmethyl)-4-piperidinamin;
(2R,4S)-2-Benzyl-1-(3,5-ditrifluormethylbenzoyl)-N-(2-chlorbenzyl)-4-piperidinamin;
(2S,4S)-2-Benzyl-1-(3,5-ditrifluormethylbenzoyl)-N-(2-chlorbenzyl)-4-piperidinamin;
(2R,4S)-2-Benzyl-1-(3,5-ditrifluormethylbenzoyl)-N-[2-(2-chlorphenyl)ethyl]-4-piperidinamin;
(2S,4S)-2-Benzyl-1-(3,5-ditrifluormethylbenzoyl)-N-[2-(2-chlorphenyl)ethyl]-4-piperidinamin;
(2R,4S)-2-Benzyl-1-(3,5-ditrifluormethylbenzoyl)-N-[3-(2-chlorphenyl)propyl]-4-piperidinamin;
(2S,4S)-2-Benzyl-1-(3,5-ditrifluormethylbenzoyl)-N-[3-(2-chlorphenyl)propyl]-4-piperidinamin;
(2R,4S)-2-Benzyl-1-(3,5-ditrifluormethylbenzoyl)-N-(3-chlorbenzyl)-4-piperidinamin;
(2S,4S)-2-Benzyl-1-(3,5-ditrifluormethylbenzoyl)-N-(3-chlorbenzyl)-4-piperidinamin;
(2R,4S)-2-Benzyl-1-(3,5-ditrifluormethylbenzoyl)-N-[2-(3-chlorphenyl)ethyl]-4-piperidinamin;

(2S,4S)-2-Benzyl-1-(3,5-ditrifluormethylbenzoyl)-N-[2-(3-chlorphenyl)ethyl]-4-piperidinamin;
(2R,4S)-2-Benzyl-1-(3,5-ditrifluormethylbenzoyl)-N-[3-(3-chlorphenyl)propyl]-4-piperidinamin;
(2S,4S)-2-Benzyl-1-(3,5-ditrifluormethylbenzoyl)-N-[3-(3-chlorphenyl)propyl]-4-piperidinamin;
(2R,4S)-2-Benzyl-1-(3,5-ditrifluormethylbenzoyl)-N-(4-chlorbenzyl)-4-piperidinamin;
(2S,4S)-2-Benzyl-1-(3,5-ditrifluormethylbenzoyl)-N-(4-chlorbenzyl)-4-piperidinamin;
(2R,4S)-2-Benzyl-1-(3,5-ditrifluormethylbenzoyl)-N-[2-(4-chlorphenyl)ethyl]-4-piperidinamin;
(2S,4S)-2-Benzyl-1-(3,5-ditrifluormethylbenzoyl)-N-[2-(4-chlorphenyl)ethyl]-4-piperidinamin;
(2R,4S)-2-Benzyl-1-(3,5-ditrifluormethylbenzoyl)-N-[3-(4-chlorphenyl)propyl]-4-piperidinamin;
(2S,4S)-2-Benzyl-1-(3,5-ditrifluormethylbenzoyl)-N-[3-(4-chlorphenyl)propyl]-4-piperidinamin;
(2R,4S)-2-Benzyl-1-(3,5-ditrifluormethylbenzoyl)-N-(4-methoxynaphth-1-ylmethyl)-4-piperidinamin;
(2S,4S)-2-Benzyl-1-(3,5-ditrifluormethylbenzoyl)-N-(4-methoxynaphth-1-ylmethyl)-4-piperidinamin;
(2R,4S)-2-Benzyl-1-(3,5-ditrifluormethylbenzoyl)-N-(3,4-ethylendioxybenzyl)-4-piperidinamin;
(2S,4S)-2-Benzyl-1-(3,5-ditrifluormethylbenzoyl)-N-(3,4-ethylendioxybenzyl)-4-piperidinamin;
(2R,4S)-2-Benzyl-1-(3,5-dichlorbenzoyl)-N-(indol-2-ylmethyl)-4-piperidinamin;
(2S,4S)-2-Benzyl-1-(3,5-dichlorbenzoyl)-N-(indol-2-ylmethyl)-4-piperidinamin;
(2R,4S)-2-Benzyl-1-(3,5-dichlorbenzoyl)-N-(indol-3-ylmethyl)-4-piperidinamin;
(2S,4S)-2-Benzyl-1-(3,5-dichlorbenzoyl)-N-(indol-3-ylmethyl)-4-piperidinamin;
(2R,4S)-2-Benzyl-1-(3,5-dichlorbenzoyl)-N-(chinolin-2-ylmethyl)-4-piperidinamin;
(2S,4S)-2-Benzyl-1-(3,5-dichlorbenzoyl)-N-(chinolin-2-ylmethyl)-4-piperidinamin;
(2R,4S)-2-Benzyl-1-(3,5-dichlorbenzoyl)-N-(chinolin-3-ylmethyl)-4-piperidinamin;
(2S,4S)-2-Benzyl-1-(3,5-dichlorbenzoyl)-N-(chinolin-3-ylmethyl)-4-piperidinamin;
(2R,4S)-2-Benzyl-1-(3,5-dichlorbenzoyl)-N-(2-chlorbenzyl)-4-piperidinamin;
(2S,4S)-2-Benzyl-1-(3,5-dichlorbenzoyl)-N-(2-chlorbenzyl)-4-piperidinamin;
(2R,4S)-2-Benzyl-1-(3,5-dichlorbenzoyl)-N-[2-(2-chlorphenyl)ethyl]-4-piperidinamin;
(2S,4S)-2-Benzyl-1-(3,5-dichlorbenzoyl)-N-[2-(2-chlorphenyl)ethyl]-4-piperidinamin;
(2R,4S)-2-Benzyl-1-(3,5-dichlorbenzoyl)-N-[3-(2-chlorphenyl)propyl]-4-piperidinamin;
(2S,4S)-2-Benzyl-1-(3,5-dichlorbenzoyl)-N-[3-(2-chlorphenyl)propyl]-4-piperidinamin;
(2R,4S)-2-Benzyl-1-(3,5-dichlorbenzoyl)-N-(3-chlorbenzyl)-4-piperidinamin;
(2S,4S)-2-Benzyl-1-(3,5-dichlorbenzoyl)-N-(3-chlorbenzyl)-4-piperidinamin;
(2R,4S)-2-Benzyl-1-(3,5-dichlorbenzoyl)-N-[2-(3-chlorphenyl)ethyl]-4-piperidinamin;
(2S,4S)-2-Benzyl-1-(3,5-dichlorbenzoyl)-N-[2-(3-chlorphenyl)ethyl]-4-piperidinamin;
(2R,4S)-2-Benzyl-1-(3,5-dichlorbenzoyl)-N-[3-(3-chlorphenyl)propyl]-4-piperidinamin;
(2S,4S)-2-Benzyl-1-(3,5-dichlorbenzoyl)-N-[3-(3-chlorphenyl)propyl]-4-piperidinamin;
(2R,4S)-2-Benzyl-1-(3,5-dichlorbenzoyl)-N-(4-chlorbenzyl)-4-piperidinamin;
(2S,4S)-2-Benzyl-1-(3,5-dichlorbenzoyl)-N-(4-chlorbenzyl)-4-piperidinamin;
(2R,4S)-2-Benzyl-1-(3,5-dichlorbenzoyl)-N-[2-(4-chlorphenyl)ethyl]-4-piperidinamin;
(2S,4S)-2-Benzyl-1-(3,5-dichlorbenzoyl)-N-[2-(4-chlorphenyl)ethyl]-4-piperidinamin;
(2R,4S)-2-Benzyl-1-(3,5-dichlorbenzoyl)-N-[3-(4-chlorphenyl)propyl]-4-piperidinamin;
(2S,4S)-2-Benzyl-1-(3,5-dichlorbenzoyl)-N-[3-(4-chlorphenyl)propyl]-4-piperidinamin;
(2R,4S)-2-Benzyl-1-(3,5-dichlorbenzoyl)-N-(4-methoxynaphth-1-ylmethyl)-4-piperidinamin;
(2S,4S)-2-Benzyl-1-(3,5-dichlorbenzoyl)-N-(4-methoxynaphth-1-ylmethyl)-4-piperidinamin;
(2R,4S)-2-Benzyl-1-(3,5-dichlorbenzoyl)-N-(3,4-ethylendioxybenzyl)-4-piperidinamin;
(2S,4S)-2-Benzyl-1-(3,5-dichlorbenzoyl)-N-(3,4-ethylendioxybenzyl)-4-piperidinamin;
(2R,4S)-2-Benzyl-1-(3,5-dimethoxybenzoyl)-N-(indol-2-ylmethyl)-4-piperidinamin;
(2S,4S)-2-Benzyl-1-(3,5-dimethoxybenzoyl)-N-(indol-2-ylmethyl)-4-piperidinamin;
(2R,4S)-2-Benzyl-1-(3,5-dimethoxybenzoyl)-N-(indol-3-ylmethyl)-4-piperidinamin;
(2S,4S)-2-Benzyl-1-(3,5-dimethoxybenzoyl)-N-(indol-3-ylmethyl)-4-piperidinamin;
(2R,4S)-2-Benzyl-1-(3,5-dimethoxybenzoyl)-N-(chinolin-2-ylmethyl)-4-piperidinamin;
(2S,4S)-2-Benzyl-1-(3,5-dimethoxybenzoyl)-N-(chinolin-2-ylmethyl)-4-piperidinamin;
(2R,4S)-2-Benzyl-1-(3,5-dimethoxybenzoyl)-N-(chinolin-3-ylmethyl)-4-piperidinamin;
(2S,4S)-2-Benzyl-1-(3,5-dimethoxybenzoyl)-N-(chinolin-3-ylmethyl)-4-piperidinamin;
(2R,4S)-2-Benzyl-1-(3,5-dimethoxybenzoyl)-N-(2-chlorbenzyl)-4-piperidinamin;
(2S,4S)-2-Benzyl-1-(3,5-dimethoxybenzoyl)-N-(2-chlorbenzyl)-4-piperidinamin;
(2R,4S)-2-Benzyl-1-(3,5-dimethoxybenzoyl)-N-[2-(2-chlorphenyl)ethyl]-4-piperidinamin;
(2S,4S)-2-Benzyl-1-(3,5-dimethoxybenzoyl)-N-[2-(2-chlorphenyl)ethyl]-4-piperidinamin;
(2R,4S)-2-Benzyl-1-(3,5-dimethoxybenzoyl)-N-[3-(2-chlorphenyl)propyl]-4-piperidinamin;
(2S,4S)-2-Benzyl-1-(3,5-dimethoxybenzoyl)-N-[3-(2-chlorphenyl)propyl]-4-piperidinamin;
(2R,4S)-2-Benzyl-1-(3,5-dimethoxybenzoyl)-N-(3-chlorbenzyl)-4-piperidinamin;

(2S,4S)-2-Benzyl-1-(3,5-dimethoxybenzoyl)-N-(3-chlorbenzyl)-4-piperidinamin;
(2R,4S)-2-Benzyl-1-(3,5-dimethoxybenzoyl)-N-[2-(3-chlorphenyl)ethyl]-4-piperidinamin;
(2S,4S)-2-Benzyl-1-(3,5-dimethoxybenzoyl)-N-[2-(3-chlorphenyl)ethyl]-4-piperidinamin;
(2R,4S)-2-Benzyl-1-(3,5-dimethoxybenzoyl)-N-[3-(3-chlorphenyl)propyl]-4-piperidinamin;
(2S,4S)-2-Benzyl-1-(3,5-dimethoxybenzoyl)-N-[3-(3-chlorphenyl)propyl]-4-piperidinamin;
(2R,4S)-2-Benzyl-1-(3,5-dimethoxybenzoyl)-N-(4-chlorbenzyl)-4-piperidinamin;
(2S,4S)-2-Benzyl-1-(3,5-dimethoxybenzoyl)-N-(4-chlorbenzyl)-4-piperidinamin;
(2R,4S)-2-Benzyl-1-(3,5-dimethoxybenzoyl)-N-[2-(4-chlorphenyl)ethyl]-4-piperidinamin;
(2S,4S)-2-Benzyl-1-(3,5-dimethoxybenzoyl)-N-[2-(4-chlorphenyl)ethyl]-4-piperidinamin;
(2R,4S)-2-Benzyl-1-(3,5-dimethoxybenzoyl)-N-[3-(4-chlorphenyl)propyl]-4-piperidinamin;
(2S,4S)-2-Benzyl-1-(3,5-dimethoxybenzoyl)-N-[3-(4-chlorphenyl)propyl]-4-piperidinamin;
(2R,4S)-2-Benzyl-1-(3,5-dimethoxybenzoyl)-N-(4-methoxynaphth-1-ylmethyl)-4-piperidinamin;
(2S,4S)-2-Benzyl-1-(3,5-dimethoxybenzoyl)-N-(4-methoxynaphth-1-ylmethyl)-4-piperidi namin;
(2R,4S)-2-Benzyl-1-(3,5-dimethoxybenzoyl)-N-(3,4-ethylendioxybenzyl)-4-piperidinamin oder
(2S,4S)-2-Benzyl-1-(3,5-dimethoxybenzoyl)-N-(3,4-ethylendioxybenzyl)-4-piperidinamin oder jeweils ein Salz davon.

**50.** (2R,4S) und (2R,4R)-2-Benzyl-1-(3,5-dimethylbenzoyl)-N-(2-phenethyl)-4-piperidinamin oder ein Salz davon.

**51.** (2R,4S)-2-Benzyl-1-(3,5-bis-(trifluormethyl)-benzoyl)-N-(4-chinolylmethyl)-4-piperidinamin oder ein Salz davon.

**52.** (2R,4S)-2-Benzyl-1-(3,5-dichlorbenzoyl)-N-(4-chinolylmethyl)-4-piperidinamin oder ein Salz davon.

**53.** (2R*,4S*)-2-Benzyl-1-(2,4-dichlorbenzoyl)-N-(4-chinolylmethyl)-4-piperidinamin oder ein Salz davon.

**54.** (2R*,4S*)-2-Benzyl-1-(phenylacetyl)-N-(4-chinolylmethyl)-4-piperidinamin oder ein Salz davon.

**55.** (2R*,4S*)-2-Benzyl-1-(2,6-dichlorbenzoyl)-N-(4-chinolylmethyl)-4-piperidinamin oder ein Salz davon.

**56.** (2R*,4S*)-2-Benzyl-1-(3,5-dibrombenzoyl)-N-(4-chinolylmethyl)-4-piperidinamin oder ein Salz davon.

**57.** (2R*,4S*)-2-Benzyl-1-(9-fluorenoyl)-N-(4-chinolylmethyl)-4-piperidinamin oder ein Salz davon.

**58.** (2R*,4S*)-2-Benzyl-1-(3-toluoyl)-N-(4-chinolylmethyl)-4-piperidinamin oder ein Salz davon.

**59.** (2R*,4S*)-2-Benzyl-1-(3-brombenzoyl)-N-(4-chinolylmethyl)-4-piperidinamin oder ein Salz davon.

**60.** (2R*,4S*)-2-Benzyl-1-(3,5-dihydroxybenzoyl)-N-(4-chinolylmethyl)-4-piperidinamin oder ein Salz davon.

**61.** (2R*,4S*)-2-Benzyl-1-(3-cyanbenzoyl)-N-(4-chinolylmethyl)-4-piperidinamin oder ein Salz davon.

**62.** (2R*,4S*)-2-Benzyl-1-(2-chlorbenzoyl)-N-(4-chinolylmethyl)-4-piperidinamin oder ein Salz davon.

**63.** (2R*,4S*)-2-Benzyl-1-(4-chlorbenzoyl)-N-(4-chinolylmethyl)-4-piperidinamin oder ein Salz davon.

**64.** (2R*,4S*)-2-Benzyl-1-(3,5-dichlorbenzoyl)-N-(4-chinolylmethyl)-N-methyl-4-piperidinamin oder ein Salz davon.

**65.** (2R*,4S*)-2-Benzyl-1-(3,5-dichlorbenzoyl)-N-(4-chinolylmethyl)-N-cyclohexylcarbamoyl-4-piperidinamin oder ein Salz davon.

**66.** (2R*,4S*)-2-Benzyl-1-(3,5-dichlorbenzoyl)-N-(4-chinolylmethyl)-N-phenylcarbamoyl-4-piperidinamin oder ein Salz davon.

**67.** (2R*,4S*)-2-Benzyl-1-(3,5-dichlorbenzoyl)-N-(2-phenylethyl)-4-piperidinamin oder ein Salz davon.

**68.** (2R*,4S*)-2-Benzyl-1-(3,5-bis-(trifluormethyl)-benzoyl)-N-(2-phenylethyl)-4-piperidinamin oder ein Salz davon.

**69.** (2R*,4S*)-2-Benzyl-1-(3,5-dichlorbenzoyl)-N-(2-naphthoyl)-4-piperidinamin oder ein Salz davon.

**70.** (2R*,4S*)-2-Benzyl-1-(3,5-dichlorbenzoyl)-N-(3,5-dimethylbenzoyl)-4-piperidinamin oder ein Salz davon.

**71.** (2R*,4S*)-2-Benzyl-1-(3,5-dichlorbenzoyl)-N-(4-chinolylcarbonyl)-4-piperidinamin oder ein Salz davon.

**72.** (2R*,4S*)-2-Benzyl-1-(3,5-dichlorbenzoyl)-N-(3-indolylcarbonyl)-4-piperidinamin oder ein Salz davon.

**73.** (2R*,4S*)-2-Benzyl-1-(3,5-dichlorbenzoyl)-N-(2-indolylcarbonyl)-4-piperidinamin oder ein Salz davon.

**74.** (2R*,4S*)-2-Benzyl-1-(3,5-dichlorbenzoyl)-N-(5-methoxy-2-indolylcarbonyl)-4-piperidinamin oder ein Salz davon.

**75.** (2R*,4S*)-2-Benzyl-1-(3,5-dichlorbenzoyl)-N-(1-naphthoyl)-4-piperidinamin oder ein Salz davon.

**76.** (2R*,4S*)-2-Benzyl-1-(3,5-dichlorbenzoyl)-N-(phenylacetyl)-4-piperidinamin oder ein Salz davon.

**77.** (2R*,4S*)-2-Benzyl-1-(3,5-dichlorbenzoyl)-N-(2-methoxybenzyl)-4-piperidinamin oder ein Salz davon.

**78.** (2R*,4S*)-2-Benzyl-1-(3,5-dichlorbenzoyl)-N-(3-(N-acetyl)-indolylmethyl)-4-piperidinamin oder ein Salz davon.

**79.** (2R*,4S*)-2-Benzyl-1-(3,5-dichlorbenzoyl)-N-(2-benzo[b]furanylmethyl)-4-piperidinamin oder ein Salz davon.

**80.** (2R*,4S*)-2-Benzyl-1-(3,5-dichlorbenzoyl)-N-[(3-methylbenzo[b]thiophen-2-ylmethyl]-4-piperidinamin oder ein Salz davon.

**81.** (2R*,4S*)-2-Benzyl-1-(3,5-dichlorbenzoyl)-N-(5-methoxyindol-3-ylmethyl)-4-piperidinamin oder ein Salz davon.

**82.** (2R*,4S*)-2-Benzyl-1-(3,5-dichlorbenzoyl)-N-(3-indolylmethyl)-4-piperidinamin oder ein Salz davon.

**83.** (2R*,4S*)-2-Benzyl-1-(3,5-dichlorbenzoyl)-N-phenylcarbamoyl-4-piperidinamin oder ein Salz davon.

**84.** (2R*,4S*)-2-Benzyl-1-(3,5-dichlorbenzoyl)-N-diphenylmethyl-4-piperidinamin oder ein Salz davon.

**85.** (2R*,4S*)-2-Benzyl-1-(3,5-dichlorbenzoyl)-N-(3,4-dihydro-2H-1-benzopyran-2-carbonyl)-4-piperidinamin oder ein Salz davon.

**86.** (2R*,4S*)-2-Benzyl-1-(4-methoxybenzoyl)-N-(4-chinolylmethyl)-4-piperidinamin oder ein Salz davon.

**87.** (2R*,4S*,1'R*)-N-Benzyl-1-(3,5-dimethylbenzoyl)-2-(1'-hydroxy-1'-phenylmethyl)-4-piperidinamin oder ein Salz davon.

**88.** (2R*,4S*,1'R*)-2-(1'-hydroxy-1'-phenyl-methyl)-1-(3,5-dimethylbenzoyl)-N-(4-chinolinylmethyl)-4-piperidinamin oder ein Salz davon.

**89.** (2R*,4S*,1'S*)-1-(3,5-Dimethylbenzoyl)-2-(1'-hydroxy-1'-phenyl-methyl)-N-(4-chinolinylmethyl)-4-piperidinamin oder ein Salz davon.

**90.** (2R*,4S*,1'R*)-2-{1'-Hydroxy-1'-(4-chlorphenyl)methyl}-1-(3,5-dimethylbenzoyl)-N-(4-chinolinylmethyl)-4-piperidinamin oder ein Salz davon.

**91.** (2R*,4S*,1'S*)-1-(3,5-Dimethylbenzoyl)-2-{1'-hydroxy-1'-(4-chlorphenyl)-methyl}-N-(4-chinolinylmethyl)-4-piperidinamin oder ein Salz davon.

**92.** (2R*,4S*,1'S*)-1-(3,5-Dimethylbenzoyl)-2-{1'-hydroxy-1'-(3,4-dichlorphenyl)methyl}-N-(4-chinolinylmethyl)-4-piperidinamin oder ein Salz davon.

**93.** (2R*,4S*,)-N-Benzyl-1-(3,5-dimethylbenzoyl)-2-benzoyl-4-piperidinamin oder ein Salz davon.

**94.** (2R*,4S*)-2-(4-Chlorbenzyl)-1-(3,5-dimethylbenzoyl)-N-(4-chinolinylmethyl)-4-piperidinamin oder ein Salz davon.

**95.** (2R*,4S*)-2-(3,4-Dichlorbenzyl)-1-(3,5-dimethylbenzoyl)-N-(4-chinolinylmethyl)-4-piperidinamin oder ein Salz davon.

**96.** (2R*,4S*)-1-(3,5-Dimethylbenzoyl)-2-phenyl-N-(4-chinolinylmethyl)-4-piperidinamin oder ein Salz davon.

**97.** (2R*,4S*)-1-(3,5-Dichlorbenzoyl)-2-phenyl-N-(4-chinolinmethyl)-4-piperidinamin oder ein Salz davon.

**98.** (2R*,4S*)-1-(1-Naphthoyl)-2-phenyl-N-(4-chinolinylmethyl)-4-piperidineamin

**99.** (2R*,4S*)-1-(3,5-Dimethylbenzoyl)-2-(1-naphthyl)-N-(4-chinolinylmethyl)-4-piperidinamin oder ein Salz davon.

**100.** Ein neues 1-Acylpiperidon der Formel X

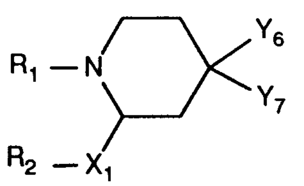

(X),

worin $R_1$ einen gegebenenfalls substituierten Aroyl-, Heteroaroyl-, Cycloalkylcarbonyl-, Aralkanoyl-, Heteroarylalkanoyl-, Aralkoxycarbonyl- oder Arylcarbamoylrest oder den Acylrest einer gegebenenfalls durch Niederalkanoyl oder Carbamoylniederalkanoyl N-substituierten $\alpha$-Aminosäure bedeutet, $R_2$ Cycloalkyl oder einen gegebenenfalls substituierten Aryl- oder Heteroarylrest darstellt, $X_1$ Hydroxymethylen darstellt und $Y_6$ und $Y_7$ gemeinsam für Oxo stehen, oder ein Salz davon.

**101.** Eine Verbindung gemäss Anspruch 100 der Formel X, worin $R_1$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Benzoyl oder unsubstituiertes Naphthoyl bedeutet, $R_2$ unsubstituiertes oder durch Halogen der Atomnummer bis und mit 35 und/oder Trifluormethyl mono- oder disubstituiertes Phenyl darstellt $X_1$ Hydroxymethylen darstellt, und $Y_6$ und $Y_7$ gemeinsam für Oxo stehen, oder ein Salz davon.

**102.** (2R*,1'R*)-1-(3,5-Dimethylbenzoyl)-2-(1'-hydroxy-1'-phenyl-methyl)-4-piperidon oder ein Salz davon.

**103.** (2R*,1'R*)-1-(3,5-Bistrifluormethylbenzoyl)-2-{1'-hydroxy-1'-(4-chlorphenyl)methyl}-4-piperidon oder ein Salz davon.

**104.** (2R*,1'R*)-1-(3,5-Dimethylbenzoyl)-2-{1'-hydroxy-1'-(3,4-dichlorphenyl)methyl}-4-piperidon oder ein Salz davon.

**105.** (2R*,1'S*)-1-(3,5-Bistrifluormethylbenzoyl)-2-{1'-hydroxy-1'-(4-chlorphenyl)methyl}-4-piperidon oder ein Salz davon.

**106.** (2R*,1'R*)-1-(3,5-Bistrifluormethylbenzoyl)-2-{1'-hydroxy-1'-(3,4-dichlorphenyl)methyl}-4-piperidon oder ein Salz davon.

**107.** (2R*,1'R*)-1-(3,5-Dimethylbenzoyl)-2-{1'-hydroxy-1'-(4-methoxyphenyl)methyl}-4-piperidon oder ein Salz davon.

**108.** (2R*,1'S*)-1-(3,5-Dimethylbenzoyl)-2-{1'-hydroxy-1'-(4-methoxyphenyl)methyl}-4-piperidon oder ein Salz davon.

**109.** (2R*,1'R*)-1-(3,5-Dimethylbenzoyl)-2-{1'-hydroxy-1'-(3-methoxyphenyl)methyl}-4-piperidon oder ein Salz davon.

**110.** (2R*,1'S*)-1-(3,5-Dimethylbenzoyl)-2-{1'-hydroxy-1'-(3-methoxyphenyl)methyl}-4-piperidon oder ein Salz davon.

**111.** (2R*,1'R*)-1-(3,5-Dimethylbenzoyl)-2-{1'-hydroxy-1'-(4-trifluormethylphenyl)methyl}-4-piperidon oder ein Salz davon.

**112.** (2R*,1'S*)-1-(3,5-Dimethylbenzoyl)-2-{1'-hydroxy-1'-(4-trifluormethylphenyl)methyl}-4-piperidon oder ein Salz davon.

**113.** (2R*,1'R*)-1-(3,5-Dimethylbenzoyl)-2-{1'-hydroxy-1'-(4-chlor-3-trifluormethylphenyl)methyl}-4-piperidon oder ein Salz davon.

**114.** (2R*,1'S*)-1-(3,5-Dimethylbenzoyl)-2-{1'-hydroxy-1'-(4-chlor-3-trifluormethylphenyl)-methyl}-4-piperidon oder ein Salz davon.

**115.** Verbindungen gemäss einem der Ansprüche 1 bis 114 zur Anwendung in einem Verfahren zur therapeutischen Behandlung des menschlichen oder tierischen Körpers.

**116.** Pharmazeutische Präparate, neben üblichen pharmazeutischen Hilfsstoffen als pharmazeutischen Wirkstoff enthaltend eine Verbindung gemäss einem der Ansprüche 1 bis 47 in freier Form oder in pharmazeutisch verwendbarer Salzform.

**117.** Pharmazeutische Präparate, neben üblichen pharmazeutischen Hilfsstoffen als pharmazeutischen Wirkstoff enthaltend eine Verbindung gemäss einem der Ansprüche 48 bis 115 in freier Form oder in pharmazeutisch verwendbarer Salzform.

**118.** Verfahren zur Herstellung neuer 1-Acylpiperidinverbindungen gemäss Anspruch 1 und ihrer Salze, dadurch gekennzeichnet, dass man

a) in eine Verbindung der Formel II

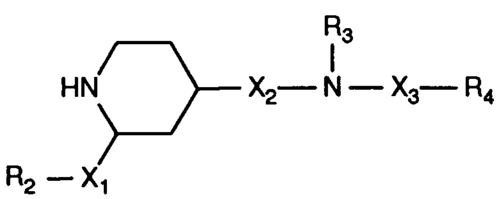

(II),

worin $R_2$, $R_3$, $R_4$, $X_1$, $X_2$ und $X_3$ die angegebenen Bedeutungen haben, den Rest $R_1$ einführt oder

b) Verbindungen der Formeln III und IV

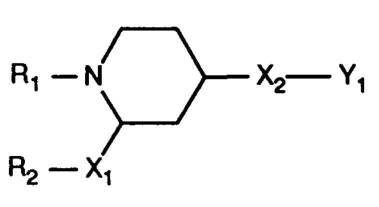

(III) und $Y_2-X_3-R_4$ (IV),

worin $Y_1$ eine Gruppe der Formel $-N(R_3)-H$ und $Y_2$ Hydroxy, reaktionsfähiges verestertes Hydroxy

oder, sofern $X_3$ für Carbonyl steht, verethertes Hydroxy darstellt oder $Y_1$ Hydroxy, reaktionsfähig verestertes Hydroxy oder, sofern $X_2$ Carbonyl bedeutet, verethertes Hydroxy und $Y_2$ eine Gruppe der Formel $-N(R_3)-H$ darstellt, wobei $R_2$, $R_3$, $R_4$, $X_1$, $X_2$ und $X_3$ die angegebenen Bedeutungen haben, oder deren Salze miteinander kondensiert oder

c) zur Herstellung von Verbindungen der Formel I, worin einer der Reste $X_2$ und $X_3$ Alkylen und der andere Alkylen, Carbonyl oder im Falle von $X_2$ eine direkte Bindung bzw. im Falle von $X_3$ einen gegebenenfalls durch Hydroxymethyl oder gegebenenfalls verestertes oder amidiertes Carboxy substituiertes Alkylenrest darstellt, in einer Verbindung der Formel V

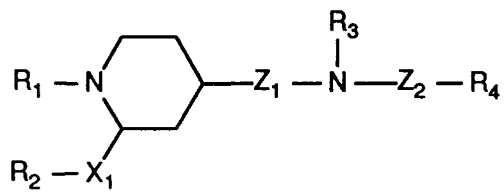

(V),

worin $Z_1$ einen in $\alpha$-Stellung zur Gruppe $-N(R_3)-$ durch Oxo oder Hydroxy substituierten Alkylenrest und $Z_2$ Alkylen, Carbonyl oder einen gegebenenfalls durch Hydroxymethyl oder gegebenenfalls verestertes oder amidiertes Carboxy substituiertes Alkylenrest bedeutet oder $Z_1$ Alkylen, Carbonyl oder eine direkte Bindung und $Z_2$ in $\alpha$-Stellung zur Gruppe $-N(R_3)-$ durch Oxo oder Hydroxy substituierten Alkylenrest darstellt und $R_2$, $R_3$, $R_4$, $X_1$, $X_2$ und $X_3$ die angegebenen Bedeutungen haben, oder in einem Salz davon die Oxo- bzw. Hydroxygruppe in $\alpha$-Stellung zur Gruppe $-N(R_3)-$ reduktiv durch Wasserstoff ersetzt bzw. in einer Verbindung der Formel VI

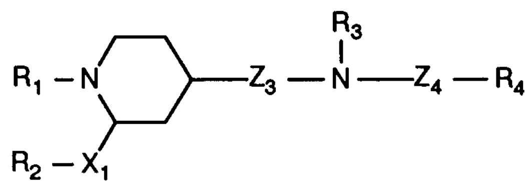

(VI),

worin $Z_3$ einen Rest der Formel $-C(R_a)=C(R_b)-$ und $Z_4$ Alkylen, Carbonyl oder einen gegebenenfalls durch Hydroxymethyl oder gegebenenfalls verestertes oder amidiertes Carboxy substituiertes Alkylenrest bedeutet oder $Z_3$ Alkylen, Carbonyl oder eine direkte Bindung und $Z_4$ einen Rest der Formel $-C(R_a)=C(R_b)-$ bedeutet, wobei $R_a$ und $R_b$ jeweils Wasserstoff oder Niederalkyl bedeuten, den Rest der Formel $-C(R_a)=C(R_b)-$ durch Reduktion der Doppelbindung zu dem entsprechenden Rest $-CH(R_a)-CH(R_b)-$ reduziert oder

d) zur Herstellung von Verbindungen der Formel I, worin $X_1$ eine Carbonyl- oder Hydroxymethylengruppe bedeutet, Verbindungen der Formeln VII und VIII

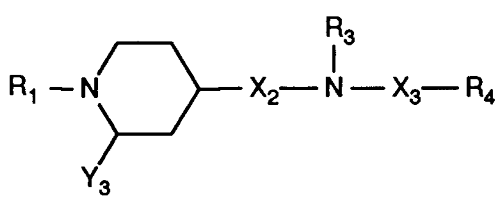

(VII) und $Y_4-R_2$ (VIII),

worin einer der Reste $Y_3$ und $Y_4$ Formyl oder eine gegebenenfalls anhydridisierte oder veresterte Carboxygruppe und der andere einen metallischen Rest darstellt und $R_2$, $R_3$, $R_4$, $X_2$ und $X_3$ die angegebenen Bedeutungen haben, miteinander kondensiert oder

e) zur Herstellung von Verbindungen der Formel I, worin $R_3$ für Wasserstoff steht, aus einer Verbindung der Formel IX

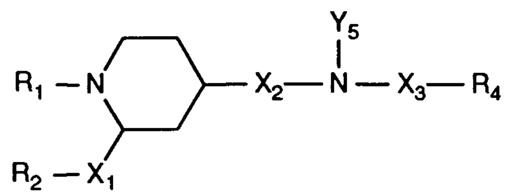 (IX),

worin $Y_5$ eine Aminoschutzgruppe bedeutet und $R_2$, $R_4$, $X_1$, $X_2$ und $X_3$ die angegebenen Bedeutungen haben, oder aus einem Salz davon die Gruppe $Y_5$ abspaltet oder

f) zur Herstellung von Verbindungen der Formel I, worin $X_3$ Alkylen bedeutet, Verbindungen der Formeln X und XI

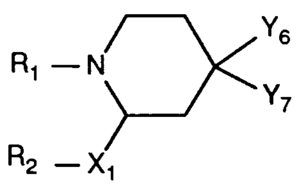 (X) und 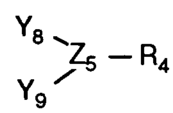 (XI) ,

worin $Y_6$ eine Gruppe der Formel -N($R_3$)-H, $Y_7$ Wasserstoff, $Y_8$ und $Y_9$ gemeinsam Oxo und $Z_5$ einen $X_3$ entsprechenden Alkanylylidenrest bedeuten oder $Y_6$ und $Y_7$ gemeinsam Oxo, $Y_8$ eine Gruppe der Formel -N($R_3$)-H, $Y_9$ Wasserstoff und $Z_5$ einen Rest $X_3$ darstellt,unter reduzierenden Bedingungen miteinander kondensiert und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

**119.** Verfahren zur Herstellung neuer 1-Acylpiperidone gemäss Anspruch 100 und ihrer Salze, dadurch gekennzeichnet, dass man eine Verbindung der Formel Xa

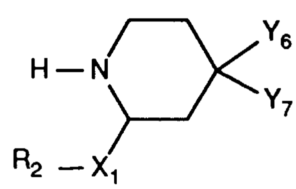 (Xa),

mit einem den Rest $R_1$ einführendem Mittel kondensiert und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

**120.** Verwendung einer Verbindung gemäss einem der Ansprüche 1 bis 115 für die Herstellung eines für die Behandlung von Erkrankungen, bei deren Entstehung Substanz P eine wesentliche Rolle spielt, bestimmten Arzneimittels.

**Patentansprüche für folgende Vertragsstaaten : ES, GR**

1. Verfahren zur Herstellung neuer 1-Acylpiperidinverbindungen der Formel I

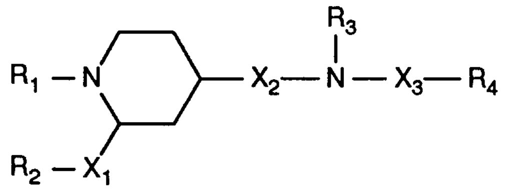

(I),

worin worin $R_1$ unsubstituiertes oder im Phenylteil durch Niederalkyl, Niederalkoxy, Diniederalkylamino, Halogen und/oder Trifluormethyl substituiertes Phenyl- oder Diphenyl-$C_1$-$C_4$-alkyl, unsubstituiertes oder im Phenyl durch Halogen und/oder Triazolyl substituiertes Phenoxy-$C_1$-$C_4$-alkyl, Pyridyl- oder Chinolinyl-$C_1$-$C_4$-alkyl, unsubstituiertes oder durch Niederalkyl, Niederalkoxy, Diniederalkylamino, Halogen und/oder Trifluormethyl substituiertes Benzoyl, unsubstituiertes oder durch Niederalkyl, Niederalkoxy, Diniederalkylamino, Halogen und/oder Trifluormethyl substituiertes Naphthoyl, unsubstituiertes oder durch Niederalkyl, Niederalkoxy, Halogen und/oder Trifluormethyl substituiertes Pyridylcarbonyl oder Chinolinylcarbonyl, unsubstituiertes oder durch Niederalkyl, Niederalkoxy, Diniederalkylamino, Halogen und/oder Trifluormethyl substituiertes 5-bis 7-gliedriges Cycloalkylcarbonyl, unsubstituiertes oder im Phenyl durch Niederalkyl, Niederalkoxy, Diniederalkylamino, Halogen und/oder Trifluormethyl substituiertes Phenyl- oder Diphenyl-$C_1$-$C_4$-alkanoyl, w unsubstituiertes oder im Phenylteil gegebenenfalls durch Niederalkyl, Niederalkoxy, Diniederalkylamino, Halogen und/oder Trifluormethyl substituiertes N-Phenylcarbamoyl oder eine Gruppe der Formel Ia

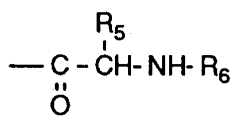

(Ia)

darstellt, in der $R_5$ Wasserstoff, unsubstituiertes oder durch Hydroxy, Mercapto, Amino, gegebenenfalls durch Hydroxy substituiertes Phenyl, Carboxy, Carbamoyl oder Ureido substituiertes $C_1$-$C_4$-Alkyl und $R_6$ $C_2$-$C_7$-Alkanoyl bedeutet, $R_2$ 5- bis 7-gliedriges Cycloalkyl oder einen unsubstituierten oder durch aromatisch gebundenes $C_1$-$C_4$-Alkyl, $C_1$-$C_4$-Alkoxy, Halogen und/oder Trifluormethyl substituierten Phenyl-, Naphthyl- oder Pyridylrest darstellt, $R_3$ für Wasserstoff, $C_1$-$C_7$-Alkyl, Carbamoyl, $C_2$-$C_7$-Alkanoyl, Carboxy-$C_1$-$C_4$-alkanoyl oder Carboxy-$C_2$-$C_4$-alkenoyl steht, $R_4$ unsubstituiertes oder durch $C_1$-$C_4$-Alkyl, $C_1$-$C_4$-Alkoxy, Halogen und/oder Trifluormethyl substituiertes Phenyl oder Naphthyl oder unsubstituiertes Pyridyl, Benzofuranyl, Indolyl, 2,3-Dihydroindolyl, Benzimidazolyl, Chinolyl oder 1,2,3,4-Tetrahydrochinolinyl bedeutet, $X_1$ Methylen, Hydroxymethylen, $C_1$-$C_4$-Alkoxymethylen, Carbonyl, Di-$C_1$-$C_4$-alkoxymethylen oder eine direkte Bindung darstellt, $X_2$ für $C_1$-$C_7$-Alkylen, Carbonyl oder eine direkte Bindung steht und $X_3$ Carbonyl, $C_1$-$C_4$-Alkylen, Carboxy-$C_1$-$C_4$-alkylen, $C_1$-$C_4$-Alkoxycarbonyl-$C_1$-$C_4$-alkylen, Carbamoyl-$C_1$-$C_4$-alkylen oder Hydroxymethyl-$C_1$-$C_4$-alkylen darstellt, Niederalkyl ist $C_1$-$C_7$-Alkyl und ihrer Salze, dadurch gekennzeichnet, dass man

a) in eine Verbindung der Formel II

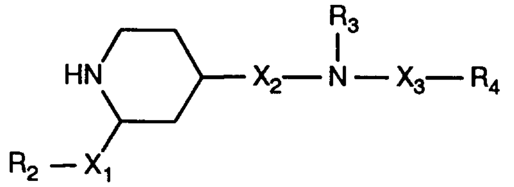

(II),

worin $R_2$, $R_3$, $R_4$, $X_1$, $X_2$ und $X_3$ die angegebenen Bedeutungen haben, den Rest $R_1$ einführt oder

b) Verbindungen der Formeln III und IV

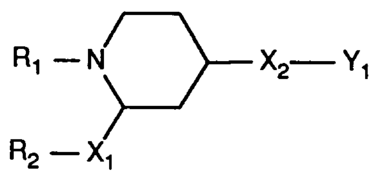

(III)

und

$Y_2$-$X_3$-$R_4$ (IV),

worin $Y_1$ eine Gruppe der Formel -N($R_3$)-H und $Y_2$ Hydroxy, reaktionsfähiges verestertes Hydroxy oder, sofern $X_3$ für Carbonyl steht, verethertes Hydroxy darstellt oder $Y_1$ Hydroxy, reaktionsfähig verestertes Hydroxy oder, sofern $X_2$ Carbonyl bedeutet, verethertes Hydroxy und $Y_2$ eine Gruppe der Formel -N($R_3$)-H darstellt, wobei $R_2$, $R_3$, $R_4$, $X_1$, $X_2$ und $X_3$ die angegebenen Bedeutungen haben, oder deren Salze miteinander kondensiert oder

c) zur Herstellung von Verbindungen der Formel I, worin einer der Reste $X_2$ und $X_3$ Alkylen und der andere Alkylen, Carbonyl oder im Falle von $X_2$ eine direkte Bindung bzw. im Falle von $X_3$ einen gegebenenfalls durch Hydroxymethyl oder gegebenenfalls verestertes oder amidiertes Carboxy substituiertes Alkylenrest darstellt, in einer Verbindung der Formel V

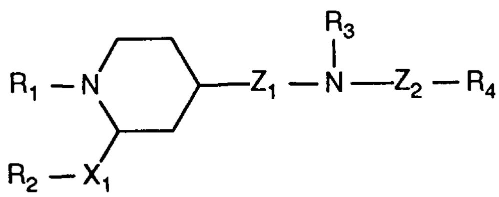

(V),

worin $Z_1$ einen in α-Stellung zur Gruppe -N($R_3$)- durch Oxo oder Hydroxy substituierten Alkylenrest und $Z_2$ Alkylen, Carbonyl oder einen gegebenenfalls durch Hydroxymethyl oder gegebenenfalls verestertes oder amidiertes Carboxy substituiertes Alkylenrest bedeutet oder $Z_1$ Alkylen, Carbonyl oder eine direkte Bindung und $Z_2$ in α-Stellung zur Gruppe -N($R_3$)- durch Oxo oder Hydroxy substituierten Alkylenrest darstellt und $R_2$, $R_3$, $R_4$, $X_1$, $X_2$ und $X_3$ die angegebenen Bedeutungen haben, oder in einem Salz davon die Oxo- bzw. Hydroxygruppe in α-Stellung zur Gruppe -N($R_3$)- reduktiv durch Wasserstoff ersetzt bzw. in einer Verbindung der Formel VI

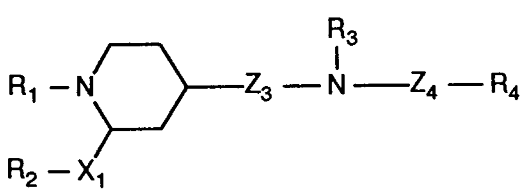

(VI),

worin $Z_3$ einen Rest der Formel -C($R_a$) = C($R_b$)- und $Z_4$ Alkylen, Carbonyl oder einen gegebenenfalls durch Hydroxymethyl oder gegebenenfalls verestertes oder amidiertes Carboxy substituiertes Alkylenrest bedeutet oder $Z_3$ Alkylen, Carbonyl oder eine direkte Bindung und $Z_4$ einen Rest der Formel -C($R_a$) = C($R_b$)- bedeutet, wobei $R_a$ und $R_b$ jeweils Wasserstoff oder Niederalkyl bedeuten, den Rest der Formel -C($R_a$) = C($R_b$)- durch Reduktion der Doppelbindung zu dem entsprechenden Rest -CH($R_a$)-CH($R_b$)- reduziert oder

d) zur Herstellung von Verbindungen der Formel I, worin $X_1$ eine Carbonyl- oder Hydroxymethylengruppe bedeutet, Verbindungen der Formeln VII und VIII

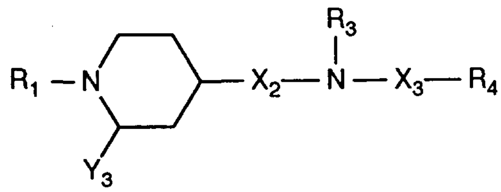

(VII)

und

$Y_4$-$R_2$ (VIII),

worin einer der Reste $Y_3$ und $Y_4$ Formyl oder eine gegebenenfalls anhydridisierte oder veresterte Carboxygruppe und der andere einen metallischen Rest darstellt und $R_2$, $R_3$, $R_4$, $X_2$ und $X_3$ die angegebenen Bedeutungen haben, miteinander kondensiert oder
e) zur Herstellung von Verbindungen der Formel I, worin $R_3$ für Wasserstoff steht, aus einer Verbindung der Formel IX

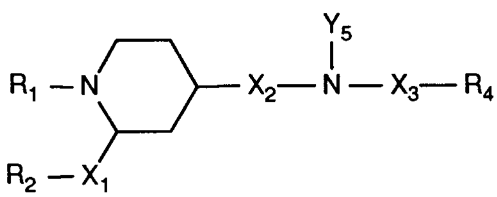

(IX),

worin $Y_5$ eine Aminoschutzgruppe bedeutet und $R_2$, $R_4$, $X_1$, $X_2$ und $X_3$ die angegebenen Bedeutungen haben, oder aus einem Salz davon die Gruppe $Y_5$ abspaltet oder
f) zur Herstellung von Verbindungen der Formel I, worin $X_3$ Alkylen bedeutet, Verbindungen der Formeln X und XI

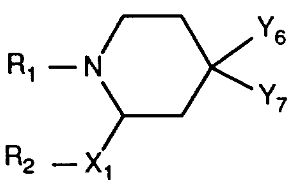

(X) und

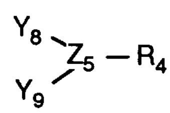

(XI) ,

worin $Y_6$ eine Gruppe der Formel -N($R_3$)-H, $Y_7$ Wasserstoff, $Y_8$ und $Y_9$ gemeinsam Oxo und $Z_5$ einen $X_3$ entsprechenden Alkanylylidenrest bedeuten oder $Y_6$ und $Y_7$ gemeinsam Oxo, $Y_8$ eine Gruppe der Formel -N($R_3$)-H, $Y_9$ Wasserstoff und $Z_5$ einen Rest $X_3$ darstellt,unter reduzierenden Bedingungen miteinander kondensiert und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

2. Verfahren gemäss Anspruch 1, dadurch gekennzeichnet, dass man eine Verbindung der Formel I, worin $R_1$ unsubstituiertes oder durch $C_1$-$C_4$-Alkyl, $C_1$-$C_4$-Alkoxy, Halogen und/oder Trifluormethyl substituiertes Benzoyl, Naphthoyl oder Phenyl-$C_1$-$C_4$-alkanoyl, unsubstituiertes Pyridylcarbonyl oder Chinolinylcarbonyl oder eine Gruppe der Formel Ia

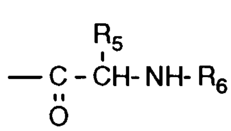

(Ia)

darstellt, in der $R_5$ Wasserstoff, unsubstituiertes oder durch Hydroxy, Mercapto, Amino, gegebenenfalls durch Hydroxy substituiertes Phenyl, Carboxy, Carbamoyl oder Ureido substituiertes $C_1$-$C_4$-Alkyl und $R_6$ $C_2$-$C_7$-Alkanoyl bedeutet, $R_2$ 5- bis 7-gliedriges Cycloalkyl oder einen unsubstituierten oder durch aromatisch gebundenes $C_1$-$C_4$-Alkyl, $C_1$-$C_4$-Alkoxy, Halogen und/oder Trifluormethyl substituierten Phenyl-, Naphthyl- oder Pyridylrest darstellt, $R_3$ für Wasserstoff, $C_1$-$C_7$-Alkyl, Carbamoyl, $C_2$-$C_7$-Alkanoyl, Carboxy-$C_1$-$C_4$-alkanoyl oder Carboxy-$C_3$-$C_5$-alkenoyl steht, $R_4$ unsubstituiertes oder durch $C_1$-$C_4$-Alkyl, $C_1$-$C_4$-Alkoxy, Halogen und/oder Trifluormethyl substituiertes Phenyl oder Naphthyl oder unsubstituiertes Pyridyl, Benzofuranyl, Indolyl, Benzimidazolyl oder Chinolyl bedeutet, $X_1$ Methylen, Hydroxymethylen, $C_1$-$C_4$-Alkoxymethylen, Carbonyl, Di-$C_1$-$C_4$-alkoxymethylen oder eine direkte Bindung darstellt, $X_2$ für $C_1$-$C_7$-Alkylen, Carbonyl oder eine direkte Bindung steht und $X_3$ Carbonyl, $C_1$-$C_4$-Alkylen, Carboxy-$C_1$-$C_4$-alkylen, $C_1$-$C_4$-Alkoxycarbonyl-$C_1$-$C_4$alkylen, Carbamoyl-$C_1$-$C_4$-alkylen, oder Hydroxymethyl-$C_1$-$C_4$-alkylen darstellt, oder ein Salz davon herstellt.

3. Verfahren gemäss Anspruch 1, dadurch gekennzeichnet, dass man eine Verbindung der Formel I, worin $R_1$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Benzoyl oder unsubstituiertes Naphthoyl oder Phenyl-$C_1$-$C_4$-alkanoyl bedeutet, $R_2$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Phenyl oder unsubstituiertes Pyridyl darstellt, $R_3$ für Wasserstoff, $C_1$-$C_4$-Alkyl, Carbamoyl oder $C_2$-$C_7$-Alkanoyl, steht, $R_4$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Phenyl oder unsubstituiertes Naphthyl, Pyridyl, Benzofuranyl, Indolyl, Benzimidazolyl oder Chinolyl bedeutet, $X_1$ Methylen, Hydroxymethylen, Carbonyl oder eine direkte Bindung darstellt, $X_2$ für eine direkte Bindung steht und $X_3$ $C_1$-$C_4$-Alkylen, darstellt, oder ein Salz davon herstellt.

4. Verfahren gemäss Anspruch 1, dadurch gekennzeichnet, dass man eine Verbindung der Formel I, worin $R_1$ unsubstituiertes oder durch $C_1$-$C_4$-Alkyl, $C_1$-$C_4$-Alkoxy, Halogen der Atomnummer bis und mit 35 und/oder Trifluormethyl mono- oder disubstituiertes Benzoyl oder unsubstituiertes Naphthoyl bedeutet, $R_2$ unsubstituiertes oder durch Halogen der Atomnummer bis und mit 35 und/oder Trifluormethyl mono- oder disubstituiertes Phenyl darstellt, $R_3$ für Wasserstoff steht, $R_4$ unsubstituiertes Chinolyl bedeutet, $X_1$ Methylen darstellt, $X_2$ für eine direkte Bindung steht und $X_3$ $C_1$-$C_4$-Alkylen darstellt, oder ein Salz davon herstellt.

5. Verfahren gemäss Anspruch 1, dadurch gekennzeichnet, dass man (2R*,4S*)-2-Benzyl-1-(2-naphthoyl)-N-(4-chinolylmethyl)-4-piperidinamin oder ein Salz davon herstellt.

6. Verfahren gemäss Anspruch 1, dadurch gekennzeichnet, dass man (2R*,4S*)-2-Benzyl-1-(3-trifluormethylbenzoyl)-N-(4-chinolylmethyl)-4-piperidinamin oder ein Salz davon herstellt.

7. Verfahren gemäss Anspruch 1, dadurch gekennzeichnet, dass man (2R*,4S*)-2-Benzyl-1-(3,5-bis-(trifluormethyl)-benzoyl)-N-(4-chinolylmethyl)-4-piperidinamin oder ein Salz davon herstellt.

8. Verfahren gemäss Anspruch 1, dadurch gekennzeichnet, dass man (2R*,4S*)-2-Benzyl-1-(3,5-dimethoxybenzoyl)-N-(4-chinolylmethyl)-4-piperidinamin oder ein Salz davon herstellt.

9. Verfahren gemäss Anspruch 1, dadurch gekennzeichnet, dass man (2R*,4S*)-2-Benzyl-1-(1-naphthoyl)-N-(4-chinolylmethyl)-4-piperidinamin oder ein Salz davon herstellt.

10. Verfahren gemäss Anspruch 1, dadurch gekennzeichnet, dass man (2R*,4S*)-2-Benzyl-1-(3,5-dichlorbenzoyl)-N-(4-chinolylmethyl)-4-piperidinamin oder ein Salz davon herstellt.

11. Verfahren gemäss Anspruch 1, dadurch gekennzeichnet, dass man (2R*,4S*)-2-Benzyl-1-(2-chinolinylcarbonyl)-N-(4-chinolylmethyl)-4-piperidinamin oder ein Salz davon herstellt.

12. Verfahren gemäss Anspruch 1, dadurch gekennzeichnet, dass man (2R*,4S*)-2-Benzyl-1-(4-chlor-phenylacetyl)-N-(4-chinolylmethyl)-4-piperidinamin oder ein Salz davon herstellt.

13. Verfahren gemäss Anspruch 1, dadurch gekennzeichnet, dass man (2R*,4S*)-2-Benzyl-1-(benzyloxycarbonyl)-N-(4-chinolylmethyl)-4-piperidinamin oder ein Salz davon herstellt.

14. Verfahren gemäss Anspruch 1, dadurch gekennzeichnet, dass man (2R*,4S*)-2-Benzyl-1-(2-phenylethyl)-N-(4-chinolylmethyl)-4-piperidinamin oder ein Salz davon herstellt.

15. Verfahren gemäss Anspruch 1, dadurch gekennzeichnet, dass man (2R*,4S*)-2-Benzyl-1-(2-naphtylacetyl)-N-(4-chinolylmethyl)-4-piperidinamin oder ein Salz davon herstellt.

16. Verfahren gemäss Anspruch 1, dadurch gekennzeichnet, dass man (2R*,4S*)-2-Benzyl-1-(4-chinolylmethyl)-N-(4-chinolylmethyl)-4-piperidinamin oder ein Salz davon herstellt.

17. Verfahren gemäss Anspruch 1, dadurch gekennzeichnet, dass man (2R*,4S*)-2-Benzyl-1-(2,4-dichlorbenzyl)-N-(4-chinolylmethyl)-4-piperidinamin oder ein Salz davon herstellt.

18. Verfahren gemäss Anspruch 1, dadurch gekennzeichnet, dass man (2R*,4S*)-2-Benzyl-1-(2,2-diphenylethyl)-N-(4-chinolylmethyl)-4-piperidinamin oder ein Salz davon herstellt.

19. Verfahren gemäss Anspruch 1, dadurch gekennzeichnet, dass man (2R*,4S*)-2-Benzyl-1-(phenylcarbamoyl)-N-(4-chinolylmethyl)-4-piperidinamin oder ein Salz davon herstellt.

20. Verfahren gemäss Anspruch 1, dadurch gekennzeichnet, dass man (2R*,4S*)-2-Benzyl-1-(diphenylacetyl)-N-(4-chinolylmethyl)-4-piperidinamin oder ein Salz davon herstellt.

21. Verfahren gemäss Anspruch 1, dadurch gekennzeichnet, dass man (2R*,4S*)-2-Benzyl-1-(2-pyridylacetyl)-N-(4-chinolylmethyl)-4-piperidinamin oder ein Salz davon herstellt.

22. Verfahren gemäss Anspruch 1, dadurch gekennzeichnet, dass man (2R*,4S*)-2-Benzyl-1-(4-pyridylacetyl)-N-(4-chinolylmethyl)-4-piperidinamin oder ein Salz davon herstellt.

23. Verfahren gemäss Anspruch 1, dadurch gekennzeichnet, dass man (2R*,4S*)-2-Benzyl-1-(2,3-diphenylpropionyl)-N-(4-chinolylmethyl)-4-piperidinamin oder ein Salz davon herstellt.

24. Verfahren gemäss Anspruch 1, dadurch gekennzeichnet, dass man (2R*,4S*)-2-Benzyl-1-((3S)-(2,3,4,9-tetrahydro-1H-pyrido[3,4-b]indol-3-yl)-carbonyl)-N-(4-chinolylmethyl)-4-piperidinamin oder ein Salz davon herstellt.

25. Verfahren gemäss Anspruch 1, dadurch gekennzeichnet, dass man (2R*,4S*)-2-Benzyl-1-(3-methoxybenzoyl)-N-(4-chinolylmethyl)-4-piperidinamin oder ein Salz davon herstellt.

26. Verfahren gemäss Anspruch 1, dadurch gekennzeichnet, dass man (2R*,4S*)-2-Benzyl-1-(3-N,N-dimethylaminobenzoyl)-N-(4-chinolylmethyl)-4-piperidinamin oder ein Salz davon herstellt.

27. Verfahren gemäss Anspruch 1, dadurch gekennzeichnet, dass man (2R*,4S*)-2-Benzyl-1-(cis,cis-3,5-dimethylcycl ohexylcarbonyl)-N-(4-chinolylmethyl)-4-piperidinamin oder ein Salz davon herstellt.

28. Verfahren gemäss Anspruch 1, dadurch gekennzeichnet, dass man (2R*,4S*)-2-Benzyl-1-(3,5-bis-(trifluormethyl)-benzyl)-N-(4-chinolylmethyl)-4-piperidinamin oder ein Salz davon herstellt.

29. Verfahren gemäss Anspruch 1, dadurch gekennzeichnet, dass man (2S*,4R*)-2-Benzyl-1-[2-(5-chlor-1H-1,2,4-triazol-1-yl)phenoxyethyl]-N-(4-chinolylmethyl)-4-piperidinarnin oder ein Salz davon herstellt.

30. Verfahren gemäss Anspruch 1, dadurch gekennzeichnet, dass man (2R*,4S*)-2-Benzyl-1-((S)-phenylalanyl)-N-(4-chinolylmethyl)-4-piperidinamin oder ein Salz davon herstellt.

31. Verfahren gemäss Anspruch 1, dadurch gekennzeichnet, dass man (2R*,4S*)-2-Benzyl-1-((R)-phenylalanyl)-N-(4-chinolylmethyl)-4-piperidinamin oder ein Salz davon herstellt.

**32.** Verfahren gemäss Anspruch 1, dadurch gekennzeichnet, dass man (2R*,4S*)-2-Benzyl-1-((S)-N-acetyl-phenylalanyl)-N-(4-chinolylmethyl)-4-piperidinamin oder ein Salz davon herstellt.

**33.** Verfahren gemäss Anspruch 1, dadurch gekennzeichnet, dass man (2R*,4S*)-2-Benzyl-1-((R)-N-acetyl-phenylalanyl)-N-(4-chinolylmethyl)-4-piperidinamin oder ein Salz davon herstellt.

**34.** Verfahren gemäss Anspruch 1, dadurch gekennzeichnet, dass man (2R*,4S*)-2-Benzyl-1-((S)-N-(4-carboxamido-but yroyl)-phenylalanyl)-N-(4-chinolylmethyl)-4-piperidinamin oder ein Salz davon herstellt.

**35.** Verfahren gemäss Anspruch 1, dadurch gekennzeichnet, dass man (2R*,4S*)-2-Benzyl-1-((R)-N-(4-carboxamido-but yroyl)-phenylalanyl)-N-(4-chinolylmethyl)-4-piperidinamin oder ein Salz davon herstellt.

**36.** Verfahren gemäss Anspruch 1, dadurch gekennzeichnet, dass man (2R*,4S*)-2-Benzyl-1-benzoyl-N-(4-chinolylmethyl)-4-piperidinamin oder ein Salz davon herstellt.

**37.** Verfahren gemäss Anspruch 1, dadurch gekennzeichnet, dass man (2R*,4S*)-2-Benzyl-1-(3-chlorbenzoyl)-N-(4-chinolylmethyl)-4-piperidinamin oder ein Salz davon herstellt.

**38.** Verfahren gemäss Anspruch 1, dadurch gekennzeichnet, dass man (2R*,4S*)-2-Benzyl-1-(3,5-dichlorbenzoyl)-N-(4-chinolylmethyl)-N-(3-carboxamido-propionyl)-4-piperidinamin oder ein Salz davon herstellt.

**39.** Verfahren gemäss Anspruch 1, dadurch gekennzeichnet, dass man (2R,4S)- oder (2R,4R)-2-Benzyl-1-(3,5-dimethylbenzoyl)-N-(4-chinolylmethyl)-4-piperidinamin oder ein Salz davon herstellt.

**40.** Verfahren gemäss Anspruch 1, dadurch gekennzeichnet, dass man (2S,4R) und (2S,4S)-2-Benzyl-1-(3,5-dimethylbenzoyl)-N-(2-phenethyl)-4-piperidinamin oder ein Salz davon herstellt.

**41.** Verfahren gemäss Anspruch 1, dadurch gekennzeichnet, dass man (2R,4S)-2-Benzyl-1-(3,5-dimethyl-benzoyl)-N-(3-chinolylmethyl)-4-piperidinamin oder ein Salz davon herstellt.

**42.** Verfahren gemäss Anspruch 1, dadurch gekennzeichnet, dass man (2R,4S)-2-Benzyl-1-(3,5-dimethyl-benzoyl)-N-(2-chinolylmethyl)-4-piperidinamin oder ein Salz davon herstellt.

**43.** Verfahren gemäss Anspruch 1, dadurch gekennzeichnet, dass man (2R,4S)- oder (2R,4R)-2-Benzyl-1-(3,5-dimethylbenzoyl)-N-benzyl-4-piperidinamin oder ein Salz davon herstellt.

**44.** Verfahren gemäss Anspruch 1, dadurch gekennzeichnet, dass man (2S,4R)- oder (2S,4S)-2-Benzyl-1-(3,5-dimethylbenzoyl)-N-benzyl-4-piperidinamin oder ein Salz davon herstellt.

**45.** Verfahren gemäss Anspruch 1, dadurch gekennzeichnet, dass man (2R,4S)- oder (2R,4R)-2-Benzyl-1-(3,5-dimethylbenzoyl)-N-(4-pyridylmethyl)-4-piperidinamin oder ein Salz davon herstellt.

**46.** Verfahren gemäss Anspruch 1, dadurch gekennzeichnet, dass man (2R,4S)- oder (2R,4R)-2-Benzyl-1-(3,5-dimethylbenzoyl)-N-(3-pyridylmethyl)-4-piperidinamin oder ein Salz davon herstellt.

**47.** Verfahren gemäss Anspruch 1, dadurch gekennzeichnet, dass man (2S,4R) und (2S,4S)-2-Benzyl-1-(3,5-dimethylbenzoyl)-N-(4-chinolylmethyl)-4-piperidinamin oder ein Salz davon herstellt.

**48.** Verfahren gemäss Anspruch 1, dadurch gekennzeichnet, dass man (2R*,4S*)-2-Benzyl-1-(3,5-dimethyl-benzoyl)-N-benzyl-N-carbamoyl-4-piperidinamin oder ein Salz davon herstellt.

**49.** Verfahren gemäss Anspruch 1, dadurch gekennzeichnet, dass man (2R,4S)-2-Benzyl-1-(3,5-dimethyl-benzoyl)-N-(3-phenylpropyl)-4-piperidinamin;
(2S,4S)-2-Benzyl-1-(3,5-dimethylbenzoyl)-N-(3-phenylpropyl)-4-piperidinamin;
(2R,4S)-2-Benzyl-1-(3,5-dimethylbenzoyl)-N-(2-methoxybenzyl)-4-piperidinamin;
(2S,4S)-2-Benzyl-1-(3,5-dimethylbenzoyl)-N-(2-methoxybenzyl)-4-piperidinamin;
(2R,4S)-2-Benzyl-1-(3,5-dimethylbenzoyl)-N-[2-(2-methoxyphenyl)ethyl]-4-piperidinamin;

(2S,4S)-2-Benzyl-1-(3,5-dimethylbenzoyl)-N-[2-(2-methoxyphenyl)ethyl]-4-piperidinamin;
(2R,4S)-2-Benzyl-1-(3,5-dimethylbenzoyl)-N-[3-(2-methoxyphenyl)propyl]-4-piperidinamin;
(2S,4S)-2-Benzyl-1-(3,5-dimethylbenzoyl)-N-[3-(2-methoxyphenyl)propyl]-4-piperidinamin;
(2R,4S)-2-Benzyl-1-(3,5-dimethylbenzoyl)-N-(2-trifluormethylbenzyl)-4-piperidinamin;
(2S,4S)-2-Benzyl-1-(3,5-dimethylbenzoyl)-N-(2-trifluormethylbenzyl)-4-piperidinamin;
(2R,4S)-2-Benzyl-1-(3,5-dimethylbenzoyl)-N-[2-(2-trifluormethylphenyl)ethyl]-4-piperidinamin;
(2S,4S)-2-Benzyl-1-(3,5-dimethylbenzoyl)-N-[2-(2-trifluormethylphenyl)ethyl]-4-piperidinamin;
(2R,4S)-2-Benzyl-1-(3,5-dimethylbenzoyl)-N-[3-(2-trifluormethylphenyl)propyl]-4-piperidinamin;
(2S,4S)-2-Benzyl-1-(3,5-dimethylbenzoyl)-N-[3-(2-trifluormethylphenyl)propyl]-4-piperidinamin;
(2R,4S)-2-Benzyl-1-(3,5-dimethylbenzoyl)-N-(3-trifluormethylbenzyl)-4-piperidinamin;
(2S,4S)-2-Benzyl-1-(3,5-dimethylbenzoyl)-N-(3-trifluormethylbenzyl)-4-piperidinamin;
(2R,4S)-2-Benzyl-1-(3,5-dimethylbenzoyl)-N-[2-(3-trifluormethylphenyl)ethyl]-4-piperidinamin;
(2S,4S)-2-Benzyl-1-(3,5-dimethylbenzoyl)-N-[2-(3-trifluormethylphenyl)ethyl]-4-piperidinamin;
(2R,4S)-2-Benzyl-1-(3,5-dimethylbenzoyl)-N-[3-(3-trifluormethylphenyl)propyl]-4-piperidinamin;
(2S,4S)-2-Benzyl-1-(3,5-dimethylbenzoyl)-N-[3-(3-trifluormethylphenyl)propyl]-4-piperidinamin;
(2R,4S)-2-Benzyl-1-(3,5-dimethylbenzoyl)-N-(4-trifluormethylbenzyl)-4-piperidinamin;
(2S,4S)-2-Benzyl-1-(3,5-dimethylbenzoyl)-N-(4-trifluormethylbenzyl)-4-piperidinamin;
(2R,4S)-2-Benzyl-1-(3,5-dimethylbenzoyl)-N-[2-(4-trifluormethylphenyl)ethyl]-4-piperidinamin;
(2S,4S)-2-Benzyl-1-(3,5-dimethylbenzoyl)-N-[2-(4-trifluormethylphenyl)ethyl]-4-piperidinamin;
(2R,4S)-2-Benzyl-1-(3,5-dimethylbenzoyl)-N-[3-(4-trifluormethylphenyl)propyl]-4-piperidinamin;
(2S,4S)-2-Benzyl-1-(3,5-dimethylbenzoyl)-N-[3-(4-trifluormethylphenyl)propyl]-4-piperidinamin;
(2R,4S)-2-Benzyl-1-(3,5-dimethylbenzoyl)-N-(2,3-dimethoxybenzyl)-4-piperidinamin;
(2S,4S)-2-Benzyl-1-(3,5-dimethylbenzoyl)-N-(2,3-dimethoxybenzyl)-4-piperidinamin;
(2R,4S)-2-Benzyl-1-(3,5-dimethylbenzoyl)-N-[2-(2,3-dimethoxyphenyl)ethyl]-4-piperidinamin;
(2S,4S)-2-Benzyl-1-(3,5-dimethylbenzoyl)-N-[2-(2,3-dimethoxyphenyl)ethyl]-4-piperidinamin;
(2R,4S)-2-Benzyl-1-(3,5-dimethylbenzoyl)-N-[3-(2,3-dimethoxyphenyl)propyl]-4-piperidinamin;
(2S,4S)-2-Benzyl-1-(3,5-dimethylbenzoyl)-N-[3-(2,3-dimethoxyphenyl)propyl]-4-piperidinamin;
(2R,4S)-2-Benzyl-1-(3,5-dimethylbenzoyl)-N-(2,4-dimethoxybenzyl)-4-piperidinamin;
(2S,4S)-2-Benzyl-1-(3,5-dimethylbenzoyl)-N-(2,4-dimethoxybenzyl)-4-piperidinamin;
(2R,4S)-2-Benzyl-1-(3,5-dimethylbenzoyl)-N-[2-(2,4-dimethoxyphenyl)ethyl]-4-piperidinamin;
(2S,4S)-2-Benzyl-1-(3,5-dimethylbenzoyl)-N-[2-(2,4-dimethoxyphenyl)ethyl]-4-piperidinamin;
(2R,4S)-2-Benzyl-1-(3,5-dimethylbenzoyl)-N-[3-(2,4-dimethoxyphenyl)propyl]-4-piperidinamin;
(2S,4S)-2-Benzyl-1-(3,5-dimethylbenzoyl)-N-[3-(2,4-dimethoxyphenyl)propyl]-4-piperidinamin;
(2R,4S)-2-Benzyl-1-(3,5-dimethylbenzoyl)-N-(2,5-dimethoxybenzyl)-4-piperidinamin;
(2S,4S)-2-Benzyl-1-(3,5-dimethylbenzoyl)-N-(2,5-dimethoxybenzyl)-4-piperidinamin;
(2R,4S)-2-Benzyl-1-(3,5-dimethylbenzoyl)-N-[2-(2,5-dimethoxyphenyl)ethyl]-4-piperidinamin;
(2S,4S)-2-Benzyl-1-(3,5-dimethylbenzoyl)-N-[2-(2,5-dimethoxyphenyl)ethyl]-4-piperidinamin;
(2R,4S)-2-Benzyl-1-(3,5-dimethylbenzoyl)-N-[3-(2,5-dimethoxyphenyl)propyl]-4-piperidinamin;
(2S,4S)-2-Benzyl-1-(3,5-dimethylbenzoyl)-N-[3-(2,5-dimethoxyphenyl)propyl]-4-piperidinamin;
(2R,4S)-2-Benzyl-1-(3,5-dimethylbenzoyl)-N-(2,6-dimethoxybenzyl)-4-piperidinamin;
(2S,4S)-2-Benzyl-1-(3,5-dimethylbenzoyl)-N-(2,6-dimethoxybenzyl)-4-piperidinamin;
(2R,4S)-2-Benzyl-1-(3,5-dimethylbenzoyl)-N-[2-(2,6-dimethoxyphenyl)ethyl]-4-piperidinamin;
(2S,4S)-2-Benzyl-1-(3,5-dimethylbenzoyl)-N-[2-(2,6-dimethoxyphenyl)ethyl]-4-piperidinamin;
(2R,4S)-2-Benzyl-1-(3,5-dimethylbenzoyl)-N-[3-(2,6-dimethoxyphenyl)propyl]-4-piperidinamin;
(2S,4S)-2-Benzyl-1-(3,5-dimethylbenzoyl)-N-[3-(2,6-dimethoxyphenyl)propyl]-4-piperidinamin;
(2R,4S)-2-Benzyl-1-(3,5-dimethylbenzoyl)-N-(2,3-methylendioxybenzyl)-4-piperidinamin;
(2S,4S)-2-Benzyl-1-(3,5-dimethylbenzoyl)-N-(2,3-methylendioxybenzyl)-4-piperidinamin;
(2R,4S)-2-Benzyl-1-(3,5-dimethylbenzoyl)-N-[2-(2,3-methylendioxyphenyl)ethyl]-4-piperidinamin;
(2S,4S)-2-Benzyl-1-(3,5-dimethylbenzoyl)-N-[2-(2,3-methylendioxyphenyl)ethyl]-4-piperidinamin;
(2R,4S)-2-Benzyl-1-(3,5-dimethylbenzoyl)-N-[3-(2,3-methylendioxyphenyl)propyl]-4-piperidinamin;
(2S,4S)-2-Benzyl-1-(3,5-dimethylbenzoyl)-N-[3-(2,3-methylendioxyphenyl)propyl]-4-piperidinamin;
(2R,4S)-2-Benzyl-1-(3,5-dimethylbenzoyl)-N-(2,4-methylendioxybenzyl)-4-piperidinamin;
(2S,4S)-2-Benzyl-1-(3,5-dimethylbenzoyl)-N-(2,4-methylendioxybenzyl)-4-piperidinamin;
(2R,4S)-2-Benzyl-1-(3,5-dimethylbenzoyl)-N-[2-(2,4-methylendioxyphenyl)ethyl]-4-piperidinamin;
(2S,4S)-2-Benzyl-1-(3,5-dimethylbenzoyl)-N-[2-(2,4-methylendioxyphenyl)ethyl]-4-piperidinamin;
(2R,4S)-2-Benzyl-1-(3,5-dimethylbenzoyl)-N-[3-(2,4-methylendioxyphenyl)propyl]-4-piperidinamin;
(2S,4S)-2-Benzyl-1-(3,5-dimethylbenzoyl)-N-[3-(2,4-methylendioxyphenyl)propyl]-4-piperidinamin;
(2R,4S)-2-Benzyl-1-(3,5-dimethylbenzoyl)-N-(indol-2-ylmethyl)-4-piperidinamin;

(2S,4S)-2-Benzyl-1-(3,5-dimethylbenzoyl)-N-(indol-2-ylmethyl)-4-piperidinamin;
(2R,4S)-2-Benzyl-1-(3,5-dimethylbenzoyl)-N-(indol-3-ylmethyl)-4-piperidinamin;
(2S,4S)-2-Benzyl-1-(3,5-dimethylbenzoyl)-N-(indol-3-ylmethyl)-4-piperidinamin;
(2S,4S)-2-Benzyl-1-(3,5-dimethylbenzoyl)-N-(chinolin-2-ylmethyl)-4-piperidinamin;
(2S,4S)-2-Benzyl-1-(3,5-dimethylbenzoyl)-N-(chinolin-3-ylmethyl)-4-piperidinamin;
(2R,4S)-2-Benzyl-1-(3,5-dimethylbenzoyl)-N-(2-chlorbenzyl)-4-piperidinamin;
(2S,4S)-2-Benzyl-1-(3,5-dimethylbenzoyl)-N-(2-chlorbenzyl)-4-piperidinamin;
(2R,4S)-2-Benzyl-1-(3,5-dimethylbenzoyl)-N-[2-(2-chlorphenyl)ethyl]-4-piperidinamin;
(2S,4S)-2-Benzyl-1-(3,5-dimethylbenzoyl)-N-[2-(2-chlorphenyl)ethyl]-4-piperidinamin;
(2R,4S)-2-Benzyl-1-(3,5-dimethylbenzoyl)-N-[3-(2-chlorphenyl)propyl]-4-piperidinamin;
(2S,4S)-2-Benzyl-1-(3,5-dimethylbenzoyl)-N-[3-(2-chlorphenyl)propyl]-4-piperidinamin;
(2R,4S)-2-Benzyl-1-(3,5-dimethylbenzoyl)-N-(3-chlorbenzyl)-4-piperidinamin;
(2S,4S)-2-Benzyl-1-(3,5-dimethylbenzoyl)-N-(3-chlorbenzyl)-4-piperidinamin;
(2R,4S)-2-Benzyl-1-(3,5-dimethylbenzoyl)-N-[2-(3-chlorphenyl)ethyl]-4-piperidinamin;
(2S,4S)-2-Benzyl-1-(3,5-dimethylbenzoyl)-N-[2-(3-chlorphenyl)ethyl]-4-piperidinamin;
(2R,4S)-2-Benzyl-1-(3,5-dimethylbenzoyl)-N-[3-(3-chlorphenyl)propyl]-4-piperidinamin;
(2S,4S)-2-Benzyl-1-(3,5-dimethylbenzoyl)-N-[3-(3-chlorphenyl)propyl]-4-piperidinamin;
(2R,4S)-2-Benzyl-1-(3,5-dimethylbenzoyl)-N-(4-chlorbenzyl)-4-piperidinamin;
(2S,4S)-2-Benzyl-1-(3,5-dimethylbenzoyl)-N-(4-chlorbenzyl)-4-piperidinamin;
(2R,4S)-2-Benzyl-1-(3,5-dimethylbenzoyl)-N-[2-(4-chlorphenyl)ethyl]-4-piperidinamin;
(2S,4S)-2-Benzyl-1-(3,5-dimethylbenzoyl)-N-[2-(4-chlorphenyl)ethyl]-4-piperidinamin;
(2R,4S)-2-Benzyl-1-(3,5-dimethylbenzoyl)-N-[3-(4-chlorphenyl)propyl]-4-piperidinamin;
(2S,4S)-2-Benzyl-1-(3,5-dimethylbenzoyl)-N-[3-(4-chlorphenyl)propyl]-4-piperidinamin;
(2R,4S)-2-Benzyl-1-(3,5-dimethylbenzoyl)-N-(4-methoxynaphth-1-ylmethyl)-4-piperidinamin;
(2S,4S)-2-Benzyl-1-(3,5-dimethylbenzoyl)-N-(4-methoxynaphth-1-ymethyl)-4-piperidina min;
(2R,4S)-2-Benzyl-1-(3,5-dimethylbenzoyl)-N-(3,4-ethylendioxybenzyl)-4-piperidinamin;
(2S,4S)-2-Benzyl-1-(3,5-dimethylbenzoyl)-N-(3,4-ethylendioxybenzyl)-4-piperidinamin;
(2R,4S)-2-Benzyl-1-(3,5-dimethylbenzoyl)-N-(chinolin-2-ylmethyl)-4-piperidinamin;
(2R,4S)-2-Benzyl-1-(3,5-dimethylbenzoyl)-N-(chinolin-3-ylmethyl)-4-piperidinamin;
(2R,4S)-2-Benzyl-1-(3,5-ditrifluormethylbenzoyl)-N-(indol-2-ylmethyl)-4-piperidinamin;
(2S,4S)-2-Benzyl-1-(3,5-ditrifluormethylbenzoyl)-N-(indol-2-ylmethyl)-4-piperidinamin;
(2R,4S)-2-Benzyl-1-(3,5-ditrifluormethylbenzoyl)-N-(indol-3-ylmethyl)-4-piperidinamin;
(2S,4S)-2-Benzyl-1-(3,5-ditrifluormethylbenzoyl)-N-(indol-3-ylmethyl)-4-piperidinamin;
(2R,4S)-2-Benzyl-1-(3,5-ditrifluourmethylbenzoyl)-N-(chinolin-2-ylmethyl)-4-piperidinamin;
(2S,4S)-2-Benzyl-1-(3,5-ditriflourmethylbenzoyl)-N-(chinolin-2-ylmethyl)-4-piperidinamin;
(2R,4S)-2-Benzyl-1-(3,5-ditrifluourmethylbenzoyl)-N-(chinolin-3-ylmethyl)-4-piperidinamin;
(2S,4S)-2-Benzyl-1-(3,5-ditrifluormethylbenzoyl)-N-(chinolin-3-ylmethyl)-4-piperidinamin;
(2R,4S)-2-Benzyl-1-(3,5-ditrifluormethylbenzoyl)-N-(2-chlorbenzyl)-4-piperidinamin;
(2S,4S)-2-Benzyl-1-(3,5-ditrifluourmethylbenzoyl)-N-(2-chlorbenzyl)-4-piperidinamin;
(2R,4S)-2-Benzyl-1-(3,5-ditrifluormethylbenzoyl)-N-[2-(2-chlorphenyl)ethyl]-4-piperidinamin;
(2S,4S)-2-Benzyl-1-(3,5-ditrifluormethylbenzoyl)-N-[2-(2-chlorphenyl)ethyl]-4-piperidinamin;
(2R,4S)-2-Benzyl-1-(3,5-ditrifluormethylbenzoyl)-N-[3-(2-chlorphenyl)propyl]-4-piperidinamin;
(2S,4S)-2-Benzyl-1-(3,5-ditrifluormethylbenzoyl)-N-[3-(2-chlorphenyl)propyl]-4-piperidinamin;
(2R,4S)-2-Benzyl-1-(3,5-ditrifluormethylbenzoyl)-N-(3-chlorbenzyl)-4-piperidinamin;
(2S,4S)-2-Benzyl-1-(3,5-ditrifluormethylbenzoyl)-N-(3-chlorbenzyl)-4-piperidinamin;
(2R,4S)-2-Benzyl-1-(3,5-ditrifluormethylbenzoyl)-N-[2-(3-chlorphenyl)ethyl]-4-piperidinamin;
(2S,4S)-2-Benzyl-1-(3,5-ditrifluormethylbenzoyl)-N-[2-(3-chlorphenyl)ethyl]-4-piperidinamin;
(2R,4S)-2-Benzyl-1-(3,5-ditrifluormethylbenzoyl)-N-[3-(3-chlorphenyl)propyl]-4-piperidinamin;
(2S,4S)-2-Benzyl-1-(3,5-ditrifluormethylbenzoyl)-N-[3-(3-chlorphenyl)propyl]-4-piperidinamin;
(2R,4S)-2-Benzyl-1-(3,5-ditrifluormethylbenzoyl)-N-(4-chlorbenzyl)-4-piperidinamin;
(2S,4S)-2-Benzyl-1-(3,5-ditrifluormethylbenzoyl)-N-(4-chlorbenzyl)-4-piperidinamin;
(2R,4S)-2-Benzyl-1-(3,5-ditrifluormethylbenzoyl)-N-[2-(4-chlorphenyl)ethyl]-4-piperidinamin;
(2S,4S)-2-Benzyl-1-(3,5-ditrifluormethylbenzoyl)-N-[2-(4-chlorphenyl)ethyl]-4-piperidinamin;
(2R,4S)-2-Benzyl-1-(3,5-ditrifluormethylbenzoyl)-N-[3-(4-chlorphenyl)propyl]-4-piperidinamin;
(2S,4S)-2-Benzyl-1-(3,5-ditrifluormethylbenzoyl)-N-[3-(4-chlorophenyl)propyl]-4-piperidinamin;
(2R,4S)-2-Benzyl-1-(3,5-ditrifluormethylbenzoyl)-N-(4-methoxynaphth-1-ylmethyl)-4-piperidinamin;
(2S,4S)-2-Benzyl-1-(3,5-ditrifluormethylbenzoyl)-N-(4-methoxynaphth-1-ylmethyl)-4-piperidinamin;
(2R,4S)-2-Benzyl-1-(3,5-ditrifluormethylbenzoyl)-N-(3,4-ethylendioxybenzyl)-4-piperidinamin;

(2S,4S)-2-Benzyl-1-(3,5-ditrifluormethylbenzoyl)-N-(3,4-ethylendioxybenzyl)-4-piperidinamin;
(2R,4S)-2-Benzyl-1-(3,5-dichlorbenzoyl)-N-(indol-2-ylmethyl)-4-piperidinamin;
(2S,4S)-2-Benzyl-1-(3,5-dichlorbenzoyl)-N-(indol-2-ylmethyl)-4-piperidinamin;
(2R,4S)-2-Benzyl-1-(3,5-dichlorbenzoyl)-N-(indol-3-ylmethyl)-4-piperidinamin;
(2S,4S)-2-Benzyl-1-(3,5-dichlorbenzoyl)-N-(indol-3-ylmethyl)-4-piperidinamin;
(2R,4S)-2-Benzyl-1-(3,5-dichlorbenzoyl)-N-(chinolin-2-ylmethyl)-4-piperidinamin;
(2S,4S)-2-Benzyl-1-(3,5-dichlorbenzoyl)-N-(chinolin-2-ylmethyl)-4-piperidinamin;
(2R,4S)-2-Benzyl-1-(3,5-dichlorbenzoyl)-N-(chinolin-3-ylmethyl)-4-piperidinamin;
(2S,4S)-2-Benzyl-1-(3,5-dichlorbenzoyl)-N-(chinolin-3-ylmethyl)-4-piperidinamin;
(2R,4S)-2-Benzyl-1-(3,5-dichlorbenzoyl)-N-(2-chlorbenzyl)-4-piperidinamin;
(2S,4S)-2-Benzyl-1-(3,5-dichlorbenzoyl)-N-(2-chlorbenzyl)-4-piperidinamin;
(2R,4S)-2-Benzyl-1-(3,5-dichlorbenzoyl)-N-[2-(2-chlorphenyl)ethyl]-4-piperidinamin;
(2S,4S)-2-Benzyl-1-(3,5-dichlorbenzoyl)-N-[2-(2-chlorphenyl)ethyl]-4-piperidinamin;
(2R,4S)-2-Benzyl-1-(3,5-dichlorbenzoyl)-N-[3-(2-chlorphenyl)propyl]-4-piperidinamin;
(2S,4S)-2-Benzyl-1-(3,5-dichlorbenzoyl)-N-[3-(2-chlorphenyl)propyl]-4-piperidinamin;
(2R,4S)-2-Benzyl-1-(3,5-dichlorbenzoyl)-N-(3-chlorbenzyl)-4-piperidinamin;
(2S,4S)-2-Benzyl-1-(3,5-dichlorbenzoyl)-N-(3-chlorbenzyl)-4-piperidinamin;
(2R,4S)-2-Benzyl-1-(3,5-dichlorbenzoyl)-N-[2-(3-chlorphenyl)ethyl]-4-piperidinamin;
(2S,4S)-2-Benzyl-1-(3,5-dichlorbenzoyl)-N-[2-(3-chlorphenyl)ethyl]-4-piperidinamin;
(2R,4S)-2-Benzyl-1-(3,5-dichlorbenzoyl)-N-[3-(3-chlorphenyl)propyl]-4-piperidinamin;
(2S,4S)-2-Benzyl-1-(3,5-dichlorbenzoyl)-N-[3-(3-chlorphenyl)propyl]-4-piperidinamin;
(2R,4S)-2-Benzyl-1-(3,5-dichlorbenzoyl)-N-(4-chlorbenzyl)-4-piperidinamin;
(2S,4S)-2-Benzyl-1-(3,5-dichlorbenzoyl)-N-(4-chlorbenzyl)-4-piperidinamin;
(2R,4S)-2-Benzyl-1-(3,5-dichlorbenzoyl)-N-[2-(4-chlorphenyl)ethyl]-4-piperidinamin;
(2S,4S)-2-Benzyl-1-(3,5-dichlorbenzoyl)-N-[2-(4-chlorphenyl)ethyl]-4-piperidinamin;
(2R,4S)-2-Benzyl-1-(3,5-dichlorbenzoyl)-N-[3-(4-chlorphenyl)propyl]-4-piperidinamin;
(2S,4S)-2-Benzyl-1-(3,5-dichlorbenzoyl)-N-[3-(4-chlorphenyl)propyl]-4-piperidinamin;
(2R,4S)-2-Benzyl-1-(3,5-dichlorbenzoyl)-N-(4-methoxynaphth-1-ylmethyl)-4-piperidinamin;
(2S,4S)-2-Benzyl-1-(3,5-dichlorbenzoyl)-N-(4-methoxynaphth-1-ylmethyl)-4-piperidinam in;
(2R,4S)-2-Benzyl-1-(3,5-dichlorbenzoyl)-N-(3,4-ethylendioxybenzyl)-4-piperidinamin;
(2S,4S)-2-Benzyl-1-(3,5-dichlorbenzoyl)-N-(3,4-ethylendioxybenzyl)-4-piperidinamin;
(2R,4S)-2-Benzyl-1-(3,5-dimethoxybenzoyl)-N-(indol-2-ylmethyl)-4-piperidinamin;
(2S,4S)-2-Benzyl-1-(3,5-dimethoxybenzoyl)-N-(indol-2-ylmethyl)-4-piperidinamin;
(2R,4S)-2-Benzyl-1-(3,5-dimethoxybenzoyl)-N-(indol-3-ylmethyl)-4-piperidinamin;
(2S,4S)-2-Benzyl-1-(3,5-dimethoxybenzoyl)-N-(indol-3-ylmethyl)-4-piperidinamin;
(2R,4S)-2-Benzyl-1-(3,5-dimethoxybenzoyl)-N-(chinolin-2-ylmethyl)-4-piperidinamin;
(2S,4S)-2-Benzyl-1-(3,5-dimethoxybenzoyl)-N-(chinolin-2-ylmethyl)-4-piperidinamin;
(2R,4S)-2-Benzyl-1-(3,5-dimethoxybenzoyl)-N-(chinolin-3-ylmethyl)-4-piperidinamin;
(2S,4S)-2-Benzyl-1-(3,5-dimethoxybenzoyl)-N-(chinolin-3-ylmethyl)-4-piperidinamin;
(2R,4S)-2-Benzyl-1-(3,5-dimethoxybenzoyl)-N-(2-chlorbenzyl)-4-piperidinamin;
(2S,4S)-2-Benzyl-1-(3,5-dimethoxybenzoyl)-N-(2-chlorbenzyl)-4-piperidinamin;
(2R,4S)-2-Benzyl-1-(3,5-dimethoxybenzoyl)-N-[2-(2-chlorphenyl)ethyl]-4-piperidinamin;
(2S,4S)-2-Benzyl-1-(3,5-dimethoxybenzoyl)-N-[2-(2-chlorphenyl)ethyl]-4-piperidinamin;
(2R,4S)-2-Benzyl-1-(3,5-dimethoxybenzoyl)-N-[3-(2-chlorphenyl)propyl]-4-piperidinamin;
(2S,4S)-2-Benzyl-1-(3,5-dimethoxybenzoyl)-N-[3-(2-chlorphenyl)propyl]-4-piperidinamin;
(2R,4S)-2-Benzyl-1-(3,5-dimethoxybenzoyl)-N-(3-chlorbenzyl)-4-piperidinamin;
(2S,4S)-2-Benzyl-1-(3,5-dimethoxybenzoyl)-N-(3-chlorbenzyl)-4-piperidinamin;
(2R,4S)-2-Benzyl-1-(3,5-dimethoxybenzoyl)-N-[2-(3-chlorphenyl)ethyl]-4-piperidinamin;
(2S,4S)-2-Benzyl-1-(3,5-dimethoxybenzoyl)-N-[2-(3-chlorphenyl)ethyl]-4-piperidinamin;
(2R,4S)-2-Benzyl-1-(3,5-dimethoxybenzoyl)-N-[3-(3-chlorphenyl)propyl]-4-piperidinamin;
(2S,4S)-2-Benzyl-1-(3,5-dimethoxybenzoyl)-N-[3-(3-chlorphenyl)propyl]-4-piperidinamin;
(2R,4S)-2-Benzyl-1-(3,5-dimethoxybenzoyl)-N-(4-chlorbenzyl)-4-piperidinamin;
(2S,4S)-2-Benzyl-1-(3,5-dimethoxybenzoyl)-N-(4-chlorbenzyl)-4-piperidinamin;
(2R,4S)-2-Benzyl-1-(3,5-dimethoxybenzoyl)-N-[2-(4-chlorphenyl)ethyl]-4-piperidinamin;
(2S,4S)-2-Benzyl-1-(3,5-dimethoxybenzoyl)-N-[2-(4-chlorphenyl)ethyl]-4-piperidinamin;
(2R,4S)-2-Benzyl-1-(3,5-dimethoxybenzoyl)-N-[3-(4-chlorphenyl)propyl]-4-piperidinamin;
(2S,4S)-2-Benzyl-1-(3,5-dimethoxybenzoyl)-N-[3-(4-chlorphenyl)propyl]-4-piperidinamin;
(2R,4S)-2-Benzyl-1-(3,5-dimethoxybenzoyl)-N-(4-methoxynaphth-1-ylmethyl)-4 piperidinamin;

(2S,4S)-2-Benzyl-1-(3,5-dimethoxybenzoyl)-N-(4-methoxynaphth-1-ylmethyl)-4-piperidi namin; (2R,4S)-2-Benzyl-1-(3,5-dimethoxybenzoyl)-N-(3,4-ethylendioxybenzyl)-4-piperidinamin oder (2S,4S)-2-Benzyl-1-(3,5-dimethoxybenzoyl)-N-(3,4-ethylendioxybenzyl)-4-piperidinamin oder jeweils ein Salz davon herstellt.

**50.** Verfahren gemäss Anspruch 1, dadurch gekennzeichnet, dass man (2R,4S) und (2R,4R)-2-Benzyl-1-(3,5-dimethylbenzoyl)-N-(2-phenethyl)-4-piperidinamin oder ein Salz davon herstellt.

**51.** Verfahren gemäss Anspruch 1, dadurch gekennzeichnet, dass man (2R,4S)-2-Benzyl-1-(3,5-bis-(trifluormethyl)-b enzoyl)-N-(4-chinolylmethyl)-4-piperidinamin oder ein Salz davon herstellt.

**52.** Verfahren gemäss Anspruch 1, dadurch gekennzeichnet, dass man (2R,4S)-2-Benzyl-1-(3,5-dichlorbenzoyl)-N-(4-chinolylmethyl)-4-piperidinamin oder ein Salz davon herstellt.

**53.** Verfahren gemäss Anspruch 1, dadurch gekennzeichnet, dass man (2R*,4S*)-2-Benzyl-1-(2,4-dichlorbenzoyl)-N-(4-chinolylmethyl)-4-piperidinamin oder ein Salz davon herstellt.

**54.** Verfahren gemäss Anspruch 1, dadurch gekennzeichnet, dass man (2R*,4S*)-2-Benzyl-1-(phenylacetyl)-N-(4-chinolylmethyl)-4-piperidinamin oder ein Salz davon herstellt.

**55.** Verfahren gemäss Anspruch 1, dadurch gekennzeichnet, dass man (2R*,4S*)-2-Benzyl-1-(2,6-dichlorbenzoyl)-N-(4-chinolylmethyl)-4-piperidinamin oder ein Salz davon herstellt.

**56.** Verfahren gemäss Anspruch 1, dadurch gekennzeichnet, dass man (2R*,4S*)-2-Benzyl-1-(3,5-dibrombenzoyl)-N-(4-chinolylmethyl)-4-piperidinamin oder ein Salz davon herstellt.

**57.** Verfahren gemäss Anspruch 1, dadurch gekennzeichnet, dass man (2R*,4S*)-2-Benzyl-1-(9-fluorenoyl)-N-(4-chinolylmethyl)-4-piperidinamin oder ein Salz davon herstellt.

**58.** Verfahren gemäss Anspruch 1, dadurch gekennzeichnet, dass man (2R*,4S*)-2-Benzyl-1-(3-toluoyl)-N-(4-chinolylmethyl)-4-piperidinamin oder ein Salz davon herstellt.

**59.** Verfahren gemäss Anspruch 1, dadurch gekennzeichnet, dass man (2R*,4S*)-2-Benzyl-1-(3-brombenzoyl)-N-(4-chinolylmethyl)-4-piperidinamin oder ein Salz davon herstellt.

**60.** Verfahren gemäss Anspruch 1, dadurch gekennzeichnet, dass man (2R*,4S*)-2-Benzyl-1-(3,5-dihydroxybenzoyl)-N-(4-chinolylmethyl)-4-piperidinamin oder ein Salz davon herstellt.

**61.** Verfahren gemäss Anspruch 1, dadurch gekennzeichnet, dass man (2R*,4S*)-2-Benzyl-1-(3-cyanbenzoyl)-N-(4-chinolylmethyl)-4-piperidinamin oder ein Salz davon herstellt.

**62.** Verfahren gemäss Anspruch 1, dadurch gekennzeichnet, dass man (2R*,4S*)-2-Benzyl-1-(2-chlorbenzoyl)-N-(4-chinolylmethyl)-4-piperidinamin oder ein Salz davon herstellt.

**63.** Verfahren gemäss Anspruch 1, dadurch gekennzeichnet, dass man (2R*,4S*)-2-Benzyl-1-(4-chlorbenzoyl)-N-(4-chinolylmethyl)-4-piperidinamin oder ein Salz davon herstellt.

**64.** Verfahren gemäss Anspruch 1, dadurch gekennzeichnet, dass man (2R*,4S*)-2-Benzyl-1-(3,5-dichlorbenzoyl)-N-(4-chinolylmethyl)-N-methyl-4-piperidinamin oder ein Salz davon herstellt.

**65.** Verfahren gemäss Anspruch 1, dadurch gekennzeichnet, dass man (2R*,4S*)-2-Benzyl-1-(3,5-dichlorbenzoyl)-N-(4-chinolylmethyl)-N-cyclohexylcarbamoyl-4-piperidinamin oder ein Salz davon herstellt.

**66.** Verfahren gemäss Anspruch 1, dadurch gekennzeichnet, dass man (2R*,4S*)-2-Benzyl-1-(3,5-dichlorbenzoyl)-N-(4-chinolylmethyl)-N-phenylcarbamoyl-4-piperidinamin oder ein Salz davon herstellt.

**67.** Verfahren gemäss Anspruch 1, dadurch gekennzeichnet, dass man (2R*,4S*)-2-Benzyl-1-(3,5-dichlorbenzoyl)-N-(2-phenylethyl)-4-piperidinamin oder ein Salz davon herstellt.

**68.** Verfahren gemäss Anspruch 1, dadurch gekennzeichnet, dass man (2R*,4S*)-2-Benzyl-1-(3,5-bis-(trifluormethyl)-benzoyl)-N-(2-phenylethyl)-4-piperidinamin oder ein Salz davon herstellt.

**69.** Verfahren gemäss Anspruch 1, dadurch gekennzeichnet, dass man (2R*,4S*)-2-Benzyl-1-(3,5-dichlorbenzoyl)-N-(2-naphthoyl)-4-piperidinamin oder ein Salz davon herstellt.

**70.** Verfahren gemäss Anspruch 1, dadurch gekennzeichnet, dass man (2R*,4S*)-2-Benzyl-1-(3,5-dichlorbenzoyl)-N-(3,5-dimethylbenzoyl)-4-piperidinamin oder ein Salz davon herstellt.

**71.** Verfahren gemäss Anspruch 1, dadurch gekennzeichnet, dass man (2R*,4S*)-2-Benzyl-1-(3,5-dichlorbenzoyl)-N-(4-chinolylcarbonyl)-4-piperidinamin oder ein Salz davon herstellt.

**72.** Verfahren gemäss Anspruch 1, dadurch gekennzeichnet, dass man (2R*,4S*)-2-Benzyl-1-(3,5-dichlorbenzoyl)-N-(3-indolylcarbonyl)-4-piperidinamin oder ein Salz davon herstellt.

**73.** Verfahren gemäss Anspruch 1, dadurch gekennzeichnet, dass man (2R*,4S*)-2-Benzyl-1-(3,5-dichlorbenzoyl)-N-(2-indolylcarbonyl)-4-piperidinamin oder ein Salz davon herstellt.

**74.** Verfahren gemäss Anspruch 1, dadurch gekennzeichnet, dass man (2R*,4S*)-2-Benzyl-1-(3,5-dichlorbenzoyl)-N-(5-methoxy-2-indolylcarbonyl)-4-piperidinamin oder ein Salz davon herstellt.

**75.** Verfahren gemäss Anspruch 1, dadurch gekennzeichnet, dass man (2R*,4S*)-2-Benzyl-1-(3,5-dichlorbenzoyl)-N-(1-naphthoyl)-4-piperidinamin oder ein Salz davon herstellt.

**76.** Verfahren gemäss Anspruch 1, dadurch gekennzeichnet, dass man (2R*,4S*)-2-Benzyl-1-(3,5-dichlorbenzoyl)-N-(phenylacetyl)-4-piperidinamin oder ein Salz davon herstellt.

**77.** Verfahren gemäss Anspruch 1, dadurch gekennzeichnet, dass man (2R*,4S*)-2-Benzyl-1-(3,5-dichlorbenzoyl)-N-(2-methoxybenzyl)-4-piperidinamin oder ein Salz davon herstellt.

**78.** Verfahren gemäss Anspruch 1, dadurch gekennzeichnet, dass man (2R*,4S*)-2-Benzyl-1-(3,5-dichlorbenzoyl)-N-(3-(N-acetyl)-indolylmethyl)-4-piperidinamin oder ein Salz davon herstellt.

**79.** Verfahren gemäss Anspruch 1, dadurch gekennzeichnet, dass man (2R*,4S*)-2-Benzyl-1-(3,5-dichlorbenzoyl)-N-(2-benzo[b]furanylmethyl)-4-piperidinamin oder ein Salz davon herstellt.

**80.** Verfahren gemäss Anspruch 1, dadurch gekennzeichnet, dass man (2R*,4S*)-2-Benzyl-1-(3,5-dichlorbenzoyl)-N-[(3-methylbenzo[b]thiophen-2-ylmethyl]-4-piperidinamin oder ein Salz davon herstellt.

**81.** Verfahren gemäss Anspruch 1, dadurch gekennzeichnet, dass man (2R*,4S*)-2-Benzyl-1-(3,5-dichlorbenzoyl)-N-(5-methoxyindol-3-ylmethyl)-4-piperidinamin oder ein Salz davon herstellt.

**82.** Verfahren gemäss Anspruch 1, dadurch gekennzeichnet, dass man (2R*,4S*)-2-Benzyl-1-(3,5-dichlorbenzoyl)-N-(3-indolylmethyl)-4-piperidinamin oder ein Salz davon herstellt.

**83.** Verfahren gemäss Anspruch 1, dadurch gekennzeichnet, dass man (2R*,4S*)-2-Benzyl-1-(3,5-dichlorbenzoyl)-N-phenylcarbamoyl-4-piperidinamin oder ein Salz davon herstellt.

**84.** Verfahren gemäss Anspruch 1, dadurch gekennzeichnet, dass man (2R*,4S*)-2-Benzyl-1-(3,5-dichlorbenzoyl)-N-diphenylmethyl-4-piperidinamin oder ein Salz davon herstellt.

**85.** Verfahren gemäss Anspruch 1, dadurch gekennzeichnet, dass man (2R*,4S*)-2-Benzyl-1-(3,5-dichlorbenzoyl)-N-(3,4-dihydro-2H-1-benzopyran-2-carbonyl)-4-piperidinamin oder ein Salz davon herstellt.

**86.** Verfahren gemäss Anspruch 1, dadurch gekennzeichnet, dass man (2R*,4S*)-2-Benzyl-1-(4-methoxybenzoyl)-N-(4-chinolylmethyl)-4-piperidinamin oder ein Salz davon herstellt.

**87.** Verfahren gemäss Anspruch 1, dadurch gekennzeichnet, dass man (2R*,4S*,1'R*)-N-Benzyl-1-(3,5-dimethylbenzoyl)-2-(1'-hydroxy-1'-phenyl-methyl)-4-piperidinamin oder ein Salz davon herstellt.

**88.** Verfahren gemäss Anspruch 1, dadurch gekennzeichnet, dass man (2R*,4S*,1'R*)-2-(1'-hydroxy-1'-phenyl-methyl)-1-(3,5-dimethylbenzoyl)-N-(4-chinolinylmethyl)-4-piperidinamin oder ein Salz davon herstellt.

**89.** Verfahren gemäss Anspruch 1, dadurch gekennzeichnet, dass man (2R*,4S*,1'S*)-1-(3,5-Dimethylbenzoyl)-2-(1'-hydroxy-1'-phenyl-methyl)-N-(4-chinolinylmethyl)-4-piperidinamin oder ein Salz davon herstellt.

**90.** Verfahren gemäss Anspruch 1, dadurch gekennzeichnet, dass man (2R*,4S*,1'R*)-2-{1'-Hydroxy-1'-(4-chlorphenyl)methyl}-1-(3,5-dimethylbenzoyl)-N-(4-chinolinylmethyl)-4-piperidinamin oder ein Salz davon herstellt.

**91.** Verfahren gemäss Anspruch 1, dadurch gekennzeichnet, dass man (2R*,4S*,1'S*)-1-(3,5-Dimethylbenzoyl)-2-{1'-hydroxy-1'-(4-chlorphenyl)methyl}-N-(4-chinolinylmethyl)-4-piperidinamin oder ein Salz davon herstellt.

**92.** Verfahren gemäss Anspruch 1, dadurch gekennzeichnet, dass man (2R*,4S*,1'S*)-1-(3,5-Dimethylbenzoyl)-2-{1'-hydroxy-1'-(3,4-dichlorphenyl)methyl}-N-(4-chinolinylmethyl)-4-piperidinamin oder ein Salz davon herstellt.

**93.** Verfahren gemäss Anspruch 1, dadurch gekennzeichnet, dass man (2R*,4S*,)-N-Benzyl-1-(3,5-dimethylbenzoyl)-2-benzoyl-4-piperidinamin oder ein Salz davon herstellt.

**94.** Verfahren gemäss Anspruch 1, dadurch gekennzeichnet, dass man (2R*,4S*)-2-(4-Chlorbenzyl)-1-(3,5-dimethylbenzoyl)-N-(4-chinolinylmethyl)-4-piperidinamin oder ein Salz davon herstellt.

**95.** Verfahren gemäss Anspruch 1, dadurch gekennzeichnet, dass man (2R*,4S*)-2-(3,4-Dichlorbenzyl)-1-(3,5-dimethylbenzoyl)-N-(4-chinolinylmethyl)-4-piperidinamin oder ein Salz davon herstellt.

**96.** Verfahren gemäss Anspruch 1, dadurch gekennzeichnet, dass man (2R*,4S*)-1-(3,5-Dimethylbenzoyl)-2-phenyl-N-(4-chinolinylmethyl)-4-piperidinamin oder ein Salz davon herstellt.

**97.** Verfahren gemäss Anspruch 1, dadurch gekennzeichnet, dass man (2R*,4S*)-1-(3,5-Dichlorbenzoyl)-2-phenyl-N-(4-chinolinmethyl)-4-piperidinamin oder ein Salz davon herstellt.

**98.** Verfahren gemäss Anspruch 1, dadurch gekennzeichnet, dass man (2R*,4S*)-1-(1-Naphthoyl)-2-phenyl-N-(4-chinolinylmethyl)-4-piperidineamin

**99.** Verfahren gemäss Anspruch 1, dadurch gekennzeichnet, dass man (2R*,4S*)-1-(3,5-Dimethylbenzoyl)-2-(1-naphthyl)-N-(4-chinolinylmethyl)-4-piperidinamin oder ein Salz davon herstellt.

**100.** Verfahren gemäss Anspruch 1, dadurch gekennzeichnet, dass man (2R*,4S*)-1-(3,5-Dimethylbenzoyl)-2-(2-naphthyl)-N-(4-chinolinylmethyl)-4-piperidinamin;
(2R*,4S*)-1-(3,5-Dimethylbenzoyl)-2-(4-methoxyphenylmethyl)-N-(4-chinolinylmethyl)-4-piperidinamin;
(2R*,4S*)-1-(3,5-Dimethylbenzoyl)-2-(3-methoxyphenylmethyl)-N-(4-chinolinylmethyl)-4-piperidinamin;
(2R*,4S*)-1-(3,5-Dimethylbenzoyl)-2-(4-nitrobenzyl)-N-(4-chinolinylmethyl)-4-piperidinamin;
(2R*,4S*)-1-(3,5-Dimethylbenzoyl)-2-(4-trifluormethylphenylmethyl)-N-(4-chinolinylmethyl)-4-piperidinamin;
(2R*,4S*)-1-(3,5-Dimethylbenzoyl)-2-(2,4-dichlorphenylmethyl)-N-(4-chinolinylmethyl)-4-piperidinamin;
(2R*,4S*)-1-(3,5-Dimethylbenzoyl)-2-(2-phenylethyl)-N-(4-chinolinylmethyl)-4-piperidinamin;
(2R*,4S*)-1-(3,5-Dimethylbenzoyl)-2-(2-phenylethenyl)-N-(4-chinolinylmethyl)-4-piperidinamin;
(2R*,4S*)-1-(3,5-Dimethylbenzoyl)-2-benzoyl-N-(4-chinolinylmethyl)-4-piperidinamin;
(2R*,4S*)-1-(3,5-Dimethylbenzoyl)-2-(4-chlorbenzoyl)-N-(4-chinolinylmethyl)-4-piperidinamin;
(2R*,4S*)-1-(3,5-Dimethylbenzoyl)-2-(2-naphthyl)-N-(4-chinolinylmethyl)-4-piperidinamin;
(2R*,4S*)-1-(3,5-Dimethylbenzoyl)-2-(4-methoxybenzyl)-N-(4-chinolinylmethyl)-4-piperidinamin;

(2R*,4S*)-1-(3,5-Dimethylbenzoyl)-2-(3-methoxybenzyl)-(4-chinolinylmethyl)-4-piperidinamin;
(2R*,4S*)-1-(3,5-Dimethylbenzoyl)-2-(4-nitrobenzyl)-(4-chinolinylmethyl)-4-piperidinamin;
(2R*,4S*)-1-(3,5-Dimethylbenzoyl)-2-(4-trifluormethylbenzyl)-(4-chinolinylmethyl)-4-piperidinamin;
(2R*,4S*)-1-(3,5-Dimethylbenzoyl)-2-(2,4-dichlorbenzyl)-(4-chinolinylmethyl)-4-piperidinamin;
(2R*,4S*)-1-(3,5-Dimethylbenzoyl)-2-(2-phenylethyl)-(4-chinolinylmethyl)-4-piperidinamin;
(2R*,4S*)-1-(3,5-Dimethylbenzoyl)-2-(2-phenylethenyl)-N-(4-chinolinylmethyl)-4-piperidinamin;
(2R*,4S*)-1-(3,5-Dimethylbenzoyl)-2-(benzoylmethyl)-N-(4-chinolinylmethyl)-4-piperidinamin oder
(2R*,4S*)-1-(3,5-Dimethylbenzoyl)-2-(4-chlorbenzoylmethyl)-N-(4-chinolinylmethyl)-4-piperidinamin oder jeweils ein Salz davon herstellt.

**101.** Verfahren zur Herstellung neuer 1-Acylpiperidone der Formel X

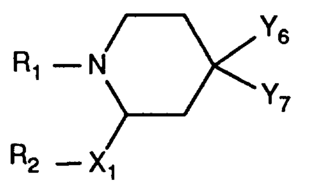

(X),

worin $R_1$ einen gegebenenfalls substituierten Aroyl-, Heteroaroyl-, Cycloalkylcarbonyl-, Aralkanoyl-, Heteroarylalkanoyl-, Aralkoxycarbonyl- oder Arylcarbamoylrest oder den Acylrest einer gegebenenfalls durch Niederalkanoyl oder Carbamoylniederalkanoyl N-substituierten α-Aminosäure bedeutet, $R_2$ Cycloalkyl oder einen gegebenenfalls substituierten Aryl- oder Heteroarylrest darstellt, $X_1$ Hydroxymethylen darstellt und $Y_6$ und $Y_7$ gemeinsam für Oxo stehen, und ihrer Salze, dadurch gekennzeichnet, dass man eine Verbindung der Formel Xa

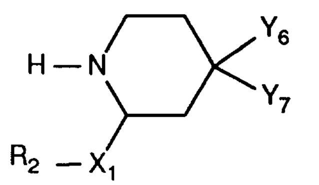

(Xa),

mit einem den Rest $R_1$ einführendem Mittel kondensiert und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

**102.** Verfahren gemäss Anspruch 101 zur Herstellung von Verbindungen der Formel X, worin $R_1$ unsubstituiertes oder durch $C_1$-$C_4$-Alkyl, $C_1$-$C_4$-Alkoxy, Halogen der Atomnummer bis und mit 35, und/oder Trifluormethyl mono- oder disubstituiertes Benzoyl oder unsubstituiertes Naphthoyl bedeutet, $R_2$ unsubstituiertes oder durch Halogen der Atomnummer bis und mit 35 und/oder Trifluormethyl mono- oder disubstituiertes Phenyl darstellt $X_1$ Hydroxymethylen darstellt, und $Y_6$ und $Y_7$ gemeinsam für Oxo stehen, und ihrer Salze.

**103.** Verfahren gemäss Anspruch 101, dadurch gekennzeichnet, dass man (2R*,1'R*)-1-(3,5-Dimethylbenzoyl)-2-(1'-hydroxy-1'-phenyl-methyl)-4-piperidon oder ein Salz davon herstellt.

**104.** Verfahren gemäss Anspruch 101, dadurch gekennzeichnet, dass man (2R*,1'R*)-1-(3,5-Bistrifluormethylbenzoyl)-2-{1'-hydroxy-1'-(4-chlorphenyl)methyl}-4-piperidon oder ein Salz davon herstellt.

**105.** Verfahren gemäss Anspruch 101, dadurch gekennzeichnet, dass man (2R*,1'R*)-1-(3,5-Dimethylbenzoyl)-2-{1'-hydroxy-1'-(3,4-dichlorphenyl)methyl}-4-piperidon oder ein Salz davon herstellt.

**106.** Verfahren gemäss Anspruch 101, dadurch gekennzeichnet, dass man (2R*,1'S*)-1-(3,5-Bistrifluormethylbenzoyl)-2-{1'-hydroxy-1'-(4-chlorphenyl)methyl}-4-piperidon oder ein Salz davon herstellt.

**107.** Verfahren gemäss Anspruch 101, dadurch gekennzeichnet, dass man (2R*,1'R*)-1-(3,5-Bistrifluormethylbenzoyl)-2-{1'-hydroxy-1'-(3,4-dichlorphenyl)methyl}-4-piperidon oder ein Salz davon herstellt.

**108.** Verfahren gemäss Anspruch 101, dadurch gekennzeichnet, dass man (2R*,1'R*)-1-(3,5-Dimethylbenzoyl)-2-{1'-hydroxy-1'-(4-methoxyphenyl)methyl}-4-piperidon oder ein Salz davon herstellt.

**109.** Verfahren gemäss Anspruch 101, dadurch gekennzeichnet, dass man (2R*,1'S*)-1-(3,5-Dimethylbenzoyl)-2-{1'-hydroxy-1'-(4-methoxyphenyl)methyl}-4-piperidon oder ein Salz davon herstellt.

**110.** Verfahren gemäss Anspruch 101, dadurch gekennzeichnet, dass man (2R*,1'R*)-1-(3,5-Dimethylbenzoyl)-2-{1'-hydroxy-1'-(3-methoxyphenyl)methyl}-4-piperidon oder ein Salz davon herstellt.

**111.** Verfahren gemäss Anspruch 101, dadurch gekennzeichnet, dass man (2R*,1'S*)-1-(3,5-Dimethylbenzoyl)-2-{1'-hydroxy-1'-(3-methoxyphenyl)methyl}-4-piperidon oder ein Salz davon herstellt.

**112.** Verfahren gemäss Anspruch 101, dadurch gekennzeichnet, dass man (2R*,1'R*)-1-(3,5-Dimethylbenzoyl)-2-{1'-hydroxy-1'-(4-trifluormethylphenyl)methyl}-4-piperidon oder ein Salz davon herstellt.

**113.** Verfahren gemäss Anspruch 101, dadurch gekennzeichnet, dass man (2R*,1'S*)-1-(3,5-Dimethylbenzoyl)-2-{1'-hydroxy-1'-(4-trifluormethylphenyl)methyl}-4-piperidon oder ein Salz davon herstellt.

**114.** Verfahren gemäss Anspruch 101, dadurch gekennzeichnet, dass man (2R*,1'R*)-1-(3,5-Dimethylbenzoyl)-2-{1'-hydroxy-1'-(4-chlor-3-trifluormethylphenyl)methyl}-4-piperidon oder ein Salz davon herstellt.

**115.** Verfahren gemäss Anspruch 101, dadurch gekennzeichnet, dass man (2R*,1'S*)-1-(3,5-Dimethylbenzoyl)-2-{1'-hydroxy-1'-(4-chlor-3-trifluormethylphenyl)-methyl}-4-piperidon oder ein Salz davon herstellt.

**116.** Verfahren zur Herstellung eines pharmazeutischen Präparates, dadurch gekennzeichnet, dass man eine 1-Acylpiperidinverbindung der Formel I oder X

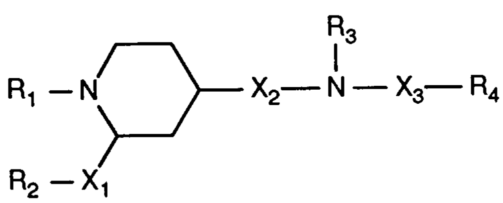 (I) oder 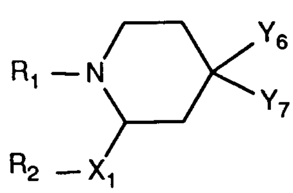 (X)

worin $R_1$ in Formel I einen gegebenenfalls substituierten Aralkyl-, Aryloxyalkyl-, Heteroaralkyl-, Aroyl-, Heteroaroyl-, Cycloalkylcarbonyl-, Aralkanoyl-, Heteroarylalkanoyl-, Aralkoxycarbonyl- oder Arylcarbamoylrest oder den Acylrest einer gegebenenfalls durch Niederalkanoyl oder Carbamoylniederalkanoyl N-substituierten α-Aminosäure bedeutet, $R_2$ Cycloalkyl oder einen gegebenenfalls substituierten Aryl- oder Heteroarylrest darstellt bzw. in Formel X einen gegebenenfalls substituierten Aroyl-, Heteroaroyl-, Cycloalkylcarbonyl-, Aralkanoyl-, Heteroarylalkanoyl-, Aralkoxycarbonyl- oder Arylcarbamoylrest oder den Acylrest einer gegebenenfalls durch Niederalkanoyl oder Carbamoylniederalkanoyl N-substituierten α-Aminosäure, $R_3$ für Wasserstoff, Alkyl, Carbamoyl oder einen gegebenenfalls durch Carboxy oder verestertes oder amidiertes Carboxy substituierten Alkanoyl- oder Alkenoylrest steht, $R_4$ einen gegebenenfalls substituierten Aryl- oder gegebenenfalls partiell hydrierten Heteroarylrest bedeutet, $X_1$ in Formel I Methylen, Ethylen, eine direkte Bindung, eine gegebenenfalls ketalisierte Carbonylgruppe oder eine gegebenenfalls veretherte Hydroxymethylengruppe bzw. in Formel X Hydroxymethylen darstellt, $X_2$ für Alkylen, Carbonyl oder eine direkte Bindung steht, $X_3$ Carbonyl, Oxoniederalkylen, Oxo(aza)-niederalkylen oder einen gegebenenfalls durch Phenyl, Hydroxymethyl, gegebenenfalls verestertes oder amidiertes Carboxy oder in höherer als der α-Stellung durch Hydroxy substituierten Alkylenrest darstellt und $Y_6$ und $Y_7$ gemeinsam für Oxo stehen, oder ein pharmazeutisch verwendbares Salz davon mit üblichen pharmazeutischen Hilfsstoffen vermischt.

## Claims

**Claims for the following Contracting States : AT, BE, CH, DE, DK, FR, GB, IE, IT LI, LU, NL, SE**

1. A 1-acylpiperidine compound of the formula I

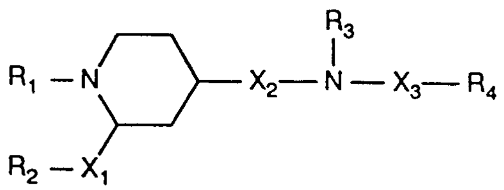

(I),

in which $R_1$ is phenyl- or diphenyl-$C_1$-$C_4$alkyl which is unsubstituted or substituted in the phenyl moiety by lower alkyl, lower alkoxy, di-lower-alkylamino, halogen and/or trifluoromethyl, phenoxy-$C_1$-$C_4$alkyl which is unsubstituted or substituted in the phenyl by halogen and/or triazolyl, pyridyl- or quinolinyl-$C_1$-$C_4$alkyl, benzoyl which is unsubstituted or substituted by lower alkyl, lower alkoxy, di-lower-alkylamino, halogen and/or trifluoromethyl, naphthoyl which is unsubstituted or substituted by lower alkyl, lower alkoxy, di-lower-alkylamino, halogen and/or trifluoromethyl, pyridylcarbonyl or quinolinylcarbonyl which is unsubstituted or substituted by lower alkyl, lower alkoxy, halogen and/or trifluoromethyl, 5- to 7-membered cycloalkylcarbonyl which is unsubstituted or substituted by lower alkyl, lower alkoxy, di-lower-alkylamino, halogen and/or trifluoromethyl, phenyl- or diphenyl-$C_1$-$C_4$alkanoyl which is unsubstituted or substituted in the phenyl by lower alkyl, lower alkoxy, di-lower-alkylamino, halogen and/or trifluoromethyl, N-phenylcarbamoyl which is unsubstituted or optionally substituted in the phenyl moiety by lower alkyl, lower alkoxy, di-lower-alkylamino, halogen and/or trifluoromethyl, or a group of the formula Ia

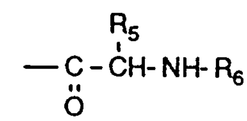

(Ia)

in which $R_5$ is hydrogen, $C_1$-$C_4$alkyl which is unsubstituted or substituted by hydroxyl, mercapto, amino, optionally hydroxy-substituted phenyl, carboxyl, carbamoyl or ureido, and $R_6$ is $C_2$-$C_7$alkanoyl, $R_2$ is 5- to 7-membered cycloalkyl or a phenyl, naphthyl or pyridyl radical which is unsubstituted or substituted by aromatically bonded $C_1$-$C_4$alkyl, $C_1$-$C_4$alkoxy, halogen and/or trifluoromethyl, $R_3$ is hydrogen, $C_1$-$C_7$alkyl, carbamoyl, $C_2$-$C_7$alkanoyl, carboxy-$C_1$-$C_4$alkanoyl or carboxy-$C_2$-$C_4$alkenoyl, $R_4$ is phenyl or naphthyl which is unsubstituted or substituted by $C_1$-$C_4$alkyl, $C_1$-$C_4$alkoxy, halogen and/or trifluoromethyl, or unsubstituted pyridyl, benzofuranyl, indolyl, 2,3-dihydroindolyl, benzimidazolyl, quinolyl or 1,2,3,4-tetrahydroquinolinyl, $X_1$ is methylene, hydroxymethylene, $C_1$-$C_4$alkoxymethylene, carbonyl, di-$C_1$-$C_4$alkoxymethylene or a direct linkage, $X_2$ is $C_1$-$C_7$alkylene, carbonyl or a direct linkage, and $X_3$ is carbonyl, $C_1$-$C_4$alkylene, carboxy-$C_1$-$C_4$alkylene, $C_1$-$C_4$alkoxycarbonyl-$C_1$-$C_4$alkylene, carbamoyl-$C_1$-$C_4$alkylene or hydroxymethyl-$C_1$-$C_4$alkylene, or a salt thereof; lower alkyl is $C_1$-$C_7$alkyl.

2. A compound according to claim 1 of the formula I in which $R_1$ is benzoyl, naphthoyl or phenyl-$C_1$-$C_4$alkanoyl which is unsubstituted or substituted by $C_1$-$C_4$alkyl, $C_1$-$C_4$alkoxy, halogen and/or trifluoromethyl, unsubstituted by pyridylcarbonyl or quinolinylcarbonyl or a group of the formula Ia

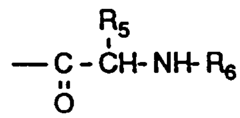

(Ia)

in which $R_5$ is hydrogen, $C_1$-$C_4$alkyl which is unsubstituted or substituted by hydroxyl, mercapto, amino, optionally hydroxy-substituted phenyl, carboxyl, carbamoyl or ureido, and $R_6$ is $C_2$-$C_7$alkanoyl,

$R_2$ is 5- to 7-membered cycloalkyl or a phenyl, naphthyl or pyridyl radical which is unsubstituted or substituted by aromatically bonded $C_1$-$C_4$alkyl, $C_1$-$C_4$alkoxy, halogen and/or trifluoromethyl, $R_3$ is hydrogen, $C_1$-$C_7$alkyl, carbamoyl, $C_2$-$C_7$alkanoyl, carboxy-$C_1$-$C_4$alkanoyl or carboxy-$C_3$-$C_5$alkenoyl, $R_4$ is phenyl or naphthyl which is unsubstituted or substituted by $C_1$-$C_4$alkyl, $C_1$-$C_4$alkoxy, halogen and/or trifluoromethyl, or unsubstituted pyridyl, benzofuranyl, indolyl, benzimidazolyl or quinolyl, $X_1$ is methylene, hydroxymethylene, $C_1$-$C_4$alkoxymethylene, carbonyl, di-$C_1$-$C_4$alkoxymethylene or a direct linkage, $X_2$ is $C_1$-$C_7$alkylene, carbonyl or a direct linkage, and $X_3$ is carbonyl, $C_1$-$C_4$alkylene, carboxy-$C_1$-$C_4$alkylene, $C_1$-$C_4$alkoxycarbonyl-$C_1$-$C_4$alkylene, carbamoyl-$C_1$-$C_4$alkylene or hydroxymethyl-$C_1$-$C_4$alkylene, or a salt thereof.

**3.** A compound according to claim 1 of the formula I in which $R_1$ is benzoyl which is unsubstituted or mono- or disubstituted by $C_1$-$C_4$alkyl, $C_1$-$C_4$alkoxy, halogen of atomic number up to and including 35 and/or trifluoromethyl, or unsubstituted naphthoyl or phenyl-$C_1$-$C_4$alkanoyl, $R_2$ is phenyl which is unsubstituted or mono- or disubstituted by $C_1$-$C_4$alkyl, $C_1$-$C_4$alkoxy, halogen of atomic number up to and including 35 and/or trifluoromethyl, or unsubstituted pyridyl, $R_3$ is hydrogen, $C_1$-$C_4$alkyl, carbamoyl or $C_2$-$C_7$alkanoyl, $R_4$ is phenyl which is unsubstituted or mono- or disubstituted by $C_1$-$C_4$alkyl, $C_1$-$C_4$alkoxy, halogen of atomic number up to and including 35 and/or trifluoromethyl, or unsubstituted naphthyl, pyridyl, benzofuranyl, indolyl, benzimidazolyl or quinolyl, $X_1$ is methylene, hydroxymethylene, carbonyl or a direct linkage, $X_2$ is a direct linkage, and $X_3$ is $C_1$-$C_4$alkylene, or a salt thereof.

**4.** A compound according to claim 1 of the formula I in which $R_1$ is benzoyl which is unsubstituted or mono- or disubstituted by $C_1$-$C_4$alkyl, $C_1$-$C_4$alkoxy, halogen of atomic number up to and including 35 and/or trifluoromethyl, or unsubstituted naphthoyl, $R_2$ is phenyl or phenyl mono- or disubstituted by halogen of atomic number up to and including 35 and/or trifluoromethyl, $R_3$ is hydrogen, $R_4$ is unsubstituted quinolinyl, $X_1$ is methylene, $X_2$ is a direct linkage and $X_3$ is $C_1$-$C_4$alkylene, or a salt thereof.

**5.** (2R*,4S*)-2-Benzyl-1-(2-naphthoyl)-N-(4-quinolylmethyl)-4-piperidinamine or a salt thereof.

**6.** (2R*,4S*)-2-Benzyl-1-(3-trifluoromethylbenzoyl)-N-(4-quinolyl-methyl)-4-piperidinamine or a salt thereof.

**7.** (2R*,4S*)-2-Benzyl-1-(3,5-bis(trifluoromethyl)benzoyl)-N-(4-quinolylmethyl)-4-piperidinamine or a salt thereof.

**8.** (2R*,4S*)-2-Benzyl-1-(3,5-dimethoxybenzoyl)-N-(4-quinolylmethyl)-4-piperidinamine or a salt thereof.

**9.** (2R*,4S*)-2-Benzyl-1-(1-naphthoyl)-N-(4-quinolylmethyl)-4-piperidinamine or a salt thereof.

**10.** (2R*,4S*)-2-Benzyl-1-(3,5-dichlorobenzoyl)-N-(4-quinolylmethyl)-4-piperidinamine or a salt thereof.

**11.** (2R*,4S*)-2-Benzyl-1-(2-quinolinylcarbonyl)-N-(4-quinolylmethyl)-4-piperidinamine or a salt thereof.

**12.** (2R*,4S*)-2-Benzyl-1-(4-chlorophenylacetyl)-N-(4-quinolylmethyl)-4-piperidinamine or a salt thereof.

**13.** (2R*,4S*)-2-Benzyl-1-(benzyloxycarbonyl)-N-(4-quinolylmethyl)-4-piperidinamine or a salt thereof.

**14.** (2R*,4S*)-2-Benzyl-1-(2-phenylethyl)-N-(4-quinolylmethyl)-4-piperidinamine or a salt thereof.

**15.** (2R*,4S*)-2-Benzyl-1-(2-naphthylacetyl)-N-(4-quionlylmethyl)-4-piperidinamine or a salt thereof.

**16.** (2R*,4S*)-2-Benzyl-1-(4-quinolylmethyl)-N-(4-quinolylmethyl)-4-piperidinamine or a salt thereof.

**17.** (2R*,4S*)-2-Benzyl-1-(2,4-dichlorobenzyl)-N-(4-quinolylmethyl)-4-piperidinamine or a salt thereof.

**18.** (2R*,4S*)-2-Benzyl-1-(2,2-diphenylethyl)-N-(4-quinolylmethyl)-4-piperidinamine or a salt thereof.

**19.** (2R*,4S*)-2-Benzyl-1-(phenylcarbamoyl)-N-(4-quinolylmethyl)-4-piperidinamine or a salt thereof.

**20.** (2R*,4S*)-2-Benzyl-1-(diphenylacetyl)-N-(4-quinolylmethyl)-4-piperidinamine or a salt thereof.

**21.** (2R*,4S*)-2-Benzyl-1-(2-pyridylacetyl)-N-(4-quinolylmethyl)-4-piperidinamine or a salt thereof.

**22.** (2R*,4S*)-2-Benzyl-1-(4-pyridylacetyl)-N-(4-quinolylmethyl)-4-piperidinamine or a salt thereof.

**23.** (2R*,4S*)-2-Benzyl-1-(2,3-diphenylpropionyl)-N-(4-quinolylmethyl)-4-piperidinamine or a salt thereof.

**24.** (2R*,4S*)-2-Benzyl-1-((3S)-(2,3,4,9-tetrahydro-1H-pyrido[3,4-b]indol-3-yl)-carbonyl)-N-(4-quinolylmethyl)-4-piperidinamine or a salt thereof.

**25.** (2R*,4S*)-2-Benzyl-1-(3-methoxybenzoyl)-N-(4-quinolylmethyl)-4-piperidinamine or a salt thereof.

**26.** (2R*,4S*)-2-Benzyl-1-(3-N,N-dimethylaminobenzoyl)-N-(4-quinolylmethyl)-4-piperidinamine or a salt thereof.

**27.** (2R*,4S*)-2-Benzyl-1-(cis,cis-3,5-dimethylcyclohexylcarbonyl)-N-(4-quinolylmethyl)-4-piperidinamine or a salt thereof.

**28.** (2R*,4S*)-2-Benzyl-1-(3,5-bis(trifluoromethyl)benzyl)-N-(4-quinolylmethyl)-4-piperidinamine or a salt thereof.

**29.** (2S*,4R*)-2-Benzyl-1-[2-(5-chloro-1H-1,2,4-triazol-1-yl)phenoxyethyl]-N-(4-quinolylmethyl)-4-piperidinamine or a salt thereof.

**30.** (2R*,4S*)-2-Benzyl-1-((S)-phenylalanyl)-N-(4-quinolylmethyl)-4-piperidinamine or a salt thereof.

**31.** (2R*,4S*)-2-Benzyl-1-((R)-phenylalanyl)-N-(4-quinolylmethyl)-4-piperidinamine or a salt thereof.

**32.** (2R*,4S*)-2-Benzyl-1-((S)-N-acetyl-phenylalanyl)-N-(4-quinolylmethyl)-4-piperidinamine or a salt thereof.

**33.** (2R*,4S*)-2-Benzyl-1-((R)-N-acetyl-phenylalanyl)-N-(4-quinolylmethyl)-4-piperidinamine or a salt thereof.

**34.** (2R*,4S*)-2-Benzyl-1-((S)-N-(4-carboxamidobutyroyl)phenylalanyl)-N-(4-quinolylmethyl)-4-piperidinamine or a salt thereof.

**35.** (2R*,4S*)-2-Benzyl-1-((R)-N-(4-carboxamidobutyroyl)phenylalanyl)-N-(4-quinolylmethyl)-4-piperidinamine or a salt thereof.

**36.** (2R*,4S*)-2-Benzyl-1-benzoyl-N-(4-quinolylmethyl)-4-piperidinamine or a salt thereof.

**37.** (2R*,4S*)-2-Benzyl-1-(3-chlorobenzoyl)-N-(4-quinolylmethyl)-4-piperidinamine or a salt thereof.

**38.** (2R*,4S*)-2-Benzyl-1-(3,5-dichlorobenzoyl)-N-(4-quinolylmethyl)-N-(3-carboxamidopropionyl)-4-piperidinamine or a salt thereof.

**39.** (2R,4S)- or (2R,4R)-2-Benzyl-1-(3,5-dimethylbenzoyl)-N-(4-quinolylmethyl)-4-piperidinamine or a salt thereof.

**40.** (2S,4R)- or (2S,4S)-2-Benzyl-1-(3,5-dimethylbenzoyl)-N-(2-phenethyl)-4-piperidinamine or a salt thereof.

**41.** (2R,4S)-2-Benzyl-1-(3,5-dimethylbenzoyl)-N-(3-quinolylmethyl)-4-piperidinamine or a salt thereof.

**42.** (2R,4S)-2-Benzyl-1-(3,5-dimethylbenzoyl)-N-(2-quinolylmethyl)-4-piperidinamine or a salt thereof.

**43.** (2R,4S)- or (2R,4R)-2-Benzyl-1-(3,5-dimethylbenzoyl)-N-benzyl-4-piperidinamine or a salt thereof.

**44.** (2S,4R)- or (2S,4S)-2-Benzyl-1-(3,5-dimethylbenzoyl)-N-benzyl-4-piperidinamine or a salt thereof.

**45.** (2R,4S)- or (2R,4R)-2-Benzyl-1-(3,5-dimethylbenzoyl)-N-(4-pyridylmethyl)-4-piperidinamine or a salt thereof.

**46.** (2R,4S)- or (2R,4R)-2-Benzyl-1-(3,5-dimethylbenzoyl)-N-(3-pyridylmethyl)-4-piperidinamine or a salt thereof.

**47.** (2S,4R)- or (2S,4S)-2-Benzyl-1-(3,5-dimethylbenzoyl)-N-(4-quinolylmethyl)-4-piperidinamine or a salt thereof.

**48.** (2R*,4S*)-2-Benzyl-1-(3,5-dimethylbenzoyl)-N-benzyl-N-carbamoyl-4-piperidinamine or a salt thereof.

**49.** (2R,4S)-2-Benzyl-1-(3,5-dimethylbenzoyl)-N-(3-phenylpropyl)-4-piperidinamine;
(2S,4S)-2-benzyl-1-(3,5-dimethylbenzoyl)-N-(3-phenylpropyl)-4-piperidinamine;
(2R,4S)-2-benzyl-1-(3,5-dimethylbenzoyl)-N-(2-methoxybenzyl)-4-piperidinamine;
(2S,4S)-2-benzyl-1-(3,5-dimethylbenzoyl)-N-(2-methoxybenzyl)-4-piperidinamine;
(2R,4S)-2-benzyl-1-(3,5-dimethylbenzoyl)-N-[2-(2-methoxyphenyl)ethyl]-4-piperidinamine;
(2S,4S)-2-benzyl-1-(3,5-dimethylbenzoyl)-N-[2-(2-methoxyphenyl)ethyl]-4-piperidinamine;
(2R,4S)-2-benzyl-1-(3,5-dimethylbenzoyl)-N-[3-(2-methoxyphenyl)propyl]-4-piperidinamine;
(2S,4S)-2-benzyl-1-(3,5-dimethylbenzoyl)-N-[3-(2-methoxyphenyl)propyl]-4-piperidinamine;
(2R,4S)-2-benzyl-1-(3,5-dimethylbenzoyl)-N-(2-trifluoromethylbenzyl)-4-piperidinamine;
(2S,4S)-2-benzyl-1-(3,5-dimethylbenzoyl)-N-(2-trifluoromethylbenzyl)-4-piperidinamine;
(2R,4S)-2-benzyl-1-(3,5-dimethylbenzoyl)-N-[2-(2-trifluoromethylphenyl)ethyl]-4-piperidinamine;
(2S,4S)-2-benzyl-1-(3,5-dimethylbenzoyl)-N-[2-(2-trifluoromethylphenyl)ethyl]-4-piperidinamine;
(2R,4S)-2-benzyl-1-(3,5-dimethylbenzoyl)-N-[3-(2-trifluoromethylphenyl)propyl]-4-piperidinamine;
(2S,4S)-2-benzyl-1-(3,5-dimethylbenzoyl)-N-[3-(2-trifluoromethylphenyl)propyl]-4-piperidinamine;
(2R,4S)-2-benzyl-1-(3,5-dimethylbenzoyl)-N-(3-trifluoromethylbenzyl)-4-piperidinamine;
(2S,4S)-2-benzyl-1-(3,5-dimethylbenzoyl)-N-(3-trifluoromethylbenzyl)-4-piperidinamine;
(2R,4S)-2-benzyl-1-(3,5-dimethylbenzoyl)-N-[2-(3-trifluoromethylphenyl)ethyl]-4-piperidinamine;
(2S,4S)-2-benzyl-1-(3,5-dimethylbenzoyl)-N-[2-(3-trifluoromethylphenyl)ethyl]-4-piperidinamine;
(2R,4S)-2-benzyl-1-(3,5-dimethylbenzoyl)-N-[3-(3-trifluoromethylphenyl)propyl]-4-piperidinamine;
(2S,4S)-2-benzyl-1-(3,5-dimethylbenzoyl)-N-[3-(3-trifluoromethylphenyl)propyl]-4-piperidinamine;
(2R,4S)-2-benzyl-1-(3,5-dimethylbenzoyl)-N-(4-trifluoromethylbenzyl)-4-piperidinamine;
(2S,4S)-2-benzyl-1-(3,5-dimethylbenzoyl)-N-(4-trifluoromethylbenzyl)-4-piperidinamine;
(2R,4S)-2-benzyl-1-(3,5-dimethylbenzoyl)-N-[2-(4-trifluoromethylphenyl)ethyl]-4-piperidinamine;
(2S,4S)-2-benzyl-1-(3,5-dimethylbenzoyl)-N-[2-(4-trifluoromethylphenyl)ethyl]-4-piperidinamine;
(2R,4S)-2-benzyl-1-(3,5-dimethylbenzoyl)-N-[3-(4-trifluoromethylphenyl)propyl]-4-piperidinamine;
(2S,4S)-2-benzyl-1-(3,5-dimethylbenzoyl)-N-[3-(4-trifluoromethylphenyl)propyl]-4-piperidinamine;
(2R,4S)-2-benzyl-1-(3,5-dimethylbenzoyl)-N-(2,3-dimethoxybenzyl)-4-piperidinamine;
(2S,4S)-2-benzyl-1-(3,5-dimethylbenzoyl)-N-(2,3-dimethoxybenzyl)-4-piperidinamine;
(2R,4S)-2-benzyl-1-(3,5-dimethylbenzoyl)-N-[2-(2,3-dimethoxyphenyl)ethyl]-4-piperidinamine;
(2S,4S)-2-benzyl-1-(3,5-dimethylbenzoyl)-N-[2-(2,3-dimethoxyphenyl)ethyl]-4-piperidinamine;
(2R,4S)-2-benzyl-1-(3,5-dimethylbenzoyl)-N-[3-(2,3-dimethoxyphenyl)propyl]-4-piperidinamine;
(2S,4S)-2-benzyl-1-(3,5-dimethylbenzoyl)-N-[3-(2,3-dimethoxyphenyl)propyl]-4-piperidinamine;
(2R,4S)-2-benzyl-1-(3,5-dimethylbenzoyl)-N-(2,4-dimethoxybenzyl)-4-piperidinamine;
(2S,4S)-2-benzyl-1-(3,5-dimethylbenzoyl)-N-(2,4-dimethoxybenzyl)-4-piperidinamine;
(2R,4S)-2-benzyl-1-(3,5-dimethylbenzoyl)-N-[2-(2,4-dimethoxyphenyl)ethyl]-4-piperidinamine;
(2S,4S)-2-benzyl-1-(3,5-dimethylbenzoyl)-N-[2-(2,4-dimethoxyphenyl)ethyl]-4-piperidinamine;
(2R,4S)-2-benzyl-1-(3,5-dimethylbenzoyl)-N-[3-(2,4-dimethoxyphenyl)propyl]-4-piperidinamine;
(2S,4S)-2-benzyl-1-(3,5-dimethylbenzoyl)-N-[3-(2,4-dimethoxyphenyl)propyl]-4-piperidinamine;
(2R,4S)-2-benzyl-1-(3,5-dimethylbenzoyl)-N-(2,5-dimethoxybenzyl)-4-piperidinamine;
(2S,4S)-2-benzyl-1-(3,5-dimethylbenzoyl)-N-(2,5-dimethoxybenzyl)-4-piperidinamine;
(2R,4S)-2-benzyl-1-(3,5-dimethylbenzoyl)-N-[2-(2,5-dimethoxyphenyl)ethyl]-4-piperidinamine;
(2S,4S)-2-benzyl-1-(3,5-dimethylbenzoyl)-N-[2-(2,5-dimethoxyphenyl)ethyl]-4-piperidinamine;
(2R,4S)-2-benzyl-1-(3,5-dimethylbenzoyl)-N-[3-(2,5-dimethoxyphenyl)propyl]-4-piperidinamine;
(2S,4S)-2-benzyl-1-(3,5-dimethylbenzoyl)-N-[3-(2,5-dimethoxyphenyl)propyl]-4-piperidinamine;
(2R,4S)-2-benzyl-1-(3,5-dimethylbenzoyl)-N-(2,6-dimethoxybenzyl)-4-piperidinamine;
(2S,4S)-2-benzyl-1-(3,5-dimethylbenzoyl)-N-(2,6-dimethoxybenzyl)-4-piperidinamine;
(2R,4S)-2-benzyl-1-(3,5-dimethylbenzoyl)-N-[2-(2,6-dimethoxyphenyl)ethyl]-4-piperidinamine;

(2S,4S)-2-benzyl-1-(3,5-dimethylbenzoyl)-N-[2-(2,6-dimethoxyphenyl)ethyl]-4-piperidinamine;
(2R,4S)-2-benzyl-1-(3,5-dimethylbenzoyl)-N-[3-(2,6-dimethoxyphenyl)propyl]-4-piperidinamine;
(2S,4S)-2-benzyl-1-(3,5-dimethylbenzoyl)-N-[3-(2,6-dimethoxyphenyl)propyl]-4-piperidinamine;
(2R,4S)-2-benzyl-1-(3,5-dimethylbenzoyl)-N-(2,3-methylenedioxybenzyl)-4-piperidinamine;
(2S,4S)-2-benzyl-1-(3,5-dimethylbenzoyl)-N-(2,3-methylenedioxybenzyl)-4-piperidinamine;
(2R,4S)-2-benzyl-1-(3,5-dimethylbenzoyl)-N-[2-(2,3-methylenedioxyphenyl)ethyl]-4-piperidinamine;
(2S,4S)-2-benzyl-1-(3,5-dimethylbenzoy-)-N-[2-(2,3-methylenedioxyphenyl)ethyl]-4-piperidinamine;
(2R,4S)-2-benzyl-1-(3,5-dimethylbenzoyl)-N-[3-(2,3-methylenedioxyphenyl)propyl]-4-piperidinamine;
(2S,4S)-2-benzyl-1-(3,5-dimethylbenzoyl)-N-[3-(2,3-methylenedioxyphenyl)propyl]-4-piperidinamine;
(2R,4S)-2-benzyl-1-(3,5-dimethylbenzoyl)-N-(2,4-methylenedioxybenzyl)-4-piperidinamine;
(2S,4S)-2-benzyl-1-(3,5-dimethylbenzoyl)-N-(2,4-methylenedioxybenzyl)-4-piperidinamine;
(2R,4S)-2-benzyl-1-(3,5-dimethylbenzoyl)-N-[2-(2,4-methylenedioxyphenyl)ethyl]-4-piperidinamine;
(2S,4S)-2-benzyl-1-(3,5-dimethylbenzoyl)-N-[2-(2,4-methylenedioxyphenyl)ethyl]-4-piperidinamine;
(2R,4S)-2-benzyl-1-(3,5-dimethylbenzoyl)-N-[3-(2,4-methylenedioxyphenyl)propyl]-4-piperidinamine;
(2S,4S)-2-benzyl-1-(3,5-dimethylbenzoyl)-N-[3-(2,4-methylenedioxyphenyl)propyl]-4-piperidinamine;
(2R,4S)-2-benzyl-1-(3,5-dimethylbenzoyl)-N-(indol-2-ylmethyl)-4-piperidinamine;
(2S,4S)-2-benzyl-1-(3,5-dimethylbenzoyl)-N-(indol-2-ylmethyl)-4 piperidinamine;
(2R,4S)-2-benzyl-1-(3,5-dimethylbenzoyl)-N-(indol-3-ylmethyl)-4-piperidinamine;
(2S,4S)-2-benzyl-1-(3,5-dimethylbenzoyl)-N-(indol-3-ylmethyl)-4-piperidinamine;
(2S,4S)-2-benzyl-1-(3,5-dimethylbenzoyl)-N-(quinolin-2-ylmethyl)-4-piperidinamine;
(2S,4S)-2-benzyl-1-(3,5-dimethylbenzoyl)-N-(quinolin-3-ylmethyl)-4-piperidinamine;
(2R,4S)-2-benzyl-1-(3,5-dimethylbenzoyl)-N-(2-chlorobenzyl)-4-piperidinamine;
(2S,4S)-2-benzyl-1-(3,5-dimethylbenzoyl)-N-(2-chlorobenzyl)-4-piperidinamine;
(2R,4S)-2-benzyl-1-(3,5-dimethylbenzoyl)-N-[2-(2-chlorophenyl)ethyl]-4-piperidinamine;
(2S,4S)-2-benzyl-1-(3,5-dimethylbenzoyl)-N-[2-(2-chlorophenyl)ethyl]-4-piperidinamine;
(2R,4S)-2-benzyl-1-(3,5-dimethylbenzoyl)-N-[3-(2-chlorophenyl)propyl]-4-piperidinamine;
(2S,4S)-2-benzyl-1-(3,5-dimethylbenzoyl)-N-[3-(2-chlorophenyl)propyl]-4-piperidinamine;
(2R,4S)-2-benzyl-1-(3,5-dimethylbenzoyl)-N-(3-chlorobenzyl)-4-piperidinamine;
(2S,4S)-2-benzyl-1-(3,5-dimethylbenzoyl)-N-(3-chlorobenzyl)-4-piperidinamine;
(2R,4S)-2-benzyl-1-(3,5-dimethylbenzoyl)-N-[2-(3-chlorophenyl)ethyl]-4-piperidinamine;
(2S,4S)-2-benzyl-1-(3,5-dimethylbenzoyl)-N-[2-(3-chlorophenyl)ethyl]-4-piperidinamine;
(2R,4S)-2-benzyl-1-(3,5-dimethylbenzoyl)-N-[3-(3-chlorophenyl)propyl]-4-piperidinamine;
(2S,4S)-2-benzyl-1-(3,5-dimethylbenzoyl)-N-[3-(3-chlorophenyl)propyl]-4-piperidinamine;
(2R,4S)-2-benzyl-1-(3,5-dimethylbenzoyl)-N-(4-chlorobenzyl)-4-piperidinamine;
(2S,4S)-2-benzyl-1-(3,5-dimethylbenzoyl)-N-(4-chlorobenzyl)-4-piperidinamine;
(2R,4S)-2-benzyl-1-(3,5-dimethylbenzoyl)-N-[2-(4-chlorophenyl)ethyl]-4-piperidinamine;
(2S,4S)-2-benzyl-1-(3,5-dimethylbenzoyl)-N-[2-(4-chlorophenyl)ethyl]-4-piperidinamine;
(2R,4S)-2-benzyl-1-(3,5-dimethylbenzoyl)-N-[3-(4-chlorophenyl)propyl]-4-piperidinamine;
(2S,4S)-2-benzyl-1-(3,5-dimethylbenzoyl)-N-[3-(4-chlorophenyl)propyl]-4-piperidinamine;
(2R,4S)-2-benzyl-1-(3,5-dimethylbenzoyl)-N-(4-methoxynaphth-1-ylmethyl)-4-piperidinamine;
(2S,4S)-2-benzyl-1-(3,5-dimethylbenzoyl)-N-(4-methoxynaphth-1-ylmethyl)-4-piperidinamine;
(2R,4S)-2-benzyl-1-(3,5-dimethylbenzoyl)-N-(3,4-ethylenedioxybenzyl)-4-piperidinamine;
(2S,4S)-2-benzyl-1-(3,5-dimethylbenzoyl)-N-(3,4-ethylenedioxybenzyl)-4-piperidinamine;
(2R,4S)-2-benzyl-1-(3,5-dimethylbenzoyl)-N-(quinolin-2-ylmethyl)-4-piperidinamine;
(2R,4S)-2-benzyl-1-(3,5-dimethylbenzoyl)-N-(quinolin-3-ylmethyl)-4-piperidinamine;
(2R,4S)-2-benzyl-1-(3,5-ditrifluoromethylbenzoyl)-N-(quinolin-2-ylmethyl)-4-piperidinamine;
(2S,4S)-2-benzyl-1-(3,5-ditrifluoromethylbenzoyl)-N-(quinolin-2-ylmethyl)-4-piperidinamine;
(2R,4S)-2-benzyl-1-(3,5-ditrifluoromethylbenzoyl)-N-(quinolin-3-ylmethyl)-4-piperidinamine;
(2S,4S)-2-benzyl-1-(3,5-ditrifluoromethylbenzoyl)-N-(quinolin-3-ylmethyl)-4-piperidinamine;
(2R,4S)-2-benzyl-1-(3,5-ditrifluoromethylbenzoyl)-N-(2-chlorobenzyl)-4-piperidinamine;
(2S,4S)-2-benzyl-1-(3,5-ditrifluoromethylbenzoyl)-N-(2-chlorobenzyl)-4-piperidinamine;
(2R,4S)-2-benzyl-1-(3,5-ditrifluoromethylbenzoyl)-N-[2-(2-chlorophenyl)ethyl]-4-piperidinamine;
(2S,4S)-2-benzyl-1-(3,5-ditrifluoromethylbenzoyl)-N-[2-(2-chlorophenyl)ethyl]-4-piperidinamine;
(2R,4S)-2-benzyl-1-(3,5-ditrifluoromethylbenzoyl)-N-[3-(2-chlorophenyl)propyl]-4-piperidinamine;
(2S,4S)-2-benzyl-1-(3,5-ditrifluoromethylbenzoyl)-N-[3-(2-chlorophenyl)propyl]-4-piperidinamine;
(2R,4S)-2-benzyl-1-(3,5-ditrifluoromethylbenzoyl)-N-(3-chlorobenzyl)-4-piperidinamine;
(2S,4S)-2-benzyl-1-(3,5-ditrifluoromethylbenzoyl)-N-(3-chlorobenzyl)-4-piperidinamine;
(2R,4S)-2-benzyl-1-(3,5-ditrifluoromethylbenzoyl)-N-[2-(3-chlorophenyl)ethyl]-4-piperidinamine;

(2S,4S)-2-benzyl-1-(3,5-ditrifluoromethylbenzoyl)-N-[2-(3-chlorophenyl)ethyl]-4-piperidinamine;
(2R,4S)-2-benzyl-1-(3,5-ditrifluoromethylbenzoyl)-N-[3-(3-chlorophenyl)propyl]-4-piperidinamine;
(2S,4S)-2-benzyl-1-(3,5-ditrifluoromethylbenzoyl)-N-[3-(3-chlorophenyl)propyl]-4-piperidinamine;
(2R,4S)-2-benzyl-1-(3,5-ditrifluoromethylbenzoyl)-N-(4-chlorobenzyl)-4-piperidinamine;
(2S,4S)-2-benzyl-1-(3,5-ditrifluoromethylbenzoyl)-N-(4-chlorobenzyl)-4-piperidinamine;
(2R,4S)-2-benzyl-1-(3,5-ditrifluoromethylbenzoyl)-N-[2-(4-chlorophenyl)ethyl]-4-piperidinamine;
(2S,4S)-2-benzyl-1-(3,5-ditrifluoromethylbenzoyl)-N-[2-(4-chlorophenyl)ethyl]-4-piperidinamine;
(2R,4S)-2-benzyl-1-(3,5-ditrifluoromethylbenzoyl)-N-[3-(4-chlorophenyl)propyl]-4-piperidinamine;
(2S,4S)-2-benzyl-1-(3,5-ditrifluoromethylbenzoyl)-N-[3-(4-chlorophenyl)propyl]-4-piperidinamine;
(2R,4S)-2-benzyl-1-(3,5-ditrifluoromethylbenzoyl)-N-(4-methoxynaphth-1-ylmethyl)-4-piperidinamine;
(2S,4S)-2-benzyl-1-(3,5-ditrifluoromethylbenzoyl)-N-(4-methoxynaphth-1-ylmethyl)-4-piperidinamine;
(2R,4S)-2-benzyl-1-(3,5-ditrifluoromethylbenzoyl)-N-(3,4-ethylenedioxybenzyl)-4-piperidinamine;
(2S,4S)-2-benzyl-1-(3,5-ditrifluoromethylbenzoyl)-N-(3,4-ethylenedioxybenzyl)-4-piperidinamine;
(2R,4S)-2-benzyl-1-(3,5-dichlorobenzoyl)-N-(indol-2-ylmethyl)-4-piperidinamine;
(2S,4S)-2-benzyl-1-(3,5-dichlorobenzoyl)-N-(indol-2-ylmethyl)-4-piperidinamine;
(2R,4S)-2-benzyl-1-(3,5-dichlorobenzoyl)-N-(indol-3-ylmethyl)-4-piperidinamine;
(2S,4S)-2-benzyl-1-(3,5-dichlorobenzoyl)-N-(indol-3-ylmethyl)-4-piperidinamine;
(2R,4S)-2-benzyl-1-(3,5-dichlorobenzoyl)-N-(quinolin-2-ylmethyl)-4-piperidinamine;
(2S,4S)-2-benzyl-1-(3,5-dichlorobenzoyl)-N-(quinolin-2-ylmethyl)-4-piperidinamine;
(2R,4S)-2-benzyl-1-(3,5-dichlorobenzoyl)-N-(quinolin-3-ylmethyl)-4-piperidinamine;
(2S,4S)-2-benzyl-1-(3,5-dichlorobenzoyl)-N-(quinolin-3-ylmethyl)-4-piperidinamine;
(2R,4S)-2-benzyl-1-(3,5-dichlorobenzoyl)-N-(2-chlorobenzyl)-4-piperidinamine;
(2S,4S)-2-benzyl-1-(3,5-dichlorobenzoyl)-N-(2-chlorobenzyl)-4-piperidinamine;
(2R,4S)-2-benzyl-1-(3,5-dichlorobenzoyl)-N-[2-(2-chlorophenyl)ethyl]-4-piperidinamine;
(2S,4S)-2-benzyl-1-(3,5-dichlorobenzoyl)-N-[2-(2-chlorophenyl)ethyl]-4-piperidinamine;
(2R,4S)-2-benzyl-1-(3,5-dichlorobenzoyl)-N-[3-(2-chlorophenyl)propyl]-4-piperidinamine;
(2S,4S)-2-benzyl-1-(3,5-dichlorobenzoyl)-N-[3-(2-chlorophenyl)propyl]-4-piperidinamine;
(2R,4S)-2-benzyl-1-(3,5-dichlorobenzoyl)-N-(3-chlorobenzyl)-4-piperidinamine;
(2S,4S)-2-benzyl-1-(3,5-dichlorobenzoyl)-N-(3-chlorobenzyl)-4-piperidinamine;
(2R,4S)-2-benzyl-1-(3,5-dichlorobenzoyl)-N-[2-(3-chlorophenyl)ethyl]-4-piperidinamine;
(2S,4S)-2-benzyl-1-(3,5-dichlorobenzoyl)-N-[2-(3-chlorophenyl)ethyl]-4-piperidinamine;
(2R,4S)-2-benzyl-1-(3,5-dichlorobenzoyl)-N-[3-(3-chlorophenyl)propyl]-4-piperidinamine;
(2S,4S)-2-benzyl-1-(3,5-dichlorobenzoyl)-N-[3-(3-chlorophenyl)propyl]-4-piperidinamine;
(2R,4S)-2-benzyl-1-(3,5-dichlorobenzoyl)-N-(4-chlorobenzyl)-4-piperidinamine;
(2S,4S)-2-benzyl-1-(3,5-dichlorobenzoyl)-N-(4-chlorobenzyl)-4-piperidinamine;
(2R,4S)-2-benzyl-1-(3,5-dichlorobenzoyl)-N-[2-(4-chlorophenyl)ethyl]-4-piperidinamine;
(2S,4S)-2-benzyl-1-(3,5-dichlorobenzoyl)-N-[2-(4-chlorophenyl)ethyl]-4-piperidinamine;
(2R,4S)-2-benzyl-1-(3,5-dichlorobenzoyl)-N-[3-(4-chlorophenyl)propyl]-4-piperidinamine;
(2S,4S)-2-benzyl-1-(3,5-dichlorobenzoyl)-N-[3-(4-chlorophenyl)propyl]-4-piperidinamine;
(2R,4S)-2-benzyl-1-(3,5-dichlorobenzoyl)-N-(4-methoxynaphth-1-ylmethyl)-4-piperidinamine;
(2S,4S)-2-benzyl-1-(3,5-dichlorobenzoyl)-N-(4-methoxynaphth-1-ylmethyl)-4-piperidinamine;
(2R,4S)-2-benzyl-1-(3,5-dichlorobenzoyl)-N-(3,4-ethylenedioxybenzyl)-4-piperidinamine;
(2S,4S)-2-benzyl-1-(3,5-dichlorobenzoyl)-N-(3,4-ethylenedioxybenzyl)-4-piperidinamine;
(2R,4S)-2-benzyl-1-(3,5-dimethoxybenzoyl)-N-(indol-2-ylmethyl)-4-piperidinamine;
(2S,4S)-2-benzyl-1-(3,5-dimethoxybenzoyl)-N-(indol-2-ylmethyl)-4-piperidinamine;
(2R,4S)-2-benzyl-1-(3,5-dimethoxybenzoyl)-N-(indol-3-ylmethyl)-4-piperidinamine;
(2S,4S)-2-benzyl-1-(3,5-dimethoxybenzoyl)-N-(indol-3-ylmethyl)-4-piperidinamine;
(2R,4S)-2-benzyl-1-(3,5-dimethoxybenzoyl)-N-(quinolin-2-ylmethyl)-4-piperidinamine;
(2S,4S)-2-benzyl-1-(3,5-dimethoxybenzoyl)-N-(quinolin-2-ylmethyl)-4-piperidinamine;
(2R,4S)-2-benzyl-1-(3,5-dimethoxybenzoyl)-N-(quinolin-3-ylmethyl)-4-piperidinamine;
(2S,4S)-2-benzyl-1-(3,5-dimethoxybenzoyl)-N-(quinolin-3-ylmethyl)-4-piperidinamine;
(2R,4S)-2-benzyl-1-(3,5-dimethoxybenzoyl)-N-(2-chlorobenzyl)-4-piperidinamine;
(2S,4S)-2-benzyl-1-(3,5-dimethoxybenzoyl)-N-(2-chlorobenzyl)-4-piperidinamine;
(2R,4S)-2-benzyl-1-(3,5-dimethoxybenzoyl)-N-[2-(2-chlorophenyl)ethyl]-4-piperidinamine;
(2S,4S)-2-benzyl-1-(3,5-dimethoxybenzoyl)-N-[2-(2-chlorophenyl)ethyl]-4-piperidinamine;
(2R,4S)-2-benzyl-1-(3,5-dimethoxybenzoyl)-N-[3-(2-chlorophenyl)propyl]-4-piperidinamine;
(2S,4S)-2-benzyl-1-(3,5-dimethoxybenzoyl)-N-[3-(2-chlorophenyl)propyl]-4-piperidinamine;
(2R,4S)-2-benzyl-1-(3,5-dimethoxybenzoyl)-N-(3-chlorobenzyl)-4-piperidinamine;

(2S,4S)-2-benzyl-1-(3,5-dimethoxybenzoyl)-N-(3-chlorobenzyl)-4-piperidinamine;
(2R,4S)-2-benzyl-1-(3,5-dimethoxybenzoyl)-N-[2-(3-chlorophenyl)ethyl]-4-piperidinamine;
(2S,4S)-2-benzyl-1-(3,5-dimethoxybenzoyl)-N-[2-(3-chlorophenyl)ethyl]-4-piperidinamine;
(2R,4S)-2-benzyl-1-(3,5-dimethoxybenzoyl)-N-[3-(3-chlorophenyl)propyl]-4-piperidinamine;
(2S,4S)-2-benzyl-1-(3,5-dimethoxybenzoyl)-N-[3-(3-chlorophenyl)propyl]-4-piperidinamine;
(2R,4S)-2-benzyl-1-(3,5-dimethoxybenzoyl)-N-(4-chlorobenzyl)-4-piperidinamine;
(2S,4S)-2-benzyl-1-(3,5-dimethoxybenzoyl)-N-(4-chlorobenzyl)-4-piperidinamine;
(2R,4S)-2-benzyl-1-(3,5-dimethoxybenzoyl)-N-[2-(4-chlorophenyl)ethyl]-4-piperidinamine;
(2S,4S)-2-benzyl-1-(3,5-dimethoxybenzoyl)-N-[2-(4-chlorophenyl)ethyl]-4-piperidinamine;
(2R,4S)-2-benzyl-1-(3,5-dimethoxybenzoyl)-N-[3-(4-chlorophenyl)propyl]-4-piperidinamine;
(2S,4S)-2-benzyl-1-(3,5-dimethoxybenzoyl)-N-[3-(4-chlorophenyl)propyl]-4-piperidinamine;
(2R,4S)-2-benzyl-1-(3,5-dimethoxybenzoyl)-N-(4-methoxynaphth-1-ylmethyl)-4-piperidinamine;
(2S,4S)-2-benzyl-1-(3,5-dimethoxybenzoyl)-N-(4-methoxynaphth-1-ylmethyl)-4-piperidinamine;
(2R,4S)-2-benzyl-1-(3,5-dimethoxybenzoyl)-N-(3,4-ethylenedioxybenzyl)-4-piperidinamine or
(2S,4S)-2-benzyl-1-(3,5-dimethoxybenzoyl)-N-(3,4-ethylenedioxybenzyl)-4-piperidinamine or a salt thereof in each case.

**50.** (2R,4S)- or (2R,4R)-2-Benzyl-1-(3,5-dimethylbenzoyl)-N-(2-phenethyl)-4-piperidinamine or a salt thereof.

**51.** (2R,4S)-2-Benzyl-1-(3,5-bis(trifluoromethyl)benzoyl)-N-(4-quinolylmethyl)-4-piperidinamine or a salt thereof.

**52.** (2R,4S)-2-Benzyl-1-(3,5-dichlorobenzoyl)-N-(4-quinolylmethyl)-4-piperidinamine or a salt thereof.

**53.** (2R*,4S*)-2-Benzyl-1-(2,4-dichlorobenzoyl)-N-(4-quinolylmethyl)-4-piperidinamine or a salt thereof.

**54.** (2R*,4S*)-2-Benzyl-1-(phenylacetyl)-N-(4-quinolylmethyl)-4-piperidinamine or a salt thereof.

**55.** (2R*,4S*)-2-Benzyl-1-(2,6-dichlorobenzoyl)-N-(4-quinolylmethyl)-4-piperidinamine or a salt thereof.

**56.** (2R*,4S*)-2-Benzyl-1-(3,5-dibromobenzoyl)-N-(4-quinolylmethyl)-4-piperidinamine or a salt thereof.

**57.** (2R*,4S*)-2-Benzyl-1-(9-fluorenoyl)-N-(4-quinolylmethyl)-4-piperidinamine or a salt thereof.

**58.** (2R*,4S*)-2-Benzyl-1-(3-toluoyl)-N-(4-quinolylmethyl)-4-piperidinamine or a salt thereof.

**59.** (2R*,4S*)-2-Benzyl-1-(3-bromobenzoyl)-N-(4-quinolylmethyl)-4-piperidinamine or a salt thereof.

**60.** (2R*,4S*)-2-Benzyl-1-(3,5-dihydroxybenzoyl)-N-(4-quinolylmethyl)-4-piperidinamine or a salt thereof.

**61.** (2R*,4S*)-2-Benzyl-1-(3-cyanobenzoyl)-N-(4-quinolylmethyl)-4-piperidinamine or a salt thereof.

**62.** (2R*,4S*)-2-Benzyl-1-(2-chlorobenzoyl)-N-(4-quinolylmethyl)-4-piperidinamine or a salt thereof.

**63.** (2R*,4S*)-2-Benzyl-1-(4-chlorobenzoyl)-N-(4-quinolylmethyl)-4-piperidinamine or a salt thereof.

**64.** (2R*,4S*)-2-Benzyl-1-(3,5-dichlorobenzoyl)-N-(4-quinolylmethyl)-N-methyl-4-piperidinamine or a salt thereof.

**65.** (2R*,4S*)-2-Benzyl-1-(3,5-dichlorobenzoyl)-N-(4-quinolylmethyl)-N-cyclohexylcarbamoyl-4-piperidinamine or a salt thereof.

**66.** (2R*,4S*)-2-Benzyl-1-(3,5-dichlorobenzoyl)-N-(4-quinolylmethyl)-N-phenylcarbamoyl-4-piperidinamine or a salt thereof.

**67.** (2R*,4S*)-2-Benzyl-1-(3,5-dichlorobenzoyl)-N-(2-phenylethyl)-4-piperidinamine or a salt thereof.

**68.** (2R*,4S*)-2-Benzyl-1-(3,5-bis(trifluoromethyl)benzoyl)-N-(2-phenylethyl)-4-piperidinamine or a salt thereof.

**69.** (2R*,4S*)-2-Benzyl-1-(3,5-dichlorobenzoyl)-N-(2-naphthoyl)-4-piperidinamine or a salt thereof.

**70.** (2R*,4S*)-2-Benzyl-1-(3,5-dichlorobenzoyl)-N-(3,5-dimethylbenzoyl)-4-piperidinamine or a salt thereof.

**71.** (2R*,4S*)-2-Benzyl-1-(3,5-dichlorobenzoyl)-N-(4-quinolylcarbonyl)-4-piperidinamine or a salt thereof.

**72.** (2R*,4S*)-2-Benzyl-1-(3,5-dichlorobenzoyl)-N-(3-indolylcarbonyl)-4-piperidinamine or a salt thereof.

**73.** (2R*,4S*)-2-Benzyl-1-(3,5-dichlorobenzoyl)-N-(2-indolylcarbonyl)-4-piperidinamine or a salt thereof.

**74.** (2R*,4S*)-2-Benzyl-1-(3,5-dichlorobenzoyl)-N-(5-methoxy-2-indolylcarbonyl)-4-piperidinamine or a salt thereof.

**75.** (2R*,4S*)-2-Benzyl-1-(3,5-dichlorobenzoyl)-N-(1-naphthoyl)-4-piperidinamine or a salt thereof.

**76.** (2R*,4S*)-2-Benzyl-1-(3,5-dichlorobenzoyl)-N-(phenylacetyl)-4-piperidinamine or a salt thereof.

**77.** (2R*,4S*)-2-Benzyl-1-(3,5-dichlorobenzoyl)-N-(2-methoxybenzyl)-4-piperidinamine or a salt thereof.

**78.** (2R*,4S*)-2-Benzyl-1-(3,5-dichlorobenzoyl)-N-(3-(N-acetyl)indolylmethyl)-4-piperidinamine or a salt thereof.

**79.** (2R*,4S*)-2-Benzyl-1-(3,5-dichlorobenzoyl)-N-(2-benzo[b]furanylmethyl)-4-piperidinamine or a salt thereof.

**80.** (2R*,4S*)-2-Benzyl-1-(3,5-dichlorobenzoyl)-N-[(3-methylbenzo[b]thiophen-2-ylmethyl]-4-piperidinamine or a salt thereof.

**81.** (2R*,4S*)-2-Benzyl-1-(3,5-dichlorobenzoyl)-N-(5-methoxyindol-3-ylmethyl)-4-piperidinamine or a salt thereof.

**82.** (2R*,4S*)-2-Benzyl-1-(3,5-dichlorobenzoyl)-N-(3-indolylmethyl)-4-piperidinamine or a salt thereof.

**83.** (2R*,4S*)-2-Benzyl-1-(3,5-dichlorobenzoyl)-N-phenylcarbamoyl-4-piperidinamine or a salt thereof.

**84.** (2R*,4S*)-2-Benzyl-1-(3,5-dichlorobenzoyl)-N-diphenylmethyl-4-piperidinamine or a salt thereof.

**85.** (2R*,4S*)-2-Benzyl-1-(3,5-dichlorobenzoyl)-N-(3,4-dihydro-2H-1-benzopyran-2-carbonyl)-4-piperidinamine or a salt thereof.

**86.** (2R*,4S*)-2-Benzyl-1-(4-methoxybenzoyl)-N-(4-quinolylmethyl)-4-piperidinamine or a salt thereof.

**87.** (2R*,4S*,1'R*)-N-Benzyl-1-(3,5-dimethylbenzoyl)-2-(1'-hydroxy-1'-phenylmethyl)-4-piperidinamine or a salt thereof.

**88.** (2R*,4S*,1'R*)-2-(1'-Hydroxy-1'-phenylmethyl)-1-(3,5-dimethylbenzoyl)-N-(4-quinolinylmethyl)-4-piperidinamine or a salt thereof.

**89.** (2R*,4S*,1'S*)-1-(3,5-Dimethylbenzoyl)-2-(1'-hydroxy-1'-phenylmethyl)-N-(4-quinolinylmethyl)-4-piperidinamine or a salt thereof.

**90.** (2R*,4S*,1'R*)-2-{1'-Hydroxy-1'-(4-chlorophenyl)methyl}-1-(3,5-dimethylbenzoyl)-N-(4-quinolinylmethyl)-4-piperidinamine or a salt thereof.

**91.** (2R*,4S*,1'S*)-1-(3,5-Dimethylbenzoyl)-2-{1'-hydroxy-1'-(4-chlorophenyl)-methyl}-N-(4-quinolinylmethyl)-4-piperidinamine or a salt thereof.

**92.** (2R*,4S*,1'S*)-1-(3,5-Dimethylbenzoyl)-2-{1'-hydroxy-1'-(3,4-dichlorophenyl)-methyl}-N-(4-quinolinylmethyl)-4-piperidinamine or a salt thereof.

**93.** (2R*,4S*)-N-Benzyl-1-(3,5-dimethylbenzoyl)-2-benzoyl-4-piperidinamine or a salt thereof.

**94.** (2R*,4S*)-2-(4-Chlorobenzyl)-1-(3,5-dimethylbenzoyl)-N-(4-quinolinylmethyl)-4-piperidinamine or a salt thereof.

**95.** (2R*,4S*)-2-(3,4-Dichlorobenzyl)-1-(3,5-dimethylbenzoyl)-N-(4-quinolinylmethyl)-4-piperidinamine or a salt thereof.

**96.** (2R*,4S*)-1-(3,5-Dimethylbenzoyl)-2-phenyl-N-(4-quinolinylmethyl)-4-piperidinamine or a salt thereof.

**97.** (2R*,4S*)-1-(3,5-Dichlorobenzoyl)-2-phenyl-N-(4-quinolinemethyl)-4-piperidinamine or a salt thereof.

**98.** (2R*,4S*)-1-(1-Naphthoyl)-2-phenyl-N-(4-quinolinylmethyl)-4-piperidinamine.

**99.** (2R*,4S*)-1-(3,5-Dimethylbenzoyl)-2-(1-naphthyl)-N-(4-quinolinylmethyl)-4-piperidinamine or a salt thereof.

**100.** A novel 1-acylpiperidone of the formula X

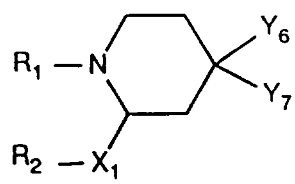 (X),

wherein $R_1$ is an optionally substituted aroyl, heteroaroyl, cycloalkylcarbonyl, aralkanoyl, heteroarylalkanoyl, aralkoxycarbonyl or arylcarbamoyl radical or the acyl radical of an $\alpha$-amino acid which is optionally N-substituted by lower alkanoyl or carbamoyl-lower-alkanoyl, $R_2$ is cycloalkyl or an optionally substituted aryl or heteroaryl radical, $X_1$ denotes hydroxymethylene and $Y_6$ and $Y_7$ together represent oxo, or a salt thereof.

**101.** A compound according to claim 100, of the formula X, wherein $R_1$ denotes benzoyl, benzoyl mono- or disubstituted by $C_1$-$C_4$alkyl, $C_1$-$C_4$alkoxy, halogen of atomic number up to and including 35 and/or trifluoromethyl, or unsubstituted naphthoyl, $R_2$ represents phenyl or phenyl mono- or disubstituted by halogen of atomic number up to and including 35 and/or trifluoromethyl, $X_1$ denotes hydroxymethylene and $Y_6$ and $Y_7$ together represent oxo, or a salt thereof.

**102.** (2R*,1'R*)-1-(3,5-Dimethylbenzoyl)-2-(1'-hydroxy-1'-phenyl-methyl)-4-piperidone or a salt thereof.

**103.** (2R*,1'R*)-1-(3,5-Bistrifluoromethylbenzoyl)-2-{1'-hydroxy-1'-(4-chlorophenyl)methyl}-4-piperidone or a salt thereof.

**104.** (2R*,1'R*)-1-(3,5-Dimethylbenzoyl)-2-{-1'-hydroxy-1'-(3,4-dichlorophenyl)methyl}-4-piperidone or a salt thereof.

**105.** (2R*,1'S*)-1-(3,5-Bistrifluoromethylbenzoyl)-2-{1'-hydroxy-1'-(4-chlorophenyl)methyl}-4-piperidone or a salt thereof.

**106.** (2R*,1'R*)-1-(3,5-Bistrifluoromethylbenzoyl)-2-{1'-hydroxy-1'-(3,4-dichlorophenyl)methyl}-4-piperidone or a salt thereof.

**107.** (2R*,1'R*)-1-(3,5-Dimethylbenzoyl)-2-{1'-hydroxy-1'-(4-methoxyphenyl)methyl}-4-piperidone or a salt thereof.

**108.** (2R*,1'S*)-1-(3,5-Dimethylbenzoyl)-2-{1'-hydroxy-1'-(4-methoxyphenyl)methyl}-4-piperidone or a salt thereof.

**109.** (2R*,1'R*)-1-(3,5-Dimethylbenzoyl)-2-{1'-hydroxy-1'-(3-methoxyphenyl)methyl}-4-piperidone or a salt thereof.

**110.** (2R*,1'S*)-1-(3,5-Dimethylbenzoyl)-2-{1'-hydroxy-1'-(3-methoxyphenyl)methyl}-4-piperidone or a salt thereof.

**111.** (2R*,1'R*)-1-(3,5-Dimethylbenzoyl)-2-{1'-hydroxy-1'-(4-trifluoromethylphenyl)methyl}-4-piperidone or a salt thereof.

**112.** (2R*,1'S*)-1-(3,5-Dimethylbenzoyl)-2-{1'-hydroxy-1'-(4-trifluoromethylphenyl)methyl}-4-piperidone or a salt thereof.

**113.** (2R*,1'R*)-1-(3,5-Dimethylbenzoyl)-2-{1'-hydroxy-1'-(4-chloro-3-trifluoromethylphenyl)methyl}-4-piperidone or a salt thereof.

**114.** (2R*,1'S*)-1-(3,5-Dimethylbenzoyl)-2-{1'-hydroxy-1'-(4-chloro-3-trifluoromethylphenyl)methyl}-4-piperidone or a salt thereof.

**115.** Compounds according to any of claims 1 to 114 for use in a method for the therapeutic treatment of the human or animal body.

**116.** A pharmaceutical composition comprising a compound according to any of claims 1 to 47 in free form or in pharmaceutically utilisable salt form in addition to customary pharmaceutical ancillary substances.

**117.** A pharmaceutical composition comprising a compound according to any of claims 48 to 115 in free form or in pharmaceutically utilisable salt form in addition to customary pharmaceutical ancillary substances.

**118.** Process for the preparation of novel 1-acylpiperidine compounds according to claim 1, and their salts, characterised in that

a) the radical $R_1$ is introduced into a compound of the formula II

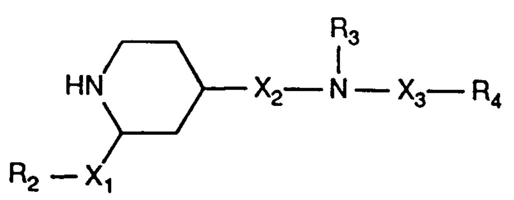

(II),

in which $R_2$, $R_3$, $R_4$, $X_1$, $X_2$ and $X_3$ have the indicated meanings, or

b) compounds of the formulae III and IV

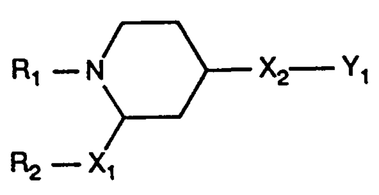

(III)

and

$Y_2$-$X_3$-$R_4$ (IV),

in which $Y_1$ is a group of the formula -N($R_3$)-H and $Y_2$ is hydroxyl, reactive esterified hydroxyl or, if $X_3$ is carbonyl, is etherified hydroxyl, or $Y_1$ is hydroxyl, reactively esterified hydroxyl or, if $X_2$ is carbonyl, is etherified hydroxyl and $Y_2$ is a group of the formula -N($R_3$)-H, where $R_2$, $R_3$, $R_4$, $X_1$, $X_2$ and $X_3$ have the indicated meanings, or the salts thereof, are condensed together or

c) for the preparation of compounds of the formula I in which one of the radicals $X_2$ and $X_3$ is alkylene and the other is alkylene, carbonyl or, in the case of $X_2$, a direct linkage or, in the case of $X_3$, an alkylene radical which is optionally substituted by hydroxymethyl or optionally esterified or amidated carboxyl, in a compound of the formula V

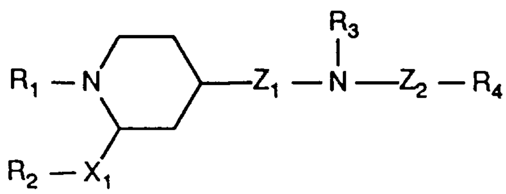

(V),

in which $Z_1$ is an alkylene radical which is substituted by oxo or hydroxyl in the $\alpha$ position to the group -N($R_3$)-, and $Z_2$ is alkylene, carbonyl or an alkylene radical which is optionally substituted by hydroxymethyl or optionally esterified or amidated carboxyl, or $Z_1$ is alkylene, carbonyl or a direct linkage and $Z_2$ is an alkylene radical substituted by oxo or hydroxyl in the $\alpha$ position to the group -N($R_3$)-, and $R_2$, $R_3$, $R_4$, $X_1$, $X_2$ and $X_3$ have the indicated meanings, or in a salt thereof, the oxo or hydroxyl group in the $\alpha$ position to the group -N($R_3$)-is replaced by hydrogen by reduction, or in a compound of the formula VI

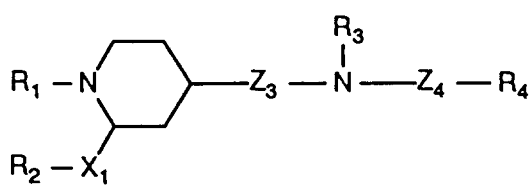

(VI),

in which $Z_3$ is a radical of the formula -C($R_a$)=C($R_b$)- and $Z_4$ is alkylene, carbonyl or an alkylene radical which is optionally substituted by hydroxymethyl or optionally esterified or amidated carboxyl, or $Z_3$ is alkylene, carbonyl or a direct linkage and $Z_4$ is a radical of the formula -C($R_a$)=C($R_b$)-, where $R_a$ and $R_b$ are each hydrogen or lower alkyl, the radical of the formula -C($R_a$)=C($R_b$)- is reduced by reduction of the double bond to the corresponding radical -CH($R_a$)=CH($R_b$)-, or

d) for the preparation of compounds of the formula I in which $X_1$ is a carbonyl or hydroxymethylene group, compounds of the formulae VII and VIII

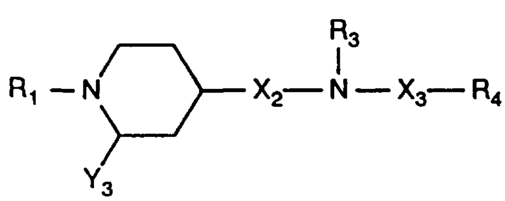

(VII)

and

$Y_4$-$R_2$ (VIII),

in which one of the radicals $Y_3$ and $Y_4$ is formyl or an optionally anhydridised or esterified carboxyl group and the other is a metallic radical, and $R_2$, $R_3$, $R_4$, $X_2$ and $X_3$ have the indicated meanings,

are condensed together or

e) for the preparation of compounds of the formula I in which $R_3$ is hydrogen, the group $Y_5$ is eliminated from a compound of the formula IX

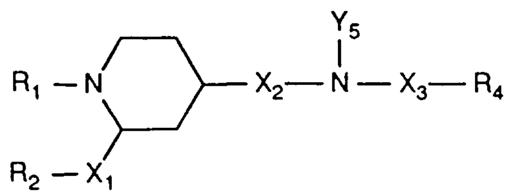

(IX),

in which $Y_5$ is an amino-protective group, and $R_2$, $R_4$, $X_1$, $X_2$ and $X_3$ have the indicated meanings, or from a salt thereof, or

f) for the preparation of compounds of the formula I in which $X_3$ is alkylene, compounds of the formulae X and XI

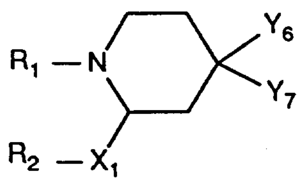

(X) and

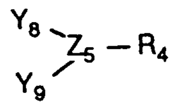

(XI),

in which $Y_6$ is a group of the formula -N($R_3$)-H, $Y_7$ is hydrogen, $Y_8$ and $Y_9$ together are oxo and $Z_5$ is an alkanylylidene radical corresponding to $X_3$, or $Y_6$ and $Y_7$ together are oxo, $Y_8$ is a group of the formula -N($R_3$)-H, $Y_9$ is hydrogen and $Z_5$ is a radical $X_3$, are condensed together under reducing conditions and, if required, a resulting compound is converted into another compound of the formula I, a mixture of isomers which can be obtained according to the process is resolved into the components, and the isomer preferred in each case is separated off and/or a free compound which can be obtained according to the process is converted into a salt, or a salt which can be obtained according to the process is converted into the corresponding free compound.

**119.** Process for the preparation of novel 1-acylpiperidones according to claim 100, and salts thereof, characterised in that a compound of the formula Xa

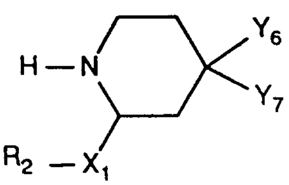

(Xa),

is condensed with an agent which introduces the radical $R_1$ and, if required, a resulting compound is converted into another compound of the formula I, a mixture of isomers which can be obtained according to the process is resolved into the components, and the isomer preferred in each case is separated off and/or a free compound which can be obtained according to the process is converted into a salt, or a salt which can be obtained according to the process is converted into the corresponding free compound.

**120.** Use of a compound according to any of claims 1 to 115 to prepare a pharmaceutical for the treatment of disorders in whose development substance P plays an essential part.

**Claims for the following Contracting States: ES, GR**

1. Process for the preparation of novel 1-acylpiperidine compounds of the formula I

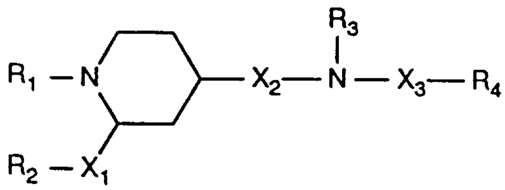

(I),

in which $R_1$ is phenyl- or diphenyl-$C_1$-$C_4$alkyl which is unsubstituted or substituted in the phenyl moiety by lower alkyl, lower alkoxy, di-lower-alkylamino, halogen and/or trifluoromethyl, phenoxy-$C_1$-$C_4$alkyl which is unsubstituted or substituted in the phenyl by halogen and/or triazolyl, pyridyl- or quinolinyl-$C_1$-$C_4$alkyl, benzoyl which is unsubstituted or substituted by lower alkyl, lower alkoxy, di-lower-alkylamino, halogen and/or trifluoromethyl, naphthoyl which is unsubstituted or substituted by lower alkyl, lower alkoxy, di-lower-alkylamino, halogen and/or trifluoromethyl, pyridylcarbonyl or quinolinylcarbonyl which is unsubstituted or substituted by lower alkyl, lower alkoxy, halogen and/or trifluoromethyl, 5- to 7-membered cycloalkylcarbonyl which is unsubstituted or substituted by lower alkyl, lower alkoxy, di-lower-alkylamino, halogen and/or trifluoromethyl, phenyl- or diphenyl-$C_1$-$C_4$alkanoyl which is unsubstituted or substituted in the phenyl by lower alkyl, lower alkoxy, di-lower-alkylamino, halogen and/or trifluoromethyl, N-phenylcarbamoyl which is unsubstituted or optionally substituted in the phenyl moiety by lower alkyl, lower alkoxy, di-lower-alkylamino, halogen and/or trifluoromethyl, or a group of the formula Ia

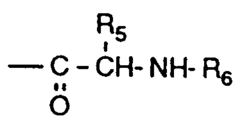

(Ia)

in which $R_5$ is hydrogen, $C_1$-$C_4$alkyl which is unsubstituted or substituted by hydroxyl, mercapto, amino, optionally hydroxy-substituted phenyl, carboxyl, carbamoyl or ureido, and $R_6$ is $C_2$-$C_7$alkanoyl, $R_2$ is 5- to 7-membered cycloalkyl or a phenyl, naphthyl or pyridyl radical which is unsubstituted or substituted by aromatically bonded $C_1$-$C_4$alkyl, $C_1$-$C_4$alkoxy, halogen and/or trifluoromethyl, $R_3$ is hydrogen, $C_1$-$C_7$alkyl, carbamoyl, $C_2$-$C_7$alkanoyl, carboxy-$C_1$-$C_4$alkanoyl or carboxy-$C_2$-$C_4$alkenoyl, $R_4$ is phenyl or naphthyl which is unsubstituted or substituted by $C_1$-$C_4$alkyl, $C_1$-$C_4$alkoxy, halogen and/or trifluoromethyl, or unsubstituted pyridyl, benzofuranyl, indolyl, 2,3-dihydroindolyl, benzimidazolyl, quinolyl or 1,2,3,4-tetrahydroquinolinyl, $X_1$ is methylene, hydroxymethylene, $C_1$-$C_4$alkoxymethylene, carbonyl, di-$C_1$-$C_4$alkoxymethylene or a direct linkage, $X_2$ is $C_1$-$C_7$alkylene, carbonyl or a direct linkage, and $X_3$ is carbonyl, $C_1$-$C_4$alkylene, carboxy-$C_1$-$C_4$alkylene, $C_1$-$C_4$alkoxycarbonyl-$C_1$-$C_4$alkylene, carbamoyl-$C_1$-$C_4$alkylene or hydroxymethyl-$C_1$-$C_4$alkylene, lower alkyl is $C_1$-$C_7$alkyl, and salts thereof, characterised in that

a) the radical $R_1$ is introduced into a compound of the formula II

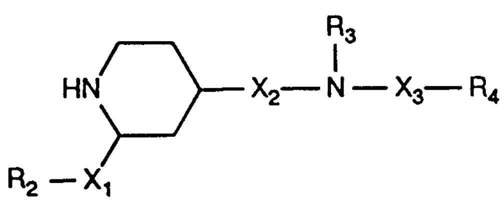

(II),

in which $R_2$, $R_3$, $R_4$, $X_1$, $X_2$ and $X_3$ have the indicated meanings, or

b) compounds of the formulae III and IV

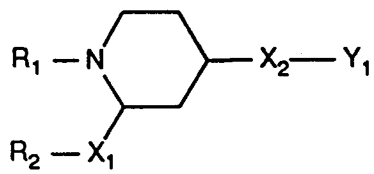

(III)

and

$Y_2$-$X_3$-$R_4$ (IV),

in which $Y_1$ is a group of the formula -N($R_3$)-H and $Y_2$ is hydroxyl, reactive esterified hydroxyl or, if $X_3$ is carbonyl, is etherified hydroxyl, or $Y_1$ is hydroxyl, reactively esterified hydroxyl or, if $X_2$ is carbonyl, is etherified hydroxyl and $Y_2$ is a group of the formula -N($R_3$)-H, where $R_2$, $R_3$, $R_4$, $X_1$, $X_2$ and $X_3$ have the indicated meanings, or the salts thereof, are condensed together or

c) for the preparation of compounds of the formula I in which one of the radicals $X_2$ and $X_3$ is alkylene and the other is alkylene, carbonyl or, in the case of $X_2$, a direct linkage or, in the case of $X_3$, an alkylene radical which is optionally substituted by hydroxymethyl or optionally esterified or amidated carboxyl, in a compound of the formula V

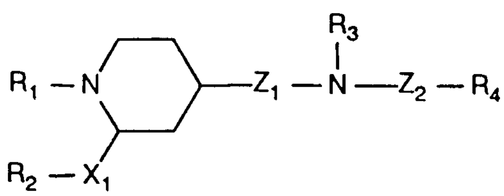

(V),

in which $Z_1$ is an alkylene radical which is substituted by oxo or hydroxyl in the $\alpha$ position to the group -N($R_3$)-, and $Z_2$ is alkylene, carbonyl or an alkylene radical which is optionally substituted by hydroxymethyl or optionally esterified or amidated carboxyl, or $Z_1$ is alkylene, carbonyl or a direct linkage and $Z_2$ is an alkylene radical substituted by oxo or hydroxyl in the $\alpha$ position to the group -N($R_3$)-, and $R_2$, $R_3$, $R_4$, $X_1$, $X_2$ and $X_3$ have the indicated meanings, or in a salt thereof, the oxo or hydroxyl group in the $\alpha$ position to the group -N($R_3$)-is replaced by hydrogen by reduction, or in a compound of the formula VI

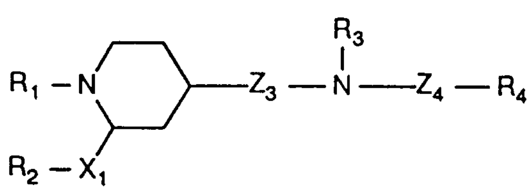

(VI),

in which $Z_3$ is a radical of the formula -C($R_a$) = C($R_b$)- and $Z_4$ is alkylene, carbonyl or an alkylene radical which is optionally substituted by hydroxymethyl or optionally esterified or amidated carboxyl, or $Z_3$ is alkylene, carbonyl or a direct linkage and $Z_4$ is a radical of the formula -C($R_a$) = C($R_b$)-, where $R_a$ and $R_b$ are each hydrogen or lower alkyl, the radical of the formula -C($R_a$) = C($R_b$)- is reduced by reduction of the double bond to the corresponding radical -CH($R_a$) = CH($R_b$)-, or

d) for the preparation of compounds of the formula I in which $X_1$ is a carbonyl or hydroxymethylene group, compounds of the formulae VII and VIII

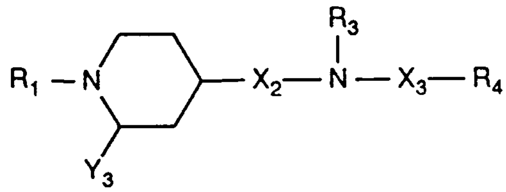 (VII)

and

$Y_4$-$R_2$ (VIII),

in which one of the radicals $Y_3$ and $Y_4$ is formyl or an optionally anhydridised or esterified carboxyl group and the other is a metallic radical, and $R_2$, $R_3$, $R_4$, $X_2$ and $X_3$ have the indicated meanings, are condensed together or

e) for the preparation of compounds of the formula I in which $R_3$ is hydrogen, the group $Y_5$ is eliminated from a compound of the formula IX

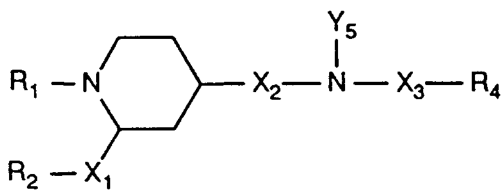 (IX),

in which $Y_5$ is an amino-protective group, and $R_2$, $R_4$, $X_1$, $X_2$ and $X_3$ have the indicated meanings, or from a salt thereof, or

f) for the preparation of compounds of the formula I in which $X_3$ is alkylene, compounds of the formulae X and XI

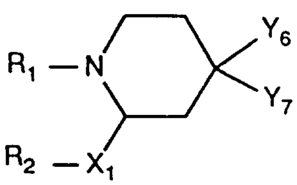 (X) and 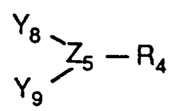 (XI),

in which $Y_6$ is a group of the formula -N($R_3$)-H, $Y_7$ is hydrogen, $Y_8$ and $Y_9$ together are oxo and $Z_5$ is an alkanylylidene radical corresponding to $X_3$, or $Y_6$ and $Y_7$ together are oxo, $Y_8$ is a group of the formula -N($R_3$)-H, $Y_9$ is hydrogen and $Z_5$ is a radical $X_3$, are condensed together under reducing conditions and, if required, a resulting compound is converted into another compound of the formula I, a mixture of isomers which can be obtained according to the process is resolved into the components, and the isomer preferred in each case is separated off and/or a free compound which can be obtained according to the process is converted into a salt, or a salt which can be obtained according to the process is converted into the corresponding free compound.

**2.** Process according to claim 1, which comprises preparing a compound of the formula I in which $R_1$ is benzoyl, naphthoyl or phenyl-$C_1$-$C_4$alkanoyl which is unsubstituted or substituted by $C_1$-$C_4$alkyl, $C_1$-$C_4$alkoxy, halogen and/or trifluoromethyl, unsubstituted pyridylcarbonyl or quinolinylcarbonyl or a group of the formula Ia

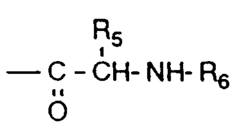 (Ia),

in which $R_5$ is hydrogen, $C_1$-$C_4$alkyl which is unsubstituted or substituted by hydroxyl, mercapto, amino, optionally hydroxy-substituted phenyl, carboxyl, carbamoyl or ureido, and $R_6$ is $C_2$-$C_7$alkanoyl, $R_2$ is 5- to 7-membered cycloalkyl or a phenyl, naphthyl or pyridyl radical which is unsubstituted or substituted by aromatically bonded $C_1$-$C_4$alkyl, $C_1$-$C_4$alkoxy, halogen and/or trifluoromethyl, $R_3$ is hydrogen, $C_1$-$C_7$alkyl, carbamoyl, $C_2$-$C_7$alkanoyl, carboxy-$C_1$-$C_4$alkanoyl or carboxy-$C_3$-$C_5$alkenoyl, $R_4$ is phenyl or naphthyl which is unsubstituted or substituted by $C_1$-$C_4$alkyl, $C_1$-$C_4$alkoxy, halogen and/or trifluoromethyl, or unsubstituted pyridyl, benzofuranyl, indolyl, benzimidazolyl or quinolyl, $X_1$ is methylene, hydroxymethylene, $C_1$-$C_4$alkoxymethylene, carbonyl, di-$C_1$-$C_4$alkoxymethylene or a direct linkage, $X_2$ is $C_1$-$C_7$alkylene, carbonyl or a direct linkage, and $X_3$ is carbonyl, $C_1$-$C_4$alkylene, carboxy-$C_1$-$C_4$alkylene, $C_1$-$C_4$alkoxycarbonyl-$C_1$-$C_4$alkylene, carbamoyl-$C_1$-$C_4$alkylene or hydroxymethyl-$C_1$-$C_4$alkylene, or a salt thereof.

**3.** Process according to claim 1, which comprises preparing a compound of the formula I in which $R_1$ is benzoyl which is unsubstituted or mono- or disubstituted by $C_1$-$C_4$alkyl, $C_1$-$C_4$alkoxy, halogen of atomic number up to and including 35 and/or trifluoromethyl, or unsubstituted naphthoyl or phenyl-$C_1$-$C_4$alkanoyl, $R_2$ is phenyl which is unsubstituted or mono- or disubstituted by $C_1$-$C_4$alkyl, $C_1$-$C_4$alkoxy, halogen of atomic number up to and including 35 and/or trifluoromethyl, or unsubstituted pyridyl, $R_3$ is hydrogen, $C_1$-$C_4$alkyl, carbamoyl or $C_2$-$C_7$alkanoyl, $R_4$ is phenyl which is unsubstituted or mono- or disubstituted by $C_1$-$C_4$alkyl, $C_1$-$C_4$alkoxy, halogen of atomic number up to and including 35 and/or trifluoromethyl, or unsubstituted naphthyl, pyridyl, benzofuranyl, indolyl, benzimidazolyl or quinolyl, $X_1$ is methylene, hydroxymethylene, carbonyl or a direct linkage, $X_2$ is a direct linkage, and $X_3$ is $C_1$-$C_4$alkylene, or a salt thereof.

**4.** Process according to claim 1, which comprises preparing a compound of the formula I in which $R_1$ is benzoyl which is unsubstituted or mono- or disubstituted by $C_1$-$C_4$alkyl, $C_1$-$C_4$alkoxy, halogen of atomic number up to and including 35 and/or trifluoromethyl, or unsubstituted naphthoyl, $R_2$ is phenyl or phenyl mono- or disubstituted by halogen of atomic number up to and including 35 and/or trifluoromethyl, $R_3$ is hydrogen, $R_4$ is unsubstituted quinolinyl, $X_1$ is methylene, $X_2$ is a direct linkage and $X_3$ is $C_1$-$C_4$alkylene, or a salt thereof.

**5.** Process according to claim 1, which comprises preparing (2R*,4S*)-2-benzyl-1-(2-naphthoyl)-N-(4-quinolylmethyl)-4-piperidinamine or a salt thereof.

**6.** Process according to claim 1, which comprises preparing (2R*,4S*)-2-benzyl-1-(3-trifluoromethylbenzoyl)-N-(4-quinolylmethyl)-4-piperidinamine or a salt thereof.

**7.** Process according to claim 1, which comprises preparing (2R*,4S*)-2-benzyl-1-(3,5-bis(trifluoromethyl)-benzoyl)-N-(4-quinolylmethyl)-4-piperidinamine or a salt thereof.

**8.** Process according to claim 1, which comprises preparing (2R*,4S*)-2-benzyl-1-(3,5-dimethoxybenzoyl)-N-(4-quinolylmethyl)-4-piperidinamine or a salt thereof.

**9.** Process according to claim 1, which comprises preparing (2R*,4S*)-2-benzyl-1-(1-naphthoyl)-N-(4-quinolylmethyl)-4-piperidinamine or a salt thereof.

**10.** Process according to claim 1, which comprises preparing (2R*,4S*)-2-benzyl-1-(3,5-dichlorobenzoyl)-N-(4-quinolylmethyl)-4-piperidinamine or a salt thereof.

**11.** Process according to claim 1, which comprises preparing (2R*,4S*)-2-benzyl-1-(2-quinolinylcarbonyl)-N-(4-quinolylmethyl)-4-piperidinamine or a salt thereof.

**12.** Process according to claim 1, which comprises preparing (2R*,4S*)-2-benzyl-1-(4-chlorophenylacetyl)-N-(4-quinolylmethyl)-4-piperidinamine or a salt thereof.

**13.** Process according to claim 1, which comprises preparing (2R*,4S*)-2-benzyl-1-(benzyloxycarbonyl)-N-(4-quinolylmethyl)-4-piperidinamine or a salt thereof.

**14.** Process according to claim 1, which comprises preparing (2R*,4S*)-2-benzyl-1-(2-phenylethyl)-N-(4-quinolylmethyl)-4-piperidinamine or a salt thereof.

**15.** Process according to claim 1, which comprises preparing (2R*,4S*)-2-benzyl-1-(2-naphthylacetyl)-N-(4-quinolylmethyl)-4-piperidinamine or a salt thereof.

**16.** Process according to claim 1, which comprises preparing (2R*,4S*)-2-benzyl-1-(4-quinolylmethyl)-N-(4-quinolylmethyl)-4-piperidinamine or a salt thereof.

**17.** Process according to claim 1, which comprises preparing (2R*,4S*)-2-benzyl-1-(2,4-dichlorobenzyl)-N-(4-quinolylmethyl)-4-piperidinamine or a salt thereof.

**18.** Process according to claim 1, which comprises preparing (2R*,4S*)-2-benzyl-1-(2,2-diphenylethyl)-N-(4-quinolylmethyl)-4-piperidinamine or a salt thereof.

**19.** Process according to claim 1, which comprises preparing (2R*,4S*)-2-benzyl-1-(phenylcarbamoyl)-N-(4-quinolylmethyl)-4-piperidinamine or a salt thereof.

**20.** Process according to claim 1, which comprises preparing (2R*,4S*)-2-benzyl-1-(diphenylacetyl)-N-(4-quinolylmethyl)-4-piperidinamine or a salt thereof.

**21.** Process according to claim 1, which comprises preparing (2R*,4S*)-2-benzyl-1-(2-pyridylacetyl)-N-(4-quinolylmethyl)-4-piperidinamine or a salt thereof.

**22.** Process according to claim 1, which comprises preparing (2R*,4S*)-2-benzyl-1-(4-pyridylacetyl)-N-(4-quinolylmethyl)-4-piperidinamine or a salt thereof.

**23.** Process according to claim 1, which comprises preparing (2R*,4S*)-2-benzyl-1-(2,3-diphenylpropionyl)-N-(4-quinolylmethyl)-4-piperidinamine or a salt thereof.

**24.** Process according to claim 1, which comprises preparing (2R*,4S*)-2-benzyl-1-((3S)-(2,3,4,9-tetrahydro-1H-pyrido[3,4-b]indol-3-yl)carbonyl)-N-(4-quinolylmethyl)4-piperidinamine or a salt thereof.

**25.** Process according to claim 1, which comprises preparing (2R*,4S*)-2-benzyl-1-(3-methoxybenzoyl)-N-(4-quinolylmethyl)-4-piperidinamine or a salt thereof.

**26.** Process according to claim 1, which comprises preparing (2R*,4S*)-2-benzyl-1-(3-N,N-dimethylaminobenzoyl)-N-(4-quinolylmethyl)-4-piperidinamine or a salt thereof.

**27.** Process according to claim 1, which comprises preparing (2R*,4S*)-2-benzyl-1-(cis,cis-3,5-dimethyl-cyclohexylcarbonyl)-N-(4-quinolylmethyl)-4-piperidinamine or a salt thereof.

**28.** Process according to claim 1, which comprises preparing (2R*,4S*)-2-benzyl-1-(3,5-bis(trifluoromethyl)-benzyl)-N-(4-quinolylmethyl)-4-piperidinamine or a salt thereof.

**29.** Process according to claim 1, which comprises preparing (2S*,4R*)-2-benzyl-1-[2-(5-chloro-1H-1,2,4-triazol-1-yl)phenoxyethyl]-N-(4-quinolylmethyl)-4-piperidinamine or a salt thereof.

**30.** Process according to claim 1, which comprises preparing (2R*,4S*)-2-benzyl-1-((S)phenylalanyl)-N-(4-quinolylmethyl)-4-piperidinamine or a salt thereof.

**31.** Process according to claim 1, which comprises preparing (2R*,4S*)-2-benzyl-1-((R)-phenylalanyl)-N-(4-quinolylmethyl)-4-piperidinamine or a salt thereof.

**32.** Process according to claim 1, which comprises preparing (2R*,4S*)-2-benzyl-1-((S)-N-acetyl-phenylalanyl)-N-(4-quinolylmethyl)-4-piperidinamine or a salt thereof.

**33.** Process according to claim 1, which comprises preparing (2R*,4S*)-2-benzyl-1-((R)-N-acetylphenylalanyl)-N-(4-quinolylmethyl)-4-piperidinamine or a salt thereof.

**34.** Process according to claim 1, which comprises preparing (2R*,4S*)-2-benzyl-1-((S)-N-(4-carboxamidobutyroyl)phenylalanyl)-N-(4-quinolylmethyl)-4-piperidinamine or a salt thereof.

**35.** Process according to claim 1, which comprises preparing (2R*,4S*)-2-benzyl-1-((R)-N-(4-carboxamidobutyroyl)phenylalanyl)-N-(4-quinolylmethyl)-4-piperidinamine or a salt thereof.

**36.** Process according to claim 1, which comprises preparing (2R*,4S*)-2-benzyl-1-benzoyl-N-(4-quinolylmethyl)-4-piperidinamine or a salt thereof.

**37.** Process according to claim 1, which comprises preparing (2R*,4S*)-2-benzyl-1-(3-chlorobenzoyl)-N-(4-quinolylmethyl)-4-piperidinamine or a salt thereof.

**38.** Process according to claim 1, which comprises preparing (2R*,4S*)-2-benzyl-1-(3,5-dichlorobenzoyl)-N-(4-quinolylmethyl)-N-(3-carboxamidopropionyl)-4-piperidinamine or a salt thereof.

**39.** Process according to claim 1, which comprises preparing (2R,4S)- or (2R,4R)-2-benzyl-1-(3,5-dimethylbenzoyl)-N-(4-quinolylmethyl)-4-piperidinamine or a salt thereof.

**40.** Process according to claim 1, which comprises preparing (2S,4R)- or (2S,4S)-2-benzyl-1-(3,5-dimethylbenzoyl)-N-(2-phenethyl)-4-piperidinamine or a salt thereof.

**41.** Process according to claim 1, which comprises preparing (2R,4S)-2-benzyl-1-(3,5-dimethylbenzoyl)-N-(3-quinolylmethyl)-4-piperidinamine or a salt thereof.

**42.** Process according to claim 1, which comprises preparing (2R,4S)-2-benzyl-1-(3,5-dimethylbenzoyl)-N-(2-quinolylmethyl)-4-piperidinamine or a salt thereof.

**43.** Process according to claim 1, which comprises preparing (2R,4S)- or (2R,4R)-2-benzyl-1-(3,5-dimethylbenzoyl)-N-benzyl-4-piperidinamine or a salt thereof.

**44.** Process according to claim 1, which comprises preparing (2S,4R)- or (2S,4S)-2-benzyl-1-(3,5,dimethylbenzoyl)-N-benzyl-4-piperidinamine or a salt thereof.

**45.** Process according to claim 1, which comprises preparing (2R,4S)- or (2R,4R)-2-benzyl-1-(3,5-dimethylbenzoyl)-N-(4-pyridylmethyl)-4-piperidinamine or a salt thereof.

**46.** Process according to claim 1, which comprises preparing (2R,4S)- or (2R,4R)-2-benzyl-1-(3,5-dimethylbenzoyl)-N-(3-pyridylmethyl)-4-piperidinamine or a salt thereof.

**47.** Process according to claim 1, which comprises preparing (2S,4R)- or (2S,4S)-2-benzyl-1-(3,5-dimethylbenzoyl)-N-(4-quinolylmethyl)-4-piperidinamine or a salt thereof.

**48.** Process according to claim 1, which comprises preparing (2R*,4S*)-2-benzyl-1-(3,5-dimethylbenzoyl)-N-benzyl-N-carbamoyl-4-piperidinamine or a salt thereof.

**49.** Process according to claim 1, which comprises preparing (2R,4S)-2-benzyl-1-(3,5-dimethylbenzoyl)-N-(3-phenylpropyl)-4-piperidinamine;
(2S,4S)-2-benzyl-1-(3,5-dimethylbenzoyl)-N-(3-phenylpropyl)-4-piperidinamine;
(2R,4S)-2-benzyl-1-(3,5-dimethylbenzoyl)-N-(2-methoxybenzyl)-4-piperidinamine;
(2S,4S)-2-benzyl-1-(3,5-dimethylbenzoyl)-N-(2-methoxybenzyl)-4-piperidinamine;
(2R,4S)-2-benzyl-1-(3,5-dimethylbenzoyl)-N-[2-(2-methoxyphenyl)ethyl]-4-piperidinamine;
(2S,4S)-2-benzyl-1-(3,5-dimethylbenzoyl)-N-[2-(2-methoxyphenyl)ethyl]-4-piperidinamine;
(2R,4S)-2-benzyl-1-(3,5-dimethylbenzoyl)-N-[3-(2-methoxyphenyl)propyl]-4-piperidinamine;
(2S,4S)-2-benzyl-1-(3,5-dimethylbenzoyl)-N-[3-(2-methoxyphenyl)propyl]-4-piperidinamine;
(2R,4S)-2-benzyl-1-(3,5-dimethylbenzoyl)-N-(2-trifluoromethylbenzyl)-4-piperidinamine;

(2S,4S)-2-benzyl-1-(3,5-dimethylbenzoyl)-N-(2-trifluoromethylbenzyl)-4-piperidinamine;
(2R,4S)-2-benzyl-1-(3,5-dimethylbenzoyl)-N-[2-(2-trifluoromethylphenyl)ethyl]-4-piperidinamine;
(2S,4S)-2-benzyl-1-(3,5-dimethylbenzoyl)-N-[2-(2-trifluoromethylphenyl)ethyl]-4-piperidinamine;
(2R,4S)-2-benzyl-1-(3,5-dimethylbenzoyl)-N-[3-(2-trifluoromethylphenyl)propyl]-4-piperidinamine;
(2S,4S)-2-benzyl-1-(3,5-dimethylbenzoyl)-N-[3-(2-trifluoromethylphenyl)propyl]-4-piperidinamine;
(2R,4S)-2-benzyl-1-(3,5-dimethylbenzoyl)-N-(3-trifluoromethylbenzyl)-4-piperidinamine;
(2S,4S)-2-benzyl-1-(3,5-dimethylbenzoyl)-N-(3-trifluoromethylbenzyl)-4-piperidinamine;
(2R,4S)-2-benzyl-1-(3,5-dimethylbenzoyl)-N-[2-(3-trifluoromethylphenyl)ethyl]-4-piperidinamine;
(2S,4S)-2-benzyl-1-(3,5-dimethylbenzoyl)-N-[2-(3-trifluoromethylphenyl)ethyl]-4-piperidinamine;
(2R,4S)-2-benzyl-1-(3,5-dimethylbenzoyl)-N-[3-(3-trifluoromethylphenyl)propyl]-4-piperidinamine;
(2S,4S)-2-benzyl-1-(3,5-dimethylbenzoyl)-N-[3-(3-trifluoromethylphenyl)propyl]-4-piperidinamine;
(2R,4S)-2-benzyl-1-(3,5-dimethylbenzoyl)-N-(4-trifluoromethylbenzyl)-4-piperidinamine;
(2S,4S)-2-benzyl-1-(3,5-dimethylbenzoyl)-N-(4-trifluoromethylbenzyl)-4-piperidinamine;
(2R,4S)-2-benzyl-1-(3,5-dimethylbenzoyl)-N-[2-(4-trifluoromethylphenyl)ethyl]-4-piperidinamine;
(2S,4S)-2-benzyl-1-(3,5-dimethylbenzoyl)-N-[2-(4-trifluoromethylphenyl)ethyl]-4-piperidinamine;
(2R,4S)-2-benzyl-1-(3,5-dimethylbenzoyl)-N-[3-(4-trifluoromethylphenyl)propyl]-4-piperidinamine;
(2S,4S)-2-benzyl-1-(3,5-dimethylbenzoyl)-N-[3-(4-trifluoromethylphenyl)propyl]-4-piperidinamine;
(2R,4S)-2-benzyl-1-(3,5-dimethylbenzoyl)-N-(2,3-dimethoxybenzyl)-4-piperidinamine;
(2S,4S)-2-benzyl-1-(3,5-dimethylbenzoyl)-N-(2,3-dimethoxybenzyl)-4-piperidinamine;
(2R,4S)-2-benzyl-1-(3,5-dimethylbenzoyl)-N-[2-(2,3-dimethoxyphenyl)ethyl]-4-piperidinamine;
(2S,4S)-2-benzyl-1-(3,5-dimethylbenzoyl)-N-[2-(2,3-dimethoxyphenyl)ethyl]-4-piperidinamine;
(2R,4S)-2-benzyl-1-(3,5-dimethylbenzoyl)-N-[3-(2,3-dimethoxyphenyl)propyl]-4-piperidinamine;
(2S,4S)-2-benzyl-1-(3,5-dimethylbenzoyl)-N-[3-(2,3-dimethoxyphenyl)propyl]-4-piperidinamine;
(2R,4S)-2-benzyl-1-(3,5-dimethylbenzoyl)-N-(2,4-dimethoxybenzyl)-4-piperidinamine;
(2S,4S)-2-benzyl-1-(3,5-dimethylbenzoyl)-N-(2,4-dimethoxybenzyl)-4-piperidinamine;
(2R,4S)-2-benzyl-1-(3,5-dimethylbenzoyl)-N-[2-(2,4-dimethoxyphenyl)ethyl]-4-piperidinamine;
(2S,4S)-2-benzyl-1-(3,5-dimethylbenzoyl)-N-[2-(2,4-dimethoxyphenyl)ethyl]-4-piperidinamine;
(2R,4S)-2-benzyl-1-(3,5-dimethylbenzoyl)-N-[3-(2,4-dimethoxyphenyl)propyl]-4-piperidinamine;
(2S,4S)-2-benzyl-1-(3,5-dimethylbenzoyl)-N-[3-(2,4-dimethoxyphenyl)propyl]-4-piperidinamine;
(2R,4S)-2-benzyl-1-(3,5-dimethylbenzoyl)-N-(2,5-dimethoxybenzyl)-4-piperidinamine;
(2S,4S)-2-benzyl-1-(3,5-dimethylbenzoyl)-N-(2,5-dimethoxybenzyl)-4-piperidinamine;
(2R,4S)-2-benzyl-1-(3,5-dimethylbenzoyl)-N-[2-(2,5-dimethoxyphenyl)ethyl]-4-piperidinamine;
(2S,4S)-2-benzyl-1-(3,5-dimethylbenzoyl)-N-[2-(2,5-dimethoxyphenyl)ethyl]-4-piperidinamine;
(2R,4S)-2-benzyl-1-(3,5-dimethylbenzoyl)-N-[3-(2,5-dimethoxyphenyl)propyl]-4-piperidinamine;
(2S,4S)-2-benzyl-1-(3,5-dimethylbenzoyl)-N-[3-(2,5-dimethoxyphenyl)propyl]-4-piperidinamine;
(2R,4S)-2-benzyl-1-(3,5-dimethylbenzoyl)-N-(2,6-dimethoxyphenyl)-4-piperidinamine;
(2S,4S)-2-benzyl-1-(3,5-dimethylbenzoyl)-N-(2,6-dimethoxyphenyl)-4-piperidinamine;
(2R,4S)-2-benzyl-1-(3,5-dimethylbenzoyl)-N-[2-(2,6-dimethoxyphenyl)ethyl]-4-piperidinamine;
(2S,4S)-2-benzyl-1-(3,5-dimethylbenzoyl)-N-[2-(2,6-dimethoxyphenyl)ethyl]-4-piperidinamine;
(2R,4S)-2-benzyl-1-(3,5-dimethylbenzoyl)-N-[3-(2,6-dimethoxyphenyl)propyl]-4-piperidinamine;
(2R,4S)-2-benzyl-1-(3,5-dimethylbenzoyl)-N-[3-(2,6-dimethoxyphenyl)propyl]-4-piperidinamine;
(2R,4S)-2-benzyl-1-(3,5-dimethylbenzoyl)-N-(2,3-methylenedioxybenzyl)-4-piperidinamine;
(2S,4S)-2-benzyl-1-(3,5-dimethylbenzoyl)-N-(2,3-methylenedioxybenzyl)-4-piperidinamine;
(2R,4S)-2-benzyl-1-(3,5-dimethylbenzoyl)-N-[2-(2,3-methylenedioxyphenyl)ethyl]-4-piperidinamine;
(2S,4S)-2-benzyl-1-(3,5-dimethylbenzoyl)-N-[2-(2,3-methylenedioxyphenyl)ethyl]-4-piperidinamine;
(2R,4S)-2-benzyl-1-(3,5-dimethylbenzoyl)-N-[3-(2,3-methylenedioxyphenyl)propyl]-4-piperidinamine;
(2S,4S)-2-benzyl-1-(3,5-dimethylbenzoyl)-N-[3-(2,3-methylenedioxyphenyl)propyl]-4-piperidinamine;
(2R,4S)-2-benzyl-1-(3,5-dimethylbenzoyl)-N-(2,4-methylenedioxybenzyl)-4-piperidinamine;
(2S,4S)-2-benzyl-1-(3,5-dimethylbenzoyl)-N-(2,4-methylenedioxybenzyl)-4-piperidinamine;
(2R,4S)-2-benzyl-1-(3,5-dimethylbenzoyl)-N-[2-(2,4-methylenedioxyphenyl)ethyl]-4-piperidinamine;
(2S,4S)-2-benzyl-1-(3,5-dimethylbenzoyl)-N-[2-(2,4-methylenedioxyphenyl)ethyl]-4-piperidinamine;
(2R,4S)-2-benzyl-1-(3,5-dimethylbenzoyl)-N-[3-(2,4-methylenedioxyphenyl)propyl]-4-piperidinamine;
(2S,4S)-2-benzyl-1-(3,5-dimethylbenzoyl)-N-[3-(2,4-methylenedioxyphenyl)propyl]-4-piperidinamine;
(2R,4S)-2-benzyl-1-(3,5-dimethylbenzoyl)-N-(indol-2-ylmethyl)-4-piperidinamine;
(2S,4S)-2-benzyl-1-(3,5-dimethylbenzoyl)-N-(indol-2-ylmethyl)-4-piperidinamine;
(2R,4S)-2-benzyl-1-(3,5-dimethylbenzoyl)-N-(indol-3-ylmethyl)-4-piperidinamine;
(2S,4S)-2-benzyl-1-(3,5-dimethylbenzoyl)-N-(indol-3-ylmethyl)-4-piperidinamine;
(2S,4S)-2-benzyl-1-(3,5-dimethylbenzoyl)-N-(quinolin-2-ylmethyl)-4-piperidinamine;

(2S,4S)-2-benzyl-1-(3,5-dimethylbenzoyl)-N-(quinolin-3-ylmethyl)-4-piperidinamine;
(2R,4S)-2-benzyl-1-(3,5-dimethylbenzoyl)-N-(2-chlorobenzyl)-4-piperidinamine;
(2S,4S)-2-benzyl-1-(3,5-dimethylbenzoyl)-N-(2-chlorobenzyl)-4-piperidinamine;
(2R,4S)-2-benzyl-1-(3,5-dimethylbenzoyl)-N-[2-(2-chlorophenyl)ethyl]-4-piperidinamine;
(2S,4S)-2-benzyl-1-(3,5-dimethylbenzoyl)-N-[2-(2-chlorophenyl)ethyl]-4-piperidinamine;
(2R,4S)-2-benzyl-1-(3,5-dimethylbenzoyl)-N-[3-(2-chlorophenyl)propyl]-4-piperidinamine;
(2S,4S)-2-benzyl-1-(3,5-dimethylbenzoyl)-N-[3-(2-chlorophenyl)propyl]-4-piperidinamine;
(2R,4S)-2-benzyl-1-(3,5-dimethylbenzoyl)-N-(3-chlorobenzyl)-4-piperidinamine;
(2S,4S)-2-benzyl-1-(3,5-dimethylbenzoyl)-N-(3-chlorobenzyl)-4-piperidinamine;
(2R,4S)-2-benzyl-1-(3,5-dimethylbenzoyl)-N-[2-(3-chlorophenyl)ethyl]-4-piperidinamine;
(2S,4S)-2-benzyl-1-(3,5-dimethylbenzoyl)-N-[2-(3-chlorophenyl)ethyl]-4-piperidinamine;
(2R,4S)-2-benzyl-1-(3,5-dimethylbenzoyl)-N-[3-(3-chlorophenyl)propyl]-4-piperidinamine;
(2S,4S)-2-benzyl-1-(3,5-dimethylbenzoyl)-N-[3-(3-chlorophenyl)propyl]-4-piperidinamine;
(2R,4S)-2-benzyl-1-(3,5-dimethylbenzoyl)-N-(4-chlorobenzyl)-4-piperidinamine;
(2S,4S)-2-benzyl-1-(3,5-dimethylbenzoyl)-N-(4-chlorobenzyl)-4-piperidinamine;
(2R,4S)-2-benzyl-1-(3,5-dimethylbenzoyl)-N-[2-(4-chlorophenyl)ethyl]-4-piperidinamine;
(2S,4S)-2-benzyl-1-(3,5-dimethylbenzoyl)-N-[2-(4-chlorophenyl)ethyl]-4-piperidinamine;
(2R,4S)-2-benzyl-1-(3,5-dimethylbenzoyl)-N-[3-(4-chlorophenyl)propyl]-4-piperidinamine;
(2S,4S)-2-benzyl-1-(3,5-dimethylbenzoyl)-N-[3-(4-chlorophenyl)propyl]-4-piperidinamine;
(2R,4S)-2-benzyl-1-(3,5-dimethylbenzoyl)-N-(4-methoxynaphth-1-ylmethyl)-4-piperidinamine;
(2S,4S)-2-benzyl-1-(3,5-dimethylbenzoyl)-N-(4-methoxynaphth-1-ylmethyl)-4-piperidinamine;
(2R,4S)-2-benzyl-1-(3,5-dimethylbenzoyl)-N-(3,4-ethylenedioxybenzyl)-4-piperidinamine;
(2S,4S)-2-benzyl-1-(3,5-dimethylbenzoyl)-N-(3,4-ethylenedioxybenzyl)-4-piperidinamine;
(2R,4S)-2-benzyl-1-(3,5-dimethylbenzoyl)-N-(quinolin-2-ylmethyl)-4-piperidinamine;
(2R,4S)-2-benzyl-1-(3,5-dimethylbenzoyl)-N-(quinolin-3-ylmethyl)-4-piperidinamine;
(2R,4S)-2-benzyl-1-(3,5-ditrifluoromethylbenzoyl)-N-(indol-2-ylmethyl)-4-piperidinamine;
(2S,4S)-2-benzyl-1-(3,5-ditrifluoromethylbenzoyl)-N-(indol-2-ylmethyl)-4-piperidinamine;
(2R,4S)-2-benzyl-1-(3,5-ditrifluoromethylbenzoyl)-N-(indol-3-ylmethyl-4-piperidinamine;
(2S,4S)-2-benzyl-1-(3,5-ditrifluoromethylbenzoyl)-N-(indol-3-ylmethyl-4-piperidinamine;
(2R,4S)-2-benzyl-1-(3,5-ditrifluoromethylbenzoyl)-N-(quinolin-2-ylmethyl)-4-piperidinamine;
(2S,4S)-2-benzyl-1-(3,5-ditrifluoromethylbenzoyl)-N-(quinolin-2-ylmethyl-4-piperidinamine;
(2R,4S)-2-benzyl-1-(3,5-ditrifluoromethylbenzoyl)-N-(quinolin-3-ylmethyl)-4-piperidinamine;
(2S,4S)-2-benzyl-1-(3,5-ditrifluoromethylbenzoyl)-N-(quinolin-3-ylmethyl)-4-piperidinamine;
(2R,4S)-2-benzyl-1-(3,5-ditrifluoromethylbenzoyl)-N-(2-chlorobenzyl)-4-piperidinamine;
(2S,4S)-2-benzyl-1-(3,5-ditrifluoromethylbenzoyl)-N-(2-chlorobenzyl)-4-piperidinamine;
(2R,4S)-2-benzyl-1-(3,5-ditrifluoromethylbenzoyl)-N-[2-(2-chlorophenyl)ethyl]-4-piperidinamine;
(2S,4S)-2-benzyl-1-(3,5-ditrifluoromethylbenzoyl)-N-[2-(2-chlorophenyl)ethyl]-4-piperidinamine;
(2R,4S)-2-benzyl-1-(3,5-ditrifluoromethylbenzoyl)-N-[3-(2-chlorophenyl)propyl]-4-piperidinamine;
(2S,4S)-2-benzyl-1-(3,5-ditrifluoromethylbenzoyl)-N-[3-(2-chlorophenyl)propyl]-4-piperidinamine;
(2R,4S)-2-benzyl-1-(3,5-ditrifluoromethylbenzoyl)-N-(3-chlorobenzyl)-4-piperidinamine;
(2S,4S)-2-benzyl-1-(3,5-ditrifluoromethylbenzoyl)-N-(3-chlorobenzyl)-4-piperidinamine;
(2R,4S)-2-benzyl-1-(3,5-ditrifluoromethylbenzoyl)-N-[2-(3-chlorophenyl)ethyl]-4-piperidinamine;
(2S,4S)-2-benzyl-1-(3,5-ditrifluoromethylbenzoyl)-N-[2-(3-chlorophenyl)ethyl]-4-piperidinamine;
(2R,4S)-2-benzyl-1-(3,5-ditrifluoromethylbenzoyl)-N-[3-(3-chlorophenyl)propyl]-4-piperidinamine;
(2S,4S)-2-benzyl-1-(3,5-ditrifluoromethylbenzoyl)-N-[3-(3-chlorophenyl)propyl]-4-piperidinamine;
(2R,4S)-2-benzyl-1-(3,5-ditrifluoromethylbenzoyl)-N-(4-chlorobenzyl)-4-piperidinamine;
(2S,4S)-2-benzyl-1-(3,5-ditrifluoromethylbenzoyl)-N-(4-chlorobenzyl)-4-piperidinamine;
(2R,4S)-2-benzyl-1-(3,5-ditrifluoromethylbenzoyl)-N-[2-(4-chlorophenyl)ethyl]-4-piperidinamine;
(2S,4S)-2-benzyl-1-(3,5-ditrifluoromethylbenzoyl)-N-[2-(4-chlorophenyl)ethyl]-4-piperidinamine;
(2R,4S)-2-benzyl-1-(3,5-ditrifluoromethylbenzoyl)-N-[3-(4-chlorophenyl)propyl]-4-piperidinamine;
(2S,4S)-2-benzyl-1-(3,5-ditrifluoromethylbenzoyl)-N-[3-(4-chlorophenyl)propyl]-4-piperidinamine;
(2R,4S)-2-benzyl-1-(3,5-ditrifluoromethylbenzoyl)-N-(4-methoxynaphth-1-ylmethyl)-4-piperidinamine;
(2S,4S)-2-benzyl-1-(3,5-ditrifluoromethylbenzoyl)-N-(4-methoxynaphth-1-ylmethyl)-4-piperidinamine;
(2R,4S)-2-benzyl-1-(3,5-ditrifluoromethylbenzoyl)-N-(3,4-ethylenedioxybenzyl)-4-piperidinamine;
(2S,4S)-2-benzyl-1-(3,5-ditrifluoromethylbenzoyl)-N-(3,4-ethylenedioxybenzyl)-4-piperidinamine;
(2R,4S)-2-benzyl-1-(3,5-dichlorobenzoyl)-N-(indol-2-ylmethyl)-4-piperidinamine;
(2S,4S)-2-benzyl-1-(3,5-dichlorobenzoyl)-N-(indol-2-ylmethyl)-4-piperidinamine;
(2R,4S)-2-benzyl-1-(3,5-dichlorobenzoyl)-N-(indol-3-ylmethyl)-4-piperidinamine;

(2S,4S)-2-benzyl-1-(3,5-dichlorobenzoyl)-N-(indol-3-ylmethyl)-4-piperidinamine;
(2R,4S)-2-benzyl-1-(3,5-dichlorobenzoyl)-N-(quinolin-2-ylmethyl)-4-piperidinamine;
(2S,4S)-2-benzyl-1-(3,5-dichlorobenzoyl)-N-(quinolin-2-ylmethyl)-4-piperidinamine;
(2R,4S)-2-benzyl-1-(3,5-dichlorobenzoyl)-N-(quinolin-3-ylmethyl)-4-piperidinamine;
(2S,4S)-2-benzyl-1-(3,5-dichlorobenzoyl)-N-(quinolin-3-ylmethyl)-4-piperidinamine;
(2R,4S)-2-benzyl-1-(3,5-dichlorobenzoyl)-N-(2-chlorobenzyl)-4-piperidinamine;
(2S,4S)-2-benzyl-1-(3,5-dichlorobenzoyl)-N-(2-chlorobenzyl)-4-piperidinamine;
(2R,4S)-2-benzyl-1-(3,5-dichlorobenzoyl)-N-[2-(2-chlorophenyl)ethyl]-4-piperidinamine;
(2S,4S)-2-benzyl-1-(3,5-dichlorobenzoyl)-N-[2-(2-chlorophenyl)ethyl]-4-piperidinamine;
(2R,4S)-2-benzyl-1-(3,5-dichlorobenzoyl)-N-[3-(2-chlorophenyl)propyl]-4-piperidinamine;
(2S,4S)-2-benzyl-1-(3,5-dichlorobenzoyl)-N-[3-(2-chlorophenyl)propyl]-4-piperidinamine;
(2R,4S)-2-benzyl-1-(3,5-dichlorobenzoyl)-N-(3-chlorobenzyl)-4-piperidinamine;
(2S,4S)-2-benzyl-1-(3,5-dichlorobenzoyl)-N-(3-chlorobenzyl)-4-piperidinamine;
(2R,4S)-2-benzyl-1-(3,5-dichlorobenzoyl)-N-[2-(3-chlorophenyl)ethyl]-4-piperidinamine;
(2S,4S)-2-benzyl-1-(3,5-dichlorobenzoyl)-N-[2-(3-chlorophenyl)ethyl]-4-piperidinamine;
(2R,4S)-2-benzyl-1-(3,5-dichlorobenzoyl)-N-[3-(3-chlorophenyl)propyl]-4-piperidinamine;
(2S,4S)-2-benzyl-1-(3,5-dichlorobenzoyl)-N-[3-(3-chlorophenyl)propyl]-4-piperidinamine;
(2R,4S)-2-benzyl-1-(3,5-dichlorobenzoyl)-N-(4-chlorobenzyl)-4-piperidinamine;
(2S,4S)-2-benzyl-1-(3,5-dichlorobenzoyl)-N-(4-chlorobenzyl)-4-piperidinamine;
(2R,4S)-2-benzyl-1-(3,5-dichlorobenzoyl)-N-[2-(4-chlorophenyl)ethyl]-4-piperidinamine;
(2S,4S)-2-benzyl-1-(3,5-dichlorobenzoyl)-N-[2-(4-chlorophenyl)ethyl]-4-piperidinamine;
(2R,4S)-2-benzyl-1-(3,5-dichlorobenzoyl)-N-[3-(4-chlorophenyl)propyl]-4-piperidinamine;
(2S,4S)-2-benzyl-1-(3,5-dichlorobenzoyl)-N-[3-(4-chlorophenyl)propyl]-4-piperidinamine;
(2R,4S)-2-benzyl-1-(3,5-dichlorobenzoyl)-N-(4-methoxynaphth-1-ylmethyl)-4-piperidinamine;
(2S,4S)-2-benzyl-1-(3,5-dichlorobenzoyl)-N-(4-methoxynaphth-1-ylmethyl)-4-piperidinamine;
(2R,4S)-2-benzyl-1-(3,5-dichlorobenzoyl)-N-(3,4-ethylenedioxybenzyl)-4-piperidinamine;
(2S,4S)-2-benzyl-1-(3,5-dichlorobenzoyl)-N-(3,4-ethylenedioxybenzyl)-4-piperidinamine;
(2R,4S)-2-benzyl-1-(3,5-dimethoxybenzoyl)-N-(indol-2-ylmethyl)-4-piperidinamine;
(2S,4S)-2-benzyl-1-(3,5-dimethoxybenzoyl)-N-(indol-2-ylmethyl)-4-piperidinamine;
(2R,4S)-2-benzyl-1-(3,5-dimethoxybenzoyl)-N-(indol-3-ylmethyl)-4-piperidinamine;
(2S,4S)-2-benzyl-1-(3,5-dimethoxybenzoyl)-N-(indol-3-ylmethyl)-4-piperidinamine;
(2R,4S)-2-benzyl-1-(3,5-dimethoxybenzoyl)-N-(quinolin-2-ylmethyl)-4-piperidinamine;
(2S,4S)-2-benzyl-1-(3,5-dimethoxybenzoyl)-N-(quinolin-2-ylmethyl)-4-piperidinamine;
(2R,4S)-2-benzyl-1-(3,5-dimethoxybenzoyl)-N-(quinolin-3-ylmethyl)-4-piperidinamine;
(2S,4S)-2-benzyl-1-(3,5-dimethoxybenzoyl)-N-(quinolin-3-ylmethyl)-4-piperidinamine;
(2R,4S)-2-benzyl-1-(3,5-dimethoxybenzoyl)-N-(2-chlorobenzyl)-4-piperidinamine;
(2S,4S)-2-benzyl-1-(3,5-dimethoxybenzoyl)-N-(2-chlorobenzyl)-4-piperidinamine;
(2R,4S)-2-benzyl-1-(3,5-dimethoxybenzoyl)-N-[2-(2-chlorophenyl)ethyl]-4-piperidinamine;
(2S,4S)-2-benzyl-1-(3,5-dimethoxybenzoyl)-N-[2-(2-chlorophenyl)ethyl]-4-piperidinamine;
(2R,4S)-2-benzyl-1-(3,5-dimethoxybenzoyl)-N-[3-(2-chlorophenyl)propyl]-4-piperidinamine;
(2S,4S)-2-benzyl-1-(3,5-dimethoxybenzoyl)-N-[3-(2-chlorophenyl)propyl]-4-piperidinamine;
(2R,4S)-2-benzyl-1-(3,5-dimethoxybenzoyl)-N-(3-chlorobenzyl)-4-piperidinamine;
(2S,4S)-2-benzyl-1-(3,5-dimethoxybenzoyl)-N-(3-chlorobenzyl)-4-piperidinamine;
(2R,4S)-2-benzyl-1-(3,5-dimethoxybenzoyl)-N-[2-(3-chlorophenyl)ethyl]-4-piperidinamine;
(2S,4S)-2-benzyl-1-(3,5-dimethoxybenzoyl)-N-[2-(3-chlorophenyl)ethyl]-4-piperidinamine;
(2R,4S)-2-benzyl-1-(3,5-dimethoxybenzoyl)-N-[3-(3-chlorophenyl)propyl]-4-piperidinamine;
(2S,4S)-2-benzyl-1-(3,5-dimethoxybenzoyl)-N-[3-(3-chlorophenyl)propyl]-4-piperidinamine;
(2R,4S)-2-benzyl-1-(3,5-dimethoxybenzoyl)-N-(4-chlorobenzyl)-4-piperidinamine;
(2S,4S)-2-benzyl-1-(3,5-dimethoxybenzoyl)-N-(4-chlorobenzyl)-4-piperidinamine;
(2R,4S)-2-benzyl-1-(3,5-dimethoxybenzoyl)-N-[2-(4-chlorophenyl)ethyl]-4-piperidinamine;
(2S,4S)-2-benzyl-1-(3,5-dimethoxybenzoyl)-N-[2-(4-chlorophenyl)ethyl]-4-piperidinamine;
(2R,4S)-2-benzyl-1-(3,5-dimethoxybenzoyl)-N-[3-(4-chlorophenyl)propyl]-4-piperidinamine;
(2S,4S)-2-benzyl-1-(3,5-dimethoxybenzoyl)-N-[3-(4-chlorophenyl)propyl]-4-piperidinamine;
(2R,4S)-2-benzyl-1-(3,5-dimethoxybenzoyl)-N-(4-methoxynaphth-1-ylmethyl)-4-piperidinamine;
(2S,4S)-2-benzyl-1-(3,5-dimethoxybenzoyl)-N-(4-methoxynaphth-1-ylmethyl)-4-piperidinamine;
(2R,4S)-2-benzyl-1-(3,5-dimethoxybenzoyl)-N-(3,4-ethylenedioxybenzyl)-4-piperidinamine or
(2S,4S)-2-benzyl-1-(3,5-dimethoxybenzoyl)-N-(3,4-ethylenedioxybenzyl)-4-piperidinamine or a salt thereof in each case.

**50.** Process according to claim 1, which comprises preparing (2R,4S)- or (2R,4R)-2-benzyl-1-(3,5-dimethylbenzoyl)-N-(2-phenethyl)-4-piperidinamine or a salt thereof.

**51.** Process according to claim 1, which comprises preparing (2R,4S)-2-benzyl-1-(3,5-bis(trifluoromethyl)-benzoyl)-N-(4-quinolylmethyl)-4-piperidinamine or a salt thereof.

**52.** Process according to claim 1, which comprises preparing (2R,4S)-2-benzyl-1-(3,5-dichlorobenzoyl)-N-(4-quinolylmethyl)-4-piperidinamine or a salt thereof.

**53.** Process according to claim 1, which comprises preparing (2R*,4S*)-2-benzyl-1-(2,4-dichlorobenzoyl)-N-(4-quinolylmethyl)-4-piperidinamine or a salt thereof.

**54.** Process according to claim 1, which comprises preparing (2R*,4S*)-2-benzyl-1-(phenylacetyl)-N-(4-quinolylmethyl)-4-piperidinamine or a salt thereof.

**55.** Process according to claim 1, which comprises preparing (2R*,4S*)-2-benzyl-1-(2,6-dichlorobenzoyl)-N-(4-quinolylmethyl)-4-piperidinamine or a salt thereof.

**56.** Process according to claim 1, which comprises preparing (2R*,4S*)-2-benzyl-1-(3,5-dibromobenzoyl)-N-(4-quinolylmethyl)-4-piperidinamine or a salt thereof.

**57.** Process according to claim 1, which comprises preparing (2R*,4S*)-2-benzyl-1-(9-fluorenoyl)-N-(4-quinolylmethyl)-4-piperidinamine or a salt thereof.

**58.** Process according to claim 1, which comprises preparing (2R*,4S*)-2-benzyl-1-(3-toluoyl)-N-(4-quinolylmethyl)-4-piperidinamine or a salt thereof.

**59.** Process according to claim 1, which comprises preparing (2R*,4S*)-2-benzyl-1-(3-bromobenzoyl)-N-(4-quinolylmethyl)-4-piperidinamine or a salt thereof.

**60.** Process according to claim 1, which comprises preparing (2R*,4S*)-2-benzyl-1-(3,5-dihydroxybenzoyl)-N-(4-quinolylmethyl)-4-piperidinamine or a salt thereof.

**61.** Process according to claim 1, which comprises preparing (2R*,4S*)-2-benzyl-1-(3-cyanobenzoyl)-N-(4-quinolylmethyl)-4-piperidinamine or a salt thereof.

**62.** Process according to claim 1, which comprises preparing (2R*,4S*)-2-benzyl-1-(2-chlorobenzoyl)-N-(4-quinolylmethyl)-4-piperidinamine or a salt thereof.

**63.** Process according to claim 1, which comprises preparing (2R*,4S*)-2-benzyl-1-(4-chlorobenzoyl)-N-(4-quinolylmethyl)-4-piperidinamine or a salt thereof.

**64.** Process according to claim 1, which comprises preparing (2R*,4S*)-2-benzyl-1-(3,5-dichlorobenzoyl)-N-(4-quinolylmethyl)-N-methyl-4-piperidinamine or a salt thereof.

**65.** Process according to claim 1, which comprises preparing (2R*,4S*)-2-benzyl-1-(3,5-dichlorobenzoyl)-N-(4-quinolylmethyl)-N-cyclohexylcarbamoyl-4-piperidinamine or a salt thereof.

**66.** Process according to claim 1, which comprises preparing (2R*,4S*)-2-benzyl-1-(3,5-dichlorobenzoyl)-N-(4-quinolylmethyl)-N-phenylcarbamoyl-4-piperidinamine or a salt thereof.

**67.** Process according to claim 1, which comprises preparing (2R*,4S*)-2-benzyl-1-(3,5-dichlorobenzoyl)-N-(2-phenylethyl)-4-piperidinamine or a salt thereof.

**68.** Process according to claim 1, which comprises preparing (2R*,4S*)-2-benzyl-1-(3,5-bis(trifluoromethyl)-benzoyl)-N-(2-phenylethyl)-4-piperidinamine or a salt thereof.

**69.** Process according to claim 1, which comprises preparing (2R*,4S*)-2-benzyl-1-(3,5-dichlorobenzoyl)-N-(2-naphthoyl)-4-piperidinamine or a salt thereof.

**70.** Process according to claim 1, which comprises preparing (2R*,4S*)-2-benzyl-1-(3,5-dichlorobenzoyl)-N-(3,5-dimethylbenzoyl)-4-piperidinamine or a salt thereof.

**71.** Process according to claim 1, which comprises preparing (2R*,4S*)-2-benzyl-1-(3,5-dichlorobenzoyl)-N-(4-quinolylcarbonyl)-4-piperidinamine or a salt thereof.

**72.** Process according to claim 1, which comprises preparing (2R*,4S*)-2-benzyl-1-(3,5-dichlorobenzoyl)-N-(3-indolylcarbonyl)-4-piperidinamine or a salt thereof.

**73.** Process according to claim 1, which comprises preparing (2R*,4S*)-2-benzyl-1-(3,5-dichlorobenzoyl)-N-(2-indolylcarbonyl)-4-piperidinamine or a salt thereof.

**74.** Process according to claim 1, which comprises preparing (2R*,4S*)-2-benzyl-1-(3,5-dichlorobenzoyl)-N-(5-methoxy-2-indolylcarbonyl)-4-piperidinamine or a salt thereof.

**75.** Process according to claim 1, which comprises preparing (2R*,4S*)-2-benzyl-1-(3,5-dichlorobenzoyl)-N-(1-naphthoyl)-4-piperidinamine or a salt thereof.

**76.** Process according to claim 1, which comprises preparing (2R*,4S*)-2-benzyl-1-(3,5-dichlorobenzoyl)-N-(phenylacetyl)-4-piperidinamine or a salt thereof.

**77.** Process according to claim 1, which comprises preparing (2R*,4S*)-2-benzyl-1-(3,5-dichlorobenzoyl)-N-(2-methoxybenzyl)-4-piperidinamine or a salt thereof.

**78.** Process according to claim 1, which comprises preparing (2R*,4S*)-2-benzyl-1-(3,5-dichlorobenzoyl)-N-(3-(N-acetyl)indolylmethyl)-4-piperidinamine or a salt thereof.

**79.** Process according to claim 1, which comprises preparing (2R*,4S*)-2-benzyl-1-(3,5-dichlorobenzoyl)-N-(2-benzo[b]furanylmethyl)-4-piperidinamine or a salt thereof.

**80.** Process according to claim 1, which comprises preparing (2R*,4S*)-2-benzyl-1-(3,5-dichlorobenzoyl)-N-[(3-methylbenzo[b]thiophen-2-ylmethyl]-4-piperidinamine or a salt thereof.

**81.** Process according to claim 1, which comprises preparing (2R*,4S*)-2-benzyl-1-(3,5-dichlorobenzoyl)-N-(5-methoxyindol-3-ylmethyl)-4-piperidinamine or a salt thereof.

**82.** Process according to claim 1, which comprises preparing (2R*,4S*)-2-benzyl-1-(3,5-dichlorobenzoyl)-N-(3-indolylmethyl)-4-piperidinamine or a salt thereof.

**83.** Process according to claim 1, which comprises preparing (2R*,4S*)-2-benzyl-1-(3,5-dichlorobenzoyl)-N-phenylcarbamoyl-4-piperidinamine or a salt thereof.

**84.** Process according to claim 1, which comprises preparing (2R*,4S*)-2-benzyl-1-(3,5-dichlorobenzoyl)-N-diphenylmethyl-4-piperidinamine or a salt thereof.

**85.** Process according to claim 1, which comprises preparing (2R*,4S*)-2-benzyl-1-(3,5-dichlorobenzoyl)-N-(3,4-dihydro-2H-1-benzopyran-2-carbonyl)-4-piperidinamine or a salt thereof.

**86.** Process according to claim 1, which comprises preparing (2R*,4S*)-2-benzyl-1-(4-methoxybenzoyl)-N-(4-quinolylmethyl)-4-piperidinamine or a salt thereof.

**87.** Process according to claim 1, which comprises preparing (2R*,4S*, 1'R*)-N-benzyl-1-(3,5-dimethylbenzoyl)-2-(1'-hydroxy-1'-phenylmethyl)-4-piperidinamine or a salt thereof.

**88.** Process according to claim 1, which comprises preparing (2R*,4S*,1'R*)-2-(1'-hydroxy-1'-phenylmethyl)-1-(3,5-dimethylbenzoyl)-N-(4-quinolinylmethyl)-4-piperidinamine or a salt thereof.

**89.** Process according to claim 1, which comprises preparing (2R*,4S*,1'S*)-1-(3,5-dimethylbenzoyl)-2-(1'-hydroxy-1'-phenyl-methyl)-N-(4-quinolinylmethyl)-4-piperidinamine or a salt thereof.

**90.** Process according to claim 1, which comprises preparing (2R*,4S*,1'R*)-2-{1'-hydroxy-1'-(4-chlorophenyl)methyl}-1-(3,5-dimethylbenzoyl)-N-(4-quinolinylmethyl)-4-piperidinamine or a salt thereof.

**91.** Process according to claim 1, which comprises preparing (2R*,4S*,1'S*)-1-(3,5-dimethylbenzoyl)-2-{1'-hydroxy-1'-(4-chlorophenyl)methyl}-N-(4-quinolinylmethyl)-4-piperidinamine or a salt thereof.

**92.** Process according to claim 1, which comprises preparing (2R*,4S*,1'S*)-1-(3,5-dimethylbenzoyl)-2-{1'-hydroxy-1'-(3,4-dichlorophenyl)-methyl}-N-(4-quinolinylmethyl)-4-piperidinamine or a salt thereof.

**93.** Process according to claim 1, which comprises preparing (2R*,4S*)-N-benzyl-1-(3,5-dimethylbenzoyl)-2-benzoyl-4-piperidinamine or a salt thereof.

**94.** Process according to claim 1, which comprises preparing (2R*,4S*)-2-(4-chlorobenzyl)-1-(3,5-dimethylbenzoyl)-N-(4-quinolinylmethyl)-4-piperidinamine or a salt thereof.

**95.** Process according to claim 1, which comprises preparing (2R*,4S*)-2-(3,4-dichlorobenzyl)-1-(3,5-dimethylbenzoyl)-N-(4-quinolinylmethyl)-4-piperidinamine or a salt thereof.

**96.** Process according to claim 1, which comprises preparing (2R*,4S*)-1-(3,5-dimethylbenzoyl)-2-phenyl-N-(4-quinolinylmethyl)-4-piperidinamine or a salt thereof.

**97.** Process according to claim 1, which comprises preparing (2R*,4S*)-1-(3,5-dichlorobenzoyl)-2-phenyl-N-(4-quinolinemethyl)-4-piperidinamine or a salt thereof.

**98.** Process according to claim 1, which comprises preparing (2R*,4S*)-1-(1-naphthoyl)-2-phenyl-N-(4-quinolinylmethyl)-4-piperidinamine.

**99.** Process according to claim 1, which comprises preparing (2R*,4S*)-1-(3,5-dimethylbenzoyl)-2-(1-naphthyl)-N-(4-quinolinylmethyl)-4-piperidinamine or a salt thereof.

**100.** Process according to claim 1, which comprises preparing (2R*,4S*)-1-(3,5-dimethylbenzoyl)-2-(2-naphthyl)-N-(4-quinolinylmethyl)-4-piperidinamine;
(2R*,4S*)-1-(3,5-dimethylbenzoyl)-2-(4-methoxyphenylmethyl)-N-(4-quinolinylmethyl)-4-piperidinamine;
(2R*,4S*)-1-(3,5-dimethylbenzoyl)-2-(3-methoxyphenylmethyl)-N-(4-quinolinylmethyl)-4-piperidinamine;
(2R*,4S*)-1-(3,5-dimethylbenzoyl)-2-(4-nitrobenzyl)-N-(4-quinolinylmethyl)-4-piperidinamine;
(2R*,4S*)-1-(3,5-dimethylbenzoyl)-2-(4-trifluoromethylphenylmethyl)-N-(4-quinolinylmethyl)-4-piperidinamine;
(2R*,4S*)-1-(3,5-dimethylbenzoyl)-2-(2,4-dichlorophenylmethyl)-N-(4-quinolinylmethyl)-4-piperidinamine;
(2R*,4S*)-1-(3,5-dimethylbenzoyl)-2-(2-phenylethyl)-N-(4-quinolinylmethyl)-4-piperidinamine;
(2R*,4S*)-1-(3,5-dimethylbenzoyl)-2-(2-phenylethenyl)-N-(4-quinolinylmethyl)-4-piperidinamine;
(2R*,4S*)-1-(3,5-dimethylbenzoyl)-2-benzoyl-N-(4-quinolinylmethyl)-4-piperidinamine;
(2R*,4S*)-1-(3,5-dimethylbenzoyl)-2-(4-chlorobenzoyl)-N-(4-quinolinylmethyl)-4-piperidinamine;
(2R*,4S*)-1-(3,5-dimethylbenzoyl)-2-(2-naphthyl)-N-(4-quinolinylmethyl)-4-piperidinamine;
(2R*,4S*)-1-(3,5-dimethylbenzoyl)-2-(4-methoxybenzyl)-N-(4-quinolinylmethyl)-4-piperidinamine;
(2R*,4S*)-1-(3,5-dimethylbenzoyl)-2-(3-methoxybenzyl)-(4-quinolinylmethyl)-4-piperidinamine;
(2R*,4S*)-1-(3,5-dimethylbenzoyl)-2-(4-nitrobenzyl)-(4-quinolinylmethyl)-4-piperidinamine;
(2R*,4S*)-1-(3,5-dimethylbenzoyl)-2-(4-trifluoromethylbenzyl)-(4-quinolinylmethyl)-4-piperidinamine;
(2R*,4S*)-1-(3,5-dimethylbenzoyl)-2-(2,4-dichlorobenzyl)-(4-quinolinylmethyl)-4-piperidinamine;
(2R*,4S*)-1-(3,5-dimethylbenzoyl)-2-(2-phenylethyl)-(4-quinolinylmethyl)-4-piperidinamine;
(2R*,4S*)-1-(3,5-dimethylbenzoyl)-2-(2-phenylethenyl)-N-(4-quinolinylmethyl)-4-piperidinamine;
(2R*,4S*)-1-(3,5-dimethylbenzoyl)-2-(benzoylmethyl)-N-(4-quinolinylmethyl)-4-piperidinamine or
(2R*,4S*)-1-(3,5-dimethylbenzoyl)-2-(4-chlorobenzoylmethyl)-N-(4-quinolinylmethyl)-4-piperidinamine or

a salt thereof in each case.

**101.** A process for the preparation of novel 1-acylpiperidones of the formula X

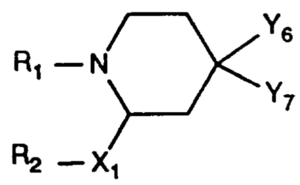

(X),

wherein $R_1$ is an optionally substituted aroyl, heteroaroyl, cycloalkylcarbonyl, aralkanoyl, heteroarylalkanoyl, aralkoxycarbonyl or arylcarbamoyl radical or the acyl radical of an α-amino acid which is optionally N-substituted by lower alkanoyl or carbamoyl-lower-alkanoyl, $R_2$ is cycloalkyl or an optionally substituted aryl or heteroaryl radical, $X_1$ denotes hydroxymethylene and $Y_6$ and $Y_7$ together represent oxo, and salts thereof, characterised in that a compound of the formula Xa

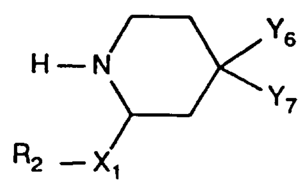

(Xa),

is condensed with an agent which introduces the radical $R_1$ and, if required, a resulting compound is converted into another compound of the formula I, a mixture of isomers which can be obtained according to the process is resolved into the components, and the isomer preferred in each case is separated off and/or a free compound which can be obtained according to the process is converted into a salt, or a salt which can be obtained according to the process is converted into the corresponding free compound.

**102.** A process according to claim 101 for the preparation of compounds of the formula X wherein $R_1$ denotes benzoyl, benzoyl mono- or disubstituted by $C_1$-$C_4$alkyl, $C_1$-$C_4$alkoxy, halogen of atomic number up to and including 35 and/or trifluoromethyl, or unsubstituted naphthoyl, $R_2$ represents phenyl or phenyl mono- or disubstituted by halogen of atomic number up to and including 35 and/or trifluoromethyl, $X_1$ denotes hydroxymethylene and $Y_6$ and $Y_7$ together represent oxo, and salts thereof.

**103.** A process according to claim 101, which comprises preparing (2R*,1'R*)-1-(3,5-dimethylbenzoyl)-2-(1'-hydroxy-1'-phenyl-methyl)-4-piperidone or a salt thereof.

**104.** A process according to claim 101, which comprises preparing (2R*,1'R*)-1-(3,5-bistrifluoromethylbenzoyl)-2-{1'-hydroxy-1'-(4-chlorophenyl)methyl}-4-piperidone or a salt thereof.

**105.** A process according to claim 101, which comprises preparing (2R*,1'R*)-1-(3,5-dimethylbenzoyl)-2-{1'-hydroxy-1'-(3,4-dichlorophenyl)methyl}-4-piperidone or a salt thereof.

**106.** A process according to claim 101, which comprises preparing (2R*,1'S*)-1-(3,5-bistrifluoromethylbenzoyl)-2-{1'-hydroxy-1'-(4-chlorophenyl)methyl}-4-piperidone or a salt thereof.

**107.** A process according to claim 101, which comprises preparing (2R*,1'R*)-1-(3,5-bistrifluoromethylbenzoyl)-2-{1'-hydroxy-1'-(3,4-dichlorophenyl)methyl}-4-piperidone or a salt thereof.

**108.** A process according to claim 101, which comprises preparing (2R*,1'R*)-1-(3,5-dimethylbenzoyl)-2-{1'-hydroxy-1'-(4-methoxy-phenyl)methyl}-4-piperidone or a salt thereof.

**109.** A process according to claim 101, which comprises preparing (2R*,1'S*)-1-(3,5-dimethylbenzoyl)-2-{1'-hydroxy-1'-(4-methoxyphenyl)methyl}-4-piperidone or a salt thereof.

**110.** A process according to claim 101, which comprises preparing (2R*,1'R*)-1-(3,5-dimethylbenzoyl)-2-{1'-hydroxy-1'-(3-methoxyphenyl)methyl}-4-piperidone or a salt thereof.

**111.** A process according to claim 101, which comprises preparing (2R*,1'S*)-1-(3,5-dimethylbenzoyl)-2-{1'-hydroxy-1'-(3-methoxyphenyl)methyl}-4-piperidone or a salt thereof.

**112.** A process according to claim 101, which comprises preparing (2R*,1'R*)-1-(3,5-dimethylbenzoyl)-2-{1'-hydroxy-1'-(4-trifluoromethylphenyl)methyl}-4-piperidone or a salt thereof.

**113.** A process according to claim 101, which comprises preparing (2R*,1'S*)-1-(3,5-dimethylbenzoyl)-2-{1'-hydroxy-1'-(4-trifluoromethylphenyl)methyl}-4-piperidone or a salt thereof.

**114.** A process according to claim 101, which comprises preparing (2R*,1'R*)-1-(3,5-dimethylbenzoyl)-2-{1'-hydroxy-1'-(4-chloro-3-trifluoromethylphenyl)methyl}-4-piperidone or a salt thereof.

**115.** A process according to claim 101, which comprises preparing (2R*,1'S*)-1-(3,5-dimethylbenzoyl)-2-{1'-hydroxy-1'-(4-chloro-3-trifluoromethylphenyl)methyl}-4-piperidone or a salt thereof.

**116.** A process for the preparation of a pharmaceutical composition, characterised in that a 1-acylpiperidine compound of the formula I or X

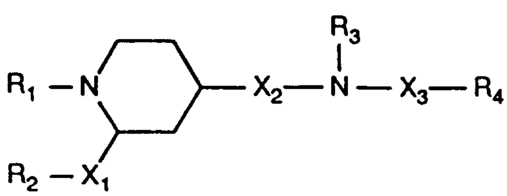 (I) or 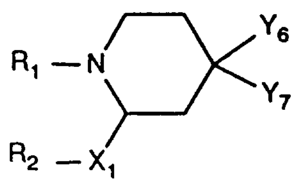 (X),

in which $R_1$ in formula I is an optionally substituted aralkyl, heteroalkyl, aroyl, heteroaroyl, cycloalkylcarbonyl, aralkanoyl, heteroarylalkanoyl, aralkoxycarbonyl or arylcarbamoyl radical or the acyl radical of an $\alpha$-amino acid which is optionally N-substituted by lower alkanoyl or carbamoyl-lower-alkanoyl, $R_2$ is cycloalkyl or an optionally substituted aryl or heteroaryl radical or in formula X is an optionally substituted aroyl, heteroaroyl, cycloalkylcarbonyl, aralkanoyl, heteroarylalkanoyl, aralkoxycarbonyl or arylcarbamoyl radical or the acyl radical of an $\alpha$-amino acid which is optionally N-substituted by lower alkanoyl or carbamoyl-lower-alkanoyl, $R_3$ is hydrogen, alkyl, carbamoyl or an alkanoyl or alkenoyl radical which is optionally substituted by carboxyl or esterified or amidated carboxyl, $R_4$ is an optionally substituted aryl or optionally partially hydrogenated heteroaryl radical, $X_1$ in formula I is methylene, ethylene, a direct linkage, an optionally ketalised carbonyl group or an optionally etherified hydroxymethylene group or in formula X is hydroxymethylene, $X_2$ is alkylene, carbonyl or a direct linkage, and $X_3$ is carbonyl, oxo-lower-alkylene, oxo(aza)-lower-alkylene or an alkylene radical which is optionally substituted by phenyl, hydroxymethyl, optionally esterified or amidated carboxyl or, in higher than the a position, by hydroxyl, and $Y_6$ and $Y_7$ together represent oxo, or a pharmaceutically utilisable salt thereof, is mixed with customary pharmaceutical ancillary substances.

**Revendications**

**Revendications pour les Etats contractants suivants : AT, BE, CH, DE, DK, FR, GB, IE, IT, LI, LU, NL, SE**

**1.** Un composé 1-acylpipéridine de formule I

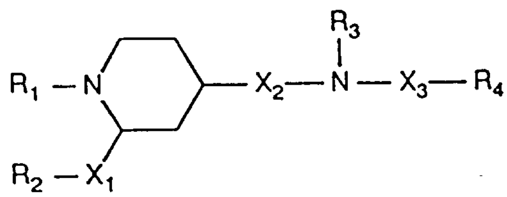

(I)

où $R_1$ représente un phénylalkyle en $C_1$-$C_4$ ou un diphénylalkyle en $C_1$-$C_4$ non substitué ou substitué dans la partie phényle par un alkyle inférieur, un alcoxy inférieur, un dialkyle inférieur amino, un halogène et/ou un trifluorométhyle, un phénoxyalkyle en $C_1$-$C_4$ non substitué ou substitué dans la partie phényle par un halogène et/ou un triazolyle, un pyridylalkyle en $C_1$-$C_4$ ou un quinoléinylalkyle en $C_1$-$C_4$, un benzoyle non substitué ou substitué par un alkyle inférieur, un alcoxy inférieur, un dialkyle inférieur amino, un halogène et/ou le trifluorométhyle, un naphtoyle non substitué ou substitué par un alkyle inférieur, un alcoxy inférieur, un dialkyle inférieur amino, un halogène et/ou le trifluorométhyle, le pyridylcarbonyle ou le quinoléinylcarbonyle non substitué ou substitué par un alkyle inférieur, un alcoxy inférieur, un halogène et/ou le trifluorométhyle, un cycloalkylcarbonyle ayant 5 à 7 chaînons non substitué ou substitué par un alkyle inférieur, un alcoxy inférieur, un dialkyle inférieur amino, un halogène et/ou le trifluorométhyle, un phénylalcanoyle en $C_1$-$C_4$ ou un diphénylalcanoyle en $C_1$-$C_4$ non substitué ou substitué dans la partie phényle par un alkyle inférieur, un alcoxy inférieur, un dialkyle inférieur amino, un halogène et/ou le trifluorométhyle, un N-phénylcarbamoyle non substitué ou substitué dans la partie phényle éventuellement par un alkyle inférieur, un alcoxy inférieur, un dialkyle inférieur amino, un halogéne et/ou le trifluorométhyle ou un groupe de formule Ia

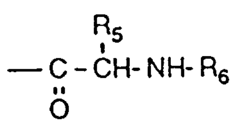

(Ia)

dans laquelle $R_5$ représente l'hydrogène, un alkyle en $C_1$-$C_4$ non substitué ou substitué par l'hydroxy, le mercapto, l'amino, un phényle éventuellement substitué par l'hydroxy, le carboxy, le carbamoyle ou l'uréido et $R_6$ représente un alcanoyle en $C_2$-$C_7$, $R_2$ représente un cycloalkyle ayant 5 à 7 chaînons ou un reste phényle, naphtyle ou pyridyle non substitué ou substitué par un alkyle en $C_1$-$C_4$, un alcoxy en $C_1$-$C_4$, un halogène et/ou le trifluorométhyle aromatiquement lié, $R_3$ représente l'hydrogène, un alkyle en $C_1$-$C_7$, le carbamoyle, un alcanoyle en $C_2$-$C_7$, un carboxyalcanoyle en $C_1$-$C_4$ ou un carboxyalcènoyle en $C_2$-$C_4$, $R_4$ représente un phényle ou un naphtyle non substitué ou substitué par un alkyle en $C_1$-$C_4$, un alcoxy en $C_1$-$C_4$, un halogène et/ou le trifluorométhyle ou le pyridyle non substitué, le benzofuranyle, l'indolyle, le 2,3-dihydroindolyle, le benzimidazolyle, le quinolyle ou le 1,2,3,4-tétrahydroquinoléinyle, $X_1$ représente le méthylène, l'hydroxyméthylène, un alcoxy en $C_1$-$C_4$ méthylène, le carbonyle ou un dialcoxy en $C_1$-$C_4$ méthylène ou une liaison directe, $X_2$ représente un alkylène en $C_1$-$C_7$, le carbonyle ou une liaison directe et $X_3$ représente le carbonyle, un alkylène en $C_1$-$C_4$, un carboxyalkylène en $C_1$-$C_4$, un alcoxy en $C_1$-$C_4$ carbonylalkylène en $C_1$-$C_4$, un carbamoylalkylène en $C_1$-$C_4$ ou un hydroxyméthylalkylène en $C_1$-$C_4$ ou l'un de ses sels, un alkyle inférieur étant un alkyle en $C_1$-$C_7$.

**2.** Un composé de formule I selon la revendication 1 où $R_1$ représente le benzoyle, le naphtoyle ou un phénylalcanoyle en $C_1$-$C_4$ non substitué ou substitué par un alkyle en $C_1$-$C_4$, un alcoxy en $C_1$-$C_4$, un halogène et/ou le trifluorométhyle, un pyridylcarbonyle ou un quinoléinylcarbonyle non substitué ou un groupe de formule Ia

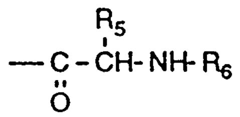

(Ia)

dans laquelle $R_5$ représente l'hydrogène, un alkyle en $C_1$-$C_4$ non substitué ou substitué par l'hydroxy, le mercapto, l'aminow un phényle éventuellement substitué par l'hydroxy, le carboxy, le carbamoyle ou l'uréido et $R_6$ représente un alcanoyle en $C_2$-$C_7$, $R_2$ représente un cycloalkyle ayant 5 à 7 chaînons ou un reste phényle, naphtyle ou pyridyle non substitué ou substitué par un alkyle en $C_1$-$C_4$, un alcoxy en $C_1$-$C_4$, un halogène et/ou le trifluorométhyle aromatiquement lié, $R_3$ représente l'hydrogène, un alkyle en $C_1$-$C_7$, un carbamoyle, un alcanoyle en $C_2$-$C_7$, un carboxyalcanoyle en $C_1$-$C_4$ ou un carboxyalcénoyle en $C_3$-$C_5$, $R_4$ représente un phényle ou un naphtyle non substitué ou substitué par un alkyle en $C_1$-$C_4$, un alcoxy en $C_1$-$C_4$, un halogène et/ou le trifluorométhyle ou le pyridyle, le benzofuranyle, l'indolyle, le benzimidazolyle ou le quinolyle non substitué, $X_1$ représente le méthylène, l'hydroxyméthylène, un alcoxy en $C_1$-$C_4$ méthylène, le carbonyle, un dialcoxy en $C_1$-$C_4$ méthylène ou une liaison directe, $X_2$ représente un alkylène en $C_1$-$C_7$, le carbonyle ou une liaison directe et $X_3$ représente un carbonyle, un alkylène en $C_1$-$C_4$, un carboxyalkylène en $C_1$-$C_4$, un alcoxy en $C_1$-$C_4$ carbonylalkylène en $C_1$-$C_4$, un carbamoylalkylène en $C_1$-$C_4$ ou un hydroxyméthylalkylène en $C_1$-$C_4$ ou l'un de ses sels.

3. Un composé de formule I selon la revendication 1 où $R_1$ représente un benzoyle non substitué ou mono- ou disubstitué par un alkyle en $C_1$-$C_4$, un alcoxy en $C_1$-$C_4$, un halogène ayant un numéro atomique allant jusqutà 35 et/ou le trifluorométhyle ou un naphoyle ou un phénylalcanoyle en $C_1$-$C_4$ non substitué, $R_2$ représente un phényle non substitué ou mono- ou disubstitué par un alkyle en $C_1$-$C_4$, un alcoxy en $C_1$-$C_4$, un halogène ayant un numéro atomique allant jusqu'à 35 et/ou le trifluorométhyle ou un pyridyle non substitué, $R_3$ représente l'hydrogène, un alkyle en $C_1$-$C_4$, un carbamoyle ou un alcanoyle en $C_2$-$C_7$, $R_4$ représente un phényle non substitué ou mono- ou disubstitué par un alkyle en $C_1$-$C_4$, un alcoxy en $C_1$-$C_4$, un halogène ayant un numéro atomique allant jusqu'à 35 et/ou le trifluorométhyle ou un naphtyle, pyridyle, benzofuranyle, indolyle, benzimidazolyle ou quinolyle non substitué, $X_1$ représente le méthylène, l'hydroxyméthylène, le carbonyle ou une liaison directe, $X_2$ représente une liaison directe et $X_3$ représente un alkylène en $C_1$-$C_4$ ou l'un de ses sels.

4. Un composé de formule I selon la revendication 1 où $R_1$ représente un benzoyle non substitué ou mono- ou disubstitué par un alkyle en $C_1$-$C_4$, un alcoxy en $C_1$-$C_4$, un halogéne ayant un numéro atomique allant jusqu'à 35 et/ou le trifluorométhyle ou un naphtoyle non substitué, $R_2$ représente un phényle non substitué ou mono- ou disubstitué par un halogène ayant un numéro atomique allant jusqu'à 35 et/ou le trifluorométhyle, $R_3$ représente l'hydrogène, $R_4$ représente un quinolyle non substitué, $X_1$ le méthylène, $X_2$ une liaison directe et $X_3$ un alkylène en $C_1$-$C_4$ ou l'un de ses sels.

5. La (2R*,4S*)-2-benzyl-1-(2-naphtoyl)-N-(4-quinolylméthyl)-4-pipéridineamine ou l'un de ses sels.

6. La (2R*,4S*)-2-benzyl-1-(3-trifluorométhylbenzoyl)-N-(4-quinolylméthyl)-4-pipéridineamine ou l'un de ses sels.

7. La (2R*,4S*)-2-benzyl-1-(3,5-bis-(trifluorométhyl)-benzoyl)-N-(4-quinolylméthyl)-4-pipéridineamine ou l'un de ses sels.

8. La (2R*,4S*)-2-benzyl-1-(3,5-diméthoxybenzoyl)-N-(4-quinolylméthyl)-4-pipéridineamine ou l'un de ses sels.

9. La (2R*,4S*)-2-benzyl-1-(1-naphtoyl)-N-(4-quinolylméthyl)-4-pipéridineamine ou l'un de ses sels.

10. La (2R*,4S*)-2-benzyl-1-(3,5-dichlorobenzoyl)-N-(4-quinolylméthyl)-4-pipéridineamine ou l'un de ses sels.

11. La (2R*,4S*)-2-benzyl-1-(2-quinoléinylcarbonyl)-N-(4-quinolylméthyl)-4-pipéridineamine ou l'un de ses sels.

**12.** La (2R*,4S*)-2-benzyl-1-(4-chlorophénylacétyl)-N-(4-quinolylméthyl)-4-pipéridineamine ou l'un de ses sels.

**13.** La (2R*,4S*)-2-benzyl-1-(benzyloxycarbonyl)-N-(4-quinolylméthyl)-4-pipéridineamine ou l'un de ses sels.

**14.** La (2R*,4S*)-2-benzyl-1-(2-phényléthyl)-N-(4-quinolylméthyl)-4-pipéridineamine ou l'un de ses sels.

**15.** La (2R*,4S*)-2-benzyl-1-(2-naphtylacétyl)-N-(4-quinolylméthyl)-4-pipéridineamine ou l'un de ses sels.

**16.** La (2R*,4S*)-2-benzyl-1-(4-quinolylméthyl)-N-(4-quinolylméthyl)-4-pipéridineamine ou l'un de ses sels.

**17.** La (2R*,4S*)-2-benzyl-1-(2,4-dichlorobenzyl)-N-[4-quinolylméthyl)-4-pipéridineamine ou l'un de ses sels.

**18.** La (2R*,4S*)-2-benzyl-1-[2,2-diphényléthyl)-N-(4-quinolylméthyl)-4-pipéridineamine ou l'un de ses sels.

**19.** La (2R*,4S*)-2-benzyl-1-[phénylcarbamoyl)-N-(4-quinolylméthyl)-4-pipéridineamine ou l'un de ses sels.

**20.** La (2R*,4S*)-2-benzyl-1-(diphénylacétyl)-N-(4-quinolylméthyl)-4-pipéridineamine ou l'un de ses sels.

**21.** La (2R*,4S*)-2-benzyl-1-(2-pyridylacétyl)-N-(4-quinolylméthyl)-4-pipéridineamine ou l'un de ses sels.

**22.** La (2R*,4S*)-2-benzyl-1-(4-pyridylacétyl)-N-[4-quinolylméthyl)-4-pipéridineamine ou l'un de ses sels.

**23.** La (2R*,4S*)-2-benzyl-1-(2,3-diphénylpropionyl)-N-(4-quinolylméthyl)-4-pipéridineamine ou l'un de ses sels.

**24.** La [2R*,4S*)-2-benzyl-1-((3S)-(2,3,4,9-tétrahydro-1H-pyrido[3,4-b]indol-3-yl)-carbonyl)-N-[4-quinolylméthyl)-4-pipéridineamine ou l'un de ses sels.

**25.** La (2R*,4S*)-2-benzyl-1-(3-méthoxybenzoyl)-N-(4-quinolylméthyl)-4-pipéridineamine ou l'un de ses sels.

**26.** La (2R*,4S*)-2-benzyl-1-(3-N,N-diméthylaminobenzoyl)-N-[4-quinolylméthyl)-4-pipéridineamine ou l'un de ses sels.

**27.** La (2R*,4S*)-2-benzyl-1-[cis,cis-3,5-diméthylcyclohexylcarbonyl)-N-(4-quinolylméthyl)-4-pipéridineamine ou l'un de ses sels.

**28.** La (2R*,4S*)-2-benzyl-1-(3,5-bis-(trifluorométhyl)-benzyl)-N-[4-quinolylméthyl)-4-pipéridineamine ou l'un de ses sels.

**29.** La (2S*,4R*)-2-benzyl-1-[2-(5-chloro-1H-1,2,4-triazol-1-yl)-phénoxyéthyl]-(4-quinolylméthyl]-4-pipéridineamine ou l'un de ses sels.

**30.** La (2R*,4S*)-2-benzyl-1-((S)-phénylalanyl)-N-(4-quinolylméthyl)-4-pipéridineamine ou l'un de ses sels.

**31.** La (2R*,4S*)-2-benzyl-1-((R)-phénylalanyl)-N-[4-quinolylméthyl)-4-pipéridineamine ou l'un de ses sels.

**32.** La (2R*,4S*)-2-benzyl-1-((S)-N-acétyl-phénylalanyl)-N-(4-quinolylméthyl)-4-pipéridineamine ou l'un de ses sels.

**33.** La (2R*,4S*)-2-benzyl-1-((R)-N-acétylphénylalanyl)-N-(4-quinolylméthyl)-4-pipéridineamine ou l'un de ses sels.

**34.** La (2R*,4S*)-2-benzyl-1-((S)-N-(4-carboxamidobutyroyl)-phénylalanyl)-N-(4-quinolylméthyl)-4-pipéridineamine ou l'un de ses sels.

**35.** La (2R*,4S*)-2-benzyl-1-((R)-N-(4-carboxamidobutyroyl)-phénylalanyl)-N-(4-quinolylméthyl)-4-pipéridineamine ou l'un de ses sels.

**36.** La (2R*,4S*)-2-benzyl-1-benzoyl-N-(4-quinolylméthyl)-4-pipéridineamine ou l'un de ses sels.

**37.** La (2R*,4S*)-2-benzyl-1-(3-chlorobenzoyl)-N-(4-quinolylméthyl)-4-pipèridineamine ou l'un de ses sels.

**38.** La (2R*,4S*)-2-benzyl-1-(3,5-dichlorobenzoyl)-N-(4-quinolylméthyl)-N-(3-carboxamidopropionyl)-4-pipéridineamine ou l'un de ses sels.

**39.** La (2R,4S)- ou la (2R,4R)-2-benzyl-1-(3,5-diméthylbenzoyl)-N-(4-quinolylméthyl)-4-pipéridineamine ou l'un de leurs sels.

**40.** La (2S,4R)- ou la (2S,4S)-2-benzyl-1-(3,5-diméthylbenzoyl)-N-(2-phénèthyl)-4-pipéridineamine ou l'un de leurs sels.

**41.** La (2R,4S)-2-benzyl-1-(3,5-diméthylbenzoyl)-N-(3-quinolylméthyl)-4-pipéridineamine ou l'un de ses sels.

**42.** La (2R,4S)-2-benzyl-1-(3,5-diméthylbenzoyl)-N-(2-quinolylméthyl)-4-pipéridineamine ou l'un de ses sels.

**43.** La (2R,4S)- ou la (2R,4R)-2-benzyl-1-(3,5-diméthylbenzoyl)-N-benzyl-4-pipéridineamine ou l'un de leurs sels.

**44.** La (2S,4R)- ou la (2S,4S)-2-benzyl-1-(3,5-diméthylbenzoyl)-N-benzyl-4-pipéridineamine ou l'un de leurs sels.

**45.** La (2R,4S)- ou la (2R,4R)-2-benzyl-1-(3,5-diméthylbenzoyl)-N-(4-pyridylméthyl)-4-pipéridineamine ou l'un de leurs sels.

**46.** La (2R,4S)- ou la (2R,4R)-2-benzyl-1-(3,5-diméthylbenzoyl)-N-(3-pyridylméthyl)-4-pipéridineamine ou l'un de leurs sels.

**47.** La (2S,4R)- et la (2S,4S)-2-benzyl-1-(3,5-diméthylbenzoyl)-N-(4-quinolylméthyl)-4-pipéridineamine ou l'un de leurs sels.

**48.** La (2R*,4S*)-2-benzyl-1-(3,5-diméthylbenzoyl)-N-benzyl-N-carbamoyl-4-pipéridineamine ou l'un de ses sels.

**49.** La (2R,4S)-2-benzyl-1-(3,5-diméthylbenzoyl)-N-(3-phénylpropyl)-4-pipéridineamine ;
la (2S,4S)-2-benzyl-1-(3,5-diméthylbenzoyl)-N-(3-phénylpropyl)-4-pipéridineamine ;
la (2R,4S)-2-benzyl-1-(3,5-diméthylbenzoyl)-N-(2-méthoxybenzyl)-4-pipéridineamine ;
la (2S,4S)-2-benzyl-1-(3,5-diméthylbenzoyl)-N-(2-méthoxybenzyl)-4-pipéridineamine ;
la (2R,4S)-2-benzyl-1-(3,5-diméthylbenzoyl)-N-[2-(2-methoxyphényl)-éthyl]-4-pipéridineamine ;
la (2S,4S)-2-benzyl-1-(3,5-diméthylbenzoyl)-N-[2-(2-méthoxyphényl)-éthyl]-4-pipéridineamine ;
la (2R,4S)-2-benzyl-1-(3,5-diméthylbenzoyl)-N-[3-(2-méthoxyphényl)-propyl]-4-pipéridineamine;
la (2S,4S)-2-benzyl-1-(3,5-diméthylbenzoyl)-N-[3-(2-méthoxyphényl)-propyl]-4-pipéridineamine;
la (2R,4S)-2-benzyl-1-(3,5-diméthylbenzoyl)-N-(2-trifluorométhylbenzyl)-4-pipéridineamine;
la (2S,4S)-2-benzyl-1-(3,5-diméthylbenkzoyl)-N-(2-trifluorométhylbenzyl)-4-pipéridineamine;
la (2R,4S)-2-benzyl-1-(3,5-diméthylbenzoyl)-N-[2-(2-trifluorométhylphényl)-éthyl]-4-pipéridineamine;
la (2S,4S)-2-benzyl-1-(3,5-diméthylbenzoyl)-N-[2-(2-trifluorométhylphényl)-éthyl]-4-pipéridineamine;
la (2R,4S)-2-benzyl-1-(3,5-diméthylbenzoyl)-N-[3-(2-trifluorométhylphényl)-propyl]-4-pipéridineamine;
la (2S,4S)-2-benzyl-1-(3,5-diméthylbenzoyl)-N-[3-(2-trifluorométhylphényl)-propyl]-4-pipéridineamine;
la (2R,4S)-2-benzyl-1-(3,5-diméthylbenzoyl)-N-(3-trifluorométhylbenzyl)-4-pipéridineamine;
la (2S,4S)-2-benzyl-1-(3,5-diméthylbenzoyl)-N-(3-trifluorométhylbenzyl)-4-pipéridineamine;
la (2R,4S)-2-benzyl-1-(3,5-diméthylbenzoyl)-N-[2-(3-trifluorométhylphényl)-éthyl]-4-pipéridineamine;
la (2S,4S]-2-benzyl-1-(3,5-diméthylbenzoyl)-N-[2-(3-trifluorométhylphényl)-éthyl]-4-pipéridineamine;
la (2R,4S)-2-benzyl-1-(3,5-diméthylbenzoyl)-N-[3-(3-trifluorométhylphényl)-propyl]-4-pipéridineamine;
la (2S,4S)-2-benzyl-1-(3,5-diméthylbenzoyl)-N-[3-(3-trifluorométhylphényl)-propyl]-4-pipéridineamine;
la (2R,4S)-2-benzyl-1-(3,5-diméthylbenzoyl)-N-(4-trifluorométhylbenzyl)-4-pipéridineamine;
la (2S,4S)-2-benzyl-1-(3,5-diméthylbenzoyl)-N-(4-trifluorométhylbenzyl)-4-pipéridineamine;
la (2R,4S)-2-benzyl-1-(3,5-diméthylbenzoyl)-N-[2-(4-trifluorométhylphényl)-éthyl]-4-piperidineamine ;

la (2S,4S)-2-benzyl-1-(3,5-diméthylbenzoyl)-N-[2-(4-trifluorométhylphényl)-éthyl]-4-pipéridineamine ;
la (2R,4S)-2-benzyl-1-(3,5-diméthylbenzoyl)-N-[3-(4-trifluorométhylphényl)-propyl]-4-pipéridineamine ;
la (2S,4S)-2-benzyl-1-(3,5-diméthylbenzoyl)-N-[3-(4-trifluorométhylphényl)-propyl]-4-pipéridineamine ;
la (2R,4S)-2-benzyl-1-(3,5-diméthylbenzoyl)-N-(2,3-diméthoxybenzyl)-4-pipéridineamine ;
la (2S,4S)-2-benzyl-1-(3,5-diméthylbenzoyl)-N-(2,3-diméthoxybenzyl)-4-pipéridineamine ;
la (2R,4S)-2-benzyl-1-(3,5-diméthylbenzoyl)-N-[2-(2,3-diméthoxyphényl)-éthyl]-4-pipéridineamine ;
la (2R,4S)-2-benzyl-1-(3,5-diméthylbenzoyl)-N-[2-(2,3-diméthoxyphényl)-éthyl]-4-pipéridineamine ;
la (2R,4S)-2-benzyl-1-(3,5-diméthylbenzoyl)-N-[3-(2,3-diméthoxyphényl)-propyl]-4-pipéridineamine ;
la (2R,4S)-2-benzyl-1-(3,5-diméthylbenzoyl)-N-[3-(2,3-diméthoxyphényl)-propyl]4-pipéridineamine ;
la (2R,4S)-2-benzyl-1-(3,5-diméthylbenzoyl)-N-(2,4-diméthoxybenzyl)-4-pipéridineamine ;
la (2R,4S)-2-benzyl-1-(3,5-diméthylbenzoyl)-N-(2,4-diméthoxyphényl)-4-pipéridineamine ;
la (2R,4S)-2-benzyl-1-(3,5-diméthylbenzoyl)-N-[2-(2,4-diméthoxyphényl)-éthyl]-4-pipéridineamine ;
la (2R,4S)-2-benzyl-1-(3,5-diméthylbenzoyl)-N-[2-(2,4-diméthoxyphényl)-éthyl]-4-pipéridineamine ;
la (2R,4S)-2-benzyl-1-(3,5-diméthylbenzoyl)-N-[3-(2,4-diméthoxyphényl)-propyl]-4-pipéridineamine ;
la (2S,4S)-2-benzyl-1-(3,5-diméthylbenzoyl)-N-[3-(2,4-diméthoxyphényl)-propyl]-4-pipéridineamine ;
la (2R,4S)-2-benzyl-1-(3,5-diméthylbenzoyl)-N-(2,5-diméthoxybenzyl)-4-pipéridineamine ;
la (SR,4S)-2-benzyl-1-(3,5-diméthylbenzoyl)-N-(2,5-diméthoxybenzyl)-4-pipéridineamine ;
la (2R,4S)-2-benzyl-1-(3,5-diméthylbenzoyl)-N-[2-(2,5-diméthoxyphényl)-éthyl]-4-pipéridineamine ;
la (2S,4S)-2-benzyl-1-(3,5-diméthylbenzoyl)-N-[2-(2,5-diméthoxyphényl)-éthyl]-4-pipéridineamine ;
la (2R,4S)-2-benzyl-1-(3,5-diméthylbenzoyl)-N-[3-(2,5-diméthoxyphényl)-propyl]-4-pipéridineamine ;
la (2S,4S)-2-benzyl-1-(3,5-diméthylbenzoyl)-N-[3-(2,5-diméthoxyphényl)-propyl]-4-pipéridineamine ;
la (2R,4S)-2-benzyl-1-(3,5-diméthylbenzoyl)-N-(2,6-diméthoxybenzyl)-4-pipéridineamine ;
la (2S,4S)-2-benzyl-1-(3,5-diméthylbenzoyl)-N-(2,6-diméthoxybénzyl)-4-pipéridineamine ;
la (2R,4S)-2-benzyl-1-(3,5-diméthylbenzoyl)-N-[2-(2,6-diméthoxyphényl)-éthyl]-4-pipéridineamine ;
la (2S,4S)-2-benzyl-1-(3,5-diméthylbenzoyl)-N-[2-(2,6-diméthoxyphényl)-éthyl]-4-pipéridineamine ;
la (2R,4S)-2-benzyl-1-(3,5-diméthylbenzoyl)-N-[3-(2,6-diméthoxyphényl)-propyl]-4-pipéridineamine ;
la (2S,4S)-2-benzyl-1-(3,5-diméthylbenzoyl)-N-[3-(2,6-diméthoxyphényl)-propyl]-4-pipéridineamine ;
la (2R,4S)-2-benzyl-1-(3,5-diméthylbenzoyl)-N-(2,3-méthylènedioxybenzyl)-4-pipéridineamine ;
la (2S,4S)-2-benzyl-1-(3,5-diméthylbenzoyl)-N-(2,3-méthylènedioxybenzyl)-4-pipéridineamine ;
la (2R,4S)-2-benzyl-1-(3,5-diméthylbenzoyl)-N-[3-(2,3-méthylènedioxyphényl)-éthyl]-4-pipéridineamine ;
la (2S,4S)-2-benzyl-1-(3,5-diméthylbenzoyl)-N-[2-(2,3-méthylènedioxyphényl)-éthyl]-4-pipéridineamine ;
la (2R,4S)-2-benzyl-1-(3,5-diméthylbenzoyl)-N-[3-(2,3-méthylènedioxyphényl)-propyl]-4-pipéridineamine ;
la (2S,4S)-2-benzyl-1-(3,5-diméthylbenzoyl)-N-[3-(2,3-méthylènedioxyphényl)-propyl]-4-pipéridineamine ;
la (2R,4S)-2-benzyl-1-(3,5-diméthylbenzoyl)-N-(2,4-méthylènedioxybenzyl)-4-pipéridineamine ;
la (2S,4S)-2-benzyl-1-(3,5-diméthylbenzoyl)-N-(2,4-méthylènedioxybenzyl)-4-pipéridineamine ;
la (2R,4S)-2-benzyl-1-(3,5-diméthylbenzoyl)-N-[2-(2,4-méthylènedioxyphényl)-éthyl]-4-pipéridineamine ;
la (2S,4S)-2-benzyl-1-(3,5-diméthylbenzoyl)-N-[2-(2,4-méthylènedioxyphényl)-éthyl]-4-pipéridineamine ;
la (2R,4S)-2-benzyl-1-(3,5-diméthylbenzoyl)-N-[3-(2,4-méthylènedioxyphényl)-propyl]-4-pipéridineamine ;
la (2S,4S)-2-benzyl-1-(3,5-diméthylbenzoyl)-N-[3-(2,4-méthylènedioxyphényl)-propyl]-4-pipéridineamine ;
la (2R,4S)-2-benzyl-1-(3,5-diméthylbenzoyl)-N-(indol-2-ylméthyl)-4-pipéridineamine ;
la (2S,4S)-2-benzyl-1-(3,5-diméthylbenzoyl)-N-(indol-2-ylméthyl)-4-pipéridineamine ;
la (2R,4S)-2-benzyl-1-(3,5-diméthylbenzoyl)-N-(indol-3-ylméthyl)-4-pipéridineamine ;
la (2S,4S)-2-benzyl-1-(3,5-diméthylbenzoyl)-N-(indol-3-ylméthyl)-4-pipéridineamine ;
la (2S,4S)-2-benzyl-1-(3,5-diméthylbenzoyl)-N-(quinoléin-2-ylméthyl)-4-pipéridineamine ;
la (2S,4S)-2-benzyl-1-(3,5-diméthylbenzoyl)-N-(quinoléin-3-ylméthyl)-4-pipéridineamine ;
la (2R,4S)-2-benzyl-1-(3,5-diméthylbenzoyl)-N-(2-chlorobenzyl)-4-pipéridineamine ;
la (2S,4S)-2-benzyl-1-(3,5-diméthylbenzoyl)-N-(2-chlorobenzyl)-4-pipéridineamine ;
la (2R,4S)-2-benzyl-1-(3,5-diméthylbenzoyl)-N-[2-(2-chlorophényl)-éthyl]-4-pipéridineamine ;
la (2S,4S)-2-benzyl-1-(3,5-diméthylbenzoyl)-N-[2-(2-chlorophényl)-éthyl]-4-pipéridineamine ;
la (2R,4S)-2-benzyl-1-(3,5-diméthylbenzoyl)-N-[3-(2-chlorophényl)-propyl]-4-pipéridineamine ;
la (2S,4S)-2-benzyl-1-(3,5-diméthylbenzoyl)-N-[3-(2-chlorophényl)-propyl]-4-pipéridineamine ;
la (2R,4S)-2-benzyl-1-(3,5-diméthylbenzoyl)-N-(3-chlorobenzyl)-4-pipéridineamine ;
la (2S,4S)-2-benzyl-1-(3,5-diméthylbenzoyl)-N-(3-chlorobenzyl)-4-pipéridineamine ;
la (2R,4S)-2-benzyl-1-(3,5-diméthylbenzoyl)-N-[2-(3-chlorophényl)-éthyl]-4-pipéridineamine ;

la (2S,4S)-2-benzyl-1-(3,5-diméthylbenzoyl)-N-[2-(3-chlorophényl)-éthyl]-4-pipéridineamine ;
la (2R,4S)-2-benzyl-1-(3,5-diméthylbenzoyl)-N-[3-(3-chlorophényl)-propyl]-4-pipéridineamine ;
la (2S,4S)-2-benzyl-1-(3,5-diméthylbenzoyl)-N-[3-(3-chlorophényl)-propyl]-4-pipéridineamine ;
la (2R,4S)-2-benzyl-1-(3,5-diméthylbenzoyl)-N-(3-chlorobenzyl)-4-pipéridineamine ;
la (2S,4S)-2-benzyl-1-(3,5-diméthylbenzoyl)-N-(3-chlorobenzyl)-4-pipéridineamine ;
la (2R,4S)-2-benzyl-1-(3,5-diméthylbenzoyl)-N-[2-(4-chlorophényl)-éthyl]-4-pipéridineamine ;
la (2S,4S)-2-benzyl-1-(3,5-diméthylbenzoyl)-N-[2-(4-chlorophényl)-éthyl]-4-pipéridineamine ;
la (2R,4S)-2-benzyl-1-(3,5-diméthylbenzoyl)-N-[3-(4-chlorophényl)-propyl]-4-pipéridineamine ;
la (2S,4S)-2-benzyl-1-(3,5-diméthylbenzoyl)-N-[3-(4-chlorophényl)-propyl]-4-pipéridineamine ;
la (2R,4S)-2-benzyl-1-(3,5-diméthylbenzoyl)-N-(4-méthoxynapht-1-ylméthyl)-4-pipéridineamine ;
la (2S,4S)-2-benzyl-1-(3,5-diméthylbenzoyl)-N-(4-méthoxynapht-1-ylméthyl)-4-pipéridineamine ;
la (2R,4S)-2-benzyl-1-(3,5-diméthylbenzoyl)-N-(3,4-éthylènedioxybenzyl)-4-pipéridineamine ;
la (2S,4S)-2-benzyl-1-(3,5-diméthylbenzoyl)-N-(3,4-éthylènedioxybenzyl)-4-pipéridineamine ;
la (2R,4S)-2-benzyl-1-(3,5-diméthylbenzoyl)-N-(indol-2-ylméthyl)-4-pipéridineamine ;
la (2S,4S)-2-benzyl-1-(3,5-diméthylbenzoyl)-N-(indol-2-ylméthyl)-4-pipéridineamine ;
la (2R,4S)-2-benzyl-1-(3,5-diméthylbenzoyl)-N-(indol-3-ylméthyl)-4-pipéridineamine ;
la (2S,4S)-2-benzyl-1-(3,5-diméthylbenzoyl)-N-(indol-3-ylméthyl)-4-pipéridineamine ;
la (2R,4S)-2-benzyl-1-(3,5-diméthylbenzoyl)-N-(quinoléin-2-ylméthyl)-4-pipéridineamine ;
la (2S,4S)-2-benzyl-1-(3,5-diméthylbenzoyl)-N-(quinoléin-2-ylméthyl)-4-pipéridineamine ;
la (2R,4S)-2-benzyl-1-(3,5-diméthylbenzoyl)-N-(quinoléin-3-ylméthyl)-4-pipéridineamine ;
la (2S,4S)-2-benzyl-1-(3,5-diméthylbenzoyl)-N-(quinoléin-3-ylméthyl)-4-pipéridineamine ;
la (2R,4S)-2-benzyl-1-(3,5-diméthylbenzoyl)-N-(2-chlorobenzyl)-4-pipéridineamine ;
la (2S,4S)-2-benzyl-1-(3,5-diméthylbenzoyl)-N-(2-chlorobenzyl)-4-pipéridineamine ;
la (2R,4S)-2-benzyl-1-(3,5-diméthylbenzoyl)-N-[2-(2-chlorophényl)-éthyl]-4-pipéridineamine ;
la (2S,4S)-2-benzyl-1-(3,5-diméthylbenzoyl)-N-[2-(2-chlorophényl)-éthyl]-4-pipéridineamine ;
la (2R,4S)-2-benzyl-1-(3,5-diméthylbenzoyl)-N-[3-(2-chlorophényl)-propyl]-4-pipéridineamine ;
la (2S,4S)-2-benzyl-1-(3,5-diméthylbenzoyl)-N-[3-(2-chlorophényl)-propyl]-4-pipéridineamine ;
la (2R,4S)-2-benzyl-1-(3,5-diméthylbenzoyl)-N-(3-chlorobenzyl))-4-pipéridineamine ;
la (2S,4S)-2-benzyl-1-(3,5-diméthylbenzoyl)-N-(3-chlorobenzyl)-4-pipéridineamine ;
la (2R,4S)-2-benzyl-1-(3,5-diméthylbenzoyl)-N-[2-(3-chlorophényl)-éthyl]-4-pipéridineamine ;
la (2S,4S)-2-benzyl-1-(3,5-diméthylbenzoyl)-N-[2-(3-chlorophényl)-éthyl]-4-pipéridineamine ;
la (2R,4S)-2-benzyl-1-(3,5-diméthylbenzoyl)-N-[3-(3-chlorophényl)-propyl]-4-pipéridineamine ;
la (2S,4S)-2-benzyl-1-(3,5-diméthylbenzoyl)-N-[3-(3-chlorophényl)-propyl]-4-pipéridineamine ;
la (2R,4S)-2-benzyl-1-(3,5-diméthylbenzoyl)-N-[4-chlorobenzyl)-4-pipéridineamine ;
la (2S,4S)-2-benzyl-1-(3,5-diméthylbenzoyl)-N-(4-chlorobenzyl)-4-pipéridineamine ;
la (2R,4S)-2-benzyl-1-(3,5-diméthylbenzoyl)-N-[2-(4-chlorophényl)-éthyl]-4-pipéridineamine ;
la (2S,4S)-2-benzyl-1-(3,5-diméthylbenzoyl)-N-[2-(4-chlorophényl)-éthyl]-4-pipéridineamine ;
la (2R,4S)-2-benzyl-1-(3,5-diméthylbenzoyl)-N-[3-(4-chlorophényl)-propyl]-4-pipéridineamine ;
la (2S,4S)-2-(benzyl)-1-(3,5-diméthylbenzoyl)-N-[3-(4-chlorophényl)-propyl]-4-pipéridineamine ;
la (2R,4S)-2-benzyl-1-(3,5-diméthylbenzoyl)-N-(4-méthoxynapht-1-ylméthyl)-4-pipéridineamine ;
la (2S,4S)-2-benzyl-1-(3,5-diméthylbenzoyl)-N-(4-méthoxynapht-1-ylméthyl)-4-pipéridineamine ;
la (2R,4S)-2-benzyl-1-(3,5-diméthylbenzoyl)-N-(3,4-éthylènedioxybenzyl)-4-pipéridineamine ;
la (2S,4S)-2-benzyl-1-(3,5-diméthylbenzoyl)-N-(3,4-éthylènedioxybenzyl)-4-pipéridineamine ;
la (2R,4S)-2-benzyl-1-(3,5-ditrifluorométhylbenzoyl)-N-(quinoléin-2-ylméthyl)-4-pipéridineamine ;
la (2S,4S)-2-benzyl-1-(3,5-ditrifluorométhylbenzoyl)-N-(quinoléin-2-ylméthyl)-4-pipéridineamine ;
la (2R,4S)-2-benzyl-1-(3,5-ditrifluorométhylbenzoyl)-N-(quinoléin-3-ylméthyl)-4-pipéridineamine ;
la (2S,4S)-2-benzyl-1-(3,5-ditrifluorométhylbenzoyl)-N-(quinoléin-3-ylméthyl)-4-pipéridineamine ;
la (2R,4S)-2-benzyl-1-(3,5-ditrifluorométhylbenzoyl)-N-(2-chlorobenzyl)-4-pipéridineamine ;
la (2S,4S)-2-benzyl-1-(3,5-ditrifluorométhylbenzoyl)-N-(2-chlorobenzyl)-4-pipéridineamine ;
la (2R,4S)-2-benzyl-1-(3,5-ditrifluorométhylbenzoyl)-N-[2-(2-chlorophényl)-éthyl]-4-pipéridineamine ;
la (2S,4S)-2-benzyl-1-(3,5-ditrifluorométhylbenzoyl)-N-[2-(2-chlorophényl)-éthyl]-4-pipéridineamine ;
la (2R,4S)-2-benzyl-1-(3,5-ditrifluorométhylbenzoyl)-N-[3-(2-chlorophényl) propyl]-4-pipéridineamine ;
la (2S,4S)-2-benzyl-1-(3,5-ditrifluorométhylbenzoyl)-N-[3-(2-chlorophényl)-propyl]-4-pipéridineamine ;
la (2R,4S)-2-benzyl-1-(3,5-ditrifluorométhylbenzoyl)-N-(3-chlorobenzyl)-4-pipéridineamine ;
la (2S,4S)-2-benzyl-1-(3,5-ditrifluorométhylbenzoyl)-N-(3-chlorobenzyl)-4-pipéridineamine ;
la (2R,4S)-2-benzyl-1-(3,5-ditrifluorométhylbenzoyl)-N-[2-(3-chlorophényl)-éthyl]-4-pipéridineamine ;
la (2S,4S)-2-benzyl-1-(3,5-ditrifluorométhylbenzoyl)-N-[2-(3-chlorophényl)-éthyl]-4-pipéridineamine ;
la (2R,4S)-2-benzyl-1-(3,5-ditrifluorométhylbenzoyl)-N-[3-(3-chlorophényl)-propyl]-4-pipéridineamine ;

la (2S,4S)-2-benzyl-1-(3,5-ditrifluorométhylbenzoyl)-N-[3-(3-chlorophényl)-propyl]-4-pipéridineamine ;
la (2R,4S)-2-benzyl-1-(3,5-ditrifluorométhylbenzoyl)-N-(4-chlorobenzyl)-4-pipéridineamine ;
la (2S,4S)-2-benzyl-1-(3,5-ditrifluorométhylbenzoyl)-N-(4-chlorobenzyl)-4-pipéridineamine ;
la (2R,4S)-2-benzyl-1-(3,5-ditrifluorométhylbenzoyl)-N-[2-(4-chlorophényl)-éthyl]-4-pipéridineamine ;
la (2S,4S)-2-benzyl-1-(3,5-ditrifluorométhylbenzoyl)-N-[2-(4-chlorophényl)-éthyl]-4-pipéridineamine ;
la (2R,4S)-2-benzyl-1-(3,5-ditrifluorométhylbenzoyl)-N-[3-(4-chlorophényl)-propyl]-4-pipéridineamine ;
la (2S,4S)-2-benzyl-1-(3,5-ditrifluorométhylbenzoyl)-N-[3-(4-chlorophényl)-propyl]-4-pipéridineamine ;
la (2R,4S)-2-benzyl-1-(3,5-ditrifluorométhylbenzoyl)-N-(4-méthoxynapht-1-ylméthyl)-4-pipéridineamine ;
la (2S,4S)-2-benzyl-1-(3,5-ditrifluorométhylbenzoyl)-N-(4-méthoxynapht-1-ylméthyl)-4-pipéridineamine ;
la (2R,4S)-2-benzyl-1-(3,5-ditrifluorométhylbenzoyl)-N-(3,4-éthylènedioxybenzyl)-4-pipéridineamine ;
la (2S,4S)-2-benzyl-1-(3,5-ditrifluorométhylbenzoyl)-N-(3,4-éthylènedioxybenzyl)-4-pipéridineamine ;
la (2R,4S)-2-benzyl-1-(3,5-dichlorobenzoyl)-N-(indol-2-ylméthyl)-4-pipéridineamine ;
la (2S,4S)-2-benzyl-1-(3,5-dichlorobenzoyl)-N-(indol-2-ylméthyl)-4-pipéridineamine ;
la (2R,4S)-2-benzyl-1-(3,5-dichlorobenzoyl)-N-(indol-3-ylméthyl)-4-pipéridineamine ;
la (2S,4S)-2-benzyl-1-(3,5-dichlorobenzoyl)-N-(indol-3-ylméthyl)-4-pipéridineamine ;
la (2R,4S)-2-benzyl-1-(3,5-dichlorobenzoyl)-N-(quinoléin-2-ylméthyl)-4-pipéridineamine ;
la (2S,4S)-2-benzyl-1-(3,5-dichlorobenzoyl)-N-(quinoléin-2-ylméthyl)-4-pipéridineamine ;
la (2R,4S)-2-benzyl-1-(3,5-dichlorobenzoyl)-N-(quinoléin-3-ylméthyl)-4-pipéridineamine ;
la (2S,4S)-2-benzyl-1-(3,5-dichlorobenzoyl)-N-(quinoléin-3-ylméthyl)-4-pipéridineamine ;
la (2R,4S)-2-benzyl-1-(3,5-dichlorobenzoyl)-N-(2-chlorobenzyl)-4-pipéridineamine ;
la (2S,4S)-2-benzyl-1-(3,5-dichlorobenzoyl)-N-(2-chlorobenzyl)-4-pipéridineamine ;
la (2R,4S)-2-benzyl-1-(3,5-dichlorobenzoyl)-N-[2-(2-chlorophényl)-éthyl]-4-pipéridineamine ;
la (2S,4S)-2-benzyl-1-(3,5-dichlorobenzoyl)-N-[2-(2-chlorophényl)-éthyl]-4-pipéridineamine ;
la (2R,4S)-2-benzyl-1-(3,5-dichlorobenzoyl)-N-[3-(2-chlorophényl)-propyl]-4-pipéridineamine ;
la (2S,4S)-2-benzyl-1-(3,5-dichlorobenzoyl)-N-[3-(2-chlorophényl)-propyl]-4-pipéridineamine ;
la (2R,4S)-2-benzyl-1-(3,5-dichlorobenzoyl)-N-(3-chlorobenzyl)-4-pipéridineamine ;
la (2S,4S)-2-benzyl-1-(3,5-dichlorobenzoyl)-N-(3-chlorobenzyl)-4-pipéridineamine ;
la (2R,4S)-2-benzyl-1-(3,5-dichlorobenzoyl)-N-[2-(3-chlorophényl)-éthyl]-4-pipéridineamine ;
la (2S,4S)-2-benzyl-1-(3,5-dichlorobenzoyl)-N-[2-(3-chlorophényl)-éthyl]-4-pipéridineamine ;
la (2R,4S)-2-benzyl-1-(3,5-dichlorobenzoyl)-N-[3-(3-chlorophényl)-propyl]-4-pipéridineamine ;
la (2S,4S)-2-benzyl-1-(3,5-dichlorobenzoyl)-N-[3-(3-chlorophényl)-propyl]-4-pipéridineamine ;
la (2R,4S)-2-benzyl-1-(3,5-dichlorobenzoyl)-N-(4-chlorobenzyl)-4-pipéridineamine ;
la (2S,4S)-2-benzyl-1-(3,5-dichlorobenzoyl)-N-(4-chlorobenzyl)-4-pipéridineamine ;
la (2R,4S)-2-benzyl-1-(3,5-dichlorobenzoyl)-N-[2-(4-chlorophényl)-éthyl]-4-pipéridineamine ;
la (2S,4S)-2-benzyl-1-(3,5-dichlorobenzoyl)-N-[2-(4-chlorophényl)-éthyl]-4-pipéridineamine ;
la (2R,4S)-2-benzyl-1-(3,5-dichlorobenzoyl)-N-[3-(4-chlorophényl)-propyl]-4-pipéridineamine ;
la (2S,4S)-2-benzyl-1-(3,5-dichlorobenzoyl)-N-[3-(4-chlorophényl)-propyl]-4-pipéridineamine ;
la (2R,4S)-2-benzyl-1-(3,5-dichlorobenzoyl)-N-(4-méthoxynapht-1-yl)-4-pipéridineamine ;
la (2S,4S)-2-benzyl-1-(3,5-dichlorobenzoyl)-N-(4-méthoxynapht-1-ylméthyl)-4-pipéridineamine ;
la (2R,4S)-2-benzyl-1-(3,5-dichlorobenzoyl)-N-(3,4-éthylènedioxybenzyl)-4-pipéridineamine ;
la (2S,4S)-2-benzyl-1-(3,5-dichlorobenzoyl)-N-(3,4-éthylènedioxybenzyl)-4-pipéridineamine ;
la (2R,4S)-2-benzyl-1-(3,5-diméthoxybenzoyl)-N-(indol-2-ylméthyl)-4-pipéridineamine ;
la (2S,4S)-2-benzyl-1-(3,5-diméthoxybenzoyl)-N-(indol-2-ylméthyl)-4-pipéridineamine ;
la (2R,4S)-2-benzyl-1-(3,5-diméthoxybenzoyl)-N-(indol-3-ylméthyl)-4-pipéridineamine ;
la (2S,4S)-2-benzyl-1-(3,5-diméthoxybenzoyl)-N-(indol-3-ylméthyl)-4-pipéridineamine ;
la (2R,4S)-2-benzyl-1-(3,5-diméthoxybenzoyl)-N-(quinoléin-2-ylméthyl)-4-pipéridineamine ;
la (2S,4S)-2-benzyl-1-(3,5-diméthoxybenzoyl)-N-(quinoléin-2-ylméthyl)-4-pipéridineamine ;
la (2R,4S)-2-benzyl-1-(3,5-diméthoxybenzoyl)-N-(quinoléin-3-ylméthyl)-4-pipéridineamine ;
la (2S,4S)-2-benzyl-1-(3,5-diméthoxybenzoyl)-N-(quinoléin-3-ylméthyl)-4-pipéridineamine ;
la (2R,4S)-2-benzyl-1-(3,5-diméthoxybenzoyl)-N-(2-chlorobenzyl)-4-pipéridineamine ;
la (2S,4S)-2-benzyl-1-(3,5-diméthoxybenzoyl)-N-(2-chlorobenzyl)-4-pipéridineamine ;
la (2R,4S)-2-benzyl-1-(3,5-dichlorobenzoyl)-N-[2-(2-chlorophényl)-éthyl]-4-pipéridineamine ;
la (2S,4S)-2-benzyl-1-(3,5-diméthoxybenzoyl)-N-[2-(2-chlorophényl)-éthyl]-4-pipéridineamine ;
la (2R,4S)-2-benzyl-1-(3,5-diméthoxybenzoyl)-N-[3-(2-chlorophényl)-propyl]-4-pipéridineamine ;
la (2S,4S)-2-benzyl-1-(3,5-diméthoxybenzoyl)-N-[3-(2-chlorophényl)-propyl]-4-pipéridineamine ;
la (2R,4S)-2-benzyl-1-(3,5-diméthoxybenzoyl)-N-(3-chlorobenzyl)-4-pipéridineamine ;
la (2S,4S)-2-benzyl-1-(3,5-diméthoxybenzoyl)-N-(3-chlorobenzyl)-4-pipéridineamine ;
la (2R,4S)-2-benzyl-1-(3,5-diméthoxybenzoyl)-N-[3-(2-chlorophényl)-éthyl]-4-pipéridineamine ;

la (2S,4S)-2-benzyl-1-(3,5-diméthoxybenzoyl)-N-[2-(3-chlorophényl)-éthyl]-4-pipéridineamine ;
la (2R,4S)-2-benzyl-1-(3,5-diméthoxybenzoyl)-N-[3-(3-chlorophényl)-propyl]-4-pipéridineamine ;
la (2S,4S)-2-benzyl-1-(3,5-diméthoxybenzoyl)-N-[3-(3-chlorophényl)-propyl]-4-pipéridineamine ;
la (2R,4S)-2-benzyl-1-(3,5-diméthoxybenzoyl)-N-(2-chlorobenzyl)-4-pipéridineamine ;
la (2S,4S)-2-benzyl-1-(3,5-diméthoxybenzoyl)-N-(4-chlorobenzyl)-4-pipéridineamine ;
la (2R,4S)-2-benzyl-1-(3,5-diméthoxybenzoyl)-N-[2-(4-chlorophényl)-éthyl]-4-pipéridineamine ;
la (2S,4S)-2-benzyl-1-(3,5-diméthoxybenzoyl)-N-[2-(4-chlorophényl)-éthyl]-4-pipéridineamine ;
la (2R,4S)-2-benzyl-1-(3,5-diméthoxybenzoyl)-N-[3-(4-chlorophényl)-propyl]-4-pipéridineamine ;
la (2S,4S)-2-benzyl-1-(3,5-diméthoxybenzoyl)-N-[3-(4-chlorophényl)-propyl]-4-pipéridineamine ;
la (2R,4S)-2-benzyl-1-(3,5-diméthylbenzoyl)-N-(4-méthoxynapht-1-ylméthyl)-4-pipéridineamine ;
la (2S,4S)-2-benzyl-1-(3,5-diméthoxybenzoyl)-N-(4-méthoxynapht-1-ylméthyl)-4-pipéridineamine ;
la (2R,4S)-2-benzyl-1-(3,5-diméthoxybenzoyl)-N-(3,4-éthylènedioxybenzyl)-4-pipéridineamine ; ou
la (2S,4S)-2-benzyl-1-(3,5-diméthoxybenzoyl)-N-(3,4-éthylènedioxybenzyl)-4-pipéridineamine ;
ou l'un des sels de chacun de ces composés.

**50.** La (2R,4S)- et la (2R,4R)-2-benzyl-1-(3,5-diméthylbenzoyl)-N-(2-phénéthyl)-4-pipéridineamine ou l'un de leurs sels.

**51.** La (2R,4S)-2-benzyl-1-(3,5-bis-(trifluorométhyl)-benzoyl)-N-4-quinolylméthyl)-4-pipéridineamine ou l'un de ses sels.

**52.** La (2R,4S)-2-benzyl-1-(3,5-dichlorobenzoyl)-N-(4-quinolylméthyl)-4-pipéridineamine ou l'un de ses sels.

**53.** La (2R*,4S*)-2-benzyl-1-(2,4-dichlorobenzoyl)-N-(4-quinolylméthyl)-4-pipéridineamine ou l'un de ses sels.

**54.** La (2R*,4S*)-2-benzyl-1-(phénylacétyl)-N-(4-quinolylméthyl)-4-pipéridineamine ou l'un de ses sels.

**55.** La (2R*,4S*)-2-benzyl-1-(2,6-dichlorobenzoyl)-N-(4-quinolylméthyl)-4-pipéridineamine.

**56.** La (2R*,4S*)-2-benzyl-1-(3,5-dibromobenzoyl)-N-(4-quinolylméthyl)-4-pipéridineamine ou l'un de ses sels.

**57.** La (2R*,4S*)-2-benzyl-1-(9-fluorénoyl)-N-(4-quinolylméthyl)-4-pipéridineamine ou l'un de ses sels.

**58.** La (2R*,4S*)-2-benzyl-1-(3-toluoyl)-N-(4-quinolylméthyl)-4-pipéridineamine ou l'un de ses sels.

**59.** La (2R*,4S*)-2-benzyl-1-(3-bromobenzoyl)-N-(4-quinolylméthyl)-4-pipéridineamine ou l'un de ses sels.

**60.** La (2R*,4S*)-2-benzyl-1-(3,5-dihydroxybenzoyl)-N-(4-quinolylméthyl)-4-pipéridineamine ou l'un de ses sels.

**61.** La (2R*,4S*)-2-benzyl-1-(3-cyanobenzoyl)-N-(4-quinolylméthyl)-4-pipéridineamine ou l'un de ses sels.

**62.** La (2R*,4S*)-2-benzyl-1-(2-chlorobenzoyl)-N-(4-quinolylméthyl)-4-pipéridineamine ou l'un de ses sels.

**63.** La (2R*,4S*)-2-benzyl-1-(4-chlorobenzoyl)-N-(4-quinolylméthyl)-4-pipéridineamine ou l'un de ses sels.

**64.** La (2R*,4S*)-2-benzyl-1-(3,5-dichlorobenzoyl)-N-(4-quinolylméthyl)-N-méthyl-4-pipéridineamine ou l'un de ses sels.

**65.** La (2R*,4S*)-2-benzyl-1-(3,5-dichlorobenzoyl)-N-(4-quinolylméthyl)-N-cyclohexylcarbamoyl-4-pipéridineamine ou l'un de ses sels.

**66.** La (2R*,4S*)-2-benzyl-1-(3,5-dichlorobenzoyl)-N-(4-quinolylméthyl)-N-phénylcarbamoyl-4-pipéridineamine ou l'un de ses sels.

**67.** La (2R*,4S*)-2-benzyl-1-(3,5-dichlorobenzoyl)-N-(2-phényléthyl)-4-pipéridineamine ou l'un de ses sels.

**68.** La (2R*,4S*)-2-benzyl-1-(3,5-bis-(trifluorométhyl)-benzoyl)-N-(2-phényléthyl)-4-pipéridineamine ou l'un de ses sels.

**69.** La (2R*,4S*)-2-benzyl-1-(3,5-dichlorobenzoyl)-N-(2-naphtoyl)-4-pipéridineamine ou l'un de ses sels.

**70.** La (2R*,4S*)-2-benzyl-1-(3,5-dichlorobenzoyl)-N-(3,5-diméthylbenzoyl)-4-pipéridineamine ou l'un de ses sels.

**71.** La (2R*,4S*)-2-benzyl-1-(3,5-dichlorobenzoyl)-N-(4-quinolylcarbonyl)-4-pipéridineamine ou l'un de ses sels.

**72.** La (2R*,4S*)-2-benzyl-1-(3,5-dichlorobenzoyl)-N-(3-indolyléarbonyl)-4-pipéridineamine ou l'un de ses sels.

**73.** La (2R*,4S*)-2-benzyl-1-(3,5-dichlorobenzoyl)-N-(2-indolylcarbonyl)-4-pipéridineamine ou l'un de ses sels.

**74.** La (2R*,4S*)-2-benzyl-1-(3,5-dichlorobenzoyl)-N-(5-méthoxy-2-indolylcarbonyl)-4-pipéridineamine ou l'un de ses sels.

**75.** La (2R*,4S*)-2-benzyl-1-(3,5-dichlorobenzoyl)-N-(1-naphtoyl)-4-pipéridineamine ou l'un de ses sels.

**76.** La (2R*,4S*)-2-benzyl-1-(3,5-dichlorobenzoyl)-N-(phénylacétyl)-4-pipéridineamine ou l'un de ses sels.

**77.** La (2R*,4S*)-2-benzyl-1-(3,5-dichlorobenzoyl)-N-(2-méthoxybenzyl)-4-pipéridineamine ou l'un de ses sels.

**78.** La (2R*,4S*)-2-benzyl-1-(3,5-dichlorobenzoyl)-N-(3-(N-acétyl)-indolylméthyl)-4-pipéridineamine ou l'un de ses sels.

**79.** La (2R*,4S*)-2-benzyl-1-(3,5-dichlorobenzoyl)-N-(2-benzo[b]furanylméthyl)4-pipéridineamine ou l'un de ses sels.

**80.** La (2R*,4S*)-2-benzyl-1-(3,5-dichlorobenzoyl)-N-[(3-méthylbenzo[b]thlophén-2-ylméthyl]-4-pipéridineamine ou l'un de ses sels.

**81.** La (2R*,4S*)-2-benzyl-1-(3,5-dichlorobenzoyl)-N-(5-méthoxyindol-3-ylméthyl)-4-pipéridineamine ou l'un de ses sels.

**82.** La (2R*,4S*)-2-benzyl-1-(3,5-dichlorobenzoyl)-N-(3-indolylméthyl)-4-pipéridine-amine ou l'un de ses sels.

**83.** La (2R*,4S*)-2-benzyl-1-(3,5-dichlorobenzoyl)-N-phénylcarbamoyl-4-pipéridineamine ou l'un de ses sels.

**84.** La (2R*,4S*)-2-benzyl-1-(3,5-dichlorobenzoyl)-N-diphénylméthyl-4-pipéridineamine ou l'un de ses sels.

**85.** La (2R*,4S*)-2-benzyl-1-(3,5-dichlorobenzoyl)-N-(3,4-dihydro-2H-1-benzopyranne-2-carbonyl)-4-pipéridineamine ou l'un de ses sels.

**86.** La (2R*,4S*)-2-benzyl-1-(4-méthoxybenzoyl)-N-(4-quinolylméthyl)-4-pipéridineamine ou l'un de ses sels.

**87.** La (2R*,4S*,1'R*)-N-benzyl-1-(3,5-diméthylbenzoyl)-2-(1'-hydroxy-1'-phénylméthyl)-4-pipéridineamine ou l'un de ses sels.

**88.** La (2R*,4S*,1'R*)-2-(1'-hydroxy-1'-phénylméthyl)-1-(3,5-diméthylbenzoyl)-N-(4-quinoléthylméthyl)-4-pipéridineamine ou l'un de ses sels.

**89.** La (2R*,4S*,1'S*)-1-(3,5-diméthylbenzoyl)-2-(1'-hydroxy-1'-phénylméthyl)-N-(4-quinoléinylméthyl)-4-pipéridineamine ou l'un de ses sels.

**90.** La (2R*,4S*,1'R*)-2-{1'-hydroxy-1'-(4-chlorophényl)-méthyl}-1-(3,5-diméthylbenzoyl)-N-(4-quinoléinylméthyl)-4-pipéridineamine ou l'un de ses sels.

**91.** La (2R*,4S*,1'S*)-1-(3,5-diméthylbenzoyl)-2-{1'-hydroxy-1'-(4-chlorophényl)-méthyl}-N-(4-quinoléinylméthyl)-4-pipéridineamine ou l'un de ses sels.

**92.** La (2R*,4S*,1'S*)-1-(3,5-diméthylbenzoyl)-2-{1'-hydroxy-1'-(3,4-dlchlorophényl)-méthyl}-N-(4-quinoléinylméthyl)-4-pipéridineamine ou l'un de ses sels.

**93.** La (2R*,4S*)-N-benzyl-1-(3,5-diméthylbenzoyl)-2-benzoyl-4-pipéridineamine ou l'un de ses sels.

**94.** La (2R*,4S*)-2-(4-chlorobenzyl)-1-(3,5-diméthylbenzoyl)-N-(4-quinoléinylméthyl)-4-pipéridineamine ou l'un de ses sels.

**95.** La (2R*,4S*)-2-(3,4-dichlorobenzyl)-1-(3,5-diméthylbenzoyl)-N-(4-quinoléinylméthyl)-4-pipéridineamine ou l'un de ses sels.

**96.** La (2R*,4S*)-1-(3,5-diméthylbenzoyl)-2-phényl-N-(4-quinoléinylméthyl)-4-pipéridineamine ou l'un de ses sels.

**97.** La (2R *,4S*)-1-(3,5-dichlorobenzoyl)-2-phényl-N-(4-quinoléineméthyl)-4-pipéridineamine ou l'un de ses sels.

**98.** La (2R*,4S*)-1-(1-naphtoyl)-2-phényl-N-(4-quinoléinylméthyl)-4-pipéridineamine.

**99.** La (2R*,4S*)-1-(3,5-diméthylbenzoyl)-2-(1-naphtyl)-N-(4-quinoléinylméthyl)-4-pipéridineamine ou l'un de ses sels.

**100.** Une nouvelle 1-acylpipéridone de formule X

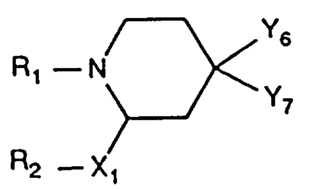

(X)

où $R_1$ représente un reste aroyle, hétéroaroyle, cycloalkylcarbonyle, aralcanoyle, hétéroarylalcanoyle, aralcoxycarbonyle ou arylcarbamoyle éventuellement substitué ou le reste acyle d'un $\alpha$-amino-acide éventuellement N-substitué par un alcanoyle inférieur ou par un carbamoylalcanoyle inférieur, $R_2$ représente un cycloalkyle ou un reste aryle ou hétéroaryle éventuellement substitué, $X_1$ représente l'hydroxméthylène et $Y_6$ et $Y_7$ représentent ensemble un oxo ou l'un de ses sels.

**101.** Un composé de formule X selon la revendication 100 où $R_1$ représente un benzoyle non substitué ou mono- ou disubstitué par un alkyle en $C_1$-$C_4$, un alcoxy en $C_1$-$C_4$, un halogène ayant un numéro atomique allant jusqu'à 35 et/ou le trifluorométhyle ou un naphtoyle non substitué, $R_2$ représente un phényle non substitué ou mono- ou disubstitué, par un halogène ayant un numéro atomique allant jusqu'à 35 et/ou le trifluorométhyle, $X_1$ représente l'hydroxyméthylène et $Y_6$ et $Y_7$ représentent ensemble un oxo ou l'un de ses sels.

**102.** La (2R*,1'R*)-1-(3,5-diméthylbenzoyl)-2-(1'-hydroxy-1'-phénylméthyl)-4-pipéridone ou l'un de ses sels.

**103.** La (2R*,1'R*)-1-(3,5-bis-trifluorométhylbenzoyl)-2-{1'-hydroxy-1'-(4-chlorophényl)-méthyl}-4-pipéridone ou l'un de ses sels.

**104.** La (2R*,1'R*)-1-(3,5-diméthylbenzoyl)-2-{1'-hydroxy-1'-(3,4-dichlorophénylmèthyl)-4-pipéridone ou l'un de ses sels.

**105.** La (2R*,1'S*)-1-(3,5-bis-trifluorométhylbenzoyl)-2-{1'-hydroxy-1'-(4-chlorophényl)-méthyl}-4-pipéridone ou l'un de ses sels.

**106.** La (2R*,1'R*)-1-(3,5-bis-trifluorométhylbenzoyl)-2-{1'-hydroxy-1'-(3,4-dichlorophényl)-méthyl}-4-pipéridone ou l'un de ses sels.

**107.** La (2R*,1'R*)-1-(3,5-diméthylbenzoyl)-2-{1'-hydroxy-1'-(4-méthoxyphényl)-méthyl}-4-pipéridone ou l'un de ses sels.

**108.** La (2R*,1'S*)-1-(3,5-diméthylbenzoyl)-2-{1'-hydroxy-1'-(4-méthoxyphènyl)-méthyl)-4-pipéridone ou l'un de ses sels.

**109.** La (2R*,1'R*)-1-(3,5-diméthylbenzoyl)-2-{1'-hydroxy-1'-(3-méthoxyphényl) méthyl}-4-pipéridone ou l'un de ses sels.

**110.** La (2R*,1'S*)-1-(3,5-diméthylbenzoyl)-2-{1'-hydroxy-1'-(3-méthoxyphényl)-méthyl}-4-pipéridone ou l'un de ses sels.

**111.** La (2R*,1'R*)-1-(3,5-diméthylbenzoyl)-2-{1'-hydroxy-1'-(4-trifluorométhylphényl)-méthyl}-4-pipéridone ou l'un de ses sels.

**112.** La (2R*,1'S*)-1-(3,5-diméthylbenzoyl)-2-{1'-hydroxy-1'-(4-trifluorométhylphényl)-méthyl}-4-pipéridone ou l'un de ses sels.

**113.** La (2R*,1'R*)-1-(3,5-diméthylbenzoyl)-2-{1'-hydroxy-1'-(4-chloro-3-trifluorométhylphényl)-méthyl}-4-pipéridone ou l'un de ses sels.

**114.** La (2R*,1'S*)-1-(3,5-diméthylbenzoyl)-2-{1'-hydroxy-1'-(4-chloro-3-trifluorométhylphényl)-méthyl})-4-pipéridone ou l'un de ses sels.

**115.** Composés selon l'une des revendications 1 à 114 destinés à être utilisés dans un procédé pour le traitement thérapeutique du corps humain ou animal.

**116.** Préparations pharmaceutiques contenant comme substance active pharmaceutique, à côté des adjuvants pharmaceutiques usuels, un composé selon l'une des revendications 1 à 47 sous forme libre ou sous la forme d'un sel pharmaceutiquement utilisable.

**117.** Préparations pharmaceutiques contenant comme substance active pharmaceutique, à côté des adjuvants pharmaceutiques usuels, un composé selon l'une des revendications 48 à 115 sous forme libre ou sous la forme d'un sel pharmaceutiquement utilisable.

**118.** Procédé pour la préparation des nouveaux composés 1-acylpipéridines selon la revendication 1 et de leurs sels, caractérisé

a) en ce que l'on introduit dans un composé de formule II

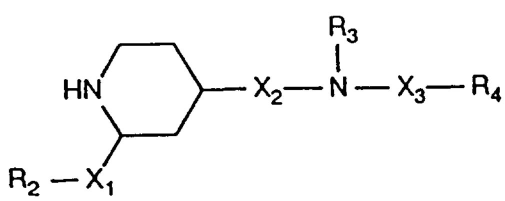

(II)

où $R_2$, $R_3$, $R_4$, $X_1$, $X_2$ et $X_3$ ont les significations qui ont été indiquées, le reste $R_1$ ou

b) en ce que l'on condense les composés de formules III et IV

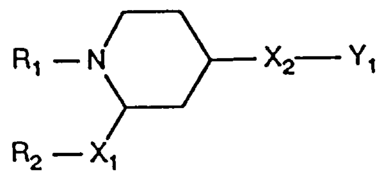

(III)

et

$Y_2$-$X_3$-$R_4$ (IV),

où $Y_1$ représente un groupe de formule -N($R_3$)-H et $Y_2$ représente un hydroxy, un hydroxy estérifié réactif ou, si $X_3$ représente un carbonyle, un hydroxy étherifié ou $Y_1$ représente un hydroxy, un hydroxy estérifié réactif ou, si $X_2$ représente un carbonyle, un hydroxy éthérifié et $Y_2$ un groupe de formule -N($R_3$)-H, $R_2$, $R_3$, $R_4$, $X_1$, $X_2$ et $X_3$ ayant les significations indiquées ou leurs sels ou

c) en ce que pour la préparation des composés de formule I où l'un des restes $X_2$ et $X_3$ représente un alkylène et l'autre reste représente un alkylène, le carbonyle ou, dans le cas de $X_2$ une liaison directe ou dans le cas de $X_3$, un reste àlklylène éventuellement substitué par l'hydroxyméthyle ou par un carboxy éventuellement estérifié ou amidé, l'on remplace dans un composé de formule V

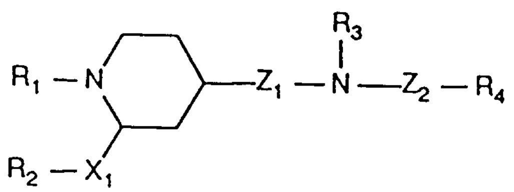

(V)

où $Z_1$ représente un reste alkylène substitué par un oxo ou par un hydroxy en position $\alpha$ par rapport au groupe -N($R_3$)- et $Z_2$ représente un alkylène, le carbonyle ou un reste alkylène éventuellement substitué par l'hydroxyméthyle ou par un carboxy éventuellement estérifié ou amidé ou $Z_1$ représente un alklylé~ne, le carbonyle ou une liaison directe et $Z_2$ représente un reste alklylène substitué par un oxo ou un hydroxy en position $\alpha$ par rapport au groupe -N($R_3$)- et $R_2$, $R_3$, $R_4$, $X_1$, $X_2$ et $X_3$ ont les significations qui ont été indiquées ou dans l'un de ses sels, le groupe oxo ou le groupe hydroxy en position $\alpha$ par rapport au groupe -N($R_3$)- par l'hydrogène par vole de réduction ou en ce que dans un composé de formule VI

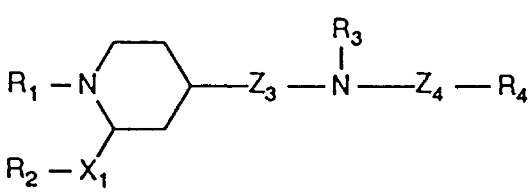

(VI)

où $Z_3$ représente un reste de formule -C($R_a$) = C($R_b$)- et $Z_4$ représente un alkylène, le carbonyle ou un reste alkylène éventuellement substitué par l'hydroxyméthyle ou par un carboxy éventuellement estérifié ou amidé ou $Z_3$ représente un alkylène, le carbonyle ou une liaison directe et $Z_4$ représente un reste de formule -C($R_a$) = C($R_b$)-, $R_a$ et $R_b$ représentant l'hydrogène ou un alkyle inférieur, l'on réduit le reste de formule -C($R_a$) = C($R_b$)- par réduction de la double liaison en un reste correspondant -CH($R_a$)-CH($R_b$)- ou

d) en ce que pour la préparation des composés de formule I où $X_1$ représente un groupe carbonyle ou un groupe hydroxyméthylène, on condense les composés de formules VII et VIII

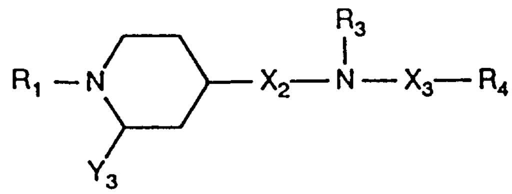 (VII)

et

$Y_4$-$R_2$ (VIII),

où l'un des restes $Y_3$ et $Y_4$ représente le formyle ou un groupe carboxy éventuellement anhydridé ou estérifié et l'autre groupe un reste métallique et $R_2$. $R_3$, $R_4$, $X_2$ et $X_3$ ont les significations qui ont été indiquées ou

e) en ce que pour la préparation des composés de formule I où $R_3$ représente l'hydrogène, l'on clive dans un composé de formule IX

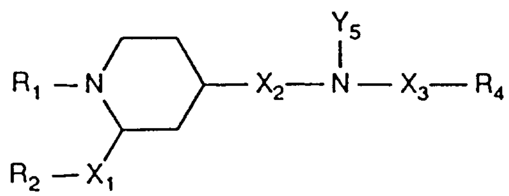 (IX)

où $Y_5$ représente un groupe protecteur de l'amino et $R_2$, $R_4$, $X_1$, $X_2$ et $X_3$ ont les significations qui ont été indiquées, ou dans l'un de ses sels le groupe $Y_5$ ou

f) en ce que pour la préparation des composés de formule I où $X_3$ représente un alkylène, on condense, dans des conditions réductrices, les composés de formules X et XI

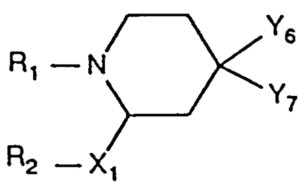 (X) et 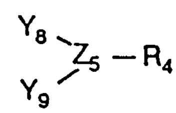 (XI) ,

où $Y_6$ représente un groupe de formule -N($R_3$)-H, $Y_7$ représente l'hydrogène, $Y_8$ et $Y_9$ représentent ensemble un oxo et $X_5$ un reste alcanylylidène correspondant $X_3$ ou $Y_6$ et $Y_7$ représentent ensemble un oxo, $Y_8$ un groupe de formule -N($R_3$)-H, $Y_9$ l'hydrogène et $Z_5$ un reste $X_3$, et en ce que, si désiré, l'on transforme le composé obtenu en un autre composé de formule I, en ce que l'on sépare le mélange d'isoméres obtenu conformément au procédé de l'invention en ses composants, en ce que l'on sépare l'isomère préféré et/ou en ce que l'on transforme le composé libre obtenu conformément au procédé de l'invention en un sel ou le sel obtenu conformément au procédé de l'invention en un composé libre correspondant.

**119.** Procédé pour la préparation des nouvelles 1-acylpipéridones selon la revendication 100 et de leurs sels, caractérisé en ce que l'on condense un composé de formule Xa

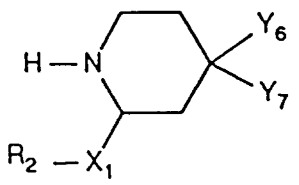

(Xa)

avec un composé introduisant le reste $R_1$ et, si désiré, en ce que l'on transforme le composé obtenu en un autre composé de formule I, en ce que l'on sépare le mélange d'isomères obtenu conformément au procédé de l'invention, en ses composants et en ce que l'on sépare l'isomére préféré et/ou en ce que l'on transforme le composé libre obtenu conformément au procédé de l'invention en un sel ou le sel obtenu par le procédé conforme à l'invention en un composé libre correspondant.

**120.** Utilisation d'un composé selon l'une des revendications 1 à 115 pour la préparation d'un médicament déterminé pour le traitement des maladies dans l'apparition desquelles la substance P joue un rôle important.

**Revendications pour les Etats contractants suivants : ES, GR**

**1.** Procédé pour la préparation des nouveaux composés 1-acylpipéridines de formule I

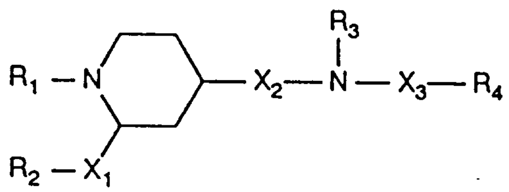

(I)

où $R_1$ représente un phénylalkyle en $C_1$-$C_4$ ou un diphénylalkyle en $C_1$-$C_4$ non substitué ou substitué dans la partie phényle par un alkyle inférieur, un alcoxy inférieur, un dialkyle inférieur amino, un halogène et/ou un trifluorométhyle, un phénoxyalkyle en $C_1$-$C_4$ non substitué ou substitué dans la partie phényle par un halogène et/ou un triazolyle, un pyridylalkyle en $C_1$-$C_4$ ou un quinoléinylalkyle en $C_1$-$C_4$, un benzoyle non substitué ou substitué par un alkyle inférieur, un alcoxy inférieur, un dialkyle inférieur amino, un halogène et/ou le trifluorométhyle, un naphtoyle non substitué ou substitué par un alkyle inférieur, un alcoxy inférieur, un dialkyle inférieur amino, un halogène et/ou le trifluorométhyle, le pyridylcarbonyle ou le quinoléinylcarbonyle non substitué ou substitué par un alkyle inférieur, un alcoxy inférieur, un halogène et/ou le trifluorométhyle, un cycloalkylcarbonyle ayant 5 à 7 chaînons non substitué ou substitué par un alkyle inférieur, un alcoxy inférieur, un dialkyle inférieur amino, un halogéne et/ou le trifluorométhyle, un phénylalcanoyle en $C_1$-$C_4$ ou un diphénylalcanoyle en $C_1$-$C_4$ non substitué ou substitué dans la partie phényle par un alkyle inférieur, un alcoxy inférieur, un dialkyle inférieur amino, un halogène et/ou le trifluorométhyle, un N-phénylcarbamoyle non substitué ou substitué dans la partie phényle éventuellement par un alkyle inférieur, un alcoxy inférieur, un dialkyle inférieur amino, un halogène et/ou le trifluorométhyle ou un groupe de formule Ia

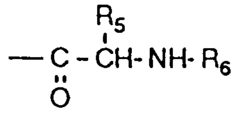

(Ia)

dans laquelle $R_5$ représente l'hydrogène, un alkyle en $C_1$-$C_4$ non substitué ou substitué par l'hydroxy, le mercapto, l'amino, un phényle éventuellement substitué par l'hydroxy, le carboxy, le carbamoyle ou l'uréido et $R_6$ représente un alcanoyle en $C_2$-$C_7$, $R_2$ représente un cycloalkyle ayant 5 à 7 chaînons ou un reste phényle, naphtyle ou pyridyle non substitué ou substitué par un alkyle en $C_1$-$C_4$, un alcoxy en

$C_1$-$C_4$, un halogène et/ou le trifluorométhyle aromatiquement lié, $R_3$ représente l'hydrogène, un alkyle en $C_1$-$C_7$, le carbamoyle, un alcanoyle en $C_2$-$C_7$, un carboxyalcanoyle en $C_1$-$C_4$ ou un carboxyalcènoyle en $C_2$-$C_4$, $R_4$ représente un phényle ou un naphtyle non substitué ou substitué par un alkyle en $C_1$-$C_4$, un alcoxy en $C_1$-$C_4$, un halogène et/ou le trifluorométhyle ou le pyridyle non substitué, le benzofuranyle, l'indolyle, le 2,3-dihydroindolyle, le benzimidazolyle, le quinolyle ou le 1,2,3,4-tétrahydroquinoléinyle, $X_1$ représente le méthylène, l'hydroxyméthylène, un alcoxy en $C_1$-$C_4$ méthylène, le carbonyle ou un dialcoxy en $C_1$-$C_4$ méthylène ou une liaison directe, $X_2$ représente un alkylène en $C_1$-$C_7$, le carbonyle ou une liaison directe et $X_3$ représente le carbonyle, un alkylène en $C_1$-$C_4$, un carboxyalkylène en $C_1$-$C_4$, un alcoxy en $C_1$-$C_4$ carbonylalkylène en $C_1$-$C_4$, un carbamoylalkylène en $C_1$-$C_4$ ou un hydroxyméthylalkylène en $C_1$-$C_4$, un alkyle inférieur étant un alkyle en $C_1$-$C_7$, et de leurs sels, caractérisé

a) en ce que l'on introduit dans un composé de formule II

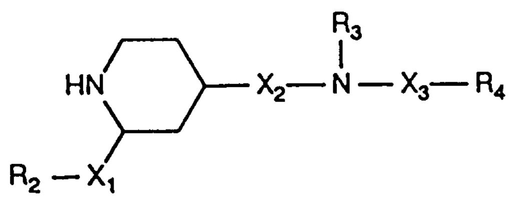

(II)

où $R_2$, $R_3$, $R_4$, $X_1$, $X_2$ et $X_3$ ont les significations qui ont été indiquées, le reste $R_1$ ou

b) en ce que l'on condense les composés de formules III et IV

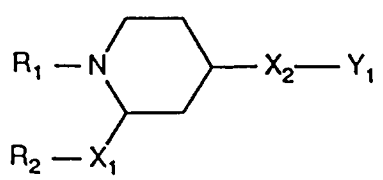

(III)

et

$Y_2$-$X_3$-$R_4$ (IV),

où $Y_1$ représente un groupe de formule -N($R_3$)-H et $Y_2$ représente un hydroxy, un hydroxy estérifié réactif ou, si $X_3$ représente un carbonyle, un hydroxy éthérifié ou $Y_1$ représente un hydroxy, un hydroxy estérifié réactif ou, si $X_2$ représente un carbonyle, un hydroxy éthérifié et $Y_2$ un groupe de formule -N($R_3$)-H, $R_2$, $R_3$, $R_4$, $X_1$, $X_2$ et $X_3$ ayant les significations indiquées ou leurs sels ou

c) en ce que pour la préparation des composés de formule I où l'un des restes $X_2$ et $X_3$ représente un alkylène et l'autre reste représente un alkylène, le carbonyle ou, dans le cas de $X_2$, une liaison directe ou dans le cas de $X_3$, un reste alkylène éventuellement substitué par l'hydroxyméthyle ou par un carboxy éventuellement estérifié ou amidé, l'on remplace dans un composé de formule V

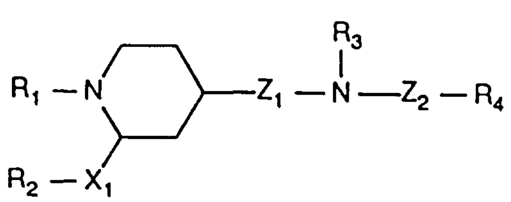

(V)

où $Z_1$ représente un reste alkylène substitué par un oxo ou par un hydroxy en position $\alpha$ par rapport au groupe -N($R_3$)- et $Z_2$ représente un alkylène, le carbonyle ou un reste alkylène éventuellement substitué par l'hydroxyméthyle ou par un carboxy éventuellement estérifié ou amidé ou $Z_1$ représen-

te un alkylène, le carbonyle ou une liaison directe et $Z_2$ représente un reste alkylène substitué par un oxo ou un hydroxy en position α par rapport au groupe $-N(R_3)-$ et $R_2$, $R_3$, $R_4$, $X_1$, $X_2$ et $X_3$ ont les significations qui ont été indiquées ou dans l'un de ses sels le groupe oxo ou le groupe hydroxy en position α par rapport au groupe $-N(R_3)-$ par l'hydrogène par voie de réduction ou en ce que dans un composé de formule VI

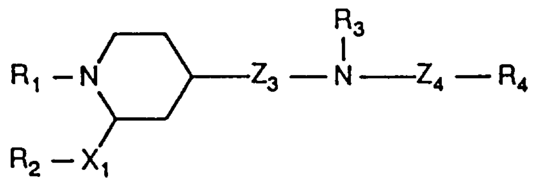 (VI)

où $Z_3$ représente un reste de formule $-C(R_a)=C(R_b)-$ et $Z_4$ représente un alkylène, le carbonyle ou un reste alkylène éventuellement substitué par l'hydroxyméthyle ou par un carboxy éventuellement estérifié ou amidé ou $Z_3$ représente un alkylène, le carbonyle ou une liaison directe et $Z_4$ représente un reste de formule $-C(R_a)=C(R_b)-$, $R_a$ et $R_b$, représentant l'hydrogène ou un alkyle inférieur, l'on réduit le reste de formule $-C(R_a)=C(R_b)-$ par réduction de la double liaison en un reste correspondant $-CH(R_a)-CH(R_b)-$ ou

d) en ce que pour la préparation des composés de formule I où $X_1$ représente un groupe carbonyle ou un groupe hydroxyméthylène, on condense les composés de formules VII et VIII

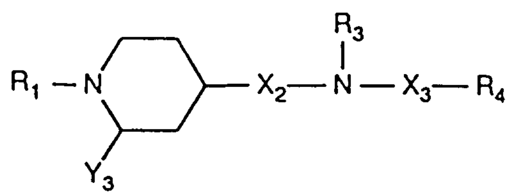 (VII)

et

$Y_4-R_2$ (VIII),

où l'un des restes $Y_3$ et $Y_4$ représente le formyle ou un groupe carboxy éventuellement anhydridé ou estérifié et l'autre groupe un reste métallique et $R_2$, $R_3$, $R_4$, $X_2$ et $X_3$ ont les significations qui ont été indiquées ou

e) en ce que pour la préparation des composés de formule I où $R_3$ représente l'hydrogène, l'on clive dans un composé de formule IX

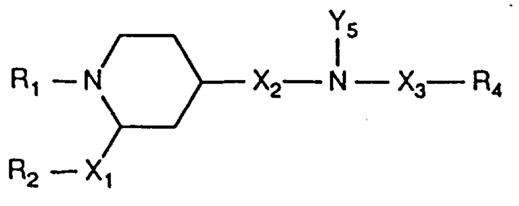 (IX)

où $Y_5$ représente un groupe protecteur de l'amino et $R_2$, $R_4$, $X_1$, $X_2$ et $X_3$ ont les significations qui ont été indiquées, ou dans l'un de ses sels le groupe $Y_5$ ou

f) en ce que pour la préparation des composés de formule I où $X_3$ représente un alkylène, on condense, dans des conditions réductrices, les composés de formule X et XI

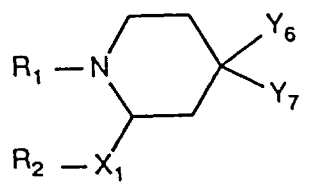 (X) et 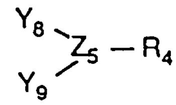 (XI) ,

où $Y_6$ représente un groupe de formule $-N(R_3)-H$, $Y_7$ représente l'hydrogène, $Y_8$ et $Y_9$ représentent ensemble un oxo et $Z_5$ un reste alcanylylidène correspondant $X_3$ ou $Y_6$ et $Y_7$ représentent ensemble un oxo, $Y_8$ un groupe de formule $-N(R_3)-H$, $Y_9$ l'hydrogène et $Z_5$ un reste $X_3$, et en ce que, si désiré, l'on transforme le composé obtenu en un autre composé de formule I, en ce que l'on sépare le mélange d'isomères obtenu conformément au procédé de l'invention, en ses composants et en ce que l'on sépare l'isomére préféré et/ou en ce que l'on transforme le composé libre obtenu conformément au procédé de l'invention en un sel ou le sel obtenu par le procédé conforme à l'invention en un composé libre correspondant.

**2.** Procédé selon la revendication 1, caractérisé en ce que l'on prépare un composé de formule I où $R_1$ représente le benzoyle, le naphtoyle ou un phénylalcanoyle en $C_1$-$C_4$ non substitué ou substitué par un alkyle en $C_1$-$C_4$, un alcoxy en $C_1$-$C_4$, un halogéne et/ou le trifluorométhyle, un pyridylcarbonyle ou un quinoldinylcarbonyle non substitué ou un groupe de formule Ia

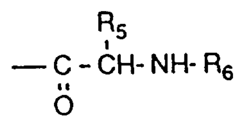 (Ia)

dans laquelle $R_5$ représente l'hydrogène, un alkyle en $C_1$-$C_4$ non substitué ou substitué par l'hydroxy, le mercapto, l'aminow un phényle éventuellement substitué par l'hydroxy, le carboxy, le carbamoyle ou l'uréido et $R_6$ représente un alcanoyle en $C_2$-$C_7$, $R_2$ représente un cycloalkyle ayant 5 à 7 chaînons ou un reste phényle, naphtyle ou pyridyle non substitué ou substitué par un alkyle en $C_1$-$C_4$, un alcoxy en $C_1$-$C_4$, un halogène et/ou le trifluorométhyle aromatiquement lié, $R_3$ représente l'hydrogène, un alkyle en $C_1$-$C_7$, un carbamoyle, un alcanoyle en $C_2$-$C_7$, un carboxyalcanoyle en $C_1$-$C_4$ ou un carboxyalcénoyle en $C_3$-$C_5$, $R_4$ représente un phényle ou un naphtyle non substitué ou substitué par un alkyle en $C_1$-$C_4$, un alcoxy en $C_1$-$C_4$, un halogène et/ou le trifluorométhyle ou le pyridyle, le benzofuranyle, l'indolyle, le benzimidazolyle ou le quinolyle non substitué, $X_1$ représente le méthylène, l'hydroxyméthylène, un alcoxy en $C_1$-$C_4$ méthylène, le carbonyle, un dialcoxy en $C_1$-$C_4$ méthylène ou une liaison directe, $X_2$ représente un alkylène en $C_1$-$C_7$, le carbonyle ou une liaison directe et $X_3$ représente un carbonyle, un alkylène en $C_1$-$C_4$, un carboxyalkylène en $C_1$-$C_4$, un alcoxy en $C_1$-$C_4$ carbonylalkylène en $C_1$-$C_4$, un carbamoylalkylène en $C_1$-$C_4$ ou un hydroxyméthylalkylène en $C_1$-$C_4$ ou l'un de ses sels.

**3.** Procédé selon la revendication 1, caractérisé en ce que l'on prépare un composé de formule I où $R_1$ représente un benzoyle non substitué ou mono- ou disubstitué par un alkyle en $C_1$-$C_4$, un alcoxy en $C_1$-$C_4$, un halogène ayant un numéro atomique allant jusqu'à 35 et/ou le trifluorométhyle ou un naphoyle ou un phénylalcanoyle en $C_1$-$C_4$ non substitué, $R_2$ représente un phényle non substitué ou mono- ou disubstitué par un alkyle en $C_1$-$C_4$, un alcoxy en $C_1$-$C_4$, un halogène ayant un numéro atomique allant jusqu'à 35 et/ou le trifluorométhyle ou un pyridyle non substitué, $R_3$ représente l'hydrogène, un alkyle en $C_1$-$C_4$, un carbamoyle ou un alcanoyle en $C_2$-$C_7$, $R_4$ représente un phényle non substitué ou mono- ou disubstitué par un alkyle en $C_1$-$C_4$, un alcoxy en $C_1$-$C_4$, un halogène ayant un numéro atomique allant jusqu'à 35 et/ou le trifluorométhyle ou un naphtyle, pyridyle, benzofuranyle, indolyle, benzimidazolyle ou quinolyle non substitué, $X_1$ représente le méthylène, l'hydroxyméthylène, le carbonyle ou une liaison directe, $X_2$ représente une liaison directe et $X_3$ représente un alkyléène en $C_1$-$C_4$ ou l'un de ses sels.

**4.** Procédé selon la revendication 1, caractérisé en ce que l'on prépare un composé de formule I ou $R_1$ représente un benzoyle non substitué ou mono- ou disubstitué par un alkyle en $C_1$-$C_4$, un alcoxy en $C_1$-$C_4$, un halogène ayant un numéro atomique allant jusqu'à 35 et/ou le trifluorométhyle ou un

naphtoyle non substitué, $R_2$ représente un phényle non substitué ou mono- ou disubstitué par un halogène ayant un numéro atomique allant jusqu'à 35 et/ou le trifluorométhyle, $R_3$ représente l'hydrogène, $R_4$ représente un quinolyle non substitué, $X_1$ le méthylène, $X_2$ une liaison directe et $X_3$ un alkylène en $C_1$-$C_4$ ou l'un de ses sels.

**5.** Procédé selon la revendication 1, caractérisé en ce que l'on prépare la (2R*,4S*)-2-benzyl-1-(2-naphtoyl)-N-(4-quinolylméthyl)-4-pipéridineamine ou l'un de ses sels.

**6.** Procédé selon la revendication 1, caractérisé en ce que l'on prépare la (2R*,4S*)-2-benzyl-1-(3-trifluorométhylbenzoyl)-N-(4-quinolylméthyl)-4-pipéridineamine ou l'un de ses sels.

**7.** Procédé selon la revendication 1, caractérisé en ce que l'on prépare la (2R*,4S*)-2-benzyl-1-(3,5-bis-(trifluorométhyl)-benzoyl)-N-(4-quinolylméthyl)-4-pipéridineamine ou l'un de ses sels.

**8.** Procédé selon la revendication 1, caractérisé en ce que l'on prépare la (2R*,4S*)-2-benzyl-1-(3,5-diméthoxybenzoyl)-N-(4-quinolylméthyl)-4-pipéridineamine ou l'un de ses sels.

**9.** Procédé selon la revendication 1, caractérisé en ce que l'on prépare la (2R*, 4S*)-2-benzyl-1-(1-naphtoyl)-N-(4-quinolylméthyl)-4-pipéridineamine ou l'un de ses sels.

**10.** Procédé selon la revendication 1, caractérisé en ce que l'on prépare la (2R*,4S*)-2-benzyl-1-(3,5-dichlorobenzoyl)-N-(4-quinolylméthyl)-4-pipéridineamine ou l'un de ses sels.

**11.** Procédé selon la revendication 1, caractérisé en ce que l'on prépare la (2R*,4S*)-2-benzyl-1-(2-quinoléinylcarbonyl)-N-(4-quinolylméthyl)-4-pipéridineamine ou l'un de ses sels.

**12.** Procédé selon la revendication 1, caractérisé en ce que l'on prépare la (2R*,4S*)-2-benzyl-1-(4-chlorophénylacétyl)-N-(4-quinolylméthyl)-4-pipéridineamine ou l'un de ses sels.

**13.** Procédé selon la revendication 1, caractérisé en ce que l'on prépare la (2R*,4S*)-2-benzyl-1-(benzyloxy-sarbonyl)-N-(4-quinolylméthyl)-4-pipéridineamine ou l'un de ses sels.

**14.** Procédé selon la revendication 1, caractérisé en ce que l'on prépare la (2R*,4S*)-2-benzyl-1-(2-phényléthyl)-N-(4-quinolylméthyl)-4-pipdridineamine ou l'un de ses sels.

**15.** Procédé selon la revendication 1, caractérisé en ce que l'on prépare la (2R*,4S*)-2-benzyl-1-(2-naphtylacétyl)-N-(4-quinolylméthyl)-4-pipéridineamine ou l'un de ses sels.

**16.** Procédé selon la revendication 1, caractérisé en ce que l'on prépare la (2R*,4S*)-2-benzyl-1-(4-quinolylmdthyl)-N-(4-quinolylméthyl)-4-pipéridineamine ou l'un de ses sels.

**17.** Procédé selon la revendication 1, caractérisé en ce que l'on prépare la (2R*,4S*)-2-benzyl-1-(2,4-dichlorobenzyl)-N-(4-quinolylméthyl)-4-pipéridineamine ou l'un de ses sels.

**18.** Procédé selon la revendication 1, caractérisé en ce que l'on prépare la (2R*,4S*)-2-benzyl-1-(2,2-diphényléthyl)-N-(4-quinolylméthyl)-4-pipéridineamine ou l'un de ses sels.

**19.** Procédé selon la revendication 1, caractérisé en ce que l'on prépare la (2R*,4S*)-2-benzyl-1-(phénylcar-bamoyl)-N-(4-quinolylméthyl)-4-pipéridineamine ou l'un de ses sels.

**20.** Procédé selon la revendication 1, caractérisé en ce que l'on prépare la (2R*,4S*)-2-benzyl-1-(diphénylacétyl)-N-(4-quinolylméthyl)-4-pipéridineamine ou l'un de ses sels.

**21.** Procédé selon la revendication 1, caractérisé en ce que l'on prépare la (2R*,4S*)-2-benzyl-1-(2-pyridylacétyl)-N-(4-quinolylméthyl)-4-pipéridineamine ou l'un de ses sels.

**22.** Procédé selon la revendication 1, caractérisé en ce que l'on prépare la (2R*,4S*)-2-benzyl-1-(4-pyridylacétyl)-N-(4-quinolylméthyl)-4-pipéridineamine ou l'un de ses sels.

23. Procédé selon la revendication 1, caractérisé en ce que l'on prépare la (2R*,4S*)-2-benzyl-1-(2,3-diphénylpropionyl)-N-(4-quinolylméthyl)-4-pipéridineamine ou l'un de ses sels.

24. Procédé selon la revendication 1, caractérisé en ce que l'on prépare la (2R*,4S*)-2-benzyl-1-((3S)-(2,3,4,9-tétrahydro-1H-pyrido[3,4-b]indol-3-yl)-carbonyl)-N-(4-quinolylméthyl)-4-pipéridineamine ou l'un de ses sels.

25. Procédé selon la revendication 1, caractérisé en ce que l'on prépare la (2R*,4S*)-2-benzyl-1-(3-méthoxybenzoyl)-N-(4-quinolylméthyl)-4-pipéridineamine ou l'un de ses sels.

26. Procédé selon la revendication 1, caractérisé en ce que l'on prépare la (2R*,4S*)-2-benzyl-1-(3-N,N-diméthylaminobenzoyl)-N-(4-quinolylméthyl)-4-pipéridineamine ou l'un de ses sels.

27. Procédé selon la revendication 1, caractérisé en ce que l'on prépare la (2R*,4S*)-2-benzyl-1-(cis,cis-3,5-diméthylcyclohexylcarbonyl)-N-(4-quinolylméthyl)-4-pipéridineamine ou l'un de ses sels.

28. Procédé selon la revendication 1, caractérisé en ce que l'on prépare la (2R*,4S*)-2-benzyl-1-(3,5-bis-(trifluorométhyl)-benzyl)-N-(4-quinolylméthyl)-4-pipéridineamine ou l'un de ses sels.

29. Procédé selon la revendication 1, caractérisé en ce que l'on prépare la (2S*,4R*)-2-benzyl-1-[2-(5-chloro-1H-1,2,4-triazol-1-yl)-phénoxyéthyl]-N-(4-quinolylméthyl)-4-pipéridineamine ou l'un de ses sels.

30. Procédé selon la revendication 1, caractérisé en ce que l'on prépare la (2R*,4S*)-2-benzyl-1-((S)-phénylalanyl)-N-(4-quinolylméthyl)-4-pipéridineamine ou l'un de ses sels.

31. Procédé selon la revendication 1, caractérisé en ce que l'on prépare la (2R*,4S*)-2-benzyl-1-((R)-phénylalanyl)-N-(4-quinolylméthyl)-4-pipéridineamine ou l'un de ses sels.

32. Procédé selon la revendication 1, caractérisé en ce que l'on prépare la (2R*,4S*)-2-benzyl-1-((S)-N-acétyl-phénylalanyl)-N-(4-quinolylméthyl)-4-pipéridineamine ou l'un de ses sels.

33. Procédé selon la revendication 1, caractérisé en ce que l'on prépare la (2R*,4S*)-2-benzyl-1-((R)-N-acétyl-phénylalanyl)-N-(4-quinolylméthyl)-4-pipéridineamine ou l'un de ses sels.

34. Procédé selon la revendication 1, caractérisé en ce que l'on prépare la (2R*,4S*)-2-benzyl-1-((S)-N-(4-carboxamidobutyroyl)-phénylalanyl)-N-(4-quinolylméthyl)-4-pipéridineamine ou l'un de ses sels.

35. Procédé selon la revendication 1, caractérisé en ce que l'on prépare la (2R*,4S*)-2-benzyl-1-((R)-N-(4-carboxamidobutyroyl)-phénylalanyl)-N-(4-quinolylméthyl)-4-pipéridineamine ou l'un de ses sels.

36. Procédé selon la revendication 1, caractérisé en ce que l'on prépare la (2R*,4S*)-2-benzyl-1-benzoyl-N-(4-quinolylméthyl)-4-pipéridineamine ou l'un de ses sels.

37. Procédé selon la revendication 1, caractérisé en ce que l'on prépare la (2R*,4S*)-2-benzyl-1-(3-chlorobenzoyl)-N-(4-quinolylméthyl)-4-pipéridineamine ou l'un de ses sels.

38. Procédé selon la revendication 1, caractérisé en ce que l'on prépare la (2R*,4S*)-2-benzyl-1-(3,5-dichlorobenzoyl)-N-(4-quinolylméthyl)-N-(3-carboxamidopropionyl)-4-pipéridineamine ou l'un de ses sels.

39. Procédé selon la revendication 1, caractérisé en ce que l'on prépare la (2R*,4S*)- ou la (2R,4R)-2-benzyl-1-(3,5-diméthylbenzoyl)-N-(4-quinolylméthyl)-4-pipéridineamine ou l'un de leurs sels.

40. Procédé selon la revendication 1, caractérisé en ce que l'on prépare la (2S,4R)- ou la (2S,4S)-2-benzyl-1-(3,5-diméthylbenzoyl)-N-(2-phénéthyl)-4-pipéridineamine ou l'un de leurs sels.

41. Procédé selon la revendication 1, caractérisé en ce que l'on prépare la (2R,4S)-2-benzyl-1-(3,5-diméthylbenzoyl)-N-(3-quinolylméthyl)-4-pipéridineamine ou l'un de ses sels.

**42.** Procédé selon la revendication 1, caractérisé en ce que l'on prépare la (2R,4S)-2-benzyl-1-(3,5-diméthylbenzoyl)-N-(2-quinolylméthyl)-4-pipéridineamine ou l'un de ses sels.

**43.** Procédé selon la revendication 1, caractérisé en ce que l'on prépare la (2R,4S)- ou la (2R,4R)-2-benzyl-1-(3,5-diméthylbenzoyl)-N-benzyl-4-pipéridineamine ou l'un de leurs sels.

**44.** Procédé selon la revendication 1, caractérisé en ce que l'on prépare la (2S,4R)- ou la (2S,4S)-2-benzyl-1-(3,5-diméthylbenzoyl)-N-benzyl-4-pipéridineamine ou l'un de leurs sels.

**45.** Procédé selon la revendication 1, caractérisé en ce que l'on prépare la (2R,4S)- ou la (2R,4R)-2-benzyl-1-(3,5-diméthylbenzoyl)-N-(4-pyridylméthyl)-4-pipéridineamine ou l'un de leurs sels.

**46.** Procédé selon la revendication 1, caractérisé en ce que l'on prépare la (2R,4S)- ou la (2R,4R)-2-benzyl-1-(3,5-diméthylbenzoyl)-N-(3-pyridylméthyl)-4-pipéridineamine ou l'un de leurs sels.

**47.** Procédé selon la revendication 1, caractérisé en ce que l'on prépare la (2S,4R)- et la (2S,4S)-2-benzyl-1-(3,5-diméthylbenzoyl)-N-(4-quinolylméthyl)-4-pipéridineamine ou l'un de leurs sels.

**48.** Procédé selon la revendication 1, caractérisé en ce que l'on prépare la (2R*,4S*)-2-benzyl-1-(3,5-diméthylbenzoyl)-N-benzyl-N-carbamoyl-4-pipéridineamine ou l'un de ses sels.

**49.** Procédé selon la revendication 1, caractérisé en ce que l'on prépare la (2R,4S)-2-benzyl-1-(3,5-diméthylbenzoyl)-N-(3-phénylpropyl)-4-pipéridineamine ;
(2S,4S)-2-benzyl-1-(3,5-diméthylbenzoyl)-N-(3-phénylpropyl)-4-pipéridineamine ;
(2R,4S)-2-benzyl-1-(3,5-diméthylbenzoyl)-N-(2-méthoxybenzyl)-4-pipéridineamine ;
la (2S,4S)-2-benzyl-1-(3,5-diméthylbenzoyl)-N-(2-méthoxybenzyl)-4-pipéridineamine ;
la (2R,4S)-2-benzyl-1-(3,5-diméthylbenzoyl)-N-[2-(2-méthoxyphényl)-éthyl]-4-pipéridineamine ;
la (2S,4S)-2-benzyl-1-(3,5-diméthylbenzoyl)-N-[2-(2-méthoxyphényl)-éthyl]-4-pipéridineamine ;
la (2R,4S)-2-benzyl-1-(3,5-diméthylbenzoyl)-N-[3-(2-méthoxyphényl)-propyl]-4-pipéridineamine ;
la (2S,4S)-2-benzyl-1-(3,5-diméthylbenzoyl)-N-[3-(2-méthoxyphényl)-propyl]-4-pipéridineamine ;
la (2R,4S)-2-benzyl-1-(3,5-diméthylbenzoyl)-N-(2-trifluorométhylbenzyl)-4-pipéridineamine ;
la (2S,4S)-2-benzyl-1-(3,5-diméthylbenzoyl)-N-(2-trifluorométhylbenzyl)-4-pipéridineamine ;
la (2R,4S)2-benzyl-1-(3,5-diméthylbenzoyl)-N-[2-(2-trifluorométhylphényl)-éthyl]-4-pipéridineamine ;
la (2S,4S)-2-benzyl-1-(3,5-diméthylbenzoyl)-N-[2-(2-trifluorométhylphényl)-éthyl]-4-pipéridineamine ;
la (2R,4S)-2-benzyl-1-(3,5-diméthylbenzoyl)-N-[3-(2-trifluorométhylphényl)-propyl]-4-pipéridineamine ;
la (2S,4S)-2-benzyl-1-(3,5-diméthylbenzoyl)-N-[3-(2-trifluorométhylphényl)-propyl]-4-pipéridineamine;
la (2R,4S)-2-benzyl-1-(3,5-diméthylbenzoyl)-N-(3-trifluorométhylbenzyl)-4-pipéridineamine ;
la (2S,4S)-2-benzyl-1-(3,5-diméthylbenzoyl)-N-(3-trifluorométhylbenzyl)-4-pipéridineamine ;
la (2R,4S)-2-benzyl-1-(3,5-diméthylbenzoyl)-N-[2-(3-trifluorométhylphényl)-éthyl]-4-pipéridineamine ;
la (2S,4S)-2-benzyl-1-(3,5-diméthylbenzoyl)-N-[2-(3-trifluorométhylphényl)-éthyl]-4-pipéridineamine ;
la (2R,4S)-2-benzyl-1-(3,5-diméthylbenzoyl)-N-[3-(3-trifluorométhylphényl)-propyl]-4-pipéridineamine ;
la (2R,4S)-2-benzyl-1-(3,5-diméthylbenzoyl)-N-[3-(3-trifluorométhylphényl)-propyl]-4-pipéridineamine ;
la (2S,4S)-2-benzyl-1-(3,5-diméthylbenzoyl)-N-(4-trifluorométhylbenzyl)-4-pipéridineamine ;
la (2S,4S)-2-benzyl-1-(3,5-diméthylbenzoyl)-N-(4-trifluorométhylbenzyl)-4-pipéridineamine ;
la (2R,4S)-2-benzyl-1-(3,5-diméthylbenzoyl)-N-[2-(4-trifluorométhylphényl)-éthyl]-4-pipéridineamine ;
la (2S,4S)-2-benzyl-1-(3,5-diméthylbenzoyl)-N-[2-(4-trifluorométhylphényl)-éthyl]-4-pipéridineamine ;
la (2R,4S)-2-benzyl-1-(3,5-diméthylbenzoyl)-N-[3-(4-trifluorométhylphényl)-propyl]-4-pipéridineamine ;
la (2R,4S)-2-benzyl-1-(3,5-diméthylbenzoyl)-N-[3-(4-trifluorométhylphényl)-propyl]-4-pipéridineamine ;
la (2S,4S)-2-benzyl-1-(3,5-diméthylbenzoyl)-N-(2,3-diméthoxybenzyl)-4-pipéridineamine ;
la (2S,4S)-2-benzyl-1-(3,5-diméthylbenzoyl)-N-(2,3-diméthoxybenzyl)-4-pipéridineamine ;
la (2R,4S)-2-benzyl-1-(3,5-diméthylbenzoyl)-N-[2-(2,3-diméthoxyphényl)-éthyl]-4-pipéridineamine ;
la (2R,4S)-2-benzyl-1-(3,5-diméthylbenzoyl)-N-[2-(2,3-diméthoxyphényl)-éthyl]-4-pipéridineamine ;
la (2R,4S)-2-benzyl-1-(3,5-diméthylbenzoyl)-N-[3-(2,3-diméthoxyphényl)-propyl]-4-pipéridineamine ;
la (2S,4S)-2-benzyl-1-(3,5-diméthylbenzoyl)-N-[3-(2,3-diméthoxyphényl)-propyl]-4-pipéridineamine ;
la (2R,4S)-2-benzyl-1-(3,5-diméthylbenzoyl)-N-(2,4-diméthoxybenzyl)-4-pipéridineamine ;
la (2S,4S)-2-benzyl-1-(3,5-diméthylbenzoyl)-N-(2,4-diméthoxybenzyl)-4-pipéridineamine ;
la (2R,4S)-2-benzyl-1-(3,5-diméthylbenzoyl)-N-[2-(2,4-diméthoxyphényl)-éthyl]-4-pipéridineamine ;
la (2S,4S)-2-benzyl-1-(3,5-diméthylbenzoyl)-N-[2-(2,4-diméthoxyphényl)-éthyl]-4-pipéridineamine ;

la (2R,4S)-2-benzyl-1-(3,5-diméthylbenzoyl)-N-[3-(2,4-diméthoxyphényl)-propyl]-4-pipéridineamine ;
la (2S,4S)-2-benzyl-1-(3,5-diméthylbenzoyl)-N-[3-(2,4-diméthoxyphényl)-propyl)-4-pipéridineamine ;
la (2R,4S)-2-benzyl-1-(3,5-diméthylbenzoyl)-N-(2,5-diméthoxybenzyl)-4-pipéridineamine ;
la (2S,4S)-2-benzyl-1-(3,5-diméthylbenzoyl)-N-(2,5-diméthoxybenzyl)-4-pipéridineamine ;
la (2R,4S)-2-benzyl-1-(3,5-diméthylbenzoyl)-N-[2-(2,5-diméthoxyphényl)-éthyl]-4-pipéridineamine ;
la (2S,4S)-2-benzyl-1-(3,5-diméthylbenzoyl)-N-[2-(2,5-diméthoxyphényl)-éthyl]-4-pipéridineamine ;
la (2R,4S)-2-benzyl-1-(3,5-diméthylbenzoyl)-N-[3-(2,5-diméthoxyphényl)-propyl]-4-pipéridineamine ;
la (2S,4S)-2-benzyl-1-(3,5-diméthylbenzoyl)-N-[3-(2,5-diméthoxyphényl)-propyl]-4-pipéridineamine ;
la (2R,4S)-2-benzyl-1-(3,5-diméthylbenzoyl)-N-(2,6-diméthoxybenzyl)-4-pipéridineamine ;
la (2S,4S)-2-benzyl-1-(3,5-diméthylbenzoyl)-N-(2,6-diméthoxybenzyl)-4-pipéridineamine ;
la (2R,4S)-2-benzyl-1-(3,5-diméthylbenzoyl)-N-[2-(2,6-diméthoxyphényl)-éthyl]-4-pipéridineamine ;
la(2S,4S)-2-benzyl-1-(3,5-diméthylbenzoyl)-N-[2-(2,6-diméthoxyphényl)-éthyl]-4-pipéridineamine ;
la (2R,4S)-2-benzyl-1-(3,5-diméthylbenzoyl)-N-[3-(2,6-diméthoxyphényl)-propyl]-4-pipéridineamine ;
la (2R,4S)-2-benzyl-1-(3,5-diméthylbenzoyl)-N-[3-(2,6-diméthoxyphényl)-propyl]-4-pipéridineamine ;
la (2R,4S)-2-benzyl-1-(3,5-diméthylbenzoyl)-N-(2,3-méthylènedioxybenzyl)-4-pipéridineamine ;
la (2S,4S)-2-benzyl-1-(3,5-diméthylbenzoyl)-N-(2,3-méthylènedioxybenzyl)-4-pipéridineamine;
la (2R,4S)-2-benzyl-1-(3,5-diméthylbenzoyl)-N-[2-(2,3-méthylènedioxyphényl)-éthyl]-4-pipéridineamine ;
la (2S,4S)-2-benzyl-1-(3,5-diméthylbenzoyl)-N-[2-(2,3-méthylènedioxyphényl)-éthyl]-4-pipéridineamine ;
la (2R,4S)-2-benzyl-1-(3,5-diméthylbenzoyl)-N-[3-(2,3-méthylènedioxyphényl)-propyl]-4-pipéridineamine ;
la (2S,4S)-2-benzyl-1-(3,5-diméthylbenzoyl)-N-[3-(2,3-méthylènedioxyphényl)-propyl]-4-pipéridineamine ;
la (2R,4S)-2-benzyl-1-(3,5-diméthylbenzoyl)-N-(2,4-méthylènedioxybenzyl)-4-pipéridineamine ;
la (2S,4S)-2-benzyl-1-(3,5-diméthylbenzoyl)-N-(2,4-méthylènedioxybenzyl)-4-pipéridineamine ;
la (2R,4S)-2-benzyl-1-(3,5-diméthylbenzoyl)-N-[2-(2,4-méthylènedioxyphényl)-éthyl]-4-pipéridineamine ;
la (2S,4S)-2-benzyl-1-(3,5-diméthylbenzoyl)-N-[2-(2,4-méthylènedioxyphényl)-éthyl]-4-pipéridineamine ;
la(2R,4S)-2-benzyl-1-(3,5-diméthylbenzoyl)-N-[3-(2,4-méthylènedioxyphényl)-propyl]-4-pipéridineamine ;
la (2S,4S)-2-benzyl-1-(3,5-diméthylbenzoyl)-N-[3-(2,4-méthylènedioxyphényl)-propyl]-4-pipéridineamine ;
la (2R,4S)-2-benzyl-1-(3,5-diméthylbenzoyl)-N-(indol-2-ylméthyl)-4-pipéridineamine ;
la (2S,4S)-2-benzyl-1-(3,5-diméthylbenzoyl)-N-(indol-2-ylméthyl)-4-pipéridineamine ;
la (2R,4S)-2-benzyl-1-(3,5-diméthylbenzoyl)-N-(indol-3-ylméthyl)-4-pipéridineamine ;
la (2S,4S)-2-benzyl-1-(3,5-diméthylbenzoyl)-N-(indol-3-ylméthyl)-4-pipéridineamine ;
la (2S,4S)-2-benzyl-1-(3,5-diméthylbenzoyl)-N-(quinoléin-2-ylméthyl)-4-pipéridineamine ;
la (2S,4S)-2-benzyl-1-(3,5-diméthylbenzoyl)-N-(quinoléin-3-ylméthyl)-4-pipéridineamine ;
la (2R,4S)-2-benzyl-1-(3,5-diméthylbenzoyl)-N-(2-chlorobenzyl)-4-pipéridineamine ;
la (2S,4S)-2-benzyl-1-(3,5-diméthylbenzoyl)-N-(2-chlorobenzyl)-4-pipéridineamine ;
la (2R,4S)-2-benzyl-1-(3,5-diméthylbenzoyl)-N-[2-(2-chlorophényl)-éthyl]-4-pipéridineamine ;
la (2S,4S)-2-benzyl-1-(3,5-diméthylbenzoyl)-N-[2-(2-chlorophényl)-éthyl]-4-pipéridineamine ;
la (2R,4S)-2-benzyl-1-(3,5-diméthylbenzoyl)-N-[3-(2-chlorophényl)-propyl]-4-pipéridineamine ;
la (2S,4S)-2-benzyl-1-(3,5-diméthylbenzoyl)-N-[3-(2-chlorophényl)-propyl]-4-pipéridineamine ;
la (2R,4S)-2-benzyl-1-(3,5-diméthylbenzoyl)-N-(3-chlorobenzyl)-4-pipéridineamine ;
la (2S,4S)-2-benzyl-1-(3,5-diméthylbenzoyl)-N-(3-chlorobenzyl)-4-pipéridineamine ;
la (2R,4S)-2-benzyl-1-(3,5-diméthylbenzoyl)-N-[2-(3-chlorophényl)-éthyl]-4-pipéridineamine ;
la (2S,4S)-2-benzyl-1-(3,5-diméthylbenzoyl)-N-[2-(3-chlorophényl)-éthyl]-4-pipéridineamine ;
la (2R,4S)-2-benzyl-1-(3,5-diméthylbenzoyl)-N-[3-(3-chlorophényl)-propyl]-4-pipéridineamine ;
la (2S,4S)-2-benzyl-1-(3,5-diméthylbenzoyl)-N-[3-(3-chlorophényl)-propyl]-4-pipéridineamine ;
la (2R,4S)-2-benzyl-1-(3,5-diméthylbenzoyl)-N-(4-chlorobenzyl)-4-pipéridineamine ;
la (2S,4S)-2-benzyl-1-(3,5-diméthylbenzoyl)-N-(4-chlorobenzyl)-4-pipéridineamine ;
la (2R,4S)-2-benzyl-1-(3,5-diméthylbenzoyl)-N-[2-(4-chlorophényl)-éthyl]-4-pipéridineamine ;
la (2S,4S)-2-benzyl-1-(3,5-diméthylbenzoyl)-N-[2-(4-chlorophényl)-éthyl]-4-pipéridineamine ;
la (2R,4S)-2-benzyl-1-(3,5-diméthylbenzoyl)-N-[3-(4-chlorophényl)-propyl]-4-pipéridineamine ;
la (2S,4S)-2-benzyl-1-(3,5-diméthylbenzoyl)-N-[3-(4-chlorophényl)-propyl]-4-pipéridineamine ;
la (2R,4S)-2-benzyl-1-(3,5-diméthylbenzoyl)-N-(4-méthoxynapht-1-ylméthyl)-4-pipéridineamine ;
la (2S,4S)-2-benzyl-1-(3,5-diméthylbenzoyl)-N-(4-méthoxynapht-1-ylméthyl)-4-pipéridineamine ;
la (2R,4S)-2-benzyl-1-(3,5-diméthylbenzoyl)-N-(3,4-éthylènedioxybenzyl)-4-pipéridineamine ;
la (2S,4S)-2-benzyl-1-(3,5-diméthylbenzoyl)-N-(3,4-éthylènedioxybenzyl)-4-pipéridineamine ;
la (2R,4S)-2-benzyl-1-(3,5-diméthylbenzoyl)-N-(quinoléin-2-ylméthyl)-4-pipéridineamine ;

la (2R,4S)-2-benzyl-1-(3,5-diméthylbenzoyl)-N-(quinoléin-3-ylméthyl)-4-pipéridineamine ;
la (2R,4S)-2-benzyl-1-(3,5-ditrifluorométhylbenzoyl)-N-(indol-2-ylméthyl)-4-pipéridineamine ;
la (2S,4S)-2-benzyl-1-(3,5-ditrifluorométhylbenzoyl)-N-(indol-2-ylméthyl)-4-pipéridineamine ;
la (2R,4S)-2-benzyl-1-(3,5-ditrifluorométhylbenzoyl)-N-(indol-3-ylméthyl)-4-pipéridineamine ;
la (2S,4S)-2-benzyl-1-(3,5-ditrifluorométhylbenzoyl)-N-(indol-3-ylméthyl)-4-pipéridineamine ;
la (2R,4S)-2-benzyl-1-(3,5-ditrifluorométhylbenzoyl)-N-(quinoléin-2-ylméthyl)-4-pipéridineamine ;
la (2S,4S)-2-benzyl-1-(3,5-ditrifluorométhylbenzoyl)-N-(quinoléin-2-ylméthyl)-4-pipéridineamine ;
la (2R,4S)-2-benzyl-1-(3,5-ditrifluorométhylbenzoyl)-N-(quinoléin-3-ylméthyl)-4-pipéridineamine ;
la (2S,4S)-2-benzyl-1-(3,5-ditrifluorométhylbenzoyl)-N-(quinoléin-3-yl)-4-pipéridineamine ;
la (2R,4S)-2-benzyl-1-(3,5-ditrifluorométhylbenzoyl)-N-(2-chlorobenzyl)-4-pipéridineamine ;
la (2S,4S)-2-benzyl-1-(3,5-ditrifluorométhylbenzoyl)-N-(2-chlorobenzyl)-4-pipéridineamine ;
la (2R,4S)-2-benzyl-1-(3,5-ditrifluorométhylbenzoyl)-N-[2-(2-chlorophényl)-éthyl]-4-pipéridineamine ;
la (2S,4S)-2-benzyl-1-(3,5-ditrifluorométhylbenzoyl)-N-[2-(2-chlorophényl)-éthyl]-4-pipéridineamine ;
la (2R,4S)-2-benzyl-1-(3,5-ditrifluorométhylbenzoyl)-N-[3-(2-chlorophényl)-propyl]-4-pipéridineamine ;
la (2S,4S)-2-benzyl-1-(3,5-ditrifluorométhylbenzoyl)-N-[3-(2-chlorophényl)-propyl]-4-pipéridineamine ;
la (2R,4S)-2-benzyl-1-(3,5-ditrifluorométhylbenzoyl)-N-(3-chlorobenzyl)-4-pipéridineamine ;
la (2S,4S)-2-benzyl-1-(3,5-ditrifluorométhylbenzoyl)-N-(3-chlorobenzyl)-4-pipéridineamine ;
la (2R,4S)-2-benzyl-1-(3,5-ditrifluorométhylbenzoyl)-N-(2-(3-chlorophényl)-éthyl]-4-pipéridineamine ;
la (2S,4S)-2-benzyl-1-(3,5-ditrifluorométhylbenzoyl)-N-[2-(3-chlorophényl)-éthyl]-4-pipéridineamine ;
la (2R,4S)-2-benzyl-1-(3,5-ditrifluorométhylbenzoyl)-N-(3-(3-chlorophényl)-propyl]-4-pipéridineamine ;
la (2S,4S)-2-benzyl-1-(3,5-ditrifluorométhylbenzoyl)-N-[3-(3-chlorophényl)-propyl]-4-pipéridineamine ;
la (2R,4S)-2-benzyl-1-(3,5-ditrifluorométhylbenzoyl)-N-(4-chlorobenzyl)-4-pipéridineamine ;
la (2S,4S)-2-benzyl-1-(3,5-ditrifluorométhylbenzoyl)-N-(4-chlorobenzyl)-4-pipéridineamine ;
la (2R,4S)-2-benzyl-1-(3,5-ditrifluorométhylbenzoyl)-N-[2-(4-chlorophényl)-éthyl]-4-pipéridineamine ;
la (2S,4S)-2-benzyl-1-(3,5-ditrifluorométhylbenzoyl)-N-[2-(4-chlorophényl)-éthyl]-4-pipéridineamine ;
la (2R,4S)-2-benzyl-1-(3,5-ditrifluorométhylbenzoyl)-N-[3-(4-chlorophényl) propyl]-4-pipéridineamine ;
la (2S,4S)-2-benzyl-1-(3,5-ditrifluorométhylbenzoyl)-N-[3-(4-chlorophényl)-propyl]-4-pipéridineamine ;
la (2R,4S)-2-benzyl-1-(3,5-ditrifluorométhylbenzoyl)-N-(4-méthoxynapht-1-ylméthyl)-4-pipéridineamine ;
la (2S,4S)-2-benzyl-1-(3,5-ditrifluorométhylbenzoyl)-N-(4-méthoxynapht-1-ylméthyl)-4-pipéridineamine ;
la (2R,4S)-2-benzyl-1-(3,5-ditrifluorométhylbenzoyl)-N-(3,4-éthylènedioxybenzyl)-4-pipéridineamine ;
la (2S,4S)-2-benzyl-1-(3,5-ditrifluorométhylbenzoyl)-N-(3,4-éthylènedioxybenzyl)-4-pipéridineamine;
la (2R,4S)-2-benzyl-1-(3,5-dichlorobenzoyl)-N-(indol-2-ylméthyl)-4-pipéridineamine ;
la (2S,4S)-2-benzyl-1-(3,5-dichlorobenzoyl)-N-(indol-2-ylméthyl)-4-pipéridineamine ;
la (2R,4S)-2-benzyl-1-(3,5-dichlorobenzoyl)-N-(indol-3-ylméthyl)-4-pipéridineamine ;
la (2S,4S)-2-benzyl-1-(3,5-dichlorobenzoyl)-N-(indol-3-ylméthyl)-4-pipéridineamine ;
la (2R,4S)-2-benzyl-1-(3,5-dichlorobenzoyl)-N-(quinoléin-2-ylméthyl)-4-pipéridineamine ;
la (2S,4S)-2-benzyl-1-(3,5-dichlorobenzoyl)-N-(quinoléin-2-ylméthyl)-4-pipéridineamine ;
la (2R,4S)-2-benzyl-1-(3,5-dichlorobenzoyl)-N-(quinoléin-3-ylméthyl)-4-pipéridineamine ;
la (2S,4S)-2-benzyl-1-(3,5-dichlorobenzoyl)-N-(quinoléin-3-ylméthyl)-4-pipéridineamine ;
la (2R,4S)-2-benzyl-1-(3,5-dichlorobenzoyl)-N-(2-chlorobenzyl)-4-pipéridineamine ;
la (2S,4S)-2-benzyl-1-(3,5-dichlorobenzoyl)-N-(2-chlorobenzyl)-4-pipéridineamine ;
la (2R,4S)-2-benzyl-1-(3,5-dichlorobenzoyl)-N-[2-(2-chlorophényl)-éthyl]-4-pipéridineamine ;
la (2S,4S)-2-benzyl-1-(3,5-dichlorobenzoyl)-N-[2-(2-chlorophényl)-éthyl]-4-pipéridineamine ;
la (2R,4S)-2-benzyl-1-(3,5-dichlorobenzoyl)-N-[3-(2-chlorophényl)-propyl]-4-pipéridineamine ;
la (2S,4S)-2-benzyl-1-(3,5-dichlorobenzoyl)-N-[3-(2-chlorophényl)-propyl]-4-pipéridineamine ;
la (2R,4S)-2-benzyl-1-(3,5-dichlorobenzoyl)-N-(3-chlorobenzyl)-4-pipéridineamine ;
la (2S,4S)-2-benzyl-1-(3,5-dichlorobenzoyl)-N-(3-chlorobenzyl)-4-pipéridineamine ;
la (2R,4S)-2-benzyl-1-(3,5-dichlorobenzoyl)-N-[2-(3-chlorophényl)-éthyl]-4-pipéridineamine ;
la (2S,4S)-2-benzyl-1-(3,5-dichlorobenzoyl)-N-[2-(3-chlorophényl)-éthyl]-4)-pipéridineamine ;
la (2R,4S)-2-benzyl-1-(3,5-dichlorobenzoyl)-N-[3-(3-chlorophényl)-propyl]-4-pipéridineamine ;
la (2S,4S)-2-benzyl-1-(3,5-dichlorobenzoyl)-N-[3-(3-chlorophényl)-propyl]-4-pipéridineamine ;
la (2R,4S)-2-benzyl-1-(3,5-dichlorobenzoyl)-N-(4-chlorobenzyl)-4-pipéridineamine ;
la (2S,4S)-2-benzyl-1-(3,5-dichlorobenzoyl)-N-(4-chlorobenzyl)-4-pipéridineamine ;
la (2R,4S)-2-benzyl-1-(3,5-dichlorobenzoyl)-N-[2-(4-chlorophényl)-éthyl]-4-pipéridineamine ;
la (2S,4S)-2-benzyl-1-(3,5-dichlorobenzoyl)-N-[2-(4-chlorophényl)-éthyl]-4-pipéridineamine ;
la (2R,4S)-2-benzyl-1-(3,5-dichlorobenzoyl)-N-[3-(4-chlorophényl)-propyl]-4-pipéridineamine ;
la (2S,4S)-2-benzyl-1-(3,5-dichlorobenzoyl)-N-[3-(4-chlorophényl)-propyl]-4-pipéridineamine ;
la (2R,4S)-2-benzyl-1-(3,5-dichlorobenzoyl)-N-(4-méthoxynapht-1-ylméthyl)-4-pipéridineamine ;

la (2S,4S)-2-benzyl-1-(3,5-dichlorobenzoyl)-N-(4-méthoxynapht-1-yiméthyl)-4-pipéridineamine :
la (2R,4S)-2-benzyl-1-(3,5-dichlorobenzoyl)-N-(3,4-éthylènedioxybenzyl)-4-pipéridineamine ;
la (2S,4S)-2-benzyl-1-(3,5-dichlorobenzoyl)-N-(3,4-éthylènedioxybenzyl)-4-pipéridineamine ;
la (2R,4S)-2-benzyl-1-(3,5-diméthoxybenzoyl)-N-(indol-2-ylméthyl)-4-pipéridineamine ;
la (2S,4S)-2-benzyl-1-(3,5-diméthoxybenzoyl)-N-(indol-2-ylméthyl)-4-pipéridineamine ;
la (2R,4S)-2-benzyl-1-(3,5-diméthoxybenzoyl)-N-(indol-3-ylméthyl)-4-pipéridineamine ;
la (2S,4S)-2-benzyl-1-(3,5-diméthoxybenzoyl)-N-(indol-3-ylméthyl)-4-pipéridineamine ;
la (2R,4S)-2-benzyl-1-(3,5-diméthoxybenzoyl)-N-(quinoléin-2-ylméthyl)-4-pipéridineamine ;
la (2S,4S)-2-benzyl-1-(3,5-diméthoxybenzoyl)-N-(quinoléin-2-ylméthyl)-4-pipéridineamine ;
la (2R,4S)-2-benzyl-1-(3,5-diméthoxybenzoyl)-N-(quinoléin-3-ylméthyl)-4-pipéridineamine ;
la (2S,4S)-2-benzyl-1-(3,5-diméthoxybenzoyl)-N-(quinoléin-3-ylméthyl)-4-pipéridineamine ;
la (2R,4S)-2-benzyl-1-(3,5-diméthoxybenzoyl)-N-(2-chlorobenzyl)-4-pipéridineamine ;
la (2S,4S)-2-benzyl-1-(3,5-diméthoxybenzoyl)-N-(2-chlorobenzyl)-4-pipéridineamine ;
la (2R,4S)-2-benzyl-1-(3,5-diméthoxybenzoyl)-N-[2-(2-chlorophényl)-éthyl]-4-pipéridineamine ;
la (2S,4S)-2-benzyl-1-(3,5-diméthoxybenzoyl)-N-[2-(2-chlorophényl)-éthyl]-4-pipéridineamine ;
la (2R,4S)-2-benzyl-1-(3,5-diméthoxybenzoyl)-N-[3-(2-chlorophényl)-propyl]-4-pipéridineamine ;
la (2S,4S)-2-benzyl-1-(3,5-diméthoxybenzoyl)-N-[3-(2-chlorophényl)-propyl]-4-pipéridineamine ;
la (2R,4S)-2-benzyl-1-(3,5-diméthoxybenzoyl)-N-(3-chlorobenzyl)-4-pipéridineamine ;
la (2S,4S)-2-benzyl-1-(3,5-diméthoxybenzoyl)-N-(3-chlorobenzyl)-4-pipéridineamine ;
la (2R,4S)-2-benzyl-1-(3,5-diméthoxybenzoyl)-N-[2-(3-chlorophényl)-éthyl]-4-pipéridineamine ;
la (2S,4S)-2-benzyl-1-(3,5-diméthoxybenzoyl)-N-[2-(3-chlorophényl)-éthyl]-4-pipéridineamine ;
la (2R,4S)-2-benzyl-1-(3,5-diméthoxybenzoyl)-N-[3-(3-chlorophényl)-propyl]-4-pipéridineamine ;
la (2S,4S)-2-benzyl-1-(3,5-diméthoxybenzoyl)-N-[3-(3-chlorophényl)-propyl]-4-pipéridineamine ;
la (2R,4S)-2-benzyl-1-(3,5-diméthoxybenzoyl)-N-(4-chlorobenzyl)-4-pipéridineamine ;
la (2S,4S)-2-benzyl-1-(3,5-diméthoxylbenzoyl)-N-(4-chlorobenzyl)-4-pipéridineamine ;
la (2R,4S)-2-benzyl-1-(3,5-diméthoxybenzoyl)-N-[2-(4-chlorophényl)-éthyl]-4-pipéridineamine ;
la (2S,4S)-2-benzyl-1-(3,5-diméthoxybenzoyl)-N-[2-(4-chlorophényl)-éthyl]-4-pipéridineamine ;
la (2R,4S)-2-benzyl-1-(3,5-diméthoxybenzoyl)-N-[3-(4-chlorophényl)-propyl]-4-pipéridineamine ;
la (2S,4S)-2-benzyl-1-(3,5-diméthoxybenzoyl)-N-(3-(4-chlorophényl)-propyl]-4-pipéridineamine ;
la (2R,4S)-2-benzyl-1-(3,5-diméthoxybenzoyl)-N-(4-méthoxynapht-1-ylméthyl)-4-pipéridineamine ;
la (2S,4S)-2-benzyl-1-(3,5-diméthoxybenzoyl)-N-(4-méthoxynapht-1-ylmdthyl)-4-pipéridineamine;
la (2R,4S)-2-benzyl-1-(3,5-diméthoxybenzoyl)-N-(3,4-éthylènedioxybenzyl)-4-pipéridineamine et ou
la (2S,4S)-2-benzyl-1-(3,5-diméthoxybenzoyl)-N-(3,4-éthylènedioxybenzyl)-4-pipéridineamine.
ou l'un des sels de chacun de ces composés.

**50.** Procédé selon la revendication 1, caractérisé en ce que l'on prépare la (2R,4S)- et la (2R,4R)-2-benzyl-1-(3,5-diméthylbenzoyl)-N-(2-phénéthyl)-4-pipéridineamine ou l'un de leurs sels.

**51.** Procédé selon la revendication 1, caractérisé en ce que l'on prépare la (2R,4S)-2-benzyl-1-(3,5-bis-(trifluorométhyl)-benzoyl)-4-quinolylméthyl)-4-pipéridineamine ou l'un de ses sels.

**52.** Procédé selon la revendication 1, caractérisé en ce que l'on prépare la (2R,4S)-2-benzyl-1-(3,5-dichlorobenzoyl)-N-(4-quinolylméthyl)-4-pipéridineamine ou l'un de ses sels.

**53.** Procédé selon la revendication 1, caractérisé en ce que l'on prépare la (2R*,4S*)-2-benzyl-1-(2,4-dichlorobenzoyl)-N-(4-quinolylméthyl)-4-pipéridineamine ou l'un de ses sels.

**54.** Procédé selon la revendication 1, caractérisé en ce que l'on prépare la (2R*,4S*)-2-benzyl-1-(phénylacétyl)-N-(4-quinolylméthyl)-4-pipéridineamine ou l'un de ses sels.

**55.** Procédé selon la revendication 1, caractérisé en ce que l'on prépare la (2R*,4S*)-2-benzyl-1-(2,6-dichlorobenzoyl)-N-(4-quinolylméthyl)-4-pipéridineamine.

**56.** Procédé selon la revendication 1, caractérisé en ce que l'on prépare la (2R*,4S*)-2-benzyl-1-(3,5-dibromobenzoyl)-N-(4-quinolylméthyl)-4-pipéridineamine ou l'un de ses sels.

**57.** Procédé selon la revendication 1, caractérisé en ce que l'on prépare la (2R*,4S*)-2-benzyl-1-(9-fluorénoyl)-N-(4-quinolylméthyl)-4-pipéridineamine ou l'un de ses sels.

**58.** Procédé selon la revendication 1, caractérisé en ce que l'on prépare la (2R*,4S*)-2-benzyl-1-(3-toluoyl)-N-(4-quinolylméthyl)-4-pipéridineamine ou l'un de ses sels.

**59.** Procédé selon la revendication 1, caractérisé en ce que l'on prépare la (2R*,4S*)-2-benzyl-1-(3-bromobenzoyl)-N-(4-quinolylméthyl)-4-pipéridineamine ou l'un de ses sels.

**60.** Procédé selon la revendication 1, caractérisé en ce que l'on prépare la (2R*,4S*)-2-benzyl-1-(3,5-dihydroxybenzoyl)-N-(4-quinolylméthyl)-4-pipéridineamine ou l'un de ses sels.

**61.** Procédé selon la revendication 1, caractérisé en ce que l'on prépare la (2R*,4S*)-2-benzyl-1-(3-cyanobenzoyl)-N-(4-quinolylméthyl)-4-pipéridineamine ou l'un de ses sels.

**62.** Procédé selon la revendication 1, caractérisé en ce que l'on prépare la (2R*,4S*)-2-benzyl-1-(2-chlorobenzoyl)-N-(4-quinolylméthyl)-4-pipéridineamine ou l'un de ses sels.

**63.** Procédé selon la revendication 1, caractérisé en ce que l'on prépare la (2R*,4S*)-2-benzyl-1-(4-chlorobenzoyl)-N-(4-quinolylméthyl)-4-pipéridineamine ou l'un de ses sels.

**64.** Procédé selon la revendication 1, caractérisé en ce que l'on prépare la (2R*,4S*)-2-benzyl-1-(3,5-dichlorobenzoyl)-N-(4-quinolylméthyl)-N-méthyl-4-pipéridineamine ou l'un de ses sels.

**65.** Procédé selon la revendication 1, caractérisé en ce que l'on prépare la (2R*, 4S*)-2-benzyl-1-(3,5-dichlorobenzoyl)-N-(4-quinolylméthyl)-N-cyclohexylcarbamoyl-4-pipéridineamine ou l'un de ses sels.

**66.** Procédé selon la revendication 1, caractérisé en ce que l'on prépare la (2R*, 4S*)-2-benzyl-1-(3,5-dichlorobenzoyl)-N-(4-quinolylméthyl)-N-phénylcarbamoyl-4-pipéridineamine ou l'un de ses sels.

**67.** Procédé selon la revendication 1, caractérisé en ce que l'on prépare la (2R*,4S*)-2-benzyl-1-(3,5-dichlorobenzoyl)-N-(2-phényléthyl)-4-pipéridineamine ou l'un de ses sels.

**68.** Procédé selon la revendication 1, caractérisé en ce que l'on prépare la (2R*,4S*)-2-benzyl-1-(3,5-bis-(trifluorométhyl)-benzoyl)-N-(2-phényléthyl)-4-pipéridineamine ou l'un de ses sels.

**69.** Procédé selon la revendication 1, caractérisé en ce que l'on prépare la (2R*,4S*)-2-benzyl-1-(3,5-dichlorobenzoyl)-N-(2-naphtoyl)-4-pipéridineamine ou l'un de ses sels.

**70.** Procédé selon la revendication 1, caractérisé en ce que l'on prépare la (2R*,4S*)-2-benzyl-1-(3,5-dichlorobenzoyl)-N-(3,5-diméthylbenzoyl)-4-pipéridineamine ou l'un de ses sels.

**71.** Procédé selon la revendication 1, caractérisé en ce que l'on prépare la (2R*,4S*)-2-benzyl-1-(3,5-dichlorobenzoyl)-N-(4-quinolylcarbonyl)-4-pipéridineamine ou l'un de ses sels.

**72.** Procédé selon la revendication 1, caractérisé en ce que l'on prépare la (2R*,4S*)-2-benzyl-1-(3,5-dichlorobenzoyl)-N-(3-indolylcarbonyl)-4-pipéridineamine ou l'un de ses sels.

**73.** Procédé selon la revendication 1, caractérisé en ce que l'on prépare la (2R*,4S*)-2-benzyl-1-(3,5-dichlorobenzoyl)-N-(2-indolylcarbonyl)-4-pipéridineamine ou l'un de ses sels.

**74.** Procédé selon la revendication 1, caractérisé en ce que l'on prépare la (2R*,4S*)-2-benzyl-1-(3,5-dichlorobenzoyl)-N-(5-méthoxy-2-indolylcarbonyl)-4-pipéridineamine ou l'un de ses sels.

**75.** Procédé selon la revendication 1, caractérisé en ce que l'on prépare la (2R*,4S*)-2-benzyl-1-(3,5-dichlorobenzoyl)-N-(1-naphtoyl)-4-pipéridineamine ou l'un de ses sels.

**76.** Procédé selon la revendication 1, caractérisé en ce que l'on prépare la (2R*,4S*)-2-benzyl-1-(3,5-dichlorobenzoyl)-N-(phénylacétyl)-4-pipéridineamine ou l'un de ses sels.

**77.** Procédé selon la revendication 1, caractérisé en ce que l'on prépare la (2R*,4S*)-2-benzyl-1-(3,5-dichlorobenzoyl)-N-(2-méthoxybenzyl)-4-pipéridineamine ou l'un de ses sels.

**78.** Procédé selon la revendication 1, caractérisé en ce que l'on prépare la (2R*,4S*)-2-benzyl-1-(3,5-dichlorobenzoyl)-N-(3-(N-acétyl)-indolylméthyl)-4-pipéridineamine ou l'un de ses sels.

**79.** Procédé selon la revendication 1, caractérisé en ce que l'on prépare la (2R*,4St)-2-benzyl-1-(3,5-dichlorobenzoyl)-N-(2-benzo[b]furanylméthyl)4-pipéridineamine ou l'un de ses sels.

**80.** Procédé selon la revendication 1, caractérisé en ce que l'on prépare la (2R*,4S*)-2-benzyl-1-(3,5-dichlorobenzoyl)-N-[(3-méthylbenzo[b]thiophén-2-ylméthyl]-4-pipéridineamine ou l'un de ses sels.

**81.** Procédé selon la revendication 1, caractérisé en ce que l'on prépare la (2R*,4S*)-2-benzyl-1-(3,5-dichlorobenzoyl)-N-(5-méthoyindol-3-ylméthyl)-4-pipéridineamine ou l'un de ses sels.

**82.** Procédé selon la revendication 1, caractérisé en ce que l'on prépare la (2R*,4S*)-2-benzyl-1-(3,5-dichlorobenzoyl)-N-(3-indolylméthyl)-4-pipéridineamine ou l'un de ses sels.

**83.** Procédé selon la revendication 1, caractérisé en ce que l'on prépare la (2R*,4S*)-2-benzyl-1-(3,5-dichlorobenzoyl)-N-phénylcarbamoyl-4-pipéridineamine ou l'un de ses sels.

**84.** Procédé selon la revendication 1, caractérisé en ce que l'on prépare la (2R*,4S*)-2-benzyl-1-(3,5-dichlorobenzoyl)-N-diphénylméthyl-4-pipéridineamine ou l'un de ses sels.

**85.** Procédé selon la revendication 1, caractérisé en ce que l'on prépare la (2R*,4S*)-2-benzyl-1-(3,5-dichlorobenzoyl)-N-(3,4-dihydro-2H-1benzopyranne-2-carbonyl)-4-pipéridineamine ou l'un de ses sels.

**86.** Procédé selon la revendication 1, caractérisé en ce que l'on prépare la (2R*,4S*)-2-benzyl-1-(4-méthoxybenzoyl)-N-(4-quinolylméthyl)-4-pipéridineamine ou l'un de ses sels.

**87.** Procédé selon la revendication 1, caractérisé en ce que l'on prépare la (2R*,4S*,1'R*)-N-benzyl-1-(3,5-diméthylbenzoyl)-2-(1'-hydroxy-1'-phénylméthyl)-4-pipéridineamine ou l'un de ses sels.

**88.** Procédé selon la revendication 1, caractérisé en ce que l'on prépare la (2R*,4S*,1'R*)-2-(1'-hydroxy-1'-phénylméthyl)-1-(3,5-diméthylbenzoyl)-N-(4-quinoléinylméthyl)-4-pipéridineamine ou l'un de ses sels.

**89.** Procédé selon la revendication 1, caractérisé en ce que l'on prépare la (2R*, 4S*,1'S*)-1-(3,5-diméthyl-benzoyl)-2-(1'-hydroxy-1'-phénylméthyl)-N-(quinoléinylméthyl)-4-pipéridineamine ou l'un de ses sels.

**90.** Procédé selon la revendication 1, caractérisé en ce que l'on prépare la (2R*,4S*,1'R*)-2-{1'-hydroxy-1'-(4-chlorophényl)-méthyl}-1-(3,5-diméthylbenzoyl)-N-(4-quinoléinylméthyl)-4-pipéridineamine ou l'un de ses sels.

**91.** Procédé selon la revendication 1, caractérisé en ce que l'on prépare la (2R*,4S*,1'S*)-1-(3,5-diméthyl-benzoyl)-2-{1'-hydroxy-1'-(4-chlorophényl)-méthyl}-N-(4-quinoléinylméthyl)-4-pipéridineamine ou l'un de ses sels.

**92.** Procédé selon la revendication 1, caractérisé en ce que l'on prépare la (2R*,4S*,1'S*)-1-(3,5-diméthyl-benzoyl)-2-{1'-hydroxy-1'-(3,4-dichlorophényl)-méthyl)-N-(4-quinoléinylméthyl)-4-pipéridineamine ou l'un de ses sels.

**93.** Procédé selon la revendication 1, caractérisé en ce que l'on prépare la (2R*,4S*)-N-benzyl-1-(3,5-diméthylbenzoyl)-2-benzoyl-4-pipéridineamine ou l'un de ses sels.

**94.** Procédé selon la revendication 1, caractérisé en ce que l'on prépare la (2R*,4S*)-2-(4-chlorobenzyl)-1-(3,5-diméthylbenzoyl)-N-(4-qunioléinylméthyl)-4-pipéridineamine ou l'un de ses sels.

**95.** Procédé selon la revendication 1, caractérisé en ce que l'on prépare la (2R*,4S*)-2-(3,4-dichlorobenzyl)-1-(3,5-diméthylbenzoyl)-N-(4-quinoléinylméthyl)-4-pipéridineamine ou l'un de ses sels.

**96.** Procédé selon la revendication 1, caractérisé en ce que l'on prépare la (2R*,4S*)-1-(3,5-diméthylbenzoyl)-2-phényl-N-(4-quinoléinylméthyl)-4-pipéridineamine ou l'un de ses sels.

**97.** Procédé selon la revendication 1, caractérisé en ce que l'on prépare la (2R*,4S*)-1-(3,5-dichlorobenzoyl)-2-phényl-N-(4-quinoléineméthyl)-4-pipéridineamine ou l'un de ses sels.

**98.** Procédé selon la revendication 1, caractérisé en ce que l'on prépare la (2R*,4S*)-1-(1-naphtoyl)-2-phényl-N-(4-quinoléinylméthyl)4-pipéridineamine.

**99.** Procédé selon la revendication 1, caractérisé en ce que l'on prépare la (2R*,4S*)-1-(3,5-diméthylbenzoyl)-2-(1-naphtyl)-N-(4-quinoléinylméthyl)-4-pipéridineamine ou l'un de ses sels.

**100.** Procédé selon la revendication 1, caractérisé en ce que l'on prépare
la (2R*,4S*)-1-(3,5-diméthylbenzoyl)-2-(2-naphtyl)-N-(4-quinoléinylméthyl)-4-pipéridineamine ;
la (2R*,4S*)-1-(3,5-diméthylbenzoyl)-2-(4-méthoxyphénylméthyl)-N-(4-quinoléinylméthyl)-4-pipéridineamine ;
la (2R*,4S*)-1-(3,5-diméthylbenzoyl)-2-(3-méthoxyphénylméthyl)-N-(4-quinoléinylméthyl)-4-pipéridineamine ;
la (2R*,4S*)-1-(3,5-diméthylbenzoyl)-2-(4-nitrobenzyl)-N-(4-quinoléinylméthyl)-4-pipéridineamine ;
la (2R*,4S*)-1-(3,5-diméthylbenzoyl)-2-(4-trifluorométhylphénylméthyl)-N-(4-quinoléinylméthyl)-4-pipéridineamine ;
la (2R*,4S*)-1-(3,5-diméthylbenzoyl)-2-(2,4-dichlorophénylméthyl)-N-(4-quinoléinylméthyl)-4-pipéridineamine ;
la (2R*,4S*)-1-(3,5-diméthylbenzoyl)-2-(2-phényléthyl)-N-(4-quinoléinylméthyl)-4-pipéridineamine ;
la (2R*,4S*)-1-(3,5-diméthylbenzoyl)-2-(2-phényléthényl)-N-(4-quinoléinylméthyl)-4-pipéridineamine ;
la (2R*,4S*)-1-(3,5-diméthylbenzoyl)-2-benzyl-N-(4-quinoléinylméthyl)-4-pipéridineamine ;
la (2R*,4S*)-1-(3,5-diméthylbenzoyl)-2-(4-chlorobenzoyl)-N-(4-quinoléinylméthyl)-4-pipéridineamine ;
la (2R*,4S*)-1-(3,5-diméthylbenzoyl)-2-(2-naphtyl)-N-(4-quinoléinylméthyl)-4-pipéridineamine ;
la (2R*,4S*)-1-(3,5-diméthylbenzoyl)-2-(4-méthoxybenzyl)-N-(4-quinoléinylméthyl)-4-pipéridineamine ;
la (2R*,4S*)-1-(3,5-diméthylbenzoyl)-2-(3-méthoxybenzyl)-(4-quinoléinylméthyl)-4-pipéridineamine ;
la (2R*,4S*)-1-(3,5-diméthylbenzoyl)-2-(4-nitrobenzyl)-(4-quinoléinylméthyl)-4-pipéridineamine ;
la (2R*,4S*)-1-(3,5-diméthylbenzoyl)-2-(4-trifluorométhylbenzyl)-(4-quinoléinylméthyl)-4-pipéridineamine ;
la (2R*,4S*)-1-(3,5-diméthylbenzoyl)-2-(2,4-dichlorobenzyl)-(4-quinoléinylméthyl)-4-pipéridineamine ;
la (2R*,4S*)-1-(3,5-diméthylbenzoyl)-2-(2-phényléthyl)-N-(4-quinoléinylméthyl)-4-pipéridineamine ;
la (2R*,4S*)-1-(3,5-diméthylbenzoyl)-2-(2-phényléthényl)-N-(4-quinoléinylméthyl)-4-pipéridineamine ;
la (2R*,4S*)-1-(3,5-diméthylbenzoyl)-2-(benzoylméthyl)-N-(4-quinoléinylméthyl)-4-pipéridineamine ou
la (2R*,4S*)-1-(3,5-diméthylbenzoyl)-2-(4-chlorobenzoylméthyl)-N-(4-quinoléinylméthyl)-4-pipéridineamine
ou l'un des sels de chacun de ces composés.

**101.** Procédé pour la préparation des nouvelles 1-acylpipéridones de formule X

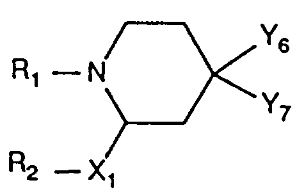

(X)

où $R_1$ représente un reste aroyle, hétéroaroyle, cycloalkylcarbonyle, aralcanoyle, hétéroarylalcanoyle, aralcoxycarbonyle ou arylcarbamoyle éventuellement substitué ou le reste acyle d'un α-amino-acide éventuellement N-substitué par un alcanoyle inférieur ou par un carbamoylalcanoyle inférieur, $R_2$ représente un cycloalkyle ou un reste aryle ou hétéroaryle éventuellement substitué, $X_1$ représente l'hydroxyméthylène et $Y_6$ et $Y_7$ représentent ensemble un oxo et de leurs sels, caractérisé en ce que

l'on condense un composé de formule Xa

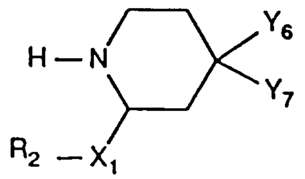

(Xa)

avec un composé introduisant le reste $R_1$ et, si désiré, en ce que l'on transforme le composé obtenu en un autre composé de formule I, en ce que l'on sépare le mélange d'isomères obtenu conformément au procédé de l'invention, en ses composants et en ce que l'on sépare l'isombre préféré et/ou en ce que l'on transforme le composé libre obtenu conformément au procédé de l'intention en un sel ou le sel obtenu par le procédé conforme à l'invention en un composé libre correspondant.

**102.** Procédé selon la revendication 101 pour la préparation des composés de formule X où $R_1$ représente un benzoyle non substitué ou mono- ou disubstitué, par un alkyle en $C_1$-$C_4$, un alcoxy en $C_1$-$C_4$, un halogène ayant un numéro atomique allant jusqu'à 35 et/ou le trifluorométhyle ou un naphtoyle non substitué, $R_2$ représente un phényle non substitué ou mono- ou disubstitué par un halogène ayant un numéro atomique allant jusqu'à 35 et/ou le trifluorométhyle, $X_1$ représente l'hydroxyméthylène et $Y_6$ et $Y_7$ représentent ensemble un oxo ou l'un de ses sels.

**103.** Procédé selon la revendication 101, caractérisé en ce que l'on prépare la (2R*,1'R*)-1-(3,5-diméthylbenzoyl)-2-(1'-hydroxy-1'-phénylméthyl)-4-pipéridone ou l'un de ses sels.

**104.** Procédé selon la revendication 101, caractérisé en ce que l'on prépare la (2R*,1'R*)-1-(3,5-bis-trifluorométhylbenzoyl)-2-{1'-hydroxy-1'-(4-chlorophényl)méthyl}-4-pipéridone ou l'un de ses sels.

**105.** Procédé selon la revendication 101, caractérisé en ce que l'on prépare la (2R*,1'R*)-1-(3,5-diméthylbenzoyl)-2-{1'-hydroxy1'-(3,4-dichlorophényl)-méthyl}-4-pipéridone ou l'un de ses sels.

**106.** Procédé selon la revendication 101, caractérisé en ce que l'on prépare la (2R*,1'S*)-1-(3,5-bis-trifluorométhylbenzoyl)-2-{1'-hydroxy-1'-(4-chlorophényl) méthyl}-4-pipéridone ou l'un de ses sels.

**107.** Procédé selon la revendication 101, caractérisé en ce que l'on prépare la (2R*,1'R*)-1-(3,5-bis-trifluorométhylbenzoyl)-2-{1'-hydroxy-1'-(3,4-dichlorophényl)-méthyl}-4-pipéridone ou l'un de ses sels.

**108.** Procédé selon la revendication 101, caractérisé en ce que l'on prépare la (2R*,1'R*)-1-(3,5-diméthylbenzoyl)-2-{1'-hydroxy-1'-(4-méthoxyphényl)-méthyl}-4-pipéridone ou l'un de ses sels.

**109.** Procédé selon la revendication 101, caractérisé en ce que l'on prépare la (2R*,1'S*)-1-(3,5-diméthylbenzoyl)-2-{1'-hydroxy-1'-(4-méthoxyphényl)-méthyl}-4-pipéridone ou l'un de ses sels.

**110.** Procédé selon la revendication 101, caractérisé en ce que l'on prépare la (2R*,1'R*)-1-(3,5-diméthylbenzoyl)-2-{1'-hydroxy-1'-(3-méthoxyphényl)-méthyl}-4-pipéridone ou l'un de ses sels.

**111.** Procédé selon la revendication 101, caractérisé en ce que l'on prépare la (2R*,1'S*)-1-(3,5-diméthylbenzoyl)-2-{1'-hydroxy-1'-(3-méthoxyphényl)-méthyl}-4-pipéridone ou l'un de ses sels.

**112.** Procédé selon la revendication 101, caractérisé en ce que l'on prépare la (2R*,1'R*)-1-(3,5-diméthylbenzoyl)-2-{1'-hydroxy-1'-(4-trifluorométhylphényl)-méthyl}-4-pipéridone ou l'un de ses sels.

**113.** Procédé selon la revendication 101, caractérisé en ce que l'on prépare la (2R*,1'S*)-1-(3,5-diméthylbenzoyl)-2-{1'-hydroxy-1'-(4-trifluorométhylphényl) méthyl}-4-pipéridone ou l'un de ses sels.

**114.** Procédé selon la revendication 101, caractérisé en ce que l'on prépare la (2R*,1'R*)-1-(3,5-diméthylbenzoyl)-2-{1'-hydroxy-1'-(4-chloro-3-trifluorométhylphényl)-méthyl}-4-pipéridone ou l'un de ses sels.

**115.** Procédé selon la revendication 101, caractérisé en ce que l'on prépare la (2R*,1'S*)-1-(3,5-diméthylbenzoyl)-2-{1'-hydroxy-1'-(4-chloro-3-trifluorométhylphényl)-méthyl)-4-pipéridone ou l'un de ses sels.

**116.** Procédé pour la préparation d'une composition pharmaceutique, caractérisé que l'on mélange un composé 1-acylpipéridine de formule

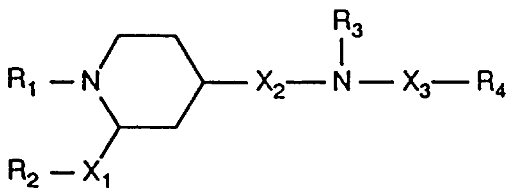
(I) ou
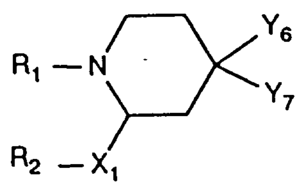
(X)

où $R_1$ représente dans la formule I un reste aralkyle, aryloxyalkyle, hétéroaralkyle, aroyle, hétéroaroyle, cycloalkylcarbonyle, aralcanoyle, hétéroarylalcanoyle, aralcoxycarbonyle ou arylcarbamoyle éventuellement substitué ou le reste acyle d'un $\alpha$-amino-acide éventuellement N-substitué par un alcanoyle inférieur ou par un carbamoylalcanoyle inférieur, $R_2$ représente un cycloalkyle ou un reste aryle ou hétéroaryle éventuellement substitué ou représente dans la formule X un reste aroyle, hétéroaroyle, cycloalkylcarbonyle, aralcanoyle, hétéroarylalcanoyle, aralcoxycarbonyle ou arylcarbamoyle éventuellement substitué ou le reste acyle d'un $\alpha$-amino-acide éventuellement N-substitué par un alcanoyle inférieur ou par un carbamoylalcanoyle inférieur, $R_3$ représente l'hydrogène, un alkyle, un carbamoyle ou un reste alcanoyle ou alcénoyle éventuellement substitué par le carboxy ou par un carboxy estérifié ou amidé, $R_4$ représente un reste aryle éventuellement substitué ou un reste hétéroaryle éventuellement partiellement hydrogéné, $X_1$ représente dans la formule I le méthylène, l'éthylène, une liaison directe, un groupe carbonyle éventuellement cétalisé ou un groupe hydroxyméthylène éventuellement éthérifié ou représente encore dans la formule X l'hydroxyméthylène, $X_2$ représente un alkylène, le carbonyle ou une liaison directe, $X_3$ représente le carbonyle, un oxoalkylène inférieur, un oxo-(aza)-alkylène inférieur ou un reste alkylène éventuellement substitué par un phényle, l'hydroxyméthyle, un carboxy éventuellement estérifié ou amidé, ou par un hydroxy au-delà de la position $\alpha$ et $Y_6$ et $Y_7$ représentent ensemble un oxo ou l'un de ses sels pharmaceutiquement utilisables avec des adjuvants pharmaceutiques usuels.